# EUROPÄISCHE PATENTSCHRIFT

(11) **EP 4 281 455 B1**
(45) Veröffentlichungstag und Bekanntmachung des Hinweises auf die Patenterteilung: **22.07.2026**
(21) Anmeldenummer: 22702222.5
(22) Anmeldetag: 24.01.2022
(51) Int. Cl.: C07D 487/04, H10K 10/00

(54) **STICKSTOFFHALTIGE VERBINDUNGEN FÜR ORGANISCHE ELEKTROLUMINESZENZVORRICHTUNGEN**
NITROGENOUS COMPOUNDS FOR ORGANIC ELECTROLUMINESCENT DEVICES
COMPOSÉS AZOTÉS POUR DISPOSITIFS ÉLECTROLUMINESCENTS ORGANIQUES

(30) Priorität: 25.01.2021 EP 21153168
(43) Veröffentlichungstag der Anmeldung: 29.11.2023
(73) Patentinhaber: Merck Patent GmbH, 64293 Darmstadt (DE)
(72) Erfinder: PARHAM, Amir Hossain, 64293 DARMSTADT (DE); EHRENREICH, Christian, 64293 DARMSTADT (DE)
(74) Vertreter: Merck Patent Association
(86) Internationale Anmeldenummer: PCT/EP2022/051417
(87) Internationale Veröffentlichungsnummer: WO 2022/157343

(56) Entgegenhaltungen:
- WO-A1-2014/003336
- KR-A- 20190 080 166

## Beschreibung

Die vorliegende Erfindung betrifft stickstoffhaltige Verbindungen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

In organischen Elektrolumineszenzvorrichtungen werden als emittierende Materialien häufig phosphoreszierende metallorganische Komplexe eingesetzt. Aus quantenmechanischen Gründen ist unter Verwendung metallorganischer Verbindungen als Phosphoreszenzemitter eine bis zu vierfache Energie- und Leistungseffizienz möglich. Generell gibt es bei Elektrolumineszenzvorrichtungen, insbesondere auch bei Elektrolumineszenzvorrichtungen, die Triplettemission (Phosphoreszenz) zeigen, immer noch Verbesserungsbedarf. Die Eigenschaften phosphoreszierender Elektrolumineszenzvorrichtungen werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu deutlichen Verbesserungen der Eigenschaften der Elektrolumineszenzvorrichtungen führen.

Aus WO 2014/010810 A1, KR 20160076881 A1, US 2015/171344 A1 sind Imidazol-Derivate beschrieben, die als Matrixmaterialien für phosphoreszierende Emitter eingesetzt werden können.

Die Imidazol-kondensierten Carbazolderivate gemäß Anspruch 1 stellen eine Auswahl aus den in KR20190080166 A und WO14003336 A1 beanspruchten Carbazolderivaten dar. Die in KR20190080166 A und WO14003336 A1 beschriebenen Verbindungen eignen sich als Matrixmaterialien zur Verwendung in organischen licht-emittierende Vorrichtungen.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot und gelb phosphoreszierende Elektrolumineszenzvorrichtungen, insbesondere für rot phosphoreszierende Elektrolumineszenzvorrichtungen und gegebenenfalls auch für blau phosphoreszierende Elektrolumineszenzvorrichtungen eignen.

Weiterhin sollten die Verbindungen, insbesondere bei ihrem Einsatz als Matrixmaterialien, als Lochtransportmaterialien oder als Elektronenblockiermaterialien in organischen Elektrolumineszenzvorrichtung zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass Verbindungen nach Anspruch 1 diese Aufgabe lösen, sich gut für die Verwendung in Elektrolumineszenzvorrichtungen eignen und zu Verbesserungen der organischen Elektrolumineszenzvorrichtung führen, insbesondere in Bezug auf die Lebensdauer, der Farbreinheit, der Effizienz und der Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung sind Verbindungen nach Anspruch 1 der Formeln (I'-5) bis (I'-22), (I'-31) bis (I'-78) und (I'-83) bis (I'-118),
wobei für die verwendeten Symbole und Indizes gilt:
- Ar^{a}: ist bei jedem Auftreten gleich oder verschieden ein aromatisches oder heteroaromatisches Ringsystem mit 5 bis 40 aromatischen Ringatomen, welches mit einem oder mehreren Resten R² substituiert sein kann;
- Ar^{b}: ist bei jedem Auftreten gleich oder verschieden ein aromatisches oder heteroaromatisches Ringsystem mit 5 bis 40 aromatischen Ringatomen, welches mit einem oder mehreren Resten R³ substituiert sein kann;
- R: ist bei jedem Auftreten gleich oder verschieden H, D, F, Cl, Br, I, N(R⁴)₂, N(Ar')₂, CN, NO₂, OR⁴, OAr`, SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann; dabei können zwei Reste R oder ein Rest R mit einem Rest R¹, R² auch miteinander ein aliphatisches, heteroaliphatisches, aromatisches oder heteroaromatisches Ringsystem bilden, vorzugsweise bilden die Reste R kein solches Ringsystem;
- R¹: ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann; dabei können zwei Reste R¹ oder ein Rest R¹ mit einem Rest R, R², R³ auch miteinander ein aliphatisches, heteroaliphatisches, aromatisches oder heteroaromatisches Ringsystem, vorzugsweise ein aliphatisches, heteroaliphatisches oder heteroaromatisches Ringsystem bilden, besonders bevorzugt bilden die Reste R¹ kein solches Ringsystem;
- R²: ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann; dabei können zwei Reste R² oder ein Rest R² mit einem Rest R, R¹, R³ auch miteinander ein aromatisches, heteroaromatisches, aliphatisches oder heteroaliphatisches Ringsystem bilden, vorzugsweise bilden die Reste R² kein solches Ringsystem;
- R³: ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann; dabei können zwei Reste R³ oder ein Rest R³ mit einem Rest R¹, R² auch miteinander ein aromatisches, heteroaromatisches, aliphatisches oder heteroaliphatisches Ring- system bilden, vorzugsweise bilden die Reste R³ kein solches Ringsystem;
- Ar': ist bei jedem Auftreten gleich oder verschieden ein aromatisches oder heteroaromatisches Ringsystem mit 5 bis 40 aromatischen Ringatomen, welches mit einem oder mehreren Resten R⁴ substituiert sein kann;
- R⁴: ist bei jedem Auftreten gleich oder verschieden H, D, F, Cl, Br, I, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁵ substituiert sein kann, wobei eine oder mehrere nicht benachbarte CH₂-Gruppen durch Si(R⁵)₂, C=O, NR⁵, O, S oder CONR⁵ ersetzt sein können, oder ein aromatisches oder heteroaromatisches Ringsystem mit 5 bis 40 aromatischen Ringatomen, das jeweils durch einen oder mehrere Reste R⁵ substituiert sein kann; dabei können zwei oder mehrere Reste R⁴ miteinander ein aromatisches, heteroaromatisches, aliphatisches oder heteroaliphatisches Ringsystem bilden, vorzugsweise ein aliphatisches Ringsystem, besonders bevorzugt bilden die Reste R⁴ kein solches Ringsystem;
- R⁵: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus **N,** O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9`-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9`-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

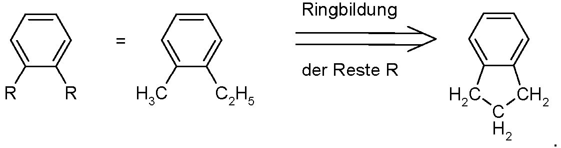

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

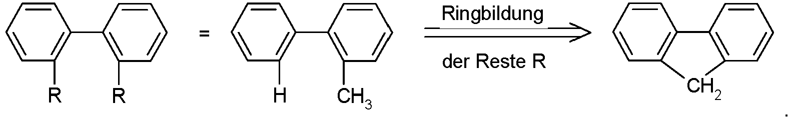

Die erfindungsgemäßen Verbindungen sind ausgewählt aus den Verbindungen der Formeln (I'-5) bis (I'-22), (I'-31) bis (I'-78) und (I'-83) bis (I'-118), 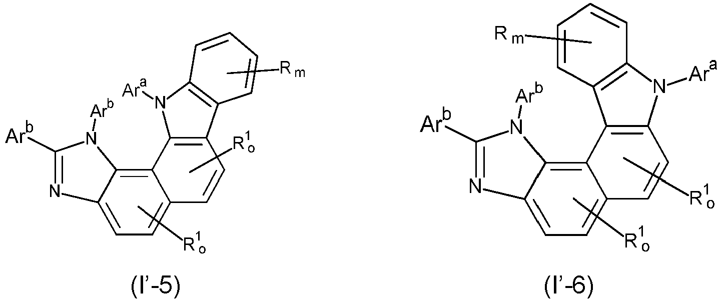 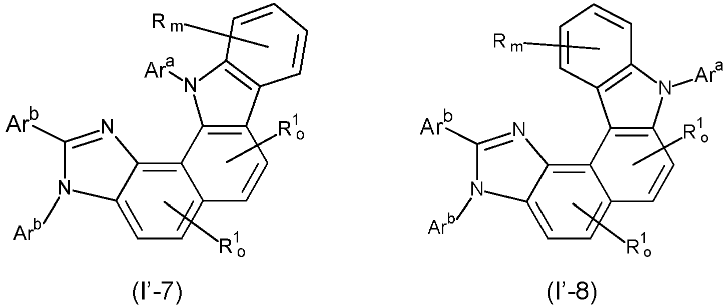 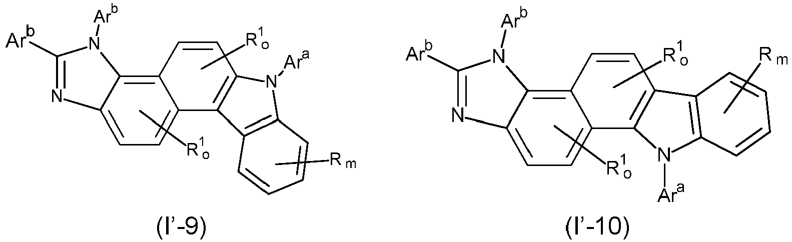 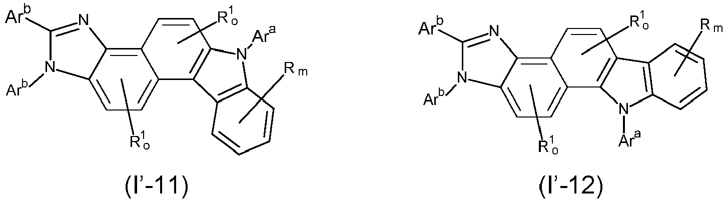 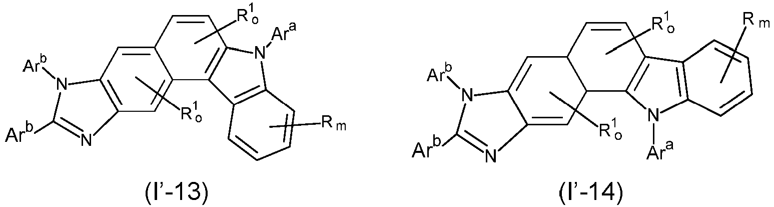 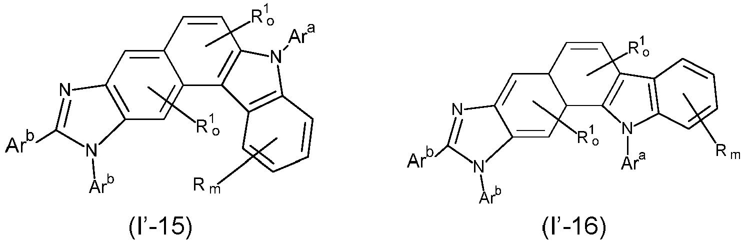 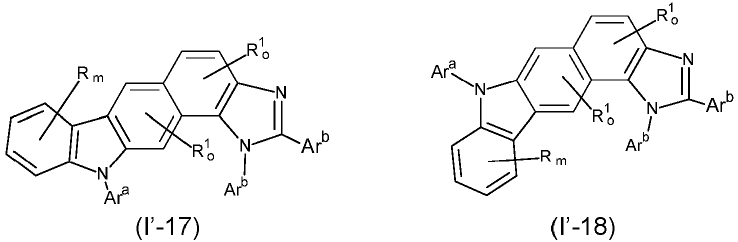 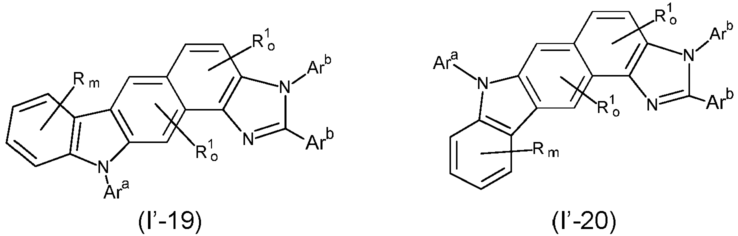 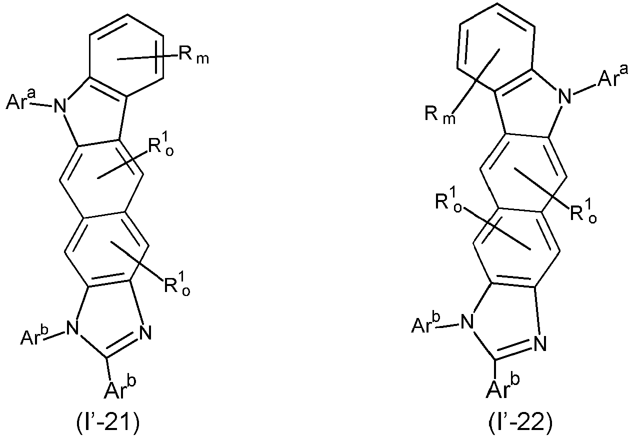 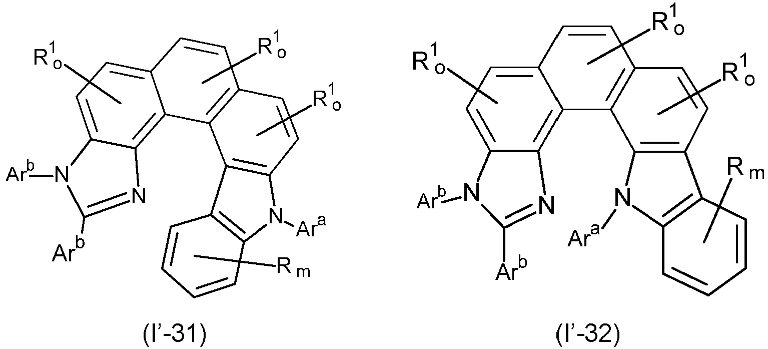 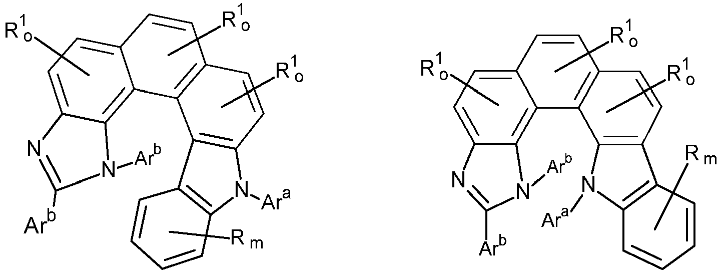 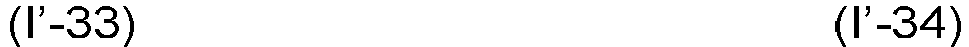 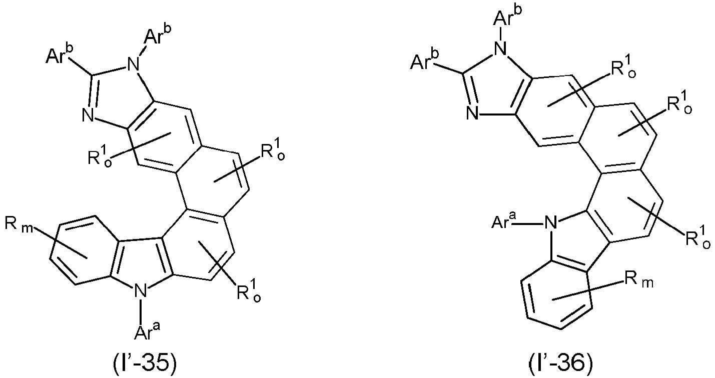 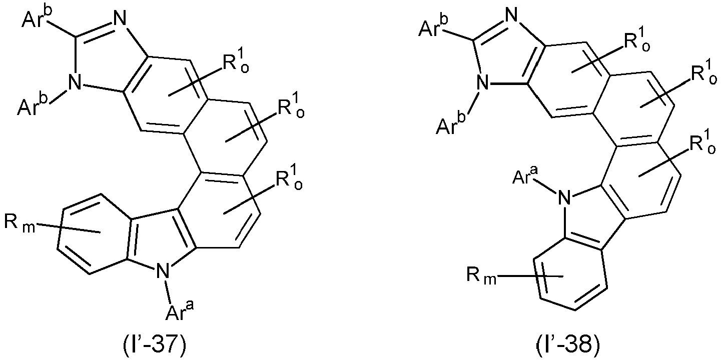 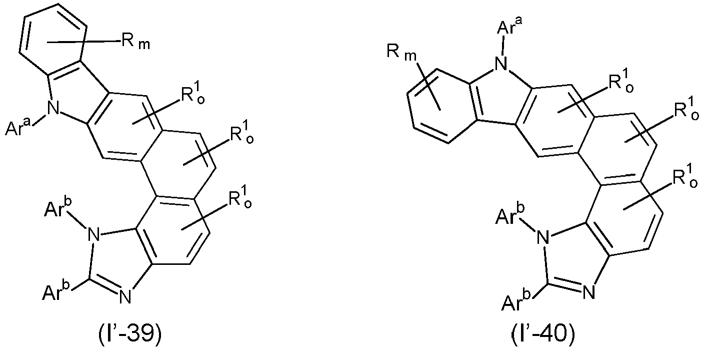 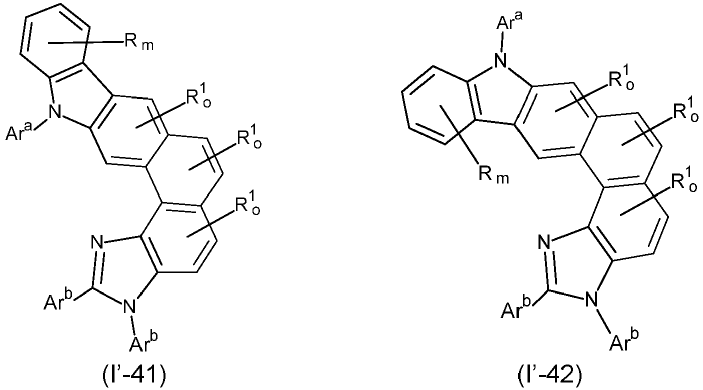 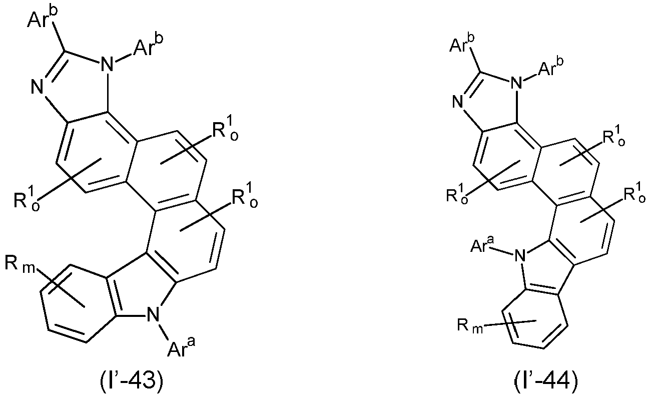 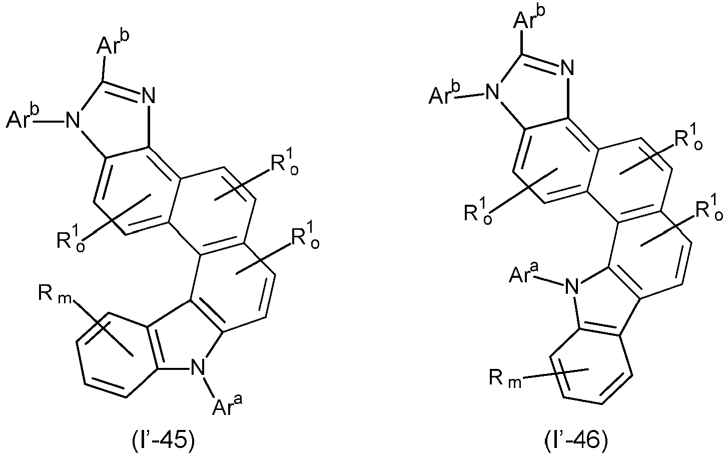 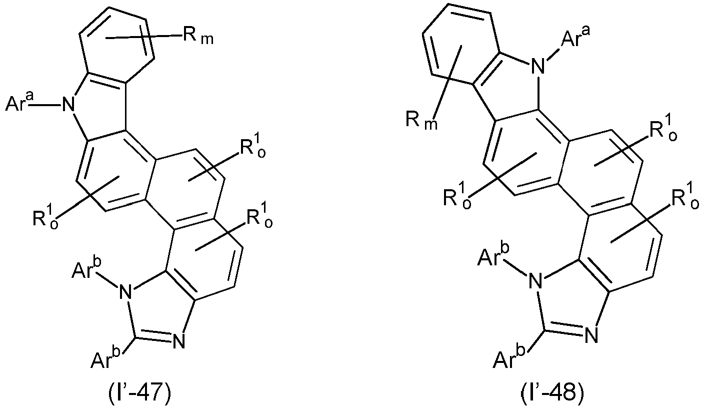 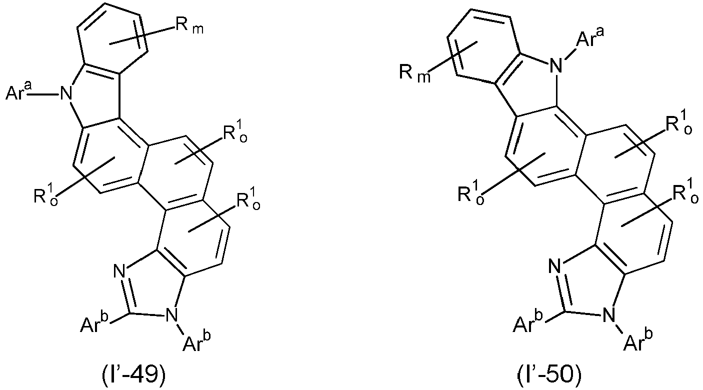 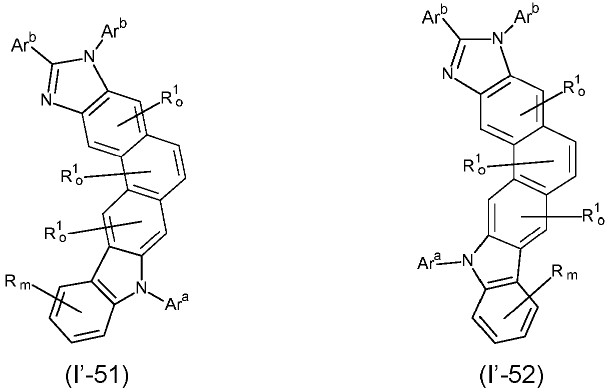 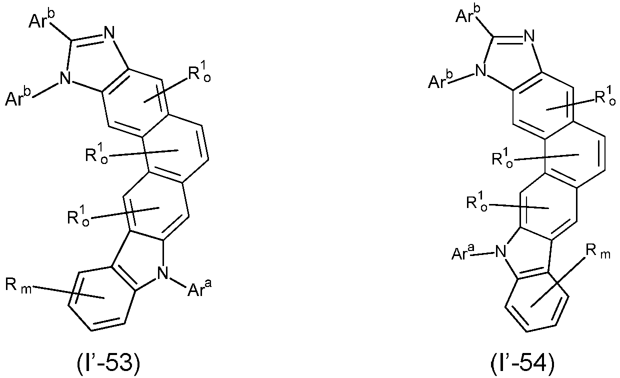 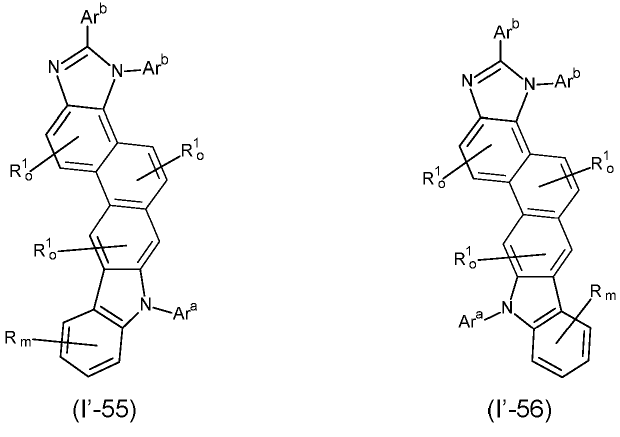 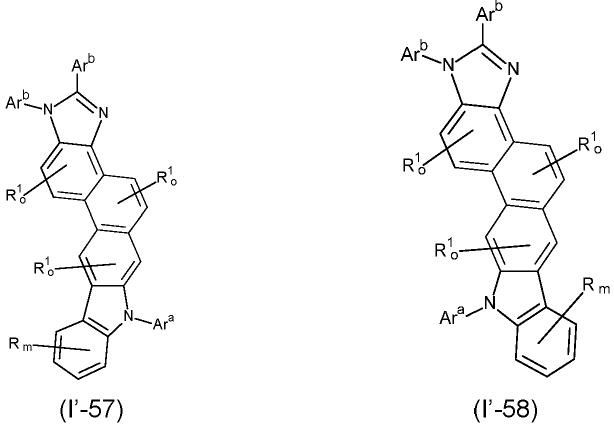 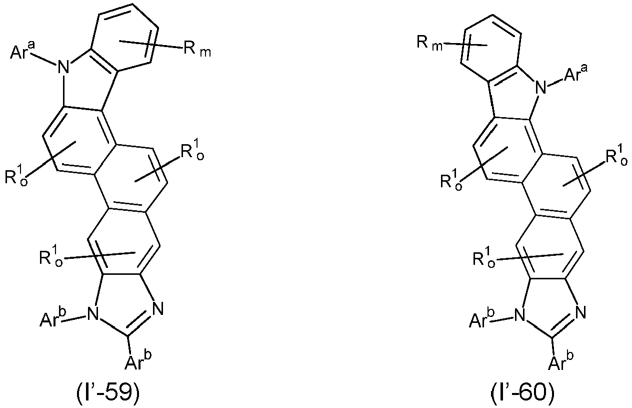 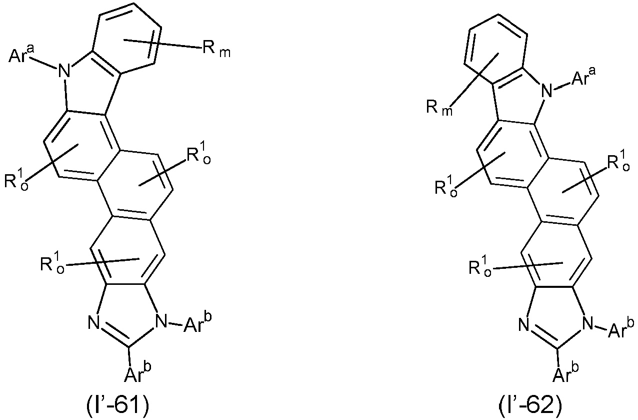 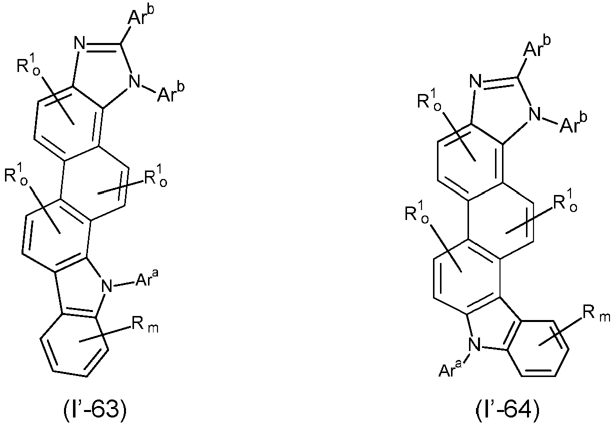 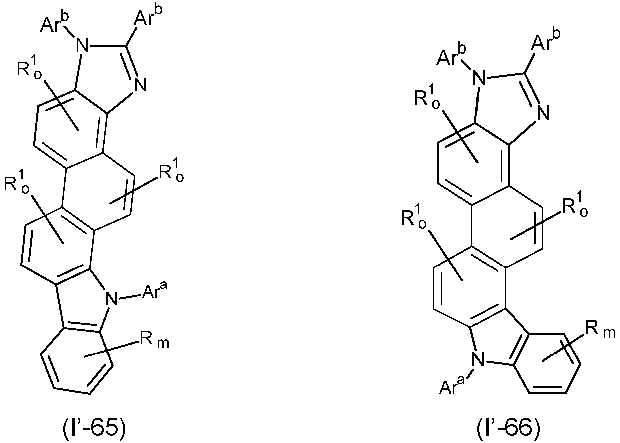 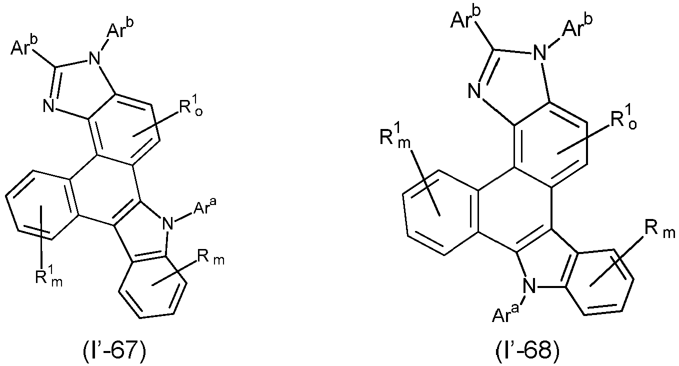 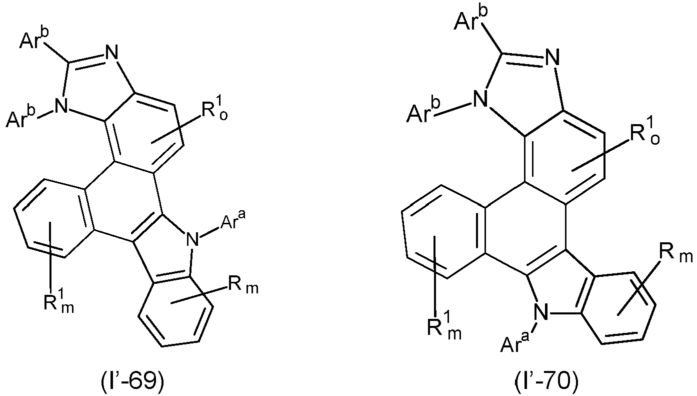 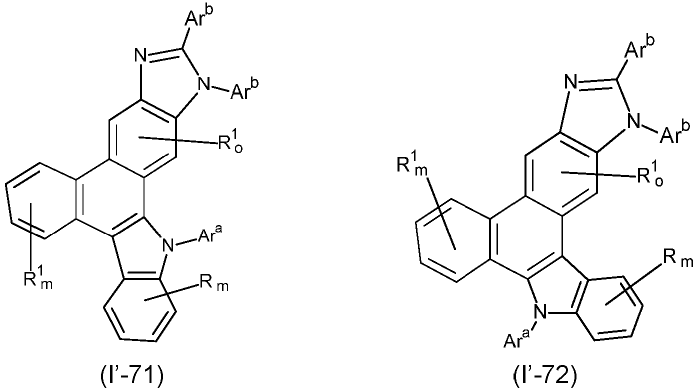 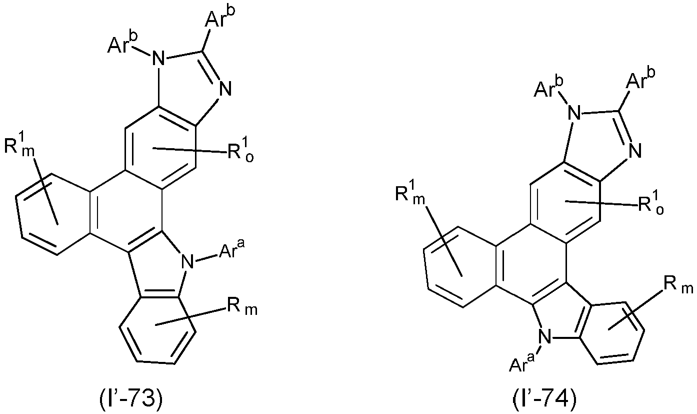 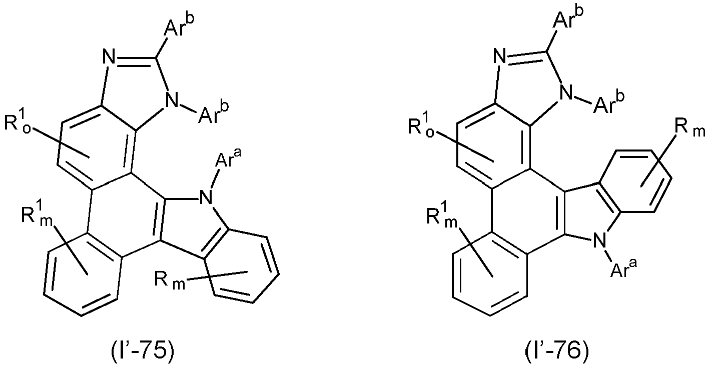 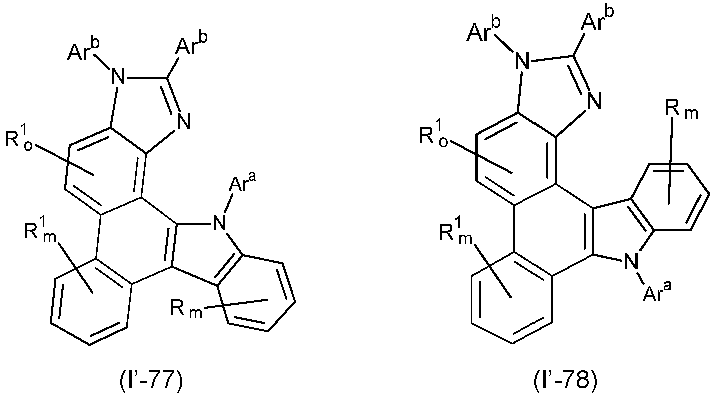 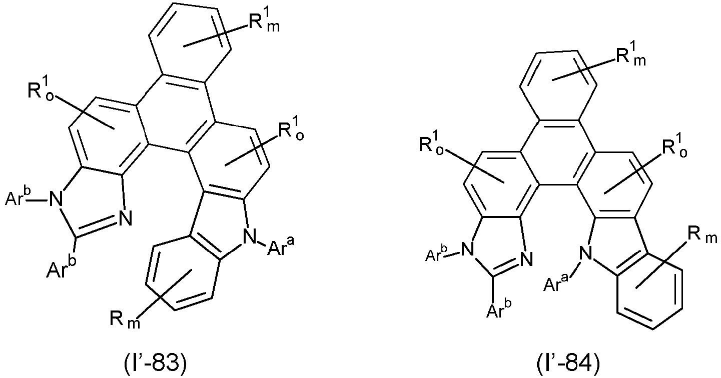 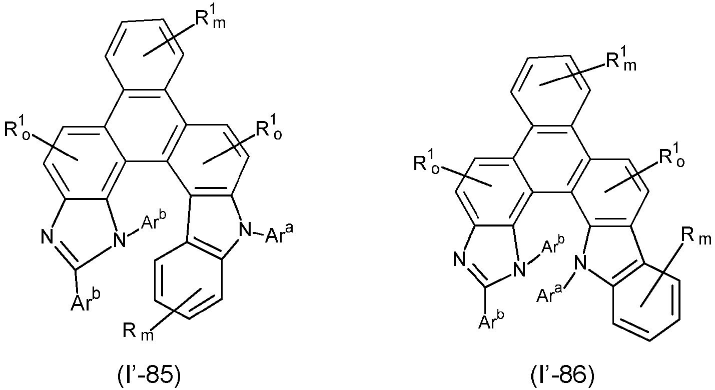 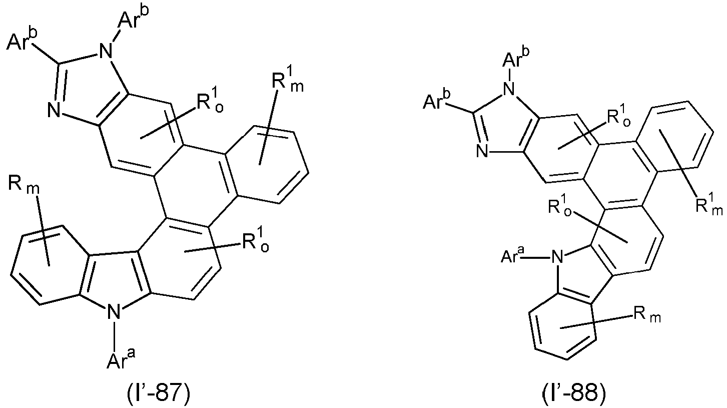 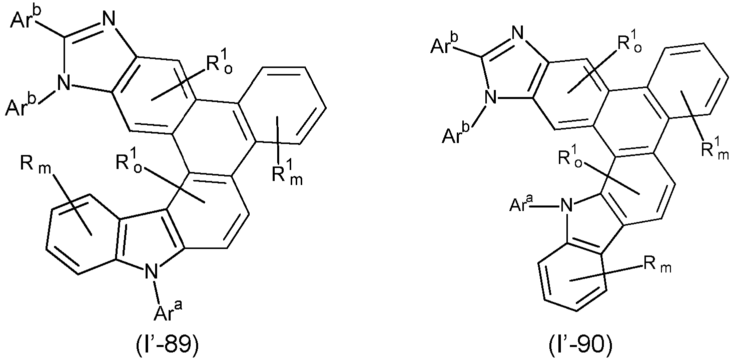 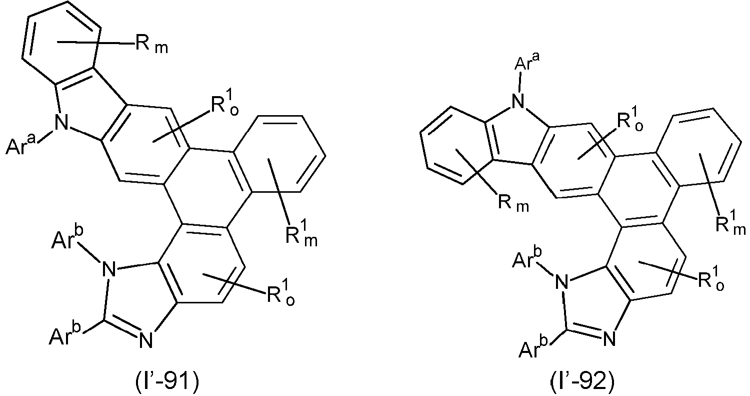 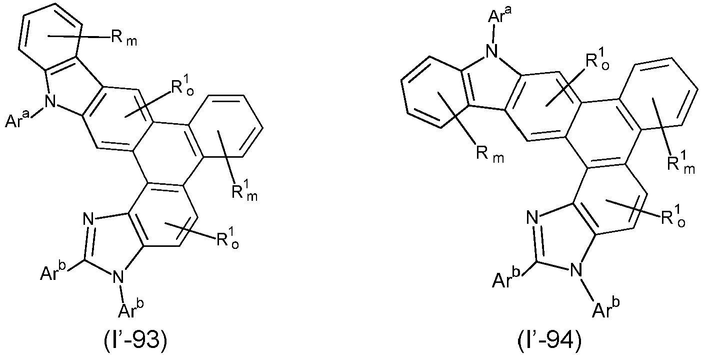 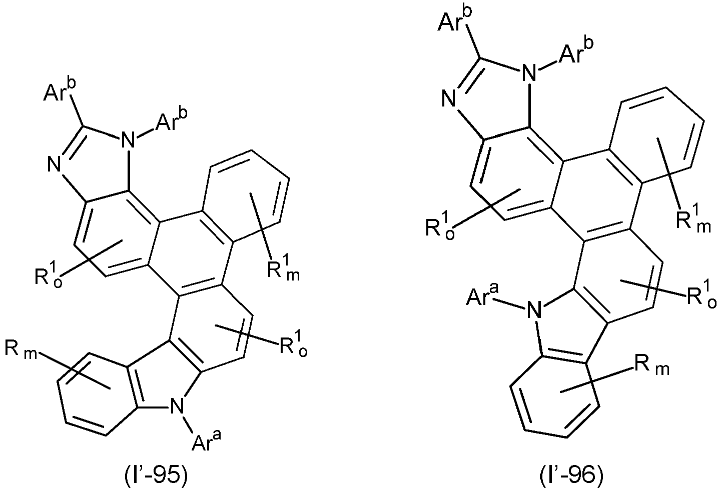 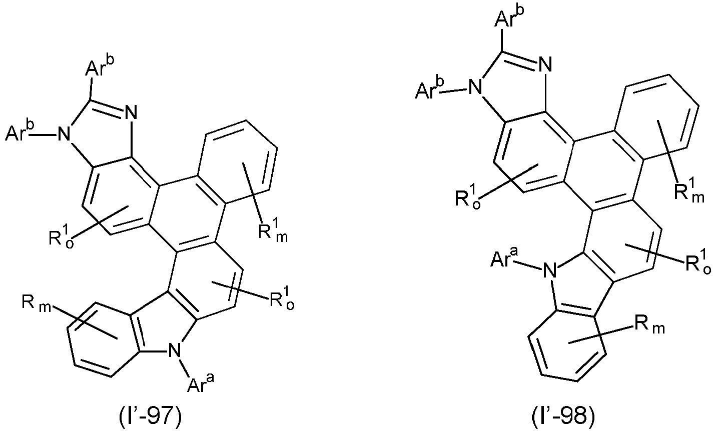 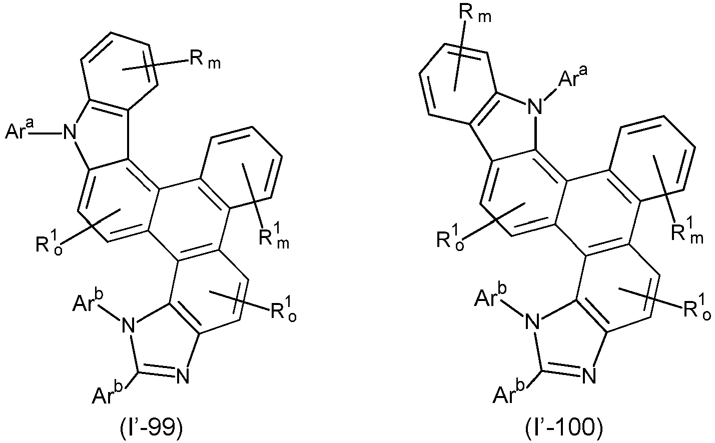 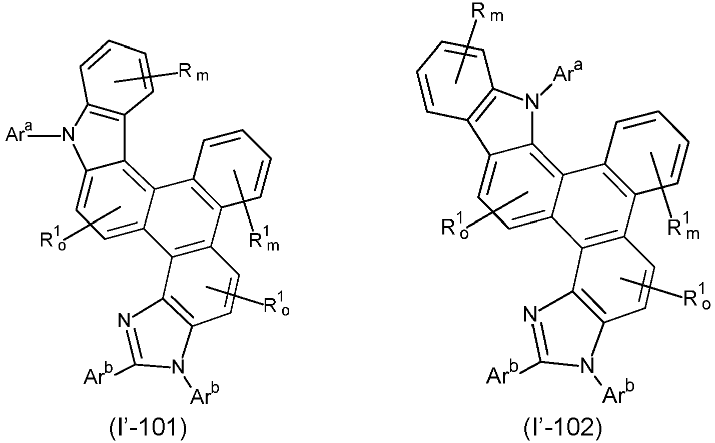 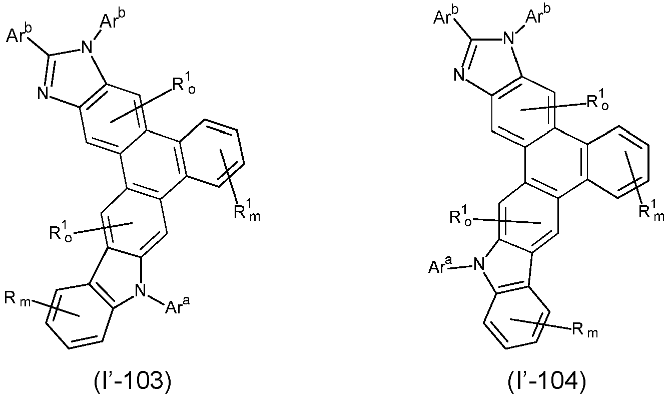 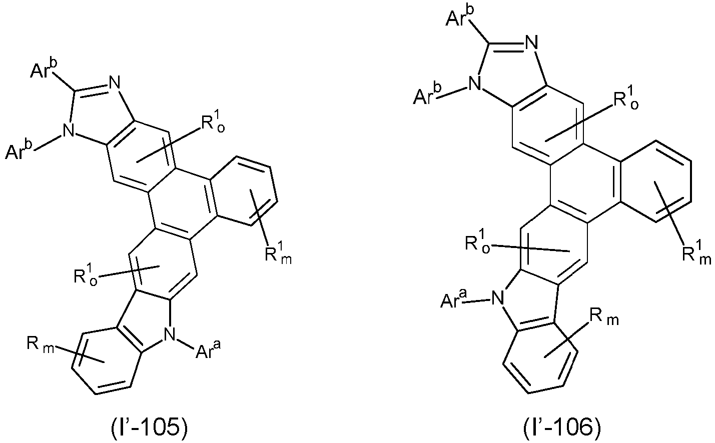 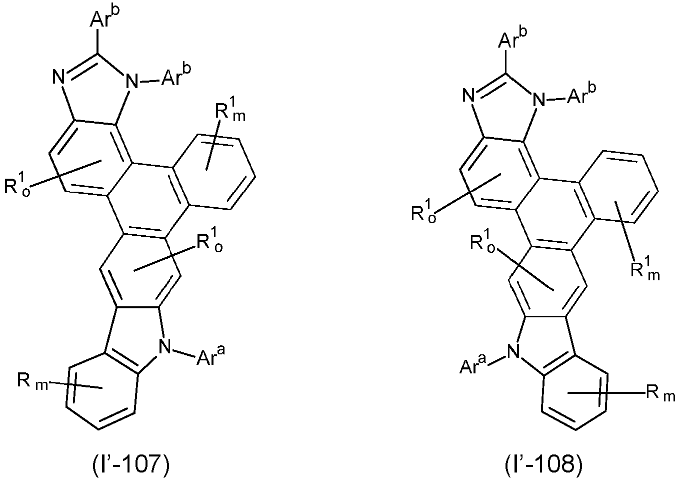 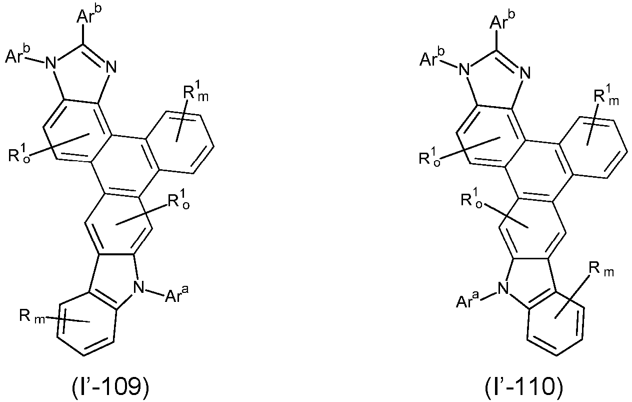 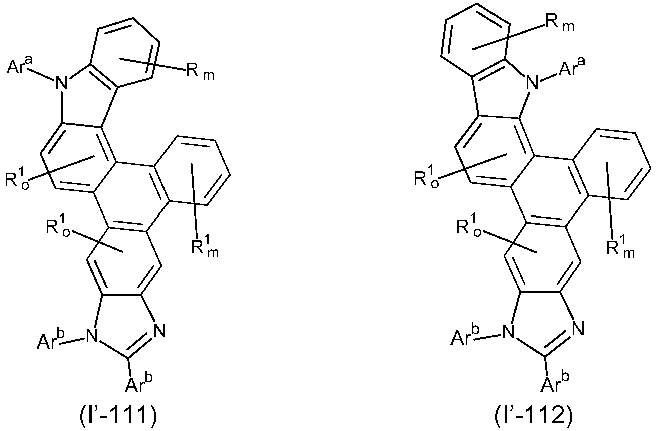 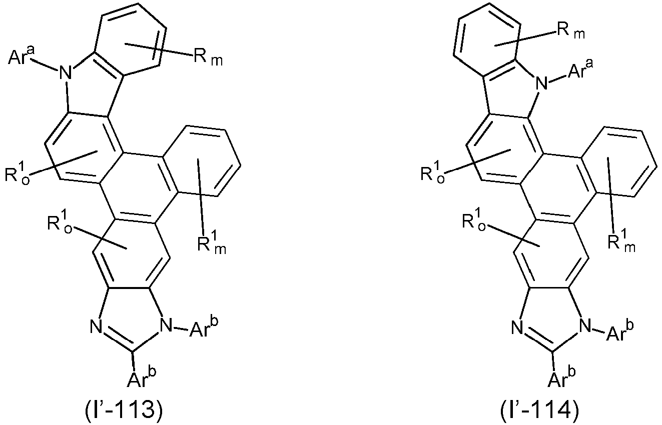 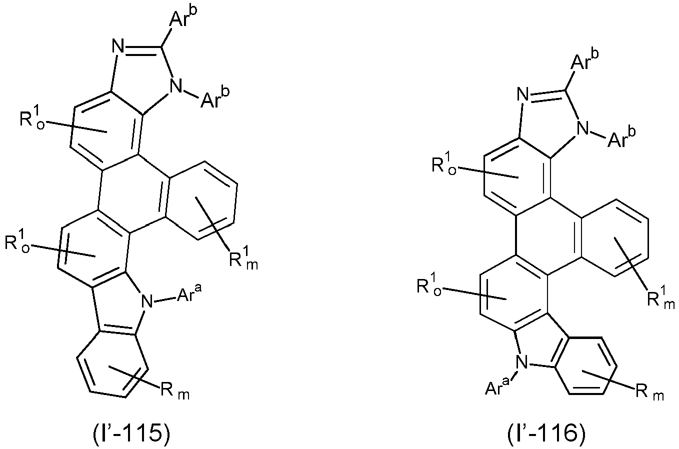 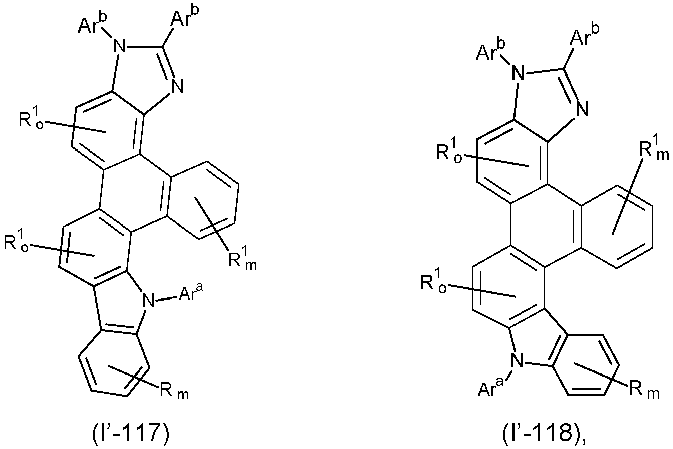 wobei die Symbole R, R¹, Ar^{a} und Ar^{b} die zuvor genannten Bedeutungen aufweisen, der Index o 0, 1 oder 2, vorzugsweise 0 oder 1 ist, der Index m 0, 1, 2, 3 oder 4, vorzugsweise 0, 1 oder 2 ist.

Die Summe der Indices m und o in Verbindungen der Formeln I'-5) bis (I'-22), (I'-31) bis (I'-78) und (I'-83) bis (I'-118)beträgt vorzugsweise höchstens 6, insbesondere bevorzugt höchstens 4 und besonders bevorzugt höchstens 2.

Bevorzugt kann vorgesehen sein, dass zwei benachbarte Gruppen R¹ kein kondensiertes aromatisches oder heteroaromatisches Ringsystem mit den Gruppen bilden, an die die Gruppen R¹ binden, wobei dies die Reste R⁴ oder R⁵ einschließt, durch die die Gruppen R¹ substituiert sein können, wobei vorzugsweise zwei Gruppen R¹ kein kondensiertes aromatisches oder heteroaromatisches Ringsystem mit den Gruppen bilden, an die die Gruppen R¹ binden.

Bevorzugte aromatische bzw. heteroaromatische Ringsysteme Ar^{a} sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R² substituiert sein können.

Bevorzugte aromatische bzw. heteroaromatische Ringsysteme Ar^{b} sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R³ substituiert sein können.

Weiterhin kann vorgesehen sein, dass die Substituenten R, R¹, R² und R³ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R⁴, R⁵ ein, die an die Reste R, R¹, R², R³ gebunden sein können.

Wenn zwei Reste, die insbesondere ausgewählt sein können aus R¹, R², R³, R⁴ und/oder R⁵, miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein.

Ferner kann vorgesehen sein, dass R, R¹, R² und/oder R³ gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-75, vorzugsweise mindestens einer der Reste R, R¹, R² und/oder R³ gleich oder verschieden bei jedem Auftreten ausgewählt ist aus den Gruppen der folgenden Formeln Ar-1 bis Ar-75 und/oder die Gruppe Ar' gleich oder verschieden bei jedem Auftreten ausgewählt ist aus den Gruppen der folgenden Formeln Ar-1 bis Ar-75, 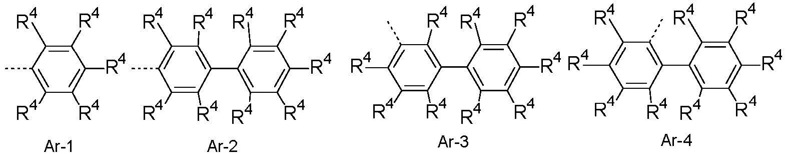 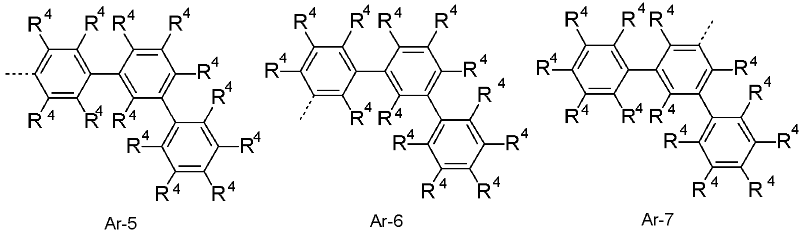 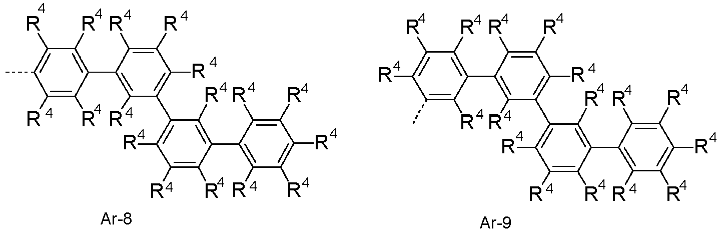 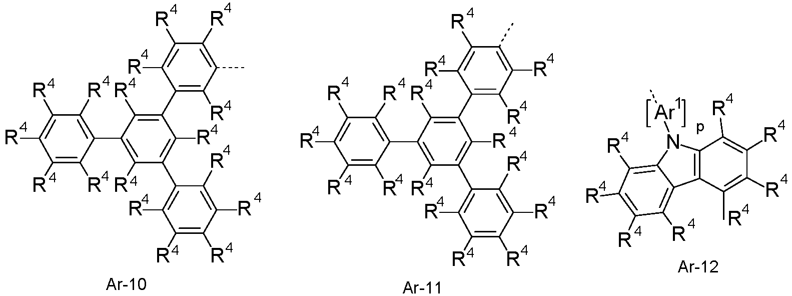 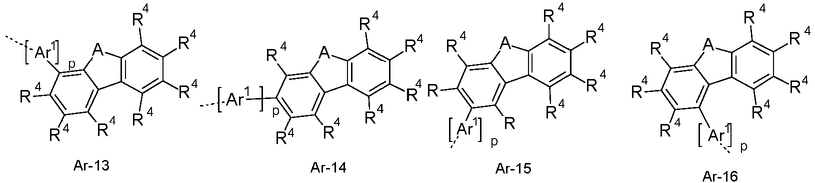 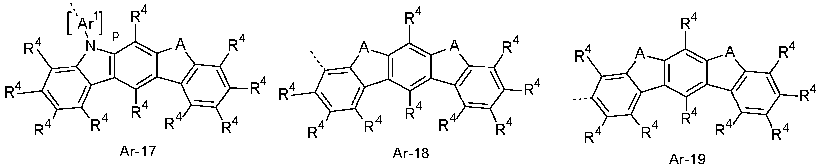 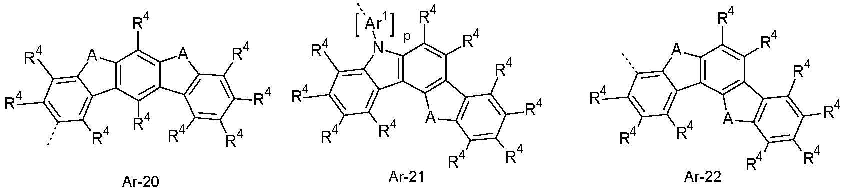 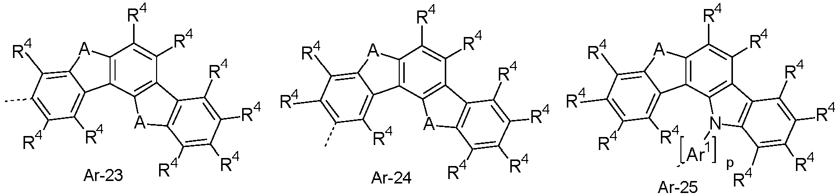 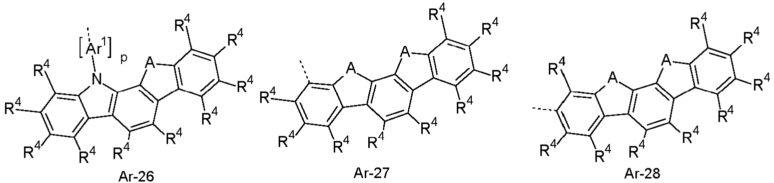 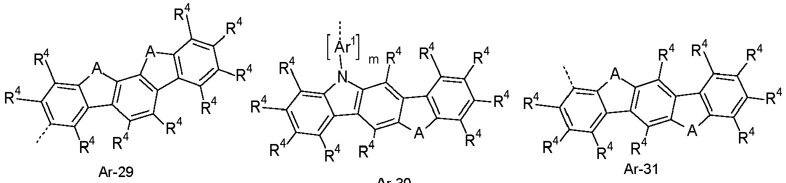 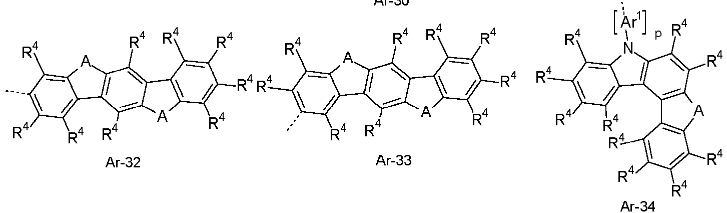 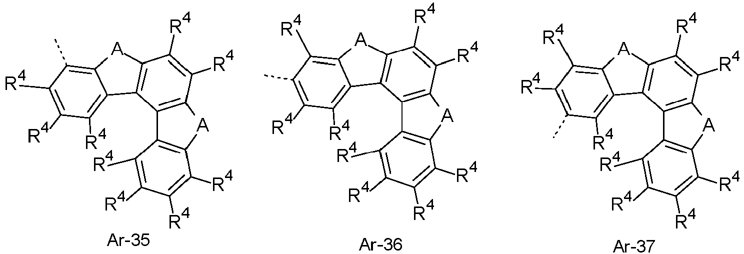 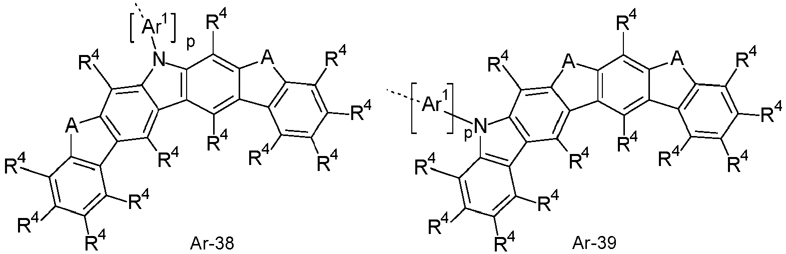 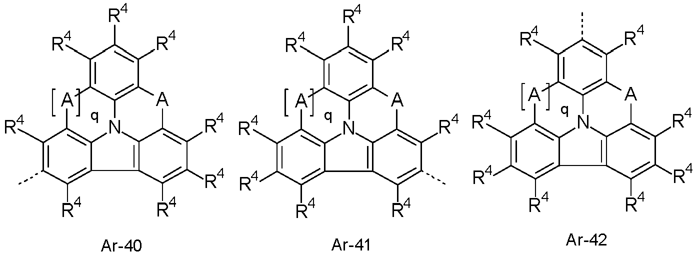 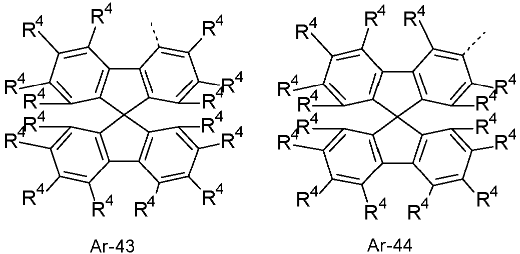 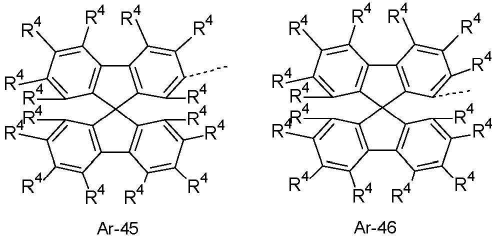 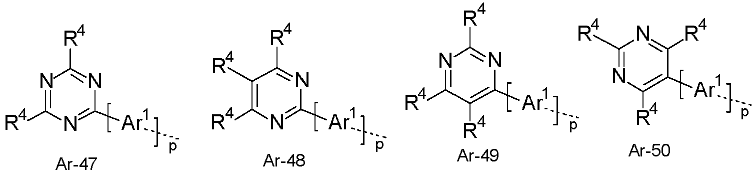 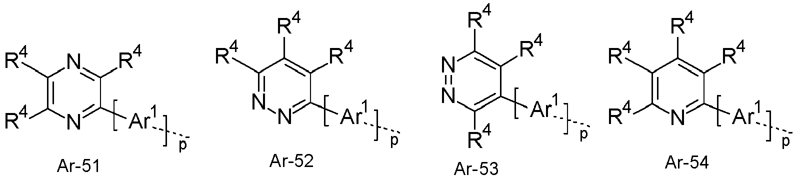 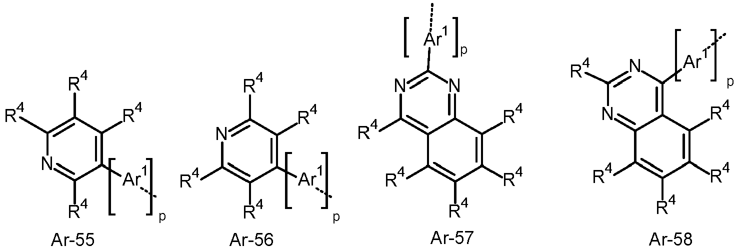 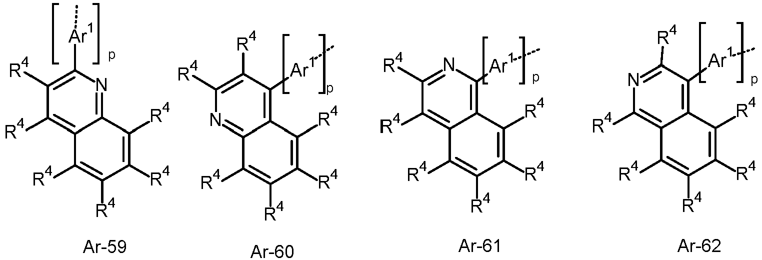 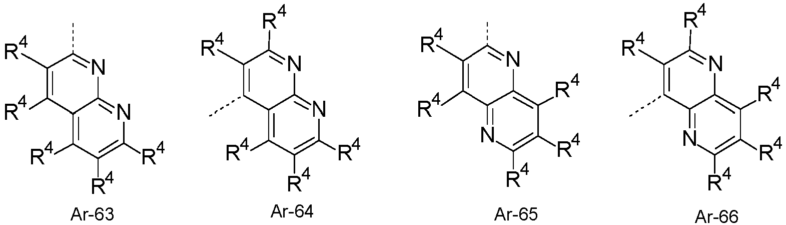 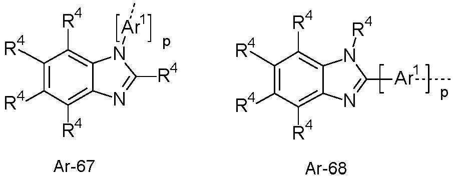 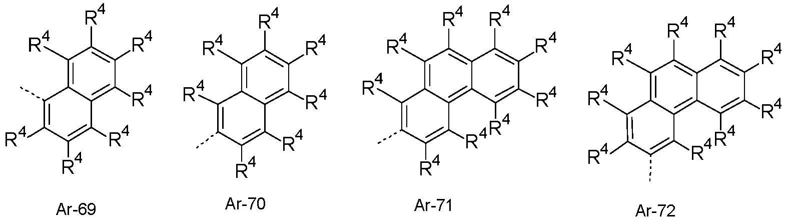 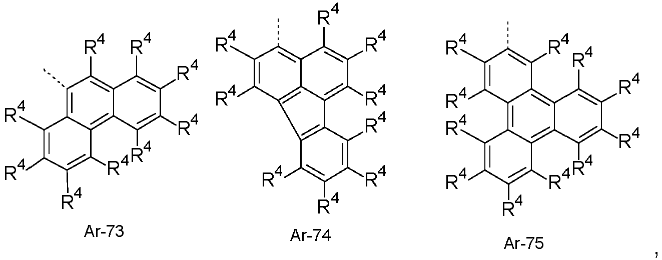 wobei R⁴ die oben genannten Bedeutungen aufweist, die gestrichelte Bindung die Anbindungsstelle darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R⁴ substituiert sein kann;
- A: ist bei jedem Auftreten gleich oder verschieden C(R⁴)₂, NR⁴, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R⁴ gebunden sind.

Die zuvor dargelegten Strukturen der Formeln (Ar-1) bis (Ar-75) stellen bevorzugte Ausgestaltungen der Reste Ar^{a}, Ar^{b} dar, wobei in diesem Fall die Substituenten R⁴ in Formeln (Ar-1) bis (Ar-75) durch R² beziehungsweise R³ zu ersetzen sind, wobei R², R³ die zuvor dargelegte Bedeutung aufweisen.

Hierbei sind Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für Ar' mehrere Gruppen A aufweisen, so kommen hierfür alle Kombinationen aus der Definition von A in Frage. Bevorzugte Ausführungsformen sind dann solche, in denen eine Gruppe A für NR⁴ und die andere Gruppe A für C(R⁴)₂ steht oder in denen beide Gruppen A für NR⁴ stehen oder in denen beide Gruppen A für O stehen.

Wenn A für NR⁴ steht, steht der Substituent R⁴, der an das Stickstoffatom gebunden ist, bevorzugt für ein aromatisches oder heteroaromatisches Ringsystem mit 5 bis 24 aromatischen Ringatomen, welches auch durch einen oder mehrere Reste R⁵ substituiert sein kann. In einer besonders bevorzugten Ausführungsform steht dieser Substituent R⁴ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R⁵ substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R⁴ durch einen oder mehrere Reste R⁵ substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R⁴ durch einen oder mehrere Reste R⁵ substituiert sein können.

Wenn A für C(R⁴)₂ steht, stehen die Substituenten R⁴, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R⁵ substituiert sein kann. Ganz besonders bevorzugt steht R⁴ für eine Methylgruppe oder für eine Phenylgruppe. Dabei können die Reste R⁴ auch miteinander ein Ringsystem bilden, was zu einem Spirosystem führt.

Bevorzugt kann die Gruppe Ar¹ mit den Gruppen, an die die Gruppe Ar¹ gemäß Formeln (Ar-1) bis (Ar-75) gebunden ist, eine durchgängige Konjugation ausbilden.

In einer weiteren bevorzugten Ausführungsform der Erfindung steht Ar¹ für ein aromatisches oder heteroaromatisches Ringsystem mit 6 bis 14 aromatischen oder heteroaromatischen Ringatomen, vorzugsweise ein aromatisches Ringsystem mit 6 bis 12 Kohlenstoffatomen, welches durch einen oder mehrere Reste R⁴ substituiert sein kann, bevorzugt aber unsubstituiert ist, wobei R⁴ die zuvor genannte Bedeutung aufweisen kann. Besonders bevorzugt steht Ar¹ für eine Bindung oder ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R⁴ substituiert sein kann, bevorzugt aber unsubstituiert ist, wobei R⁴ die zuvor genannte Bedeutung aufweisen kann.

Weiterhin bevorzugt steht das unter anderem in Formeln (Ar-1) bis (Ar-75) dargelegte Symbol Ar¹ gleich oder verschieden bei jedem Auftreten für eine Bindung oder einen Aryl- oder Heteroarylrest mit 6 bis 18 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (Ar-1) bis (Ar-75) dargelegte Gruppe Ar¹ ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme Ar¹ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R⁴ substituiert sein können, bevorzugt aber unsubstituiert sind.

Ferner kann vorgesehen sein, dass die unter anderem in Formeln (Ar-1) bis (Ar-75) dargelegte Gruppe Ar¹ höchstens 1 Stickstoffatom, bevorzugt höchstens 2 Heteroatome, insbesondere bevorzugt höchstens ein Heteroatom aufweist, welches ausgewählt ist aus O oder S.

Weiterhin kann vorgesehen sein, dass die Gruppe Ar¹ kein kondensiertes aromatisches oder heteroaromatisches Ringsystem mit den Gruppen bildet, an die die Gruppe Ar¹ bindet, wobei dies die Reste R⁴ oder R⁵ einschließt, durch die die Gruppe Ar¹ oder eine der Gruppen, an die die Gruppe Ar¹ bindet, substituiert sein können.

Besonders bevorzugt steht die Gruppe Ar¹ für eine Gruppe, die ausgewählt ist aus den Formeln (L¹-1) bis (L¹-17), 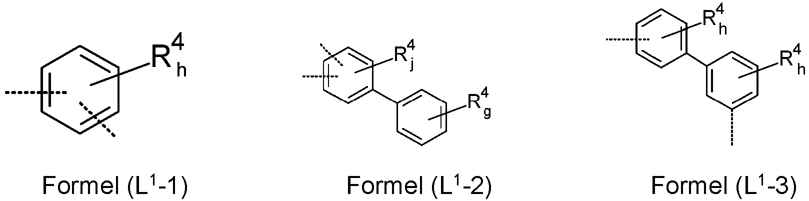 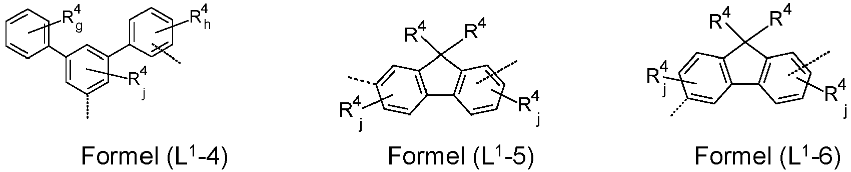 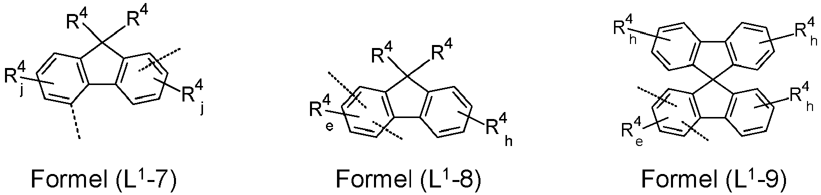 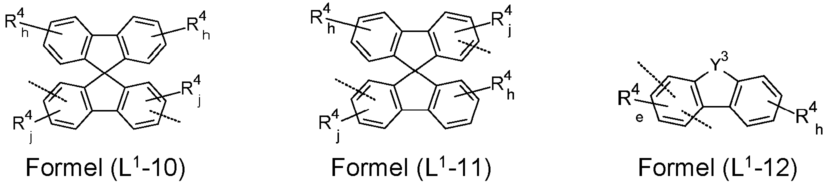 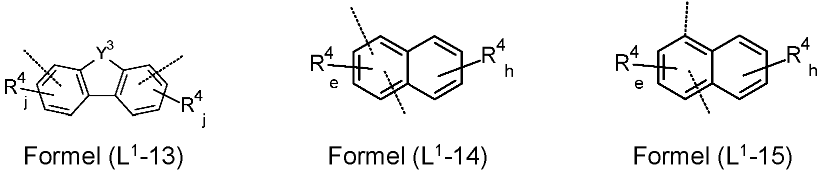 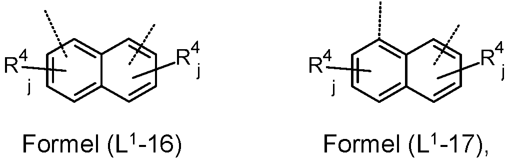 wobei die gestrichelten Bindungen jeweils die Anbindungspositionen markieren, Y³ bei jedem Auftreten gleich oder verschieden bevorzugt O, S, NAr', NR², bevorzugt O oder S ist; der Index k 0 oder 1 ist, der Index e 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; und das Symbol R⁴ die zuvor genannte Bedeutung aufweist.

Vorzugsweise stellt die Gruppe Ar¹ eine Struktur der Formeln (L¹-1) oder (L¹-12) bis (L¹-17) dar, besonders bevorzugt Formeln (L¹-1), (L¹-12) oder (L¹-15), wobei Y³ vorzugsweise jeweils für O oder S steht.

Vorzugsweise kann vorgesehen sein, dass die Verbindungen mindestens zwei Elektronenleitgruppen aufweisen, die ausgewählt sind aus Substituenten der zuvor dargestellten Formeln (Ar-47) bis (Ar-51), (Ar-57), (Ar-58) und/oder (Ar-63) bis (Ar-68).

Elektronenleitgruppen sind in der Fachwelt weithin bekannt und fördern die Fähigkeit von Verbindungen, Elektronen zu transportieren und/oder zu leiten. Zu den Elektronenleitgruppen gehören unter anderem Strukturen, die von Pyridinen, Pyrimidinen, Pyrazinen, Pyridazinen, Triazinen, Chinazolinen, Chinoxalinen, Chinolinen, Isochinolinen, Imidazolen und/oder Benzimidazolen abgeleitet sind, wobei Pyrimidin-, Triazin- und Chinazolin-Strukturen besonders bevorzugt sind.

Bevorzugt kann weiterhin vorgesehen sein, dass mindestens ein, vorzugsweise beide, Rest(e) Ar^{b} durch eine Gruppe der zuvor dargestellten Formeln (Ar-47) bis (Ar-51), (Ar-57), (Ar-58) und/oder (Ar-63) bis (Ar-68) darstellbar ist/sind, wobei die in den Formeln (Ar-47) bis (Ar-51), (Ar-57), (Ar-58) und/oder (Ar-63) bis (Ar-68) dargelegten Substituenten R⁴ durch R³ zu ersetzen sind.

Besonders bevorzugt kann weiterhin vorgesehen sein, dass die Verbindungen mindestens einen Substituenten der zuvor dargestellten Formeln (Ar-57) oder (Ar-58) aufweisen.

Bevorzugt kann weiterhin vorgesehen sein, dass der Rest Ar^{a} durch eine Gruppe der zuvor dargestellten Formeln (Ar-47) bis (Ar-51), (Ar-57), (Ar-58) und/oder (Ar-63) bis (Ar-68), vorzugsweise (Ar-47) bis (Ar-51), (Ar-57) oder (Ar-58) besonders bevorzugt (Ar-57) oder (Ar-58) darstellbar ist, wobei die in den Formeln (Ar-47) bis (Ar-51), (Ar-57), (Ar-58) und/oder (Ar-63) bis (Ar-68) dargelegten Substituenten R⁴ durch R² zu ersetzen sind. Hierbei ist p vorzugsweise 0, so dass die Verbindungsgruppe Ar¹ in Formel (Ar-47) bis (Ar-51), (Ar-57), (Ar-58), (Ar-67) und/oder (Ar-68) nicht vorhanden ist.

Im Folgenden werden bevorzugte Substituenten R, R¹, R² und R³ beschrieben.

In einer bevorzugten Ausführungsform der Erfindung ist R, R¹, R² und R³ gleich oder verschieden bei jedem Auftreten ausgewählt aus der Gruppe bestehend aus H, D, F, CN, NO₂, Si(R⁴)₃, B(OR⁴)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁴ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann.

In einer weiterhin bevorzugten Ausführungsform der Erfindung ist R, R¹, R² und R³ gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁴ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann.

In einer weiterhin bevorzugten Ausführungsform der Erfindung ist R, R¹, R² und R³ gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R⁴ substituiert sein kann, oder einer Gruppe N(Ar')₂. Besonders bevorzugt ist R, R¹, R² gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen

Ringatomen, besonders bevorzugt mit 6 bis 13 aromatischen Ringatomen, das jeweils mit einem oder mehreren Resten R⁴ substituiert sein kann.

Bevorzugte aromatische bzw. heteroaromatische Ringsystem R, R¹, R², R³ bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁴ substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind.

In einer weiteren bevorzugten Ausführungsform der Erfindung ist R⁴ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R⁵ substituiert sein kann. In einer besonders bevorzugten Ausführungsform der Erfindung ist R⁴ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁵ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁵ substituiert sein kann, bevorzugt aber unsubstituiert ist.

In einer weiteren bevorzugten Ausführungsform der Erfindung ist R⁵ gleich oder verschieden bei jedem Auftreten H**,** eine Alkylgruppe mit 1 bis 4 C-Atomen oder eine Arylgruppe mit 6 bis 10 C-Atomen.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit beispielsweise ortho-, meta-, para- oder verzweigten Terphenyl- oder Quaterphenylgruppen, substituiert sind.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

In einer weiteren Ausgestaltung der vorliegenden Erfindung kann vorgesehen sein, dass Verbindungen der Formeln (I'-119) bis (I'-130) ausgeschlossen sind, 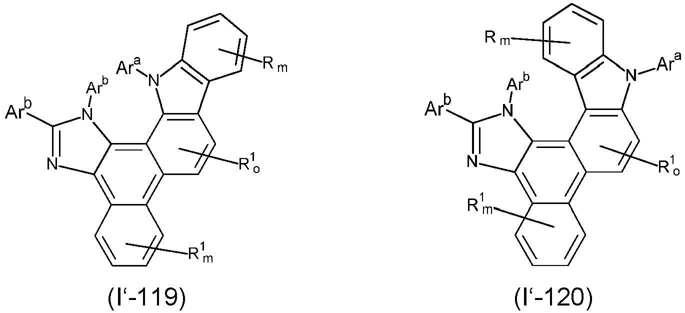 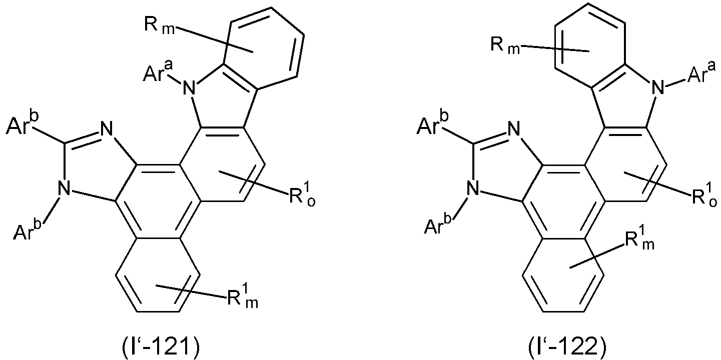 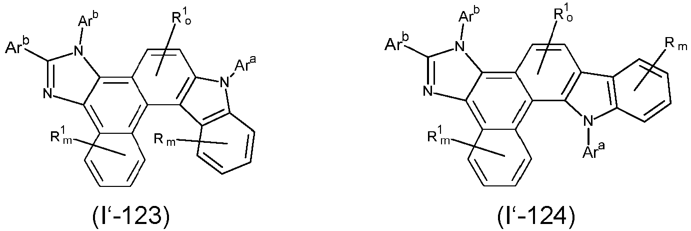 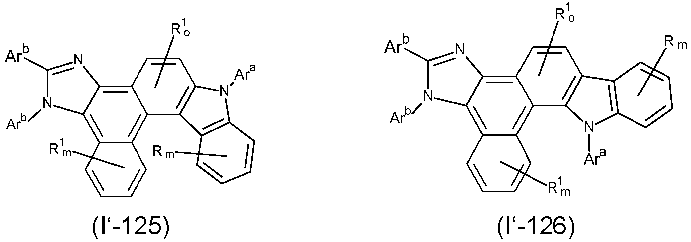 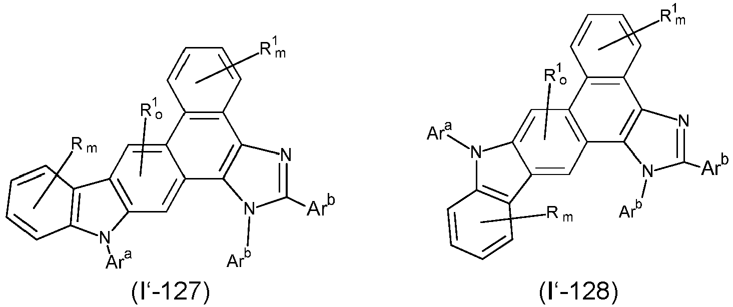 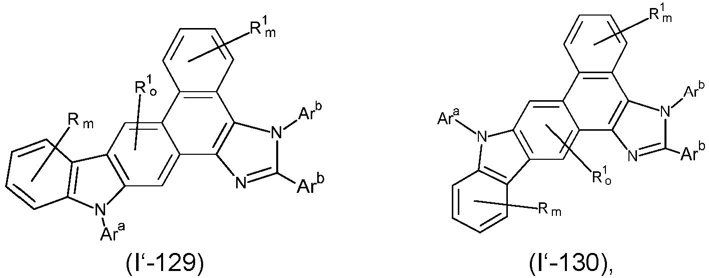 wobei R, R¹, Ar^{a} und Ar^{b} die zuvor genannten Bedeutungen aufweisen, der Index o 0, 1 oder 2, vorzugsweise 0 oder 1 ist, der Index m 0, 1, 2, 3 oder 4, vorzugsweise 0, 1 oder 2 ist.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

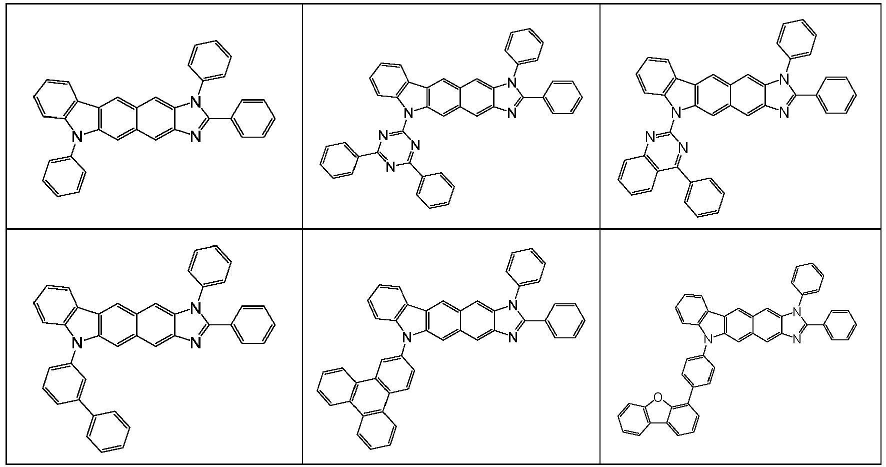

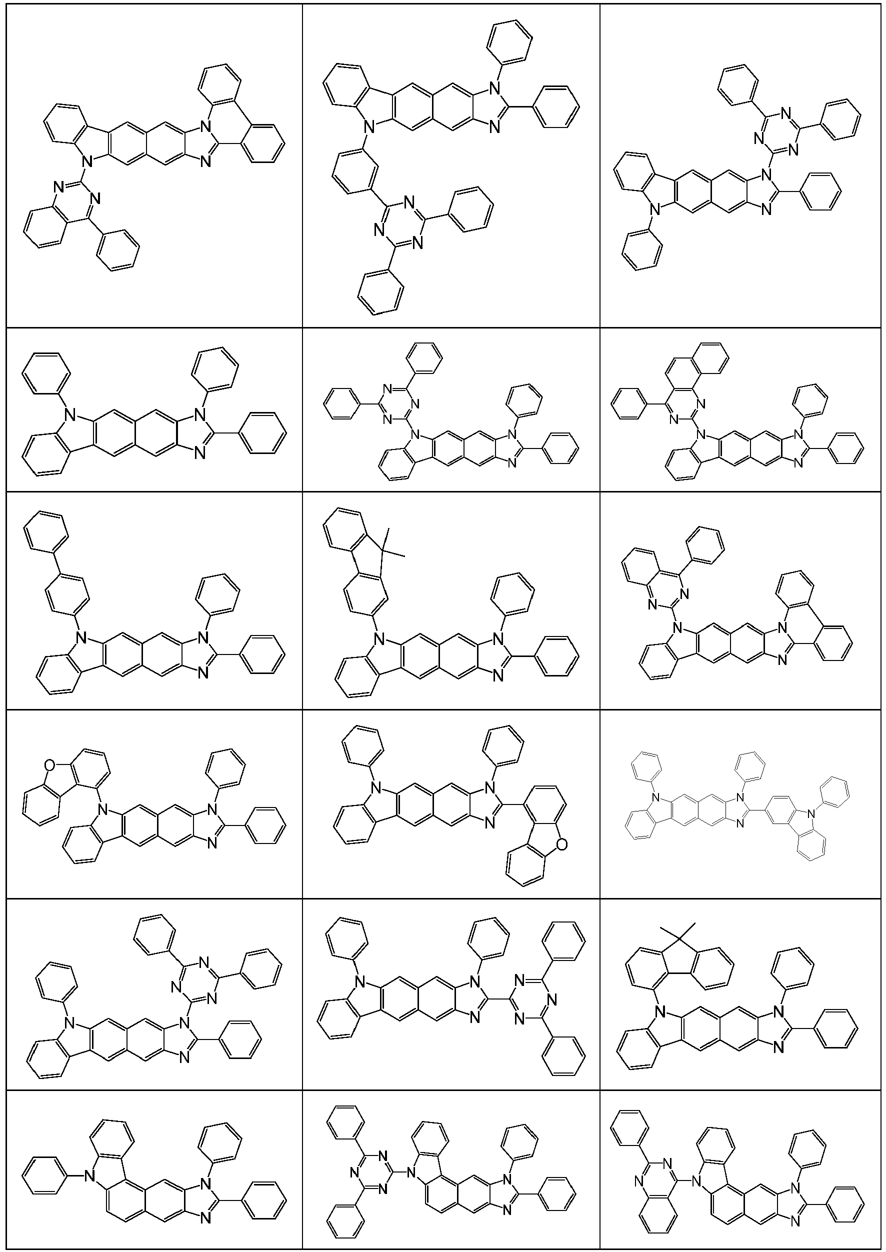

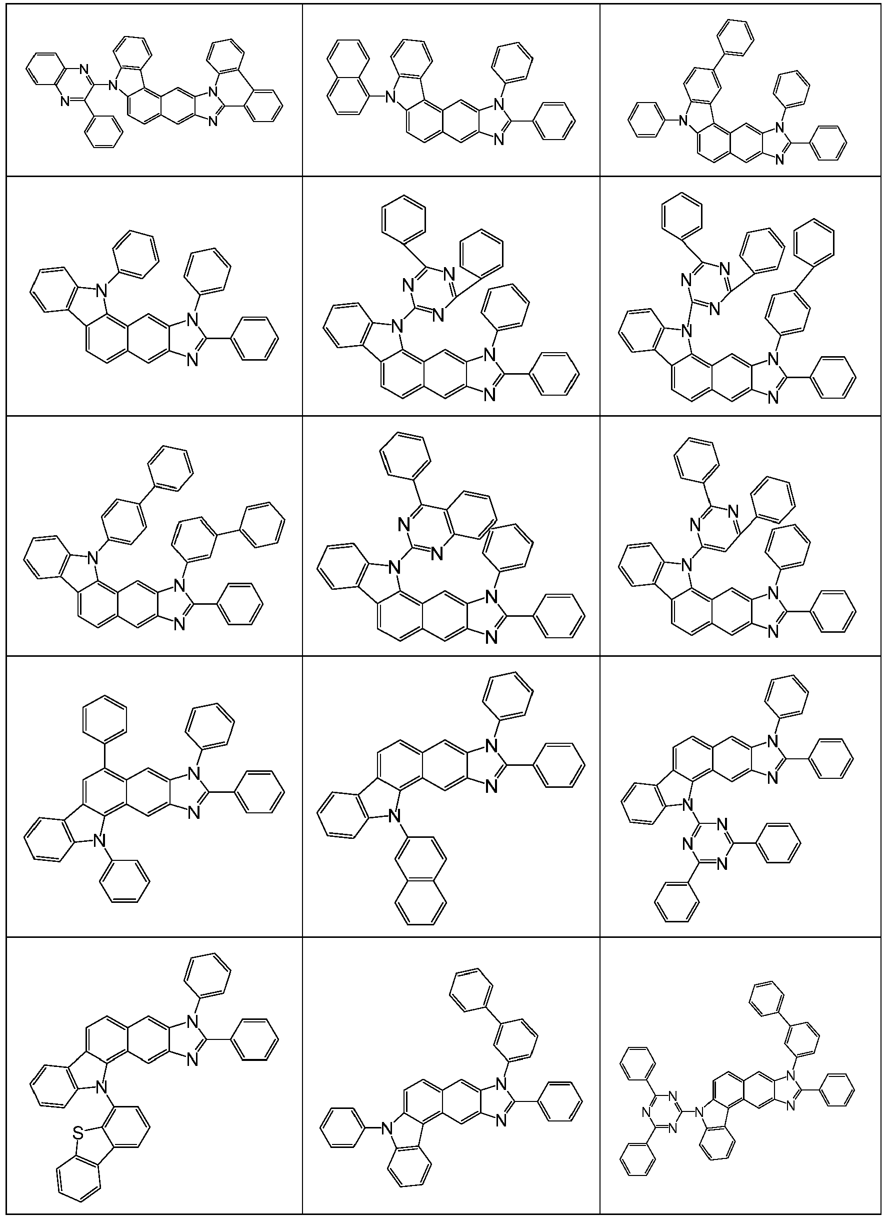

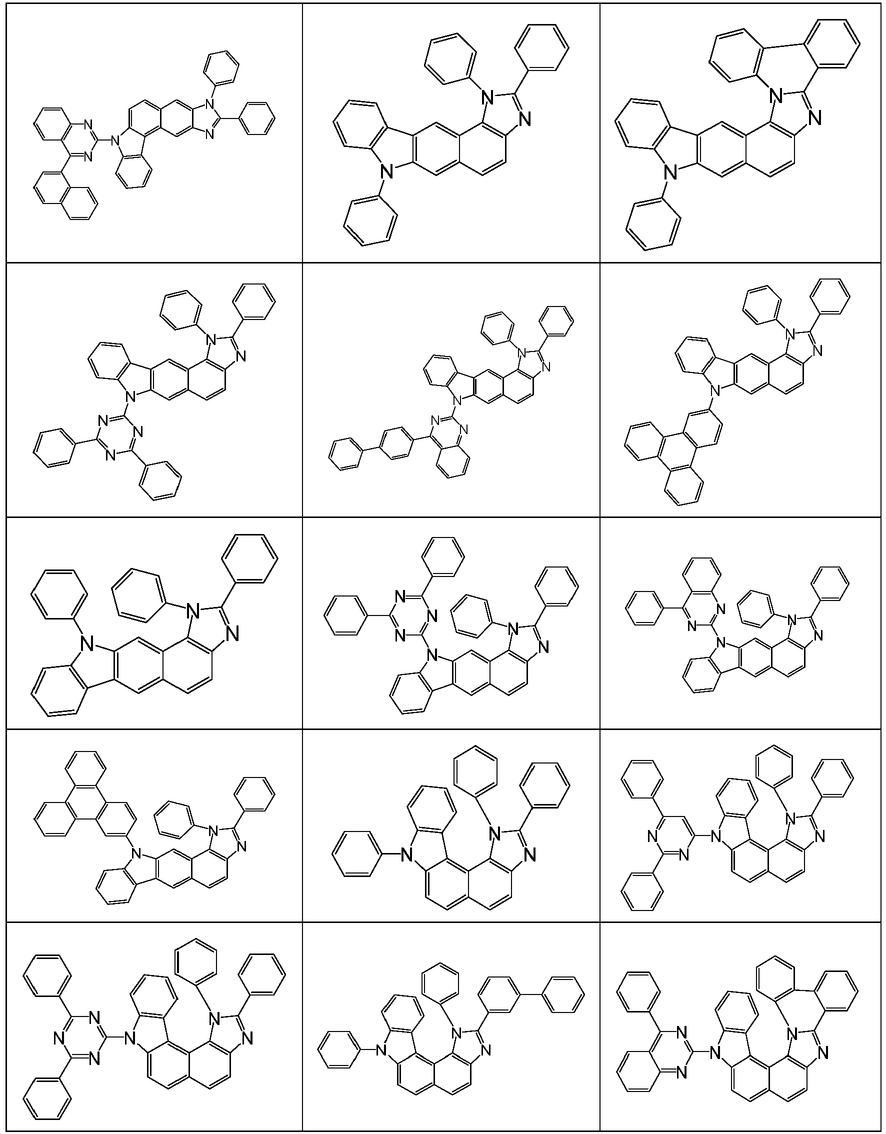

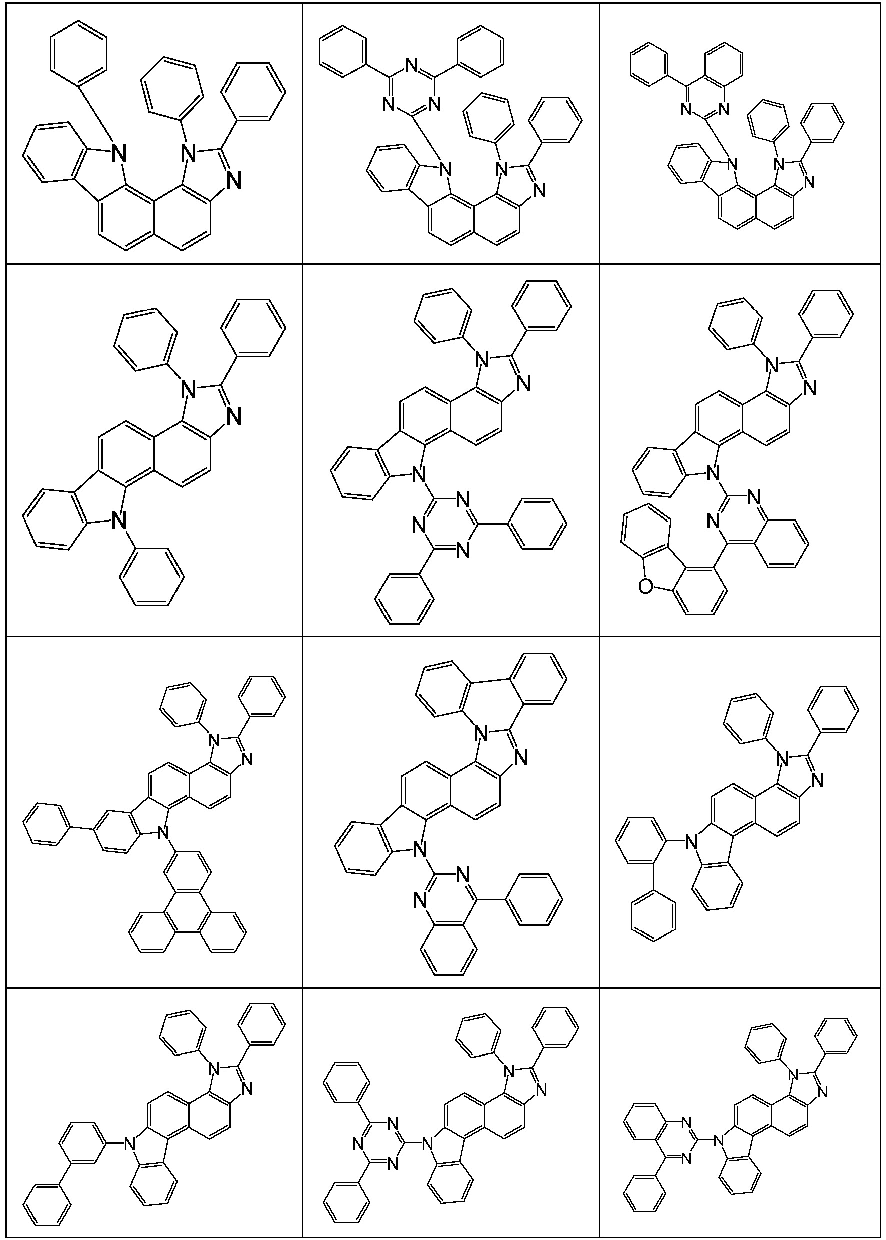

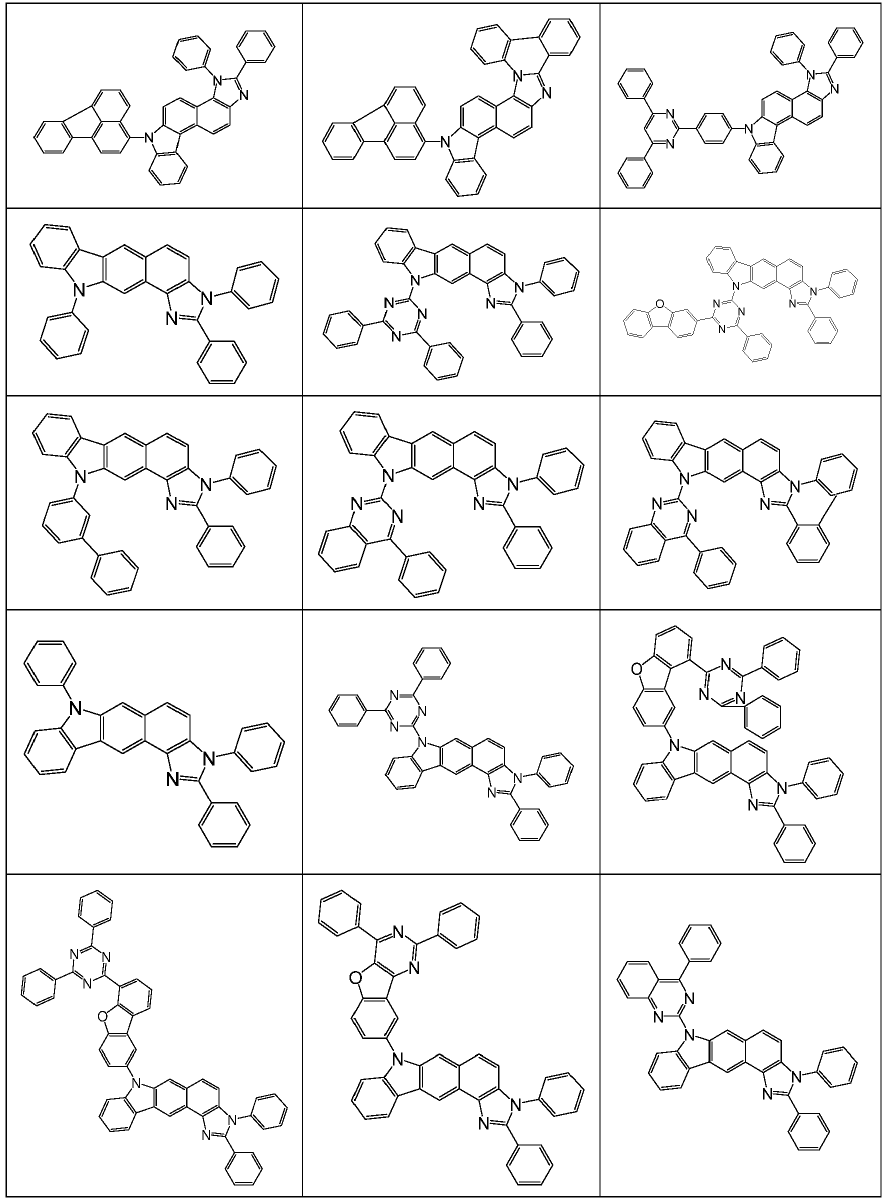

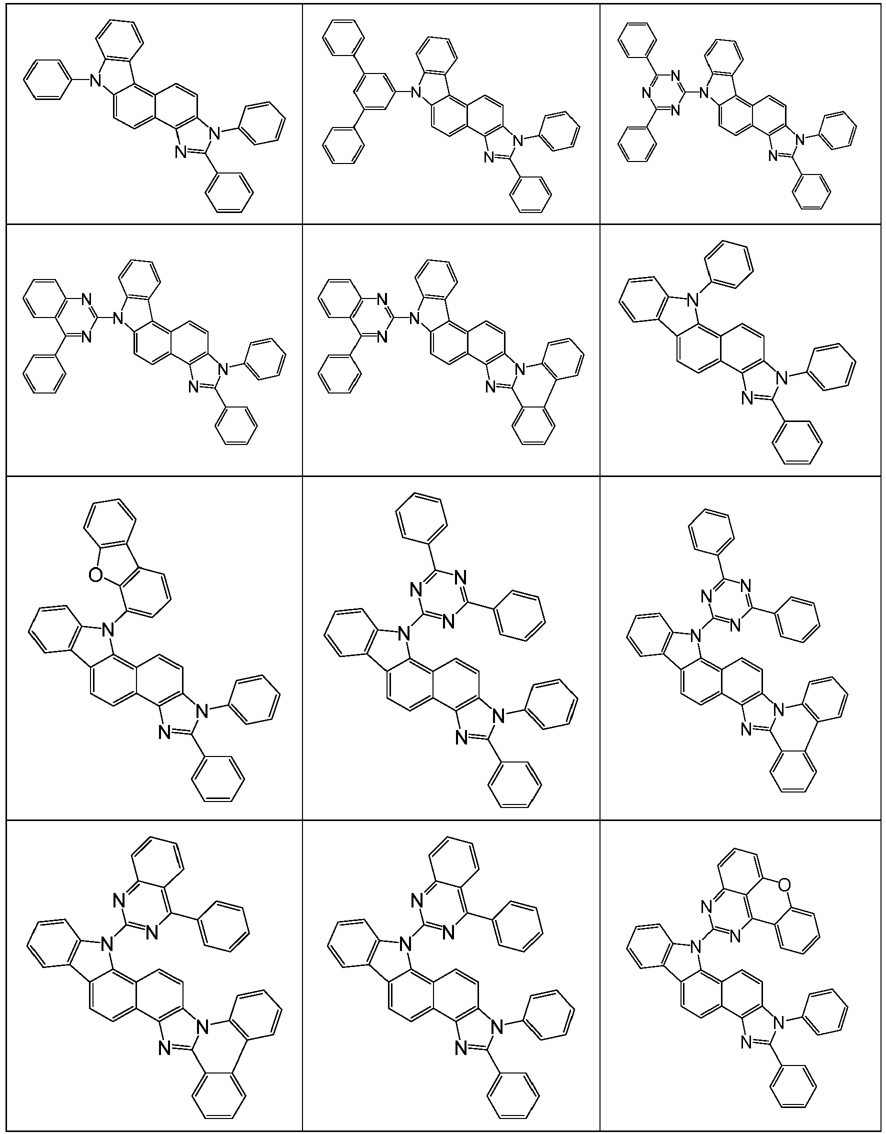

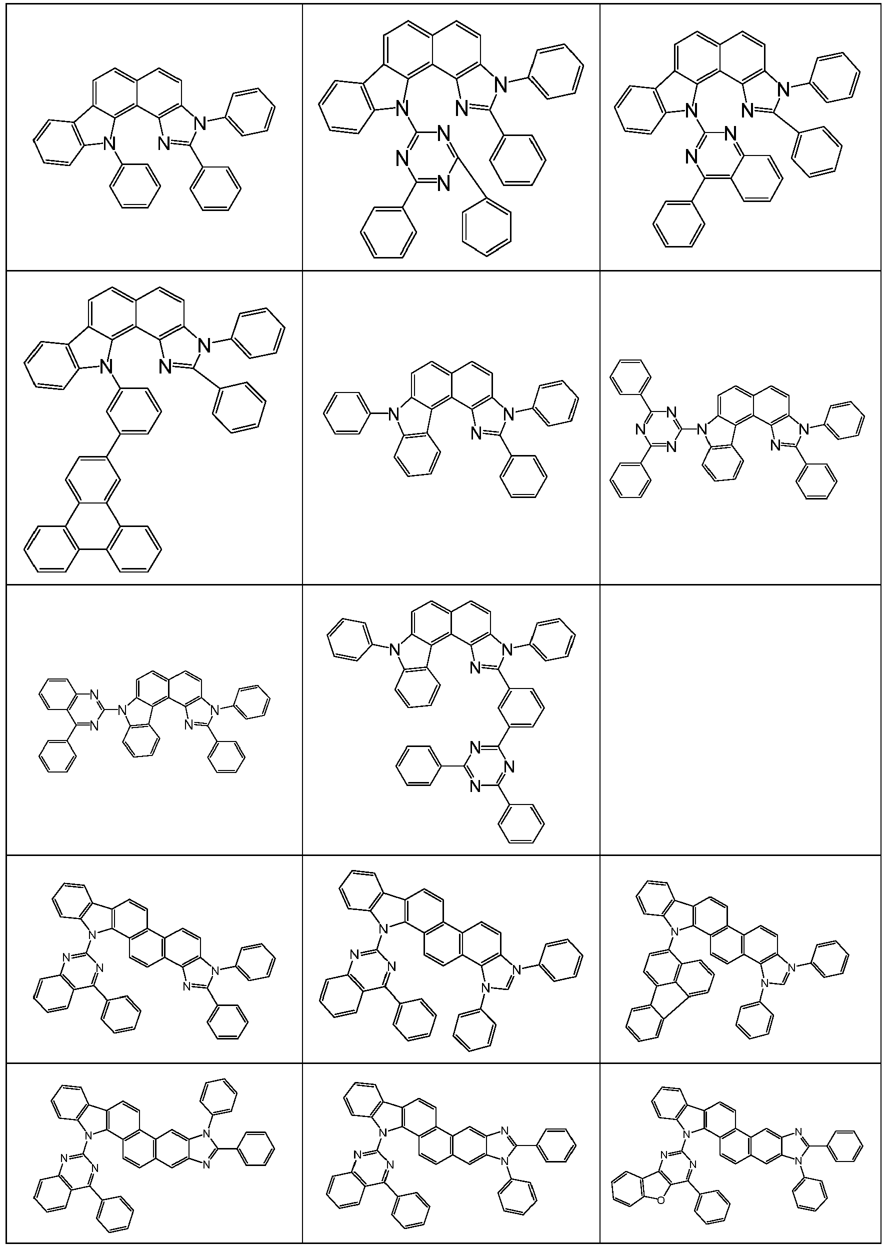

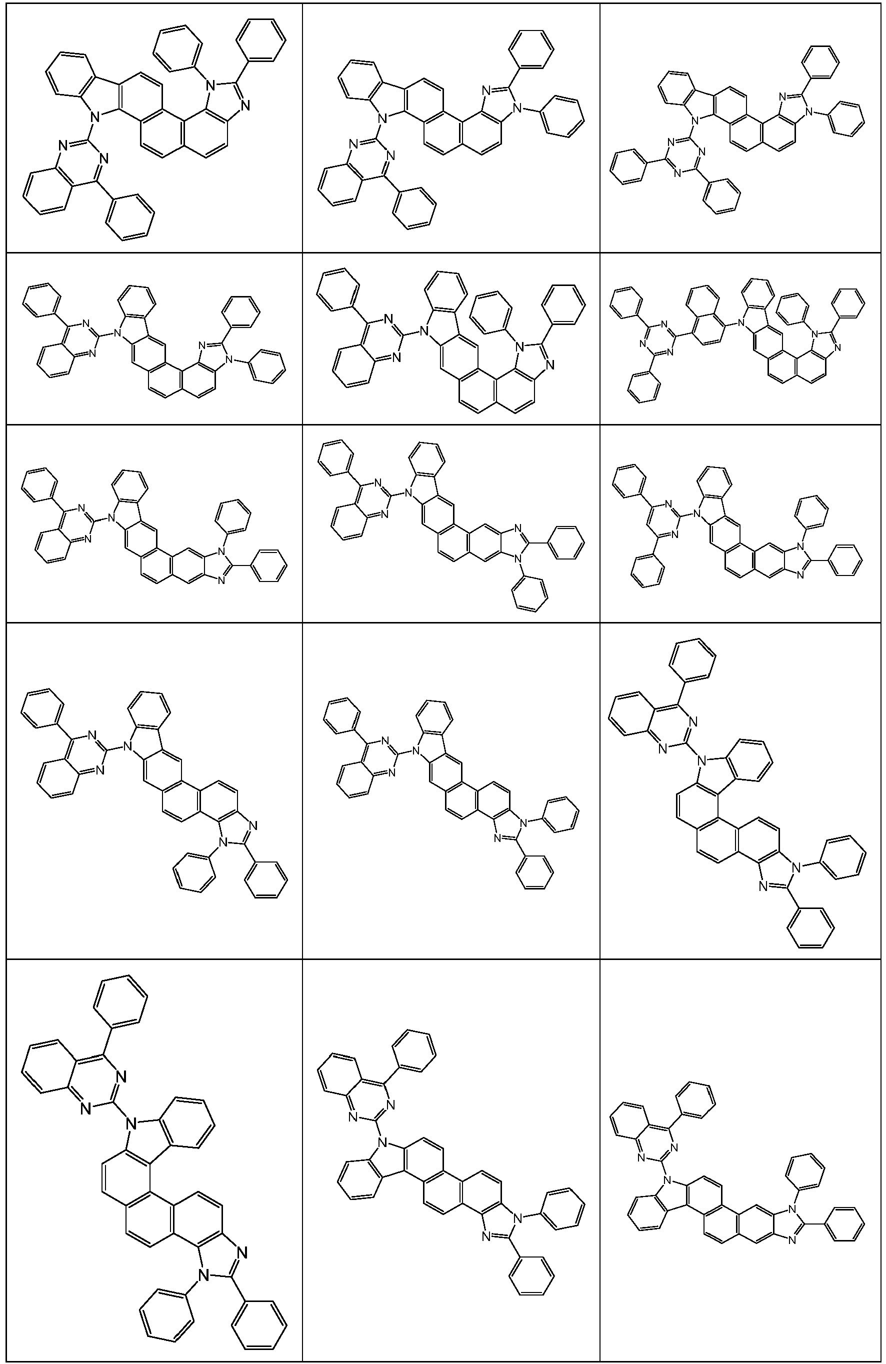

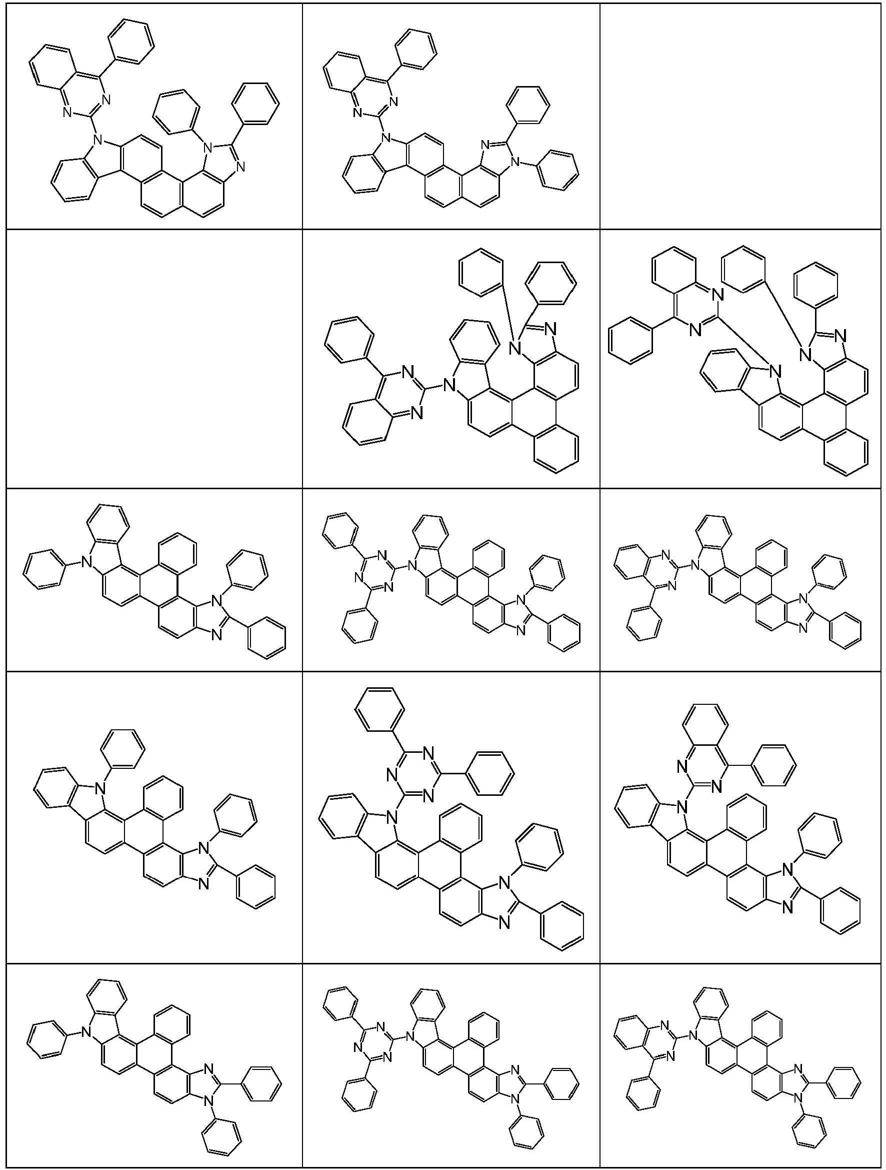

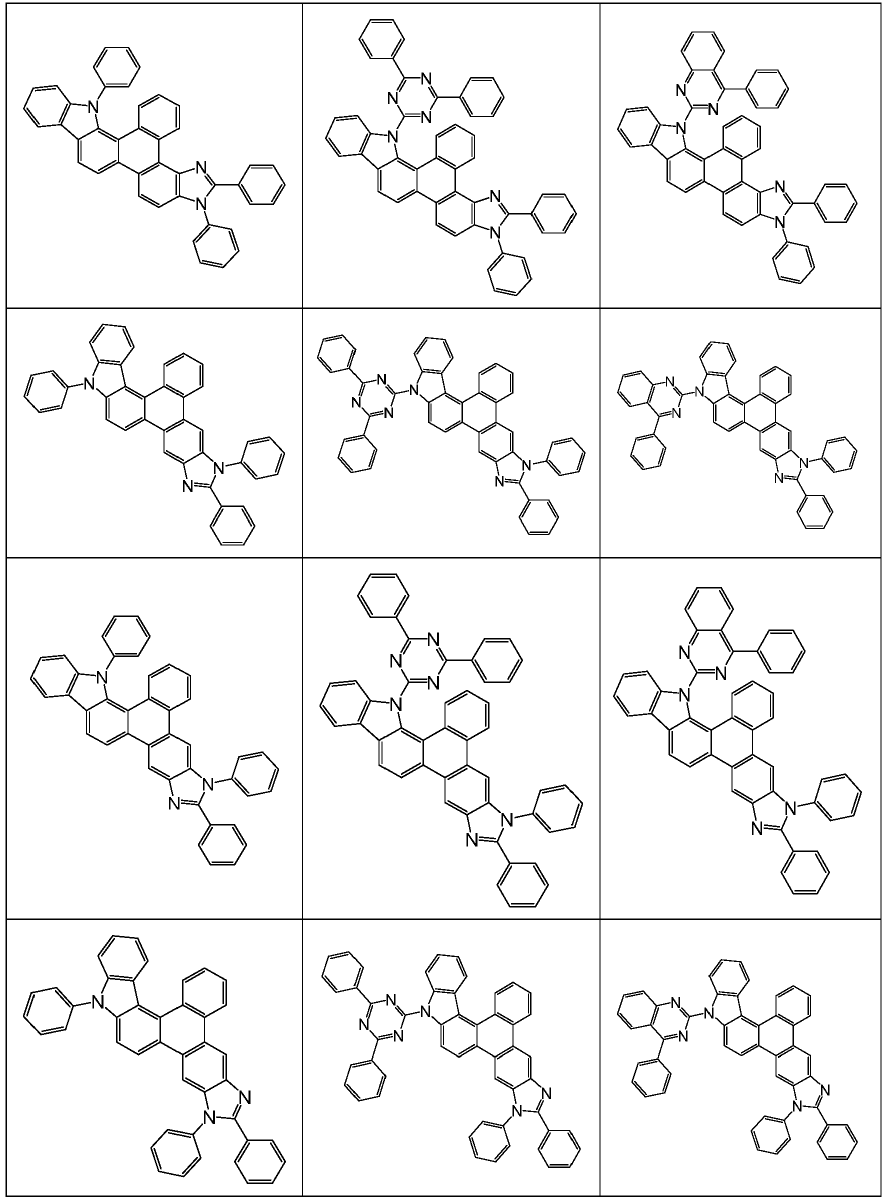

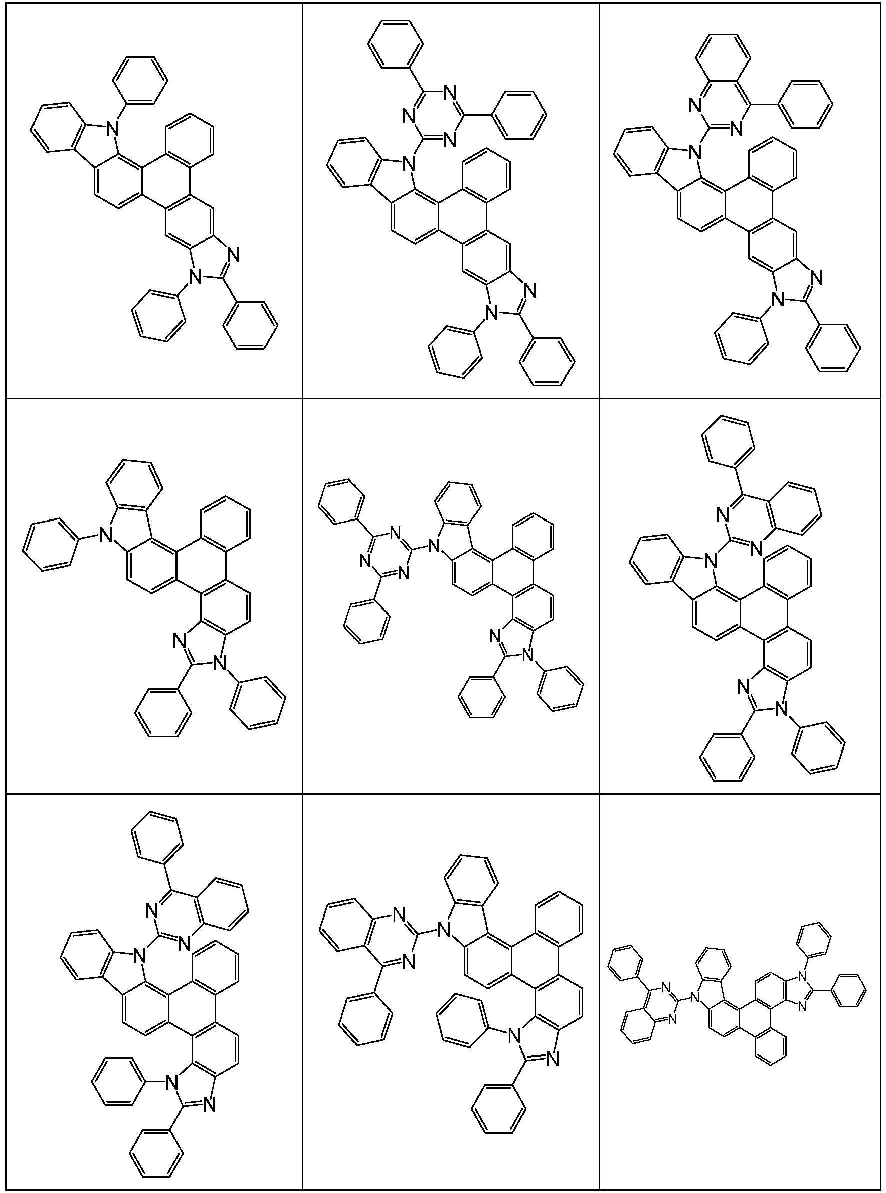

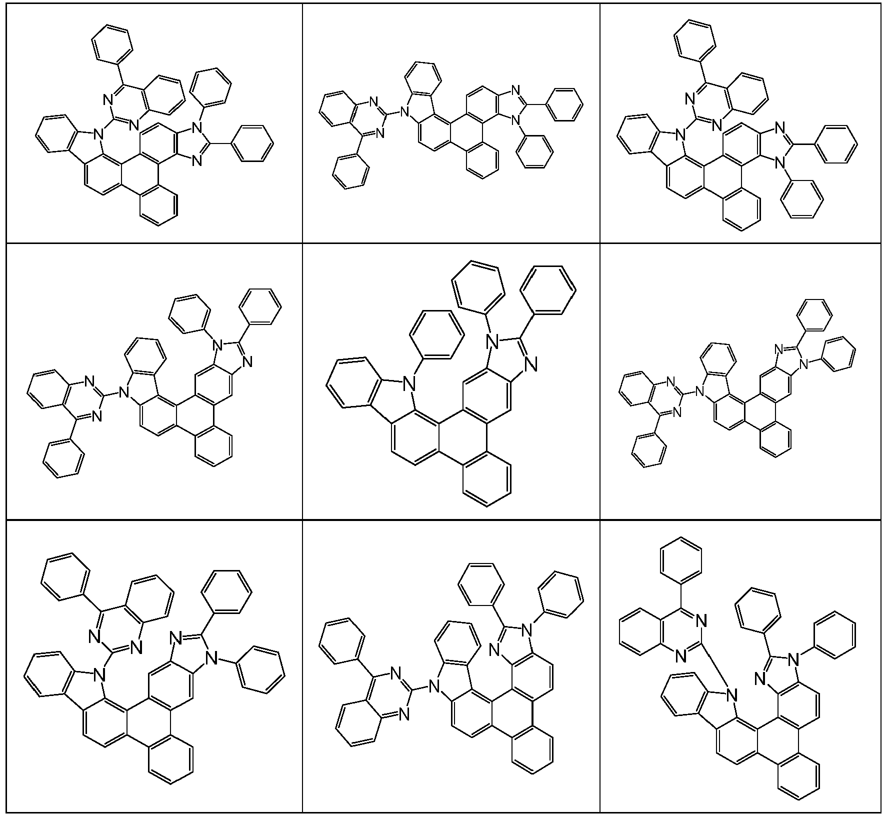

Die Grundstruktur der erfindungsgemäßen Verbindungen kann nach den in den nachfolgenden Schemata skizzierten Wegen dargestellt werden. Dabei sind die einzelnen Syntheseschritte, wie beispielsweise C-C-Kupplungsreaktionen gemäß Suzuki, C-N-Kupplungsreaktionen gemäß Hartwig-Buchwald oder Cyclisierungsreaktionen, dem Fachmann prinzipiell bekannt. Weitere Informationen zur Synthese der erfindungsgemäßen Verbindungen können den Synthesebeispielen entnommen werden. Eine mögliche Synthese der Grundstruktur sind in Schemata 1 und 2 dargestellt.

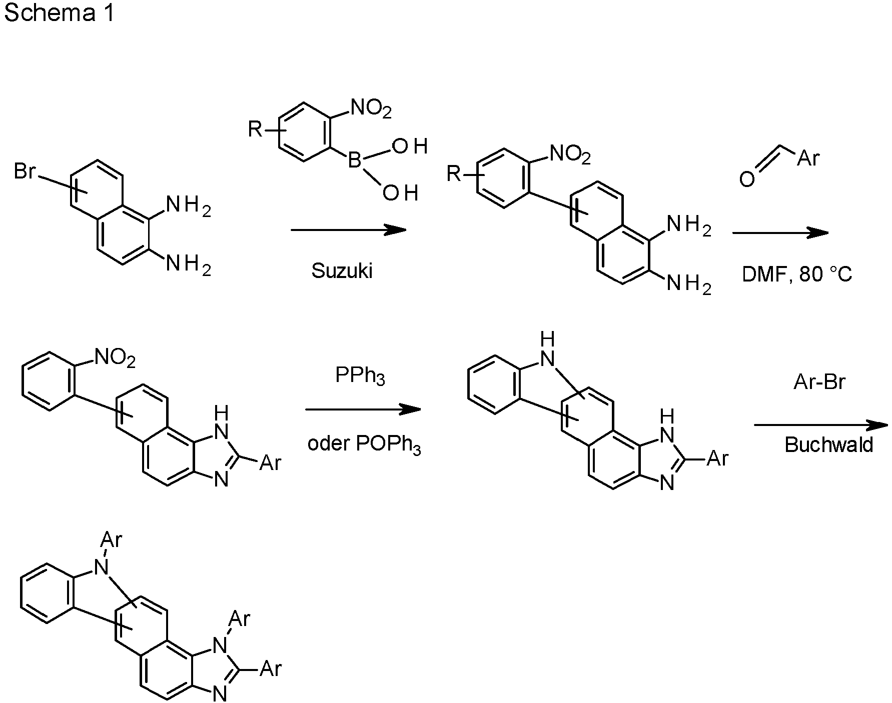

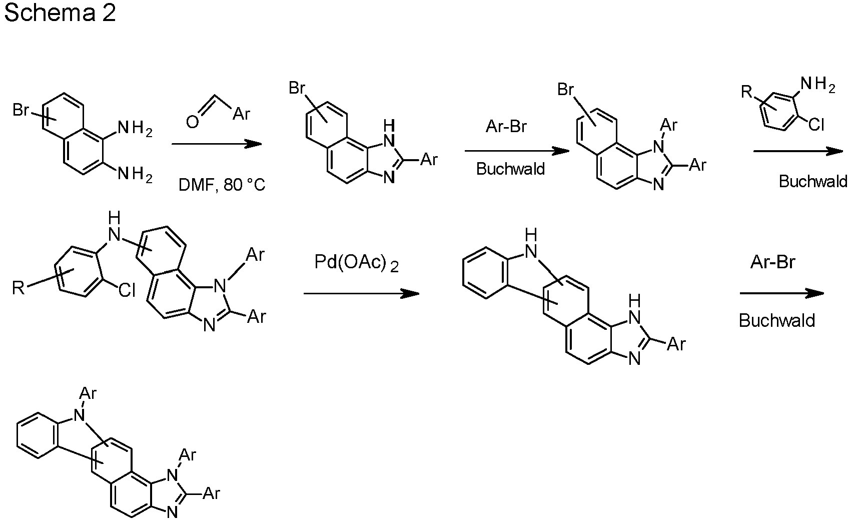

Die Bedeutung der in Schemata 1 und 2 verwendeten Symbole entspricht im Wesentlichen denen, die zuvor definiert wurden, wobei aus Gründen der Übersichtlichkeit auf eine Nummerierung sowie auf eine vollständige Darstellung aller Symbole verzichtet wurde.

Ein weiterer Gegenstand der vorliegenden Erfindung ist daher ein Verfahren zur Herstellung einer erfindungsgemäßen Verbindung, wobei eine aromatische oder heteroaromatische Verbindung mittels einer Kupplungsreaktion mit einer aromatischen oder heteroaromatischen Diaminoverbindung umgesetzt wird.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung nach Anspruch 8 oder nach Anspruch 11. Die weitere Verbindung ist in Anspruch 11 ein Lösemittel, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann prinzipiell eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Die weitere Verbindung kann auch polymer sein.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine erfindungsgemäße Verbindung bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial für phosphoreszierende Emitter, insbesondere für rot, orange, grün oder gelb, bevorzugt rot phosphoreszierende Emitter, in einer emittierenden Schicht oder als Lochtransport- bzw. Elektronenblockiermaterial in einer Lochtransport- bzw. Elektronenblockierschicht eingesetzt, besonders bevorzugt als Matrixmaterial in einer emittierenden Schicht.

Hierbei kann vorzugsweise vorgesehen sein, dass eine Verbindung einen Rest Ar^{a} und/oder Ar^{b} aufweist, der vorzugsweise eine Arylgruppe oder eine Heteroarylgruppe umfasst, wobei die Heteroatome ausgewählt sind aus O oder S, oder die Heteroarylgruppe eine Carbazolgruppe umfasst, vorzugsweise darstellt. In diesen Ausführungsformen weist die Verbindung vorzugsweise lochleitende Eigenschaften auf, wobei diese Verbindung mit lochleitenden Eigenschaften in einer bevorzugten Ausführungform mit einem elektronenleitenden Matrixmaterial kombiniert werden kann.

Ferner kann vorzugsweise vorgesehen sein, dass eine Verbindung einen Rest Ar^{a} und/oder Ar^{b} aufweist, der vorzugsweise eine Heteroarylgruppe mit ein, vorzugsweise zwei Stickstoffatomen umfasst, vorzugsweise darstellt, die vorzugsweise ausgewählt ist aus Pyridin, Pyrimidin, Pyrazin, Pyridazin, Triazin, Chinolin, Chinazolin oder Chinoxalin. Bevorzugte elektronenleitende Gruppen sind unter anderem die zuvor dargelegten Reste Ar-47 bis Ar-68. In diesen Ausführungsformen weist die Verbindung vorzugsweise elektronenleitende Eigenschaften auf, wobei diese Verbindung mit elektronenleitenden Eigenschaften in einer bevorzugten

Ausführungsform mit einem elektronenleitenden lochleitenden Matrixmaterial kombiniert werden kann.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung dabei als einziges Matrixmaterial ("single host") für den phosphoreszierenden Emitter eingesetzt.

Eine weitere Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B.

CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Ebenso kann ein weiterer phosphoreszierender Emitter, welcher kürzerwellig als der eigentliche Emitter emittiert, als Co-Host in der Mischung vorhanden sein. Besonders gute Ergebnisse werden erzielt, wenn als Emitter ein rot phoshoreszierender Emitter eingesetzt wird und als Co-Host in Kombination mit der erfindungsgemäßen Verbindung ein gelb phosphoreszierender Emitter verwendet wird.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-MatrixMaterial Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Besonders bevorzugte Co-Host-Materialien, welche in Kombination mit den erfindungsgemäßen Verbindungen eingesetzt werden können, sind Verbindungen gemäß einer der Formeln (H-1), (H-2), (H-3), (H-4) oder (H-5), 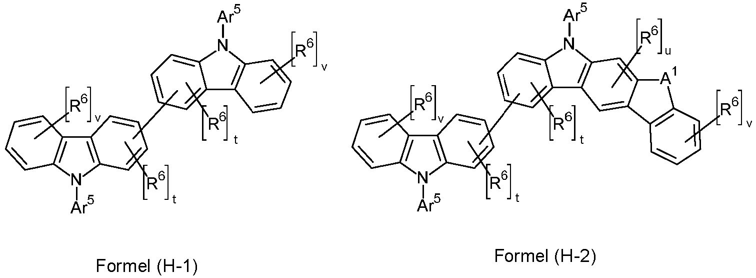 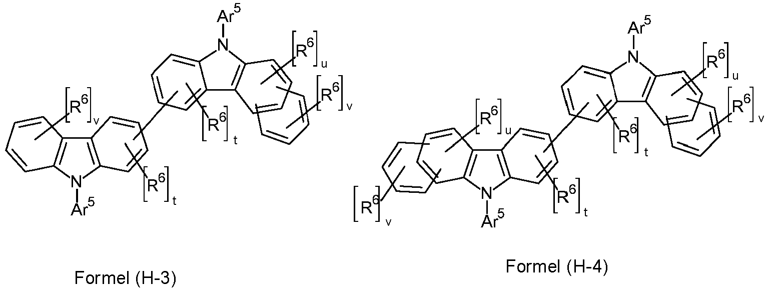 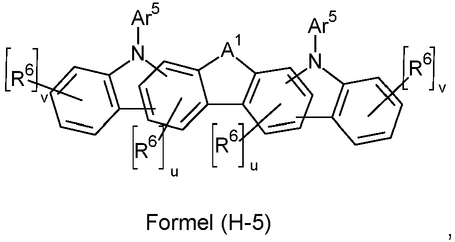 wobei für die verwendeten Symbole und Indizes gilt:
- R⁶: ist bei jedem Auftreten gleich oder verschieden H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁷)₂, C=O, NR⁷, O, S oder CONR⁷ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁷ substituiert sein kann; dabei können zwei Reste R⁶ auch miteinander ein aromatisches, heteroaromatisches, aliphatisches oder heteroaliphatisches Ringsystem bilden, vorzugsweise bilden die Reste R⁶ kein solches Ringsystem;
- Ar": ist bei jedem Auftreten gleich oder verschieden ein aromatisches oder heteroaromatisches Ringsystem mit 5 bis 40 aromatischen Ringatomen, welches mit einem oder mehreren Resten R⁷ substituiert sein kann;
- A¹: ist C(R⁷)₂, NR⁷, O oder S;
- Ar⁵: steht gleich oder verschieden bei jedem Auftreten für ein aromatisches oder heteroaromatisches Ringsystem mit 5 bis 40 aromatischen Ringatomen, welches mit einem oder mehreren Resten R⁷ substituiert sein kann;
- R⁷: ist bei jedem Auftreten gleich oder verschieden H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁸ substituiert sein kann, wobei eine oder mehrere nicht benachbarte CH₂-Gruppen durch Si(R⁸)₂, C=O, NR⁸, O, S oder CONR⁸ ersetzt sein können, oder ein aromatisches oder heteroaromatisches Ringsystem mit 5 bis 40 aromatischen Ringatomen, das jeweils durch einen oder mehrere Reste R⁸ substituiert sein kann; dabei können zwei oder mehrere Reste R⁷ miteinander ein aromatisches, heteroaromatisches, aliphatisches oder heteroaliphatisches Ringsystem bilden, vorzugsweise bilden die Reste R⁷ kein solches Ringsystem;
- R⁸: ist bei jedem Auftreten gleich oder verschieden **H, D,** F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- v: ist bei jedem Auftreten gleich oder verschieden 0, 1, 2, 3 oder 4, vorzugsweise 0 oder 1 und ganz bevorzugt 0;
- t: ist bei jedem Auftreten gleich oder verschieden 0, 1, 2, oder 3, vorzugsweise 0 oder 1 und ganz bevorzugt 0;
- u: ist bei jedem Auftreten gleich oder verschieden 0, 1 oder 2, vorzugsweise 0 oder 1 und ganz bevorzugt 0.

Die Summe der Indices v, t und u in Verbindungen der Formeln (H-1), (H-2), (H-3), (H-4) oder (H-5) beträgt vorzugsweise höchstens 6, insbesondere bevorzugt höchstens 4 und besonders bevorzugt höchstens 2.

In einer bevorzugten Ausführungsform der Erfindung ist R⁶ gleich oder verschieden bei jedem Auftreten ausgewählt aus der Gruppe bestehend aus H, D, F, CN, NO₂, Si(R⁷)₃, B(OR⁷)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁷ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁷ substituiert sein kann.

In einer weiterhin bevorzugten Ausführungsform der Erfindung ist R⁶ gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁷ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁷ substituiert sein kann.

In einer weiterhin bevorzugten Ausführungsform der Erfindung ist R⁶ gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R⁷ substituiert sein kann, oder einer Gruppe N(Ar")₂. Besonders bevorzugt ist R⁶ gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁷ substituiert sein kann.

Bevorzugte aromatische bzw. heteroaromatische Ringsystem R⁶ bzw. Ar" sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁷ substituiert sein können. Besonders bevorzugt sind die oben aufgeführten Strukturen (Ar-1) bis (Ar-75), wobei Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen (Ar-1) bis (Ar-75) sind in Bezug auf die Reste R⁶ und Ar" die Substituenten R⁴ durch die entsprechenden Reste R⁷ zu ersetzen. Die zuvor für die Gruppen R² und R³ dargelegten Bevorzugungen gelten entsprechend für die Gruppe R⁶.

Weiterhin kann vorgesehen sein, dass die Substituenten R⁶ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R⁷, R⁸ ein, die an die Reste R⁶ gebunden sein können.

Wenn A¹ für NR⁷ steht, steht der Substituent R⁷, der an das Stickstoffatom gebunden ist, bevorzugt für ein aromatisches oder heteroaromatisches Ringsystem mit 5 bis 24 aromatischen Ringatomen, welches auch durch einen oder mehrere Reste R⁸ substituiert sein kann. In einer besonders bevorzugten Ausführungsform steht dieser Substituent R⁷ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R⁸ substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R⁴ durch einen oder mehrere Reste R⁸ substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R⁴ durch einen oder mehrere Reste R⁸ substituiert sein können.

Wenn A¹ für C(R⁷)₂ steht, stehen die Substituenten R⁷, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R⁸ substituiert sein kann. Ganz besonders bevorzugt steht R⁷ für eine Methylgruppe oder für eine Phenylgruppe. Dabei können die Reste R⁷ auch miteinander ein Ringsystem bilden, was zu einem Spirosystem führt.

Weiterhin sind bevorzugte Co-Host-Materialien, welche in Kombination mit den erfindungsgemäßen Verbindungen eingesetzt werden können, Verbindungen gemäß einer der Formeln (H 6), (H 7), (H 8), (H 9), (H 10), (H 11), (H-12) oder (H-13), 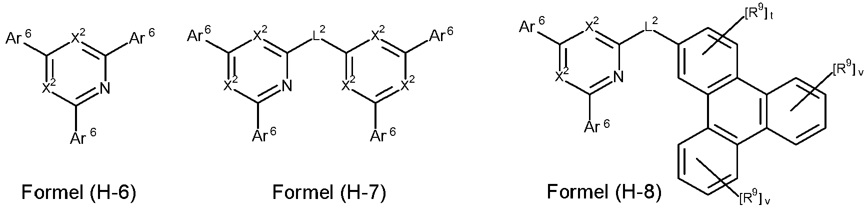 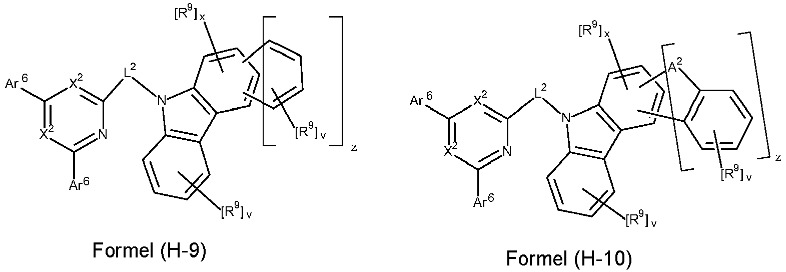 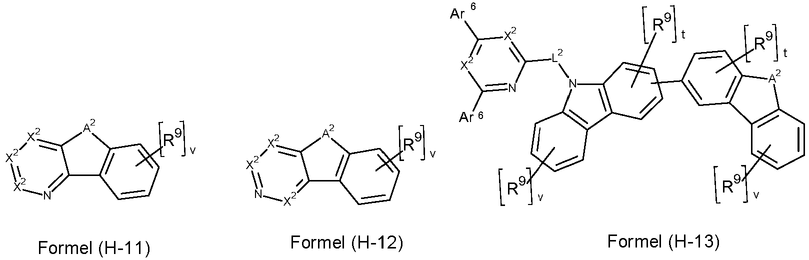 wobei für die verwendeten Symbole und Indizes gilt:
- X²: steht für N oder CR⁹, mit der Maßgabe, dass nicht mehr als zwei der Gruppen X² in einem Cyclus für N stehen, vorzugsweise steht mindestens ein X² für N;
- L²: steht für eine Verbindungsgruppe, die ausgewählt ist aus einer Bindung oder einem aromatischen oder heteroaromatischen Ringsystem mit 5 bis 40 aromatischen Ringatomen, welches mit einem oder mehreren Resten R⁹ substituiert sein kann, besonders bevorzugt eine Bindung;
- A²: ist C(R¹⁰)₂, NR¹⁰, O oder S;
- Ar⁶: steht gleich oder verschieden bei jedem Auftreten für ein aromatisches oder heteroaromatisches Ringsystem mit 5 bis 40 aromatischen Ringatomen, welches mit einem oder mehreren Resten R¹⁰ substituiert sein kann;
- R⁹: ist bei jedem Auftreten gleich oder verschieden H, D, F, Cl, Br, I, N(R¹⁰)₂, N(Ar‴)₂, CN, NO₂, OR¹⁰, SR¹⁰, COOR¹⁰, C(=O)N(R¹⁰)₂, Si(R¹⁰)₃, B(OR¹⁰)₂, C(=O)R¹⁰, P(=O)(R¹⁰)₂, S(=O)R¹⁰, S(=O)₂R¹⁰, OSO₂R¹⁰, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R¹⁰)₂, C=O, NR¹⁰, O, S oder CONR¹⁰ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹⁰ substituiert sein kann; dabei können zwei Reste R⁹ auch miteinander ein aromatisches, heteroaromatisches, aliphatisches oder heteroaliphatisches Ring- system bilden, vorzugsweise bilden die Reste R⁹ kein solches Ringsystem;
- Ar‴: ist bei jedem Auftreten gleich oder verschieden ein aromatisches oder heteroaromatisches Ringsystem mit 5 bis 40 aromatischen Ringatomen, welches mit einem oder mehreren Resten R¹⁰ substituiert sein kann;
- R¹⁰: ist bei jedem Auftreten gleich oder verschieden H, D, F, Cl, Br, I, N(R¹¹)₂, CN, NO₂, OR¹¹, SR¹¹, Si(R¹¹)₃, B(OR¹¹)₂, C(=O)R¹¹, P(=O)(R¹¹)₂, S(=O)R¹¹, S(=O)₂R¹¹, OSO₂R¹¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹¹ substituiert sein kann, wobei eine oder mehrere nicht benachbarte CH₂-Gruppen durch Si(R¹¹)₂, C=O, NR¹¹, O, S oder CONR¹¹ ersetzt sein können, oder ein aromatisches oder heteroaromatisches Ringsystem mit 5 bis 40 aromatischen Ringatomen, das jeweils durch einen oder mehrere Reste R¹¹ substituiert sein kann; dabei können zwei oder mehrere Reste R¹⁰ miteinander ein aromatisches, heteroaromatisches, aliphatisches oder heteroaliphatisches Ringsystem bilden, vorzugsweise bilden die Reste R¹⁰ kein solches Ringsystem;
- R¹¹: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- v: ist bei jedem Auftreten gleich oder verschieden 0, 1, 2, 3 oder 4, vorzugsweise 0 oder 1 und ganz bevorzugt 0;
- t: ist bei jedem Auftreten gleich oder verschieden 0, 1, 2, oder 3, vorzugsweise 0 oder 1 und ganz bevorzugt 0;
- x: ist bei jedem Auftreten gleich oder verschieden 0, 1, 2, 3 oder 4, vorzugsweise 0 oder 1 und ganz bevorzugt 0;
- z: ist bei jedem Auftreten gleich oder verschieden 0, 1 oder 2, vorzugsweise 0 oder 1 und ganz bevorzugt 0, wobei die Summe aus x und z höchstens 4, vorzugsweise höchstens 2 ist.

Die Summe der Indices v, t, x und z in Verbindungen der Formeln (H 6), (H 7), (H 8), (H 9), (H 10), (H 11), (H-12) oder (H-13) beträgt vorzugsweise höchstens 6, insbesondere bevorzugt höchstens 4 und besonders bevorzugt höchstens 2.

Die Gruppe L² steht für eine Verbindungsgruppe, die ausgewählt ist aus einer Bindung oder einem aromatischen oder heteroaromatischen Ringsystem mit 5 bis 40 aromatischen Ringatomen, welches mit einem oder mehreren Resten R⁹ substituiert sein kann, besonders bevorzugt eine Bindung.

Bevorzugt kann die Gruppe L² mit den Gruppen, an die die Gruppe L² gemäß Formeln (H 6), (H 7), (H 8), (H 9), (H 10), (H 11), (H-12) oder (H-13) oder den bevorzugten Ausführungsformen dieser Formel gebunden ist, eine durchgängige Konjugation ausbilden.

In einer weiteren bevorzugten Ausführungsform der Erfindung steht L² für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste R⁹ substituiert sein kann, bevorzugt aber unsubstituiert ist, wobei R⁹ die zuvor, insbesondere für Formeln (H 6), (H 7), (H 8), (H 9), (H 10), (H 11), (H-12) oder (H-13) genannte Bedeutung aufweisen kann. Besonders bevorzugt steht L² für eine Bindung oder ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R⁹ substituiert sein kann, bevorzugt aber unsubstituiert ist, wobei R⁹ die zuvor, insbesondere für Formeln (H 6), (H 7), (H 8), (H 9), (H 10), (H 11), (H-12) oder (H-13) genannte Bedeutung aufweisen kann.

Weiterhin bevorzugt steht das unter anderem in Formeln (H 6), (H 7), (H 8), (H 9), (H 10), (H 11), (H-12) oder (H-13) dargelegte Symbol L² gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (H 6), (H 7), (H 8), (H 9), (H 10), (H 11), (H-12) oder (H-13) dargelegte Gruppe L² ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L² sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R⁹ substituiert sein können, bevorzugt aber unsubstituiert sind.

Ferner kann vorgesehen sein, dass die unter anderem in Formeln (H 6), (H 7), (H 8), (H 9), (H 10), (H 11), (H-12) oder (H-13) dargelegte Gruppe L² höchstens 1 Stickstoffatom, bevorzugt höchstens 2 Heteroatome, insbesondere bevorzugt höchstens ein Heteroatom und besonders bevorzugt kein Heteroatom aufweist.

Weiterhin kann vorgesehen sein, dass die Gruppe L² kein kondensiertes aromatisches oder heteroaromatisches Ringsystem mit den Gruppen bildet, an die die Gruppe L² bindet, wobei dies die Reste R⁹, R¹⁰ oder R¹¹ einschließt, durch die die Gruppe L² oder eine der Gruppen, an die die Gruppe L² bindet, substituiert sein können.

Besonders bevorzugt steht die Gruppe L² für eine Bindung oder für eine Gruppe, die ausgewählt ist aus den Formeln (L¹-1) bis (L¹-17), wie diese zuvor definiert wurden, wobei die Substituenten R⁴ in den Strukturen der Formeln (L¹-1) bis (L¹-17) durch R⁹ zu ersetzen sind.

In einer bevorzugten Ausführungsform der Erfindung ist R⁹ gleich oder verschieden bei jedem Auftreten ausgewählt aus der Gruppe bestehend aus H, D, F, CN, NO₂, Si(R¹⁰)₃, B(OR¹⁰)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹⁰ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹⁰ substituiert sein kann.

In einer weiterhin bevorzugten Ausführungsform der Erfindung ist R⁹ gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹⁰ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹⁰ substituiert sein kann.

In einer weiterhin bevorzugten Ausführungsform der Erfindung ist R⁹ gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹⁰ substituiert sein kann, oder einer Gruppe N(Ar‴)₂. Besonders bevorzugt ist R⁹ gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹⁰ substituiert sein kann.

Bevorzugte aromatische bzw. heteroaromatische Ringsystem R⁹ bzw. Ar‴ sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹⁰ substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen Ar-1 bis Ar-75 sind in Bezug auf die Reste R⁶ und Ar" die Substituenten R⁴ durch die entsprechenden Reste R¹⁰ zu ersetzen. Die zuvor für die Gruppen R² und R³ dargelegten Bevorzugungen gelten entsprechend für die Gruppe R⁹.

Weiterhin kann vorgesehen sein, dass die Substituenten R⁹ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R¹⁰, R¹¹ ein, die an die Reste R⁹ gebunden sein können.

Wenn A² für NR¹⁰ steht, steht der Substituent R¹⁰, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹¹ substituiert sein kann. In einer besonders bevorzugten Ausführungsform steht dieser Substituent R¹⁰ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R¹¹ substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R⁴ durch einen oder mehrere Reste R¹¹ substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R⁴ durch einen oder mehrere Reste R¹¹ substituiert sein können.

Wenn A² für C(R¹⁰)₂ steht, stehen die Substituenten R¹⁰, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹¹ substituiert sein kann. Ganz besonders bevorzugt steht R¹⁰ für eine

Methylgruppe oder für eine Phenylgruppe. Dabei können die Reste R¹⁰ auch miteinander ein Ringsystem bilden, was zu einem Spirosystem führt.

Bevorzugte aromatische bzw. heteroaromatische Ringsysteme Ar⁵ und/oder Ar⁶ sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁷ beziehungsweise R¹⁰ substituiert sein können.

Dabei sind die Gruppen Ar⁵ und/oder Ar⁶ unabhängig voneinander besonders bevorzugt gewählt aus den Gruppen der zuvor dargelegten Formeln Ar-1 bis Ar-75, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen Ar-1 bis Ar-75 sind in Bezug auf die Reste Ar⁵ die Substituenten R⁴ durch die entsprechenden Reste R⁷ beziehungsweise R¹⁰ zu ersetzen.

In einer weiteren bevorzugten Ausführungsform der Erfindung ist R⁷ und/oder R¹⁰ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R⁸ beziehungsweise R¹¹ substituiert sein kann, oder einem aromatischen oder heteroaromatischen Ringsystem mit 6 bis 24 aromatischen Ringatomen, das jeweils durch einen oder mehrere Reste R⁸ beziehungsweise R¹¹ substituiert sein kann. In einer besonders bevorzugten Ausführungsform der Erfindung ist R⁷ und/oder R¹⁰ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁸ beziehungsweise R¹¹ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁸ beziehungsweise R¹¹ substituiert sein kann, bevorzugt aber unsubstituiert ist.

In einer weiteren bevorzugten Ausführungsform der Erfindung ist R⁸ und/oder R¹¹ gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Bevorzugte Ausführungsformen der Verbindungen der Formeln (H-1) bzw. (H-2) sind die Verbindungen der folgenden Formeln (H-1a) bzw. (H-2a), 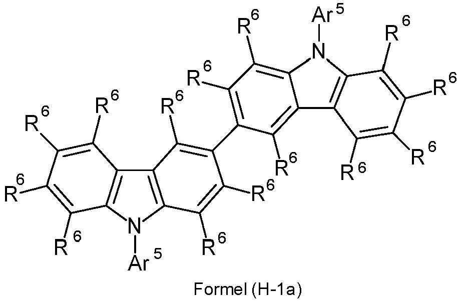 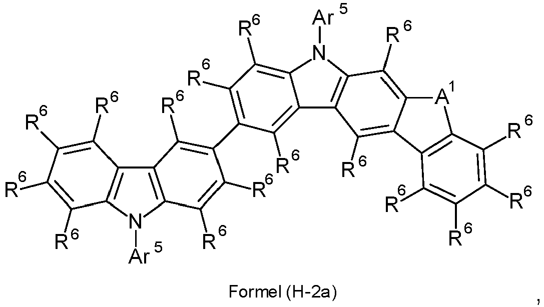 wobei R⁶, Ar⁵ und A¹ die zuvor, insbesondere für Formel (H-1) oder (H-2) genannten Bedeutungen aufweisen. In einer bevorzugten Ausführungsform der Erfindung steht A¹ in Formel (H-2a) für C(R⁷)₂.

Bevorzugte Ausführungsformen der Verbindungen der Formeln (H-1a) bzw. (H-2a) sind die Verbindungen der folgenden Formeln (H-1b) bzw. (H-2b), 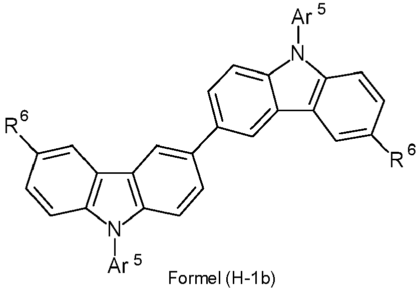 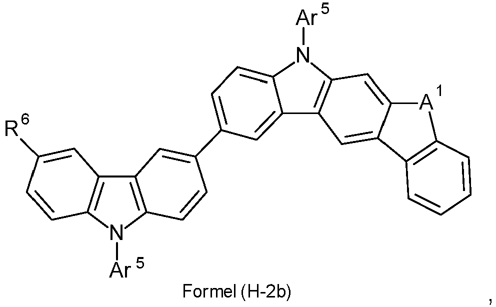 wobei R⁶, Ar⁵ und A¹ die zuvor, insbesondere für Formel (H-1) oder (H-2) genannten Bedeutungen aufweisen. In einer bevorzugten Ausführungsform der Erfindung steht A¹ in Formel (H-2b) für C(R⁷)₂.

Beispiele für geeignete Verbindungen gemäß Formel (H-1), (H-2), (H-3), (H-4) oder (H-5) sind die nachfolgend abgebildeten Verbindungen.

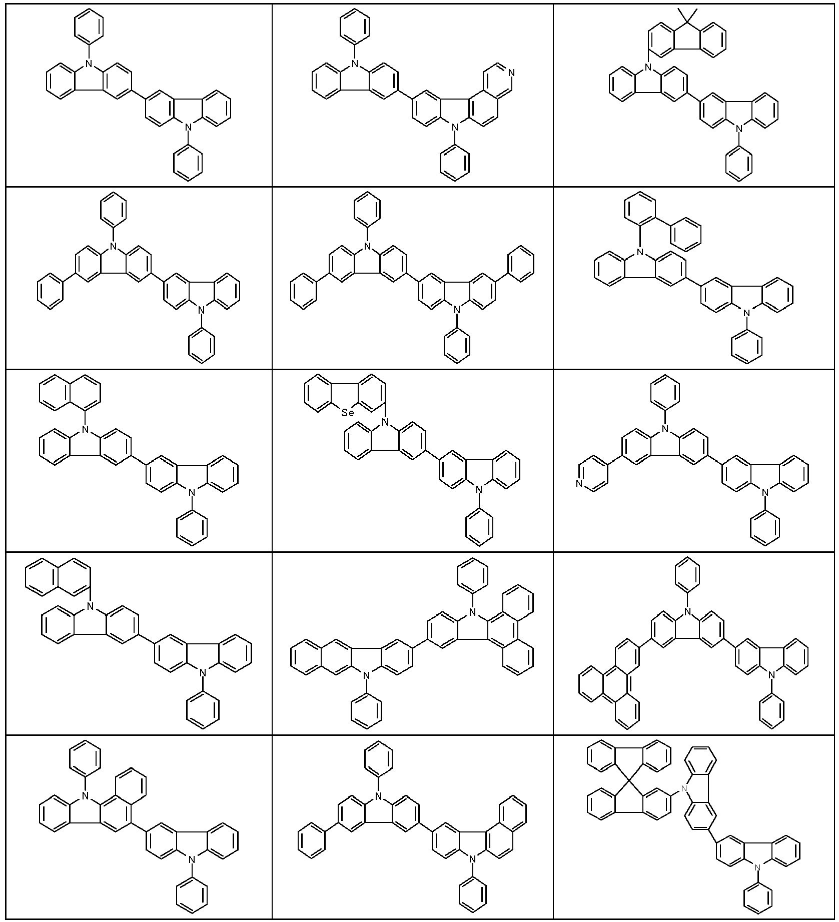

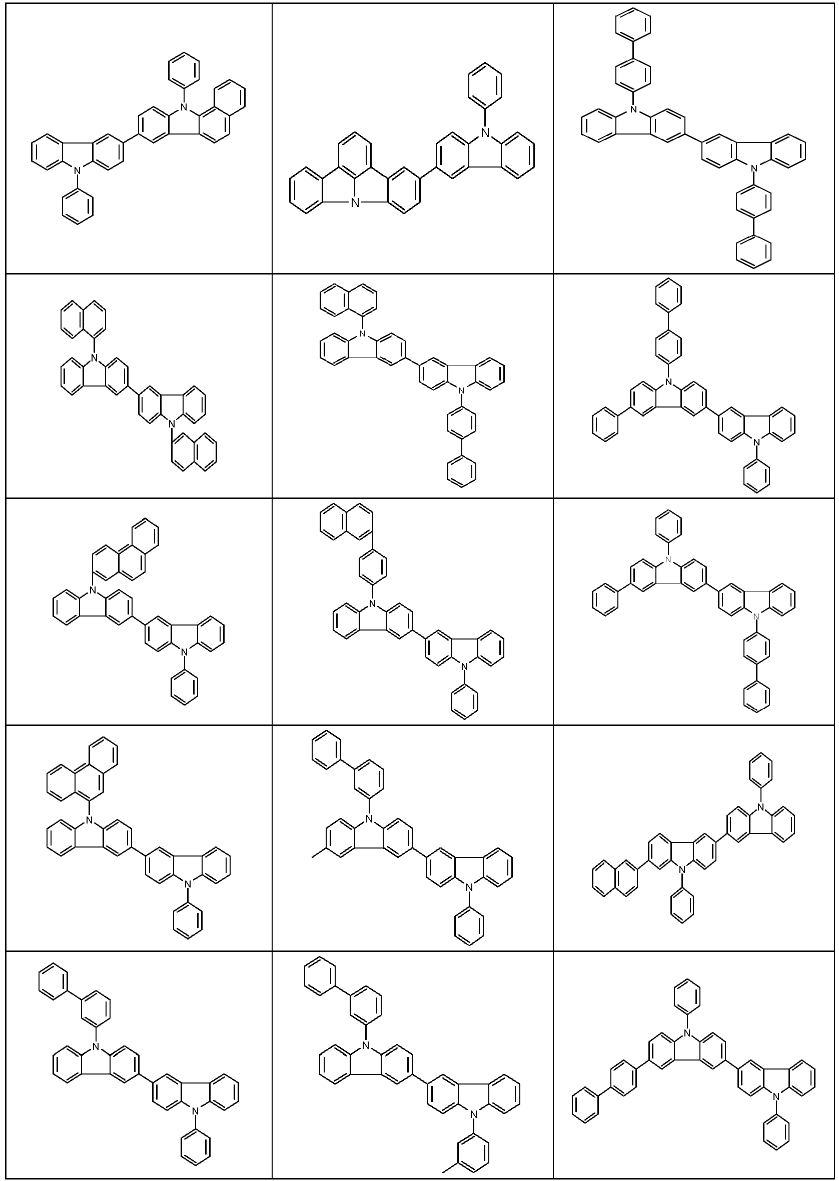

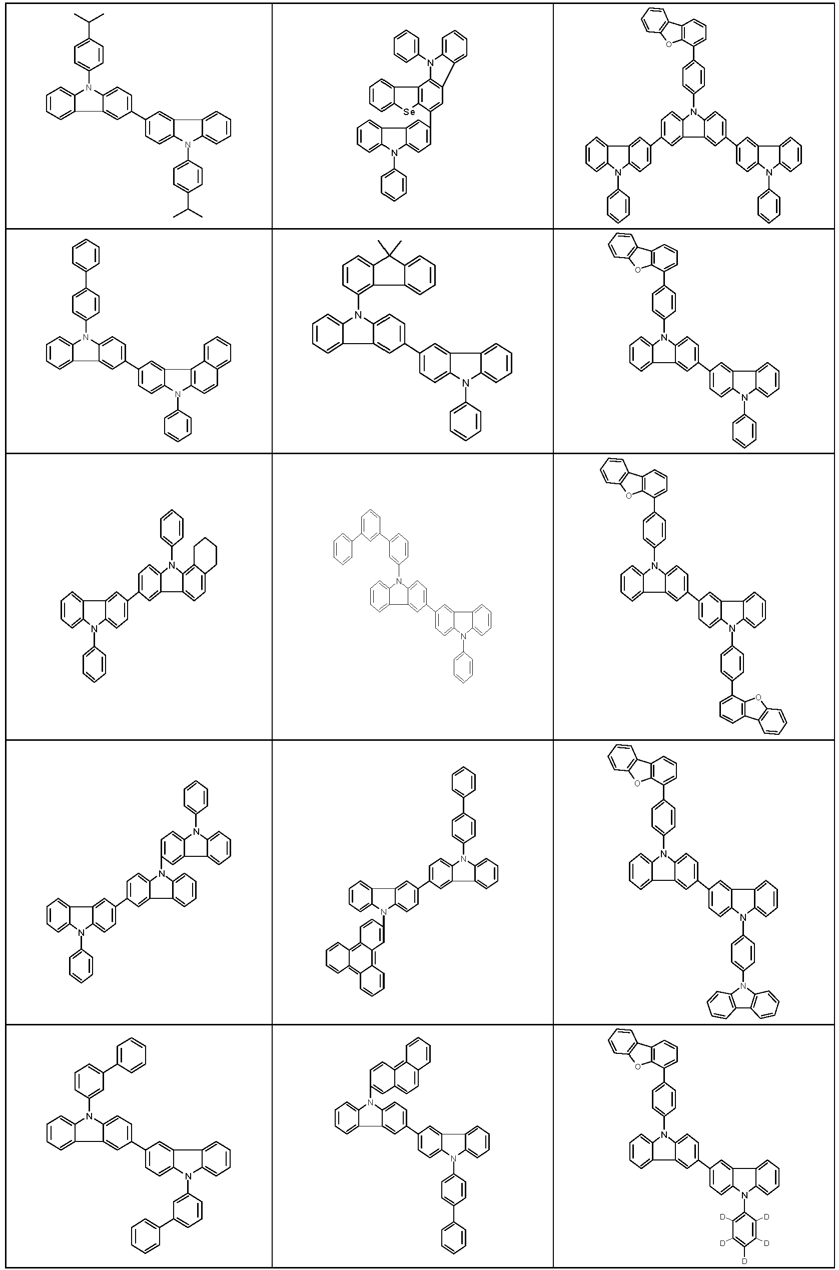

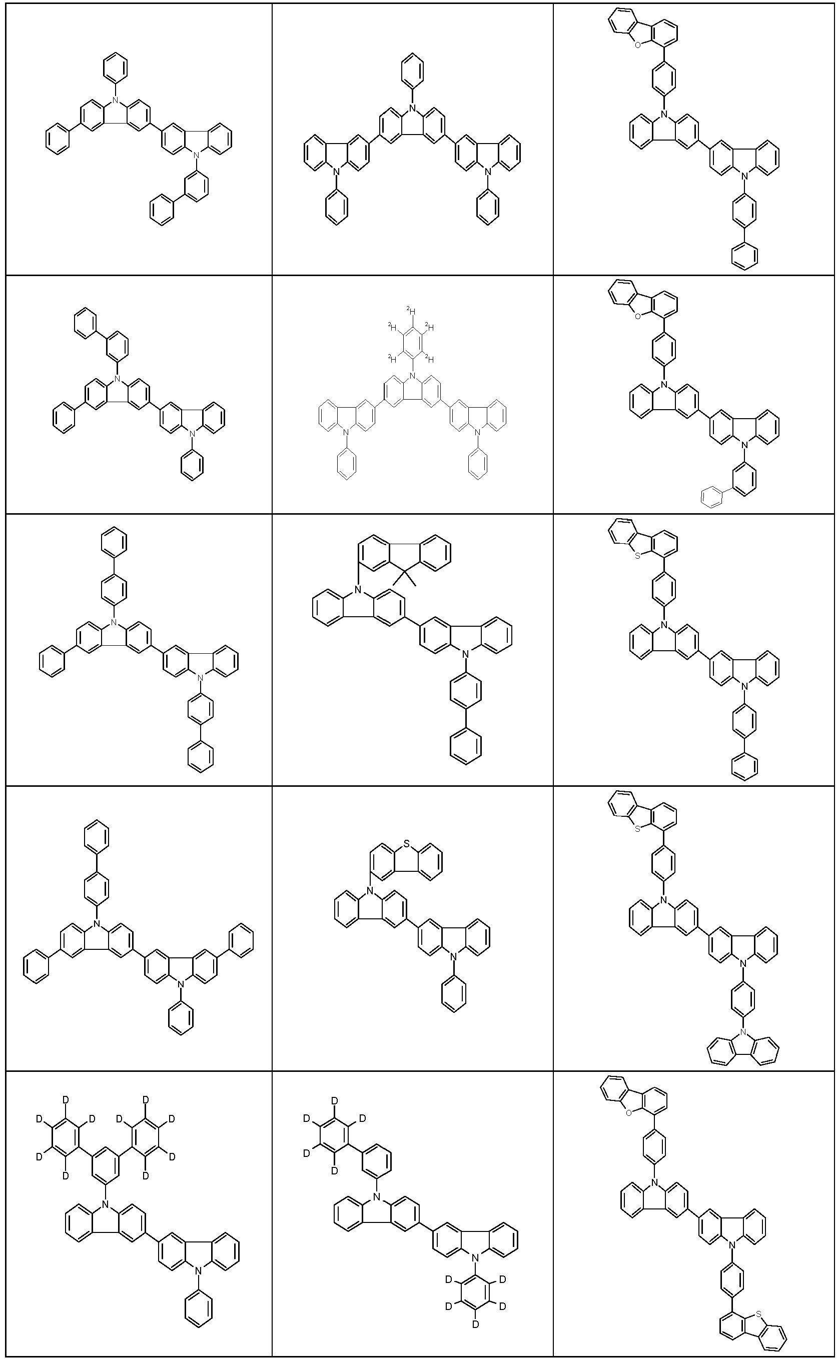

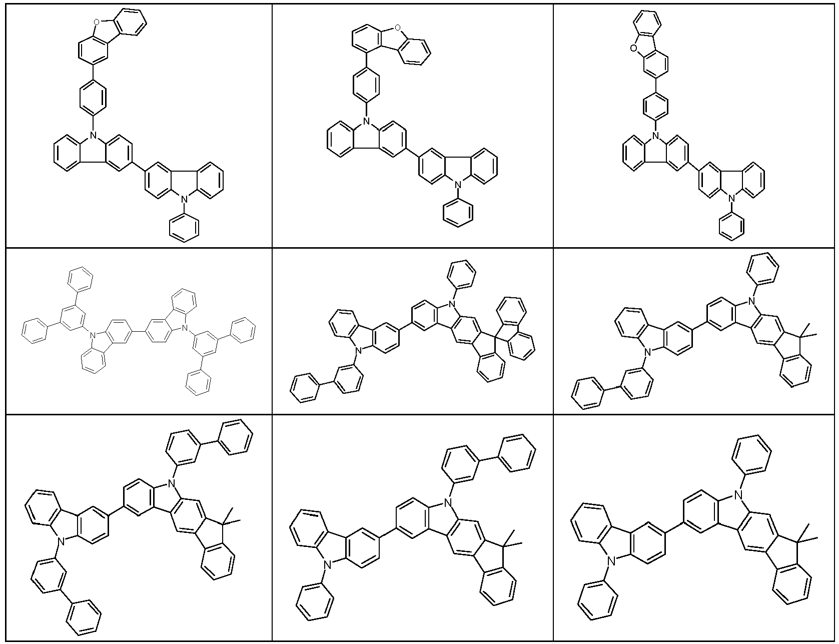

Durch die Kombination mindestens einer erfindungsgemäßen Verbindung oder deren zuvor dargelegten bevorzugten Ausführungsformen mit einer Verbindung gemäß einer der Formeln (H-1), (H-2), (H-3), (H-4) oder (H-5) können überraschende Vorteile erzielt werden. Ein weiterer Gegenstand der vorliegenden Erfindung ist daher eine Zusammensetzung, enthaltend mindestens eine erfindungsgemäße Verbindung oder deren zuvor dargelegten bevorzugten Ausführungsformen und mindestens ein weiteres Matrixmaterial, wobei das weitere Matrixmaterial ausgewählt ist aus Verbindungen gemäß einer der Formeln (H-1), (H-2), (H-3), (H-4) oder (H-5).

Vorzugsweise kann vorgesehen sein, dass die Zusammensetzung aus mindestens einer erfindungsgemäßen Verbindung oder deren zuvor dargelegten bevorzugten Ausführungsformen und mindestens einer Verbindung gemäß einer der Formeln (H-1), (H-2), (H-3), (H-4) oder (H-5) besteht. Diese Zusammensetzungen eignen sich insbesondere als sogenannte Premix-Mischungen, die gemeinsam verdampft werden können.

Durch die Kombination mindestens einer erfindungsgemäßen Verbindung oder deren zuvor dargelegten bevorzugten Ausführungsformen mit einer Verbindung gemäß einer der Formeln (H 6), (H 7), (H 8), (H 9), (H 10), (H 11), (H-12) oder (H-13) können überraschende Vorteile erzielt werden. Ein weiterer Gegenstand der vorliegenden Erfindung ist daher eine Zusammensetzung, enthaltend mindestens eine erfindungsgemäße Verbindung oder deren zuvor dargelegten bevorzugten Ausführungsformen und mindestens ein weiteres Matrixmaterial, wobei das weitere Matrixmaterial ausgewählt ist aus Verbindungen gemäß einer der Formeln (H 6), (H 7), (H 8), (H 9), (H 10), (H 11), (H-12) oder (H-13).

Vorzugsweise kann vorgesehen sein, dass die Zusammensetzung aus mindestens einer erfindungsgemäßen Verbindung oder deren zuvor dargelegten bevorzugten Ausführungsformen und mindestens einer Verbindung gemäß einer der Formeln (H 6), (H 7), (H 8), (H 9), (H 10), (H 11), (H-12) oder (H-13) besteht. Diese Zusammensetzungen eignen sich insbesondere als sogenannte Premix-Mischungen, die gemeinsam verdampft werden können.

Weiterhin kann bevorzugt vorgesehen sein, dass die Zusammensetzung aus mindestens einer erfindungsgemäßen Verbindung oder deren zuvor dargelegten bevorzugten Ausführungsformen und mindestens einer Verbindung gemäß einer der Formeln (H-1), (H-2), (H-3), (H-4), (H-5), (H 6), (H 7), (H 8), (H 9), (H 10), (H 11), (H-12) oder (H-13) besteht. Diese Zusammensetzungen eignen sich insbesondere als sogenannte Premix-Mischungen, die gemeinsam verdampft werden können.

Hierbei können die Verbindungen gemäß einer der Formeln (H-1), (H-2), (H-3), (H-4), (H-5), (H 6), (H 7), (H 8), (H 9), (H 10), (H 11), (H-12) oder (H-13) jeweils einzeln oder als Mischung von zwei, drei oder mehr Verbindungen der jeweiligen Strukturen eingesetzt werden.

Weiterhin können die Verbindungen gemäß der Formeln (H-1), (H-2), (H-3), (H-4), (H-5), (H 6), (H 7), (H 8), (H 9), (H 10), (H 11), (H-12) oder (H-13) einzeln oder als Mischung von zwei, drei oder mehr Verbindungen unterschiedlicher Strukturen eingesetzt werden.

Ferner kann vorgesehen sein, dass eine Zusammensetzung mindestens eine Verbindung gemäß der Formeln (H-1), (H-2), (H-3), (H-4), (H-5), (H 6), (H 7), (H 8), (H 9), (H 10), (H 11), (H-12) oder (H-13) und mindestens eine Verbindung der Formeln (I'-131) bis (I'-142) umfasst, 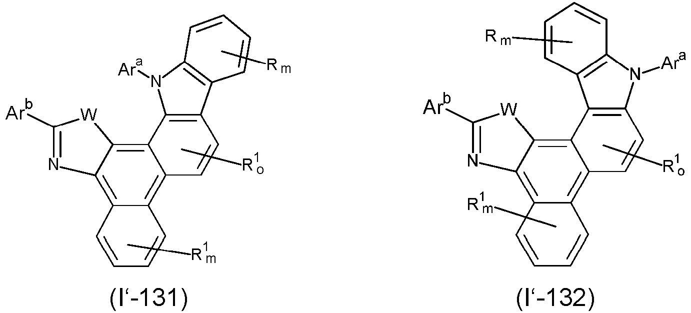 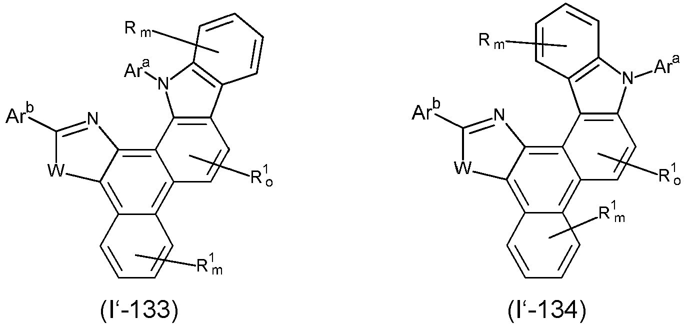 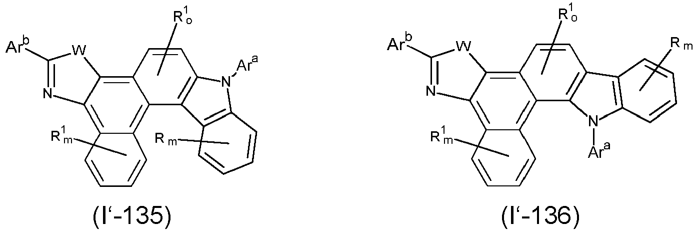 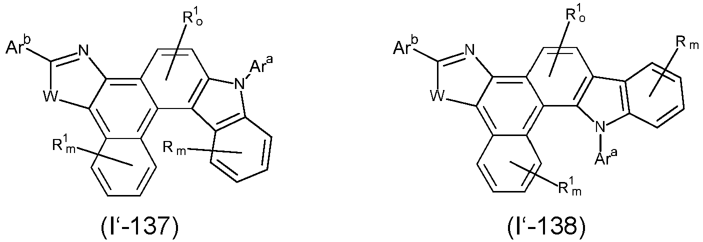 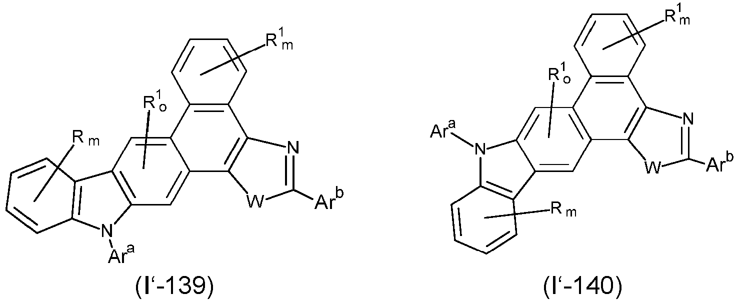 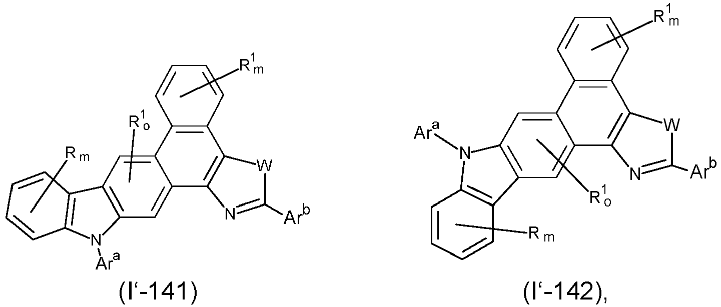 wobei R, R¹, W, Ar^{a} und Ar^{b} die zuvor genannten Bedeutungen aufweisen, der Index o 0, 1 oder 2, vorzugsweise 0 oder 1 ist, der Index m 0, 1, 2, 3 oder 4, vorzugsweise 0, 1 oder 2 ist.

Ferner kann vorgesehen sein, dass eine Zusammensetzung mindestens eine Verbindung gemäß der Formeln (H-1), (H-2), (H-3), (H-4), (H-5), (H 6), (H 7), (H 8), (H 9), (H 10), (H 11), (H-12) oder (H-13) und mindestens eine Verbindung der Formeln (I'-119) bis (I'-130) umfasst, 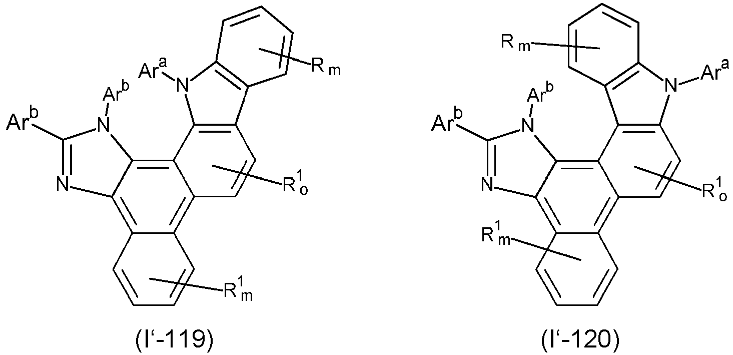 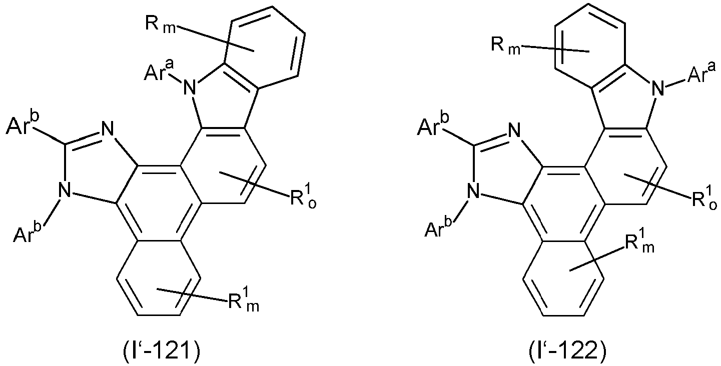 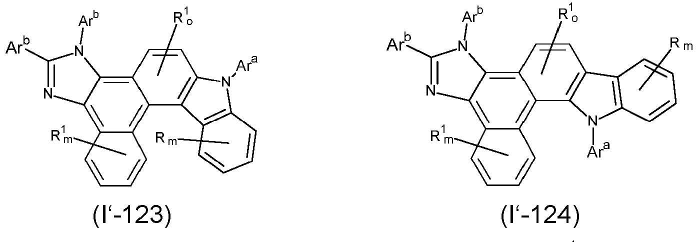 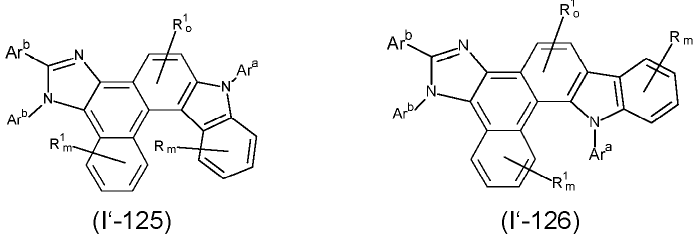 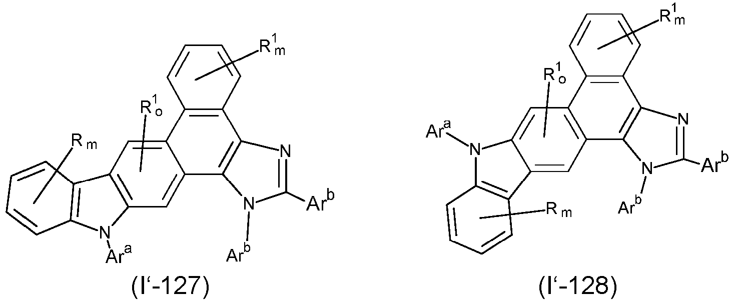 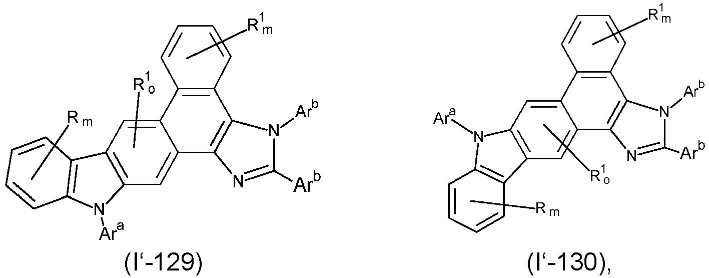 wobei R**,** R¹, Ar^{a} und Ar^{b} die zuvor genannten Bedeutungen aufweisen, der Index o 0, 1 oder 2, vorzugsweise 0 oder 1 ist, der Index m 0, 1, 2, 3 oder 4, vorzugsweise 0, 1 oder 2 ist.

Die erfindungsgemäße Verbindung weist in der Zusammensetzung vorzugsweise einen Massenanteil im Bereich von 10 Gew.-% bis 95 Gew.-%, bevorzugt im Bereich von 15 Gew.-% bis 90 Gew.-%, und ganz bevorzugt im Bereich von 40 Gew.-% bis 70 Gew.-% auf, bezogen auf die Gesamtmasse der Zusammensetzung.

Ferner kann vorgesehen sein, dass die Verbindungen gemäß einer der Formeln (H-1), (H-2), (H-3), (H-4), (H-5), (H 6), (H 7), (H 8), (H 9), (H 10), (H 11), (H-12) oder (H-13) in der Zusammensetzung einen Massenanteil im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-% aufweist, bezogen auf die gesamte Zusammensetzung.

Weiterhin kann vorgesehen sein, dass das weitere Matrixmaterial ein lochtransportierendes Matrixmaterial nach mindestens einer der Formeln (H-1), (H-2), (H-3), (H-4) oder (H-5) darstellt und das lochtransportierende Matrixmaterial einen Massenanteil im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-% aufweist, bezogen auf die gesamte Zusammensetzung.

Weiterhin kann vorgesehen sein, dass das weitere Matrixmaterial ein elektronentransportierendes Matrixmaterial nach mindestens einer der Formeln (H 6), (H 7), (H 8), (H 9), (H 10), (H 11), (H-12) oder (H-13) darstellt und das elektronentransportierende Matrixmaterial einen Massenanteil im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-% aufweist, bezogen auf die gesamte Zusammensetzung.

Darüber hinaus kann vorgesehen sein, dass die Zusammensetzung ausschließlich aus der Formel (I) oder deren zuvor dargelegten bevorzugten Ausführungsformen und einem der genannten weiteren Matrixmaterialien, vorzugsweise Verbindungen nach mindestens einer der Formeln (H-1), (H-2), (H-3), (H-4) oder (H-5) besteht.

Ferner kann vorzugsweise vorgesehen sein, dass eine erfindungsgemäße Verbindung einen Rest Ar^{a} und/oder Ar^{b} aufweist, der vorzugsweise eine Heteroarylgruppe mit ein, vorzugsweise zwei Stickstoffatomen umfasst, vorzugsweise darstellt, die vorzugsweise ausgewählt ist aus Pyridin, Pyrimidin, Pyrazin, Pyridazin, Triazin, Chinolin, Chinazolin oder Chinoxalin. In diesen Ausführungsformen weist die Verbindung vorzugsweise elektronenleitende Eigenschaften auf, wobei diese Verbindung mit elektronenleitenden Eigenschaften in einer bevorzugten Ausführungform mit einem elektronenleitenden lochleitenden Matrixmaterial, vorzugsweise einer Verbindung gemäß Formeln (H-1), (H-2), (H-3), (H-4) oder (H-5), kombiniert werden kann.

Weiterhin kann vorgesehen sein, dass die Zusammensetzung ausschließlich aus der erfindungsgemäßen Verbindung oder deren zuvor dargelegten bevorzugten Ausführungsformen und einem der genannten weiteren Matrixmaterialien, vorzugsweise Verbindungen nach mindestens einer der Formeln (H 6), (H 7), (H 8), (H 9), (H 10), (H 11), (H-12) oder (H-13) besteht.

Ferner kann vorgesehen sein, dass die Zusammensetzung ausschließlich aus der erfindungsgemäßen Verbindung oder deren zuvor dargelegten bevorzugten Ausführungsformen und einem der genannten weiteren Matrixmaterialien, vorzugsweise Verbindungen nach mindestens einer der Formeln (H-1), (H-2), (H-3), (H-4), (H-5), (H 6), (H 7), (H 8), (H 9), (H 10), (H 11), (H-12) oder (H-13) besteht.

Hierbei kann vorzugsweise vorgesehen sein, dass eine erfindungsgemäße Verbindung einen Rest Ar^{a} und/oder Ar^{b} aufweist, der vorzugsweise eine Arylgruppe oder eine Heteroarylgruppe umfasst, wobei die Heteroatome ausgewählt sind aus O oder S, oder die Heteroarylgruppe eine Carbazolgruppe umfasst, vorzugsweise darstellt. In diesen Ausführungsformen weist die Verbindung vorzugsweise lochleitende Eigenschaften auf, wobei diese Verbindung mit lochleitenden Eigenschaften in einer bevorzugten Ausführungsform mit einem elektronenleitenden Matrixmaterial, vorzugsweise einer Verbindung gemäß Formeln (H 6), (H 7), (H 8), (H 9), (H 10), (H 11), (H-12) oder (H-13) kombiniert werden kann.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und WO 2018/011186 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind in der folgenden Tabelle aufgeführt.

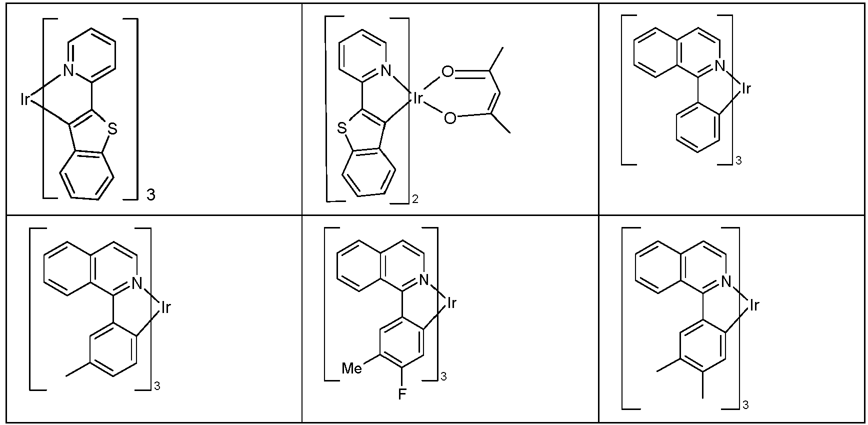

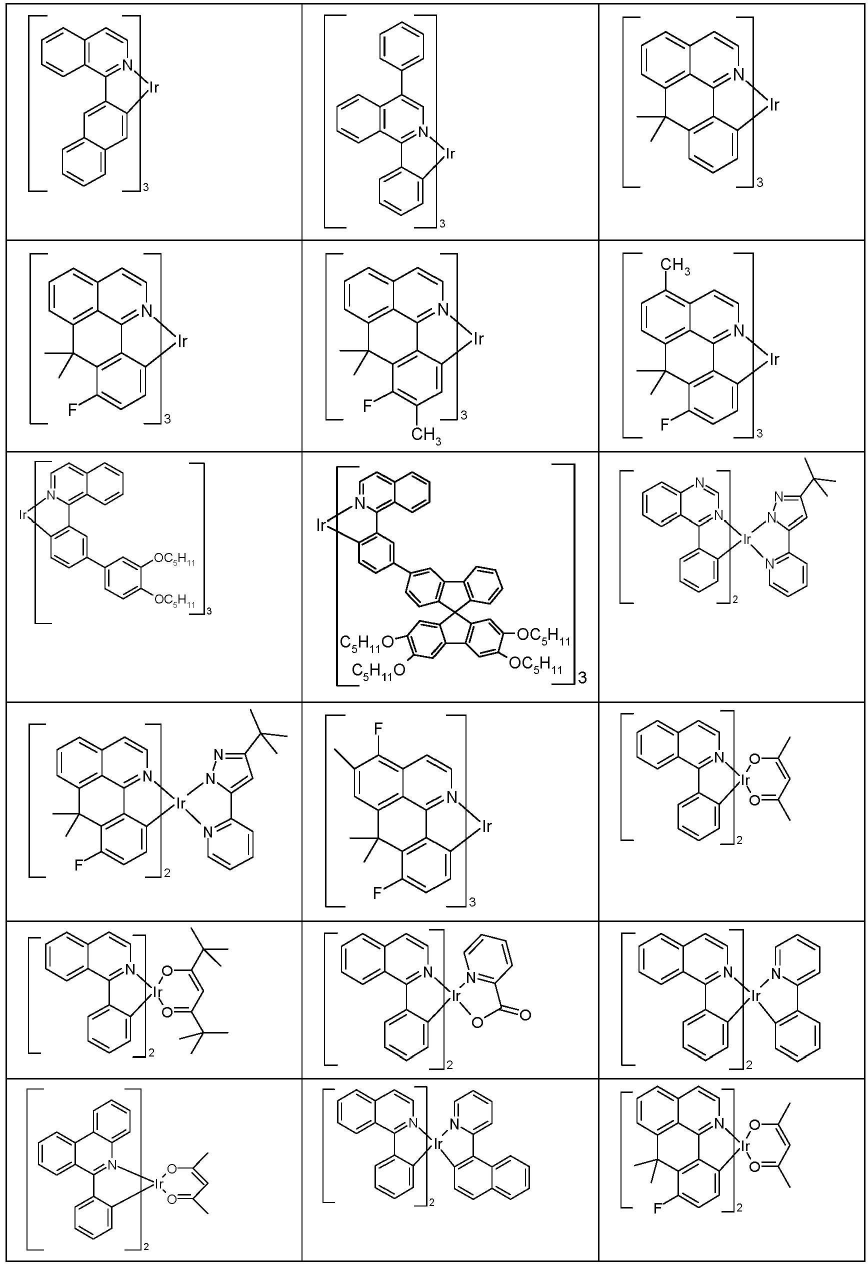

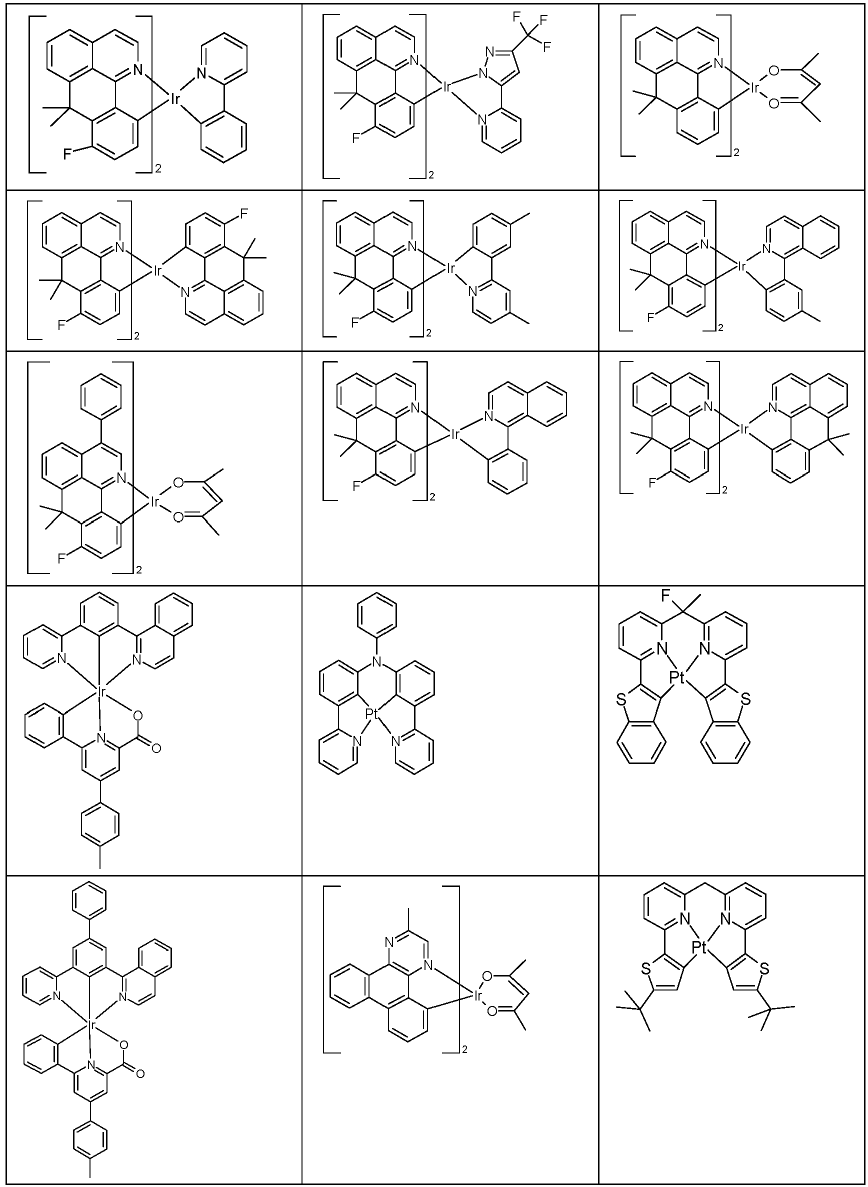

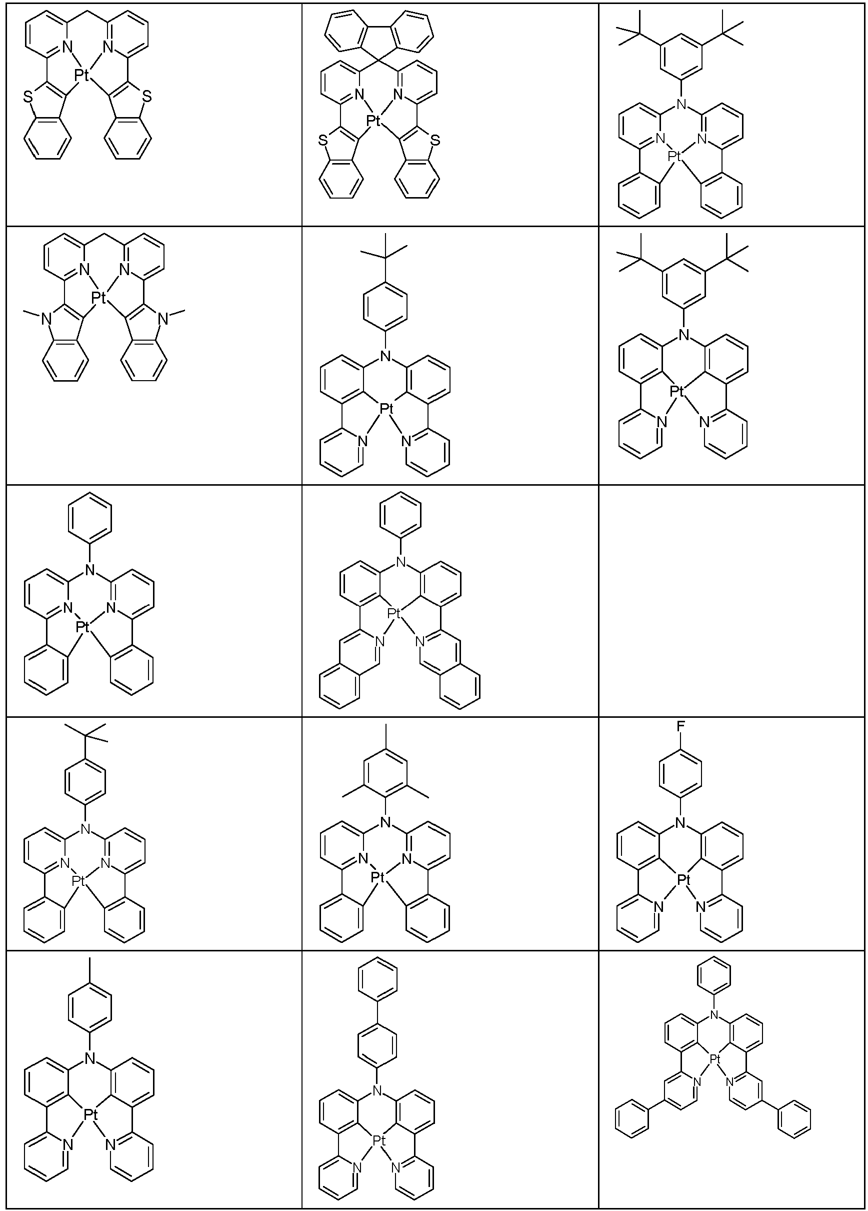

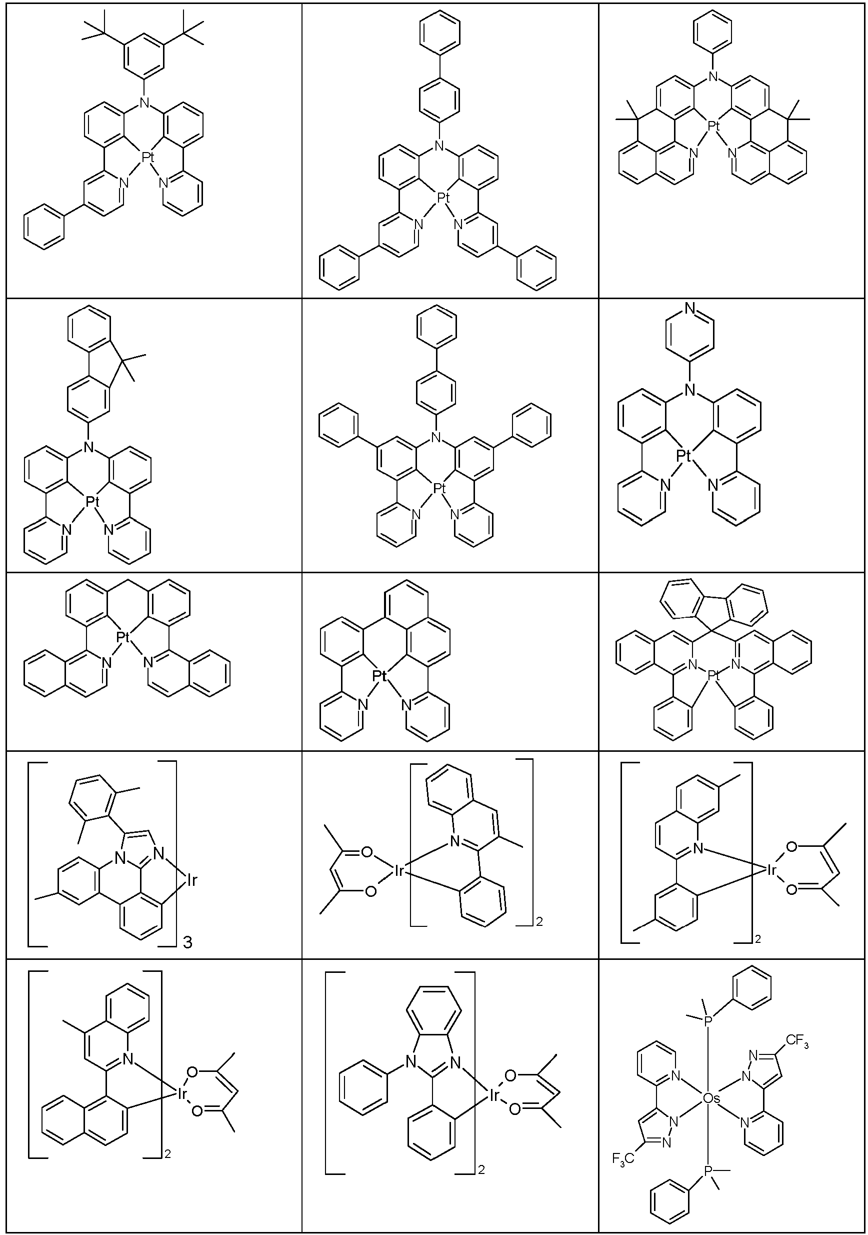

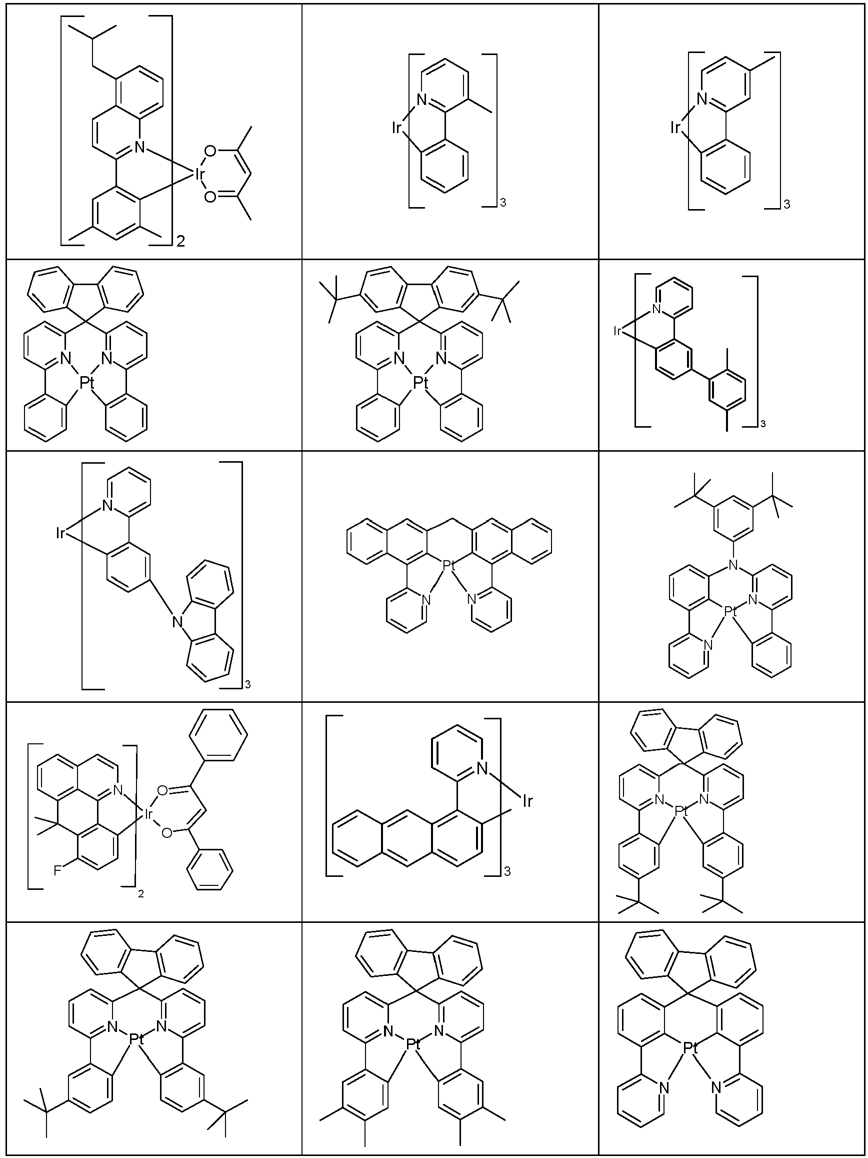

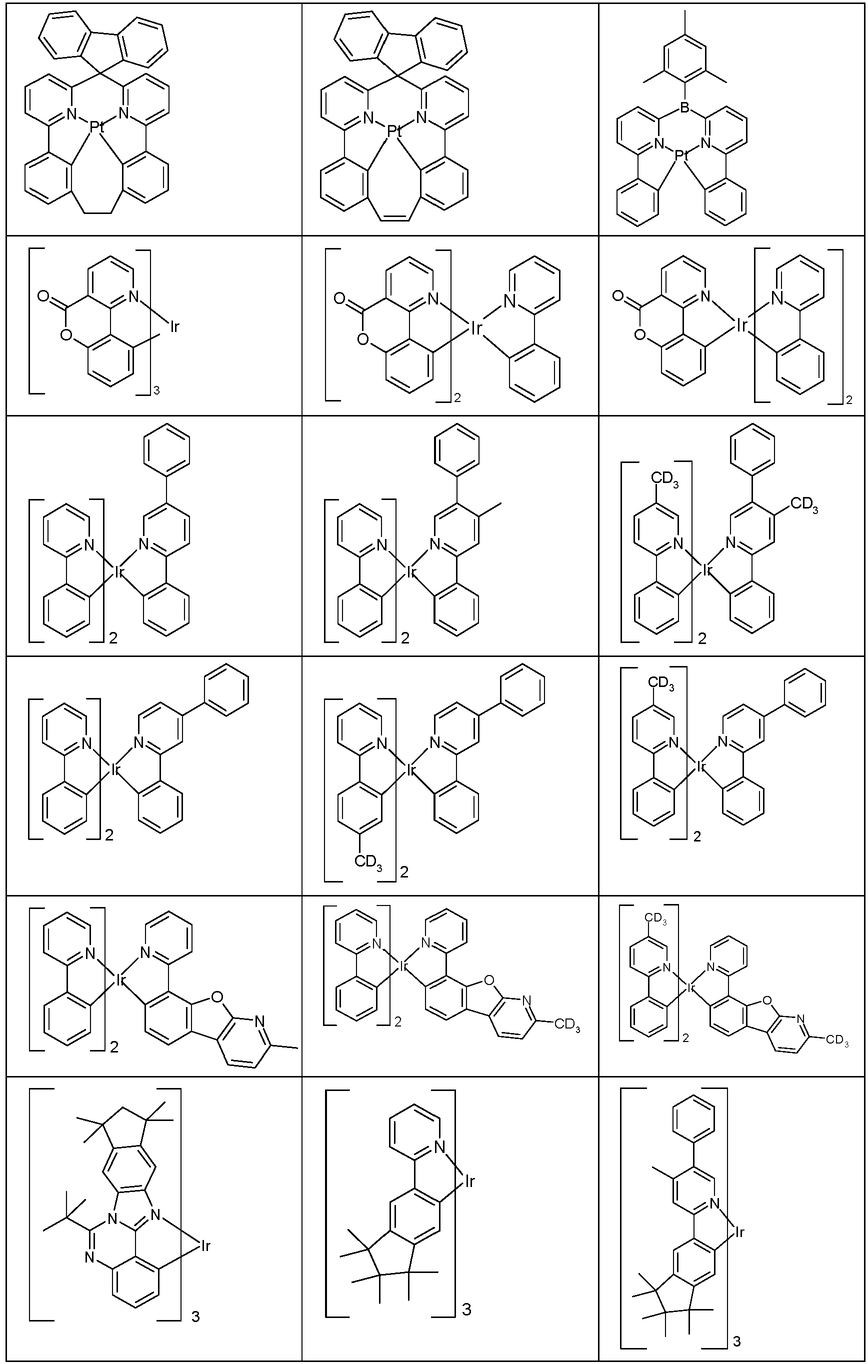

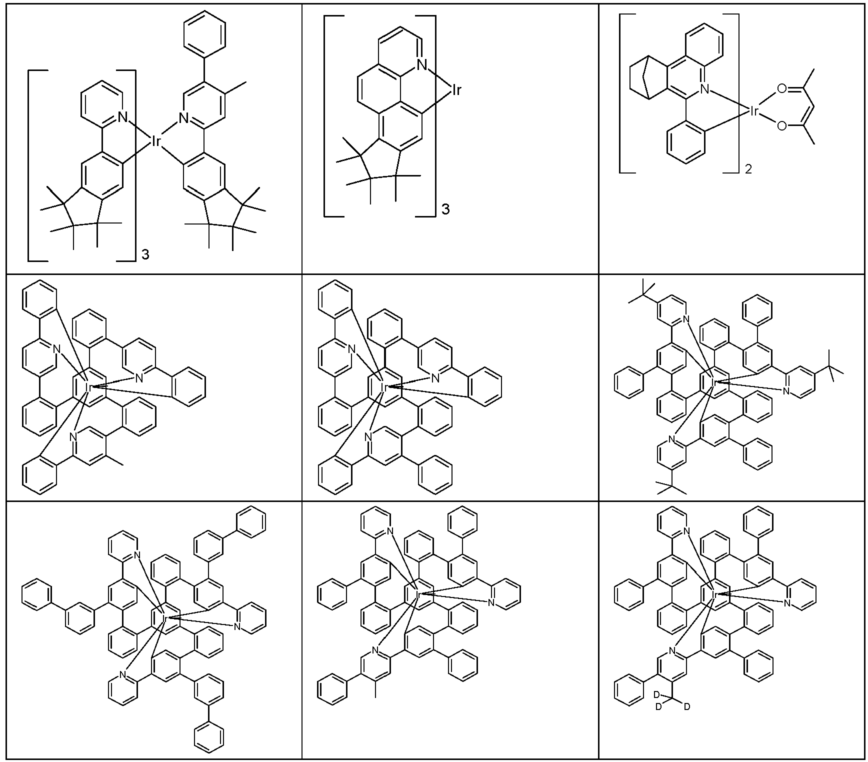

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer erfindungsgemäßen Verbindung oder deren zuvor dargelegten bevorzugten Ausführungsformen sind neu. Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind generell bekannt.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend erfindungsgemäße Verbindungen bzw. die zuvor und nachfolgend ausgeführten bevorzugten Ausführungsformen, insbesondere als Matrixmaterial oder als lochleitende beziehungsweise elektronenleitende Materialien, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
2. Elektronische Vorrichtungen, insbesondere organische Elektrolumineszenzvorrichtungen enthaltend erfindungsgemäße Verbindungen bzw. die zuvor und nachfolgend ausgeführten bevorzugten Ausführungsformen als lochleitende beziehungsweise elektronenleitende Materialien, und/oder Matrixmaterialien weisen eine hervorragende Effizienz auf. Hierbei bewirken erfindungsgemäße Verbindungen bzw. die zuvor und nachfolgend ausgeführten bevorzugten Ausführungsformen eine geringe Betriebsspannung bei Verwendung in elektronischen Vorrichtungen.
3. Die erfindungsgemäßen Verbindungen bzw. die zuvor und nachfolgend ausgeführten bevorzugten Ausführungsformen zeigen eine sehr hohe Stabilität und Lebensdauer.
4. Mit erfindungsgemäßen Verbindungen bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanälen vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
5. Erfindungsgemäße Verbindungen bzw. die zuvor und nachfolgend ausgeführten bevorzugten Ausführungsformen weisen eine ausgezeichnete Filmbildung auf.
6. Erfindungsgemäße Verbindungen bzw. die zuvor und nachfolgend ausgeführten bevorzugten Ausführungsformen bilden aus Lösungen sehr gute Filme.
7. Die erfindungsgemäßen Verbindungen bzw. die zuvor und nachfolgend ausgeführten bevorzugten Ausführungsformen weisen ein tiefes Triplett-Niveau T₁ auf, welches beispielsweise im Bereich von
   -2,22 eV bis -2.9 eV liegen kann.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Synthesebeispiele

### a) 8-Brom-1H-Triphenyleno[1,2-d]imidazole

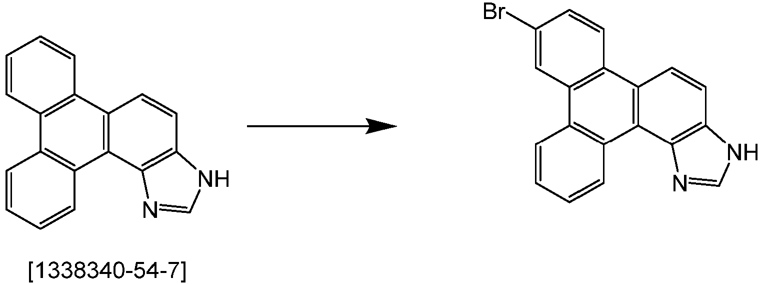

5,9 g (22 mmol) 1*H*-Triphenyleno[1,2-d]imidazol werden in 160 mL DMF vorgelegt. Anschließend tropft man unter Lichtausschluss bei Raumtemperatur eine Lösung aus 4 g (22.5 mmol) NBS in 100 ml DMF zu, lässt auf Raumtemperatur kommen und rührt 10 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 4,5 g (13,2 mmol), 60 % d. Th., Reinheit nach ¹H-NMR ca. 98 %.

Analog dazu werden die folgenden Verbindungen hergestellt:

| | **Edukt 1** | **Produkt** | **Ausbeute** |
|---|---|---|---|
| **1a** | 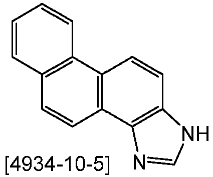 | 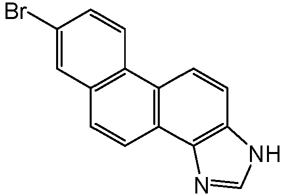 | 57% |
| **2a** | 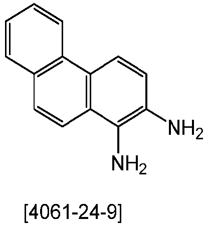 | 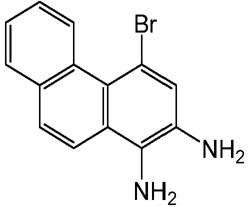 | 89% |
| **3a** | 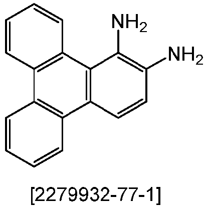 | 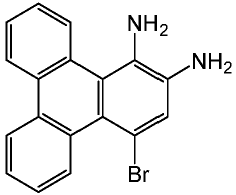 | 91% |

### b) 7-Bromo-2-phenyl-1H-benzo[e]benzimidazol

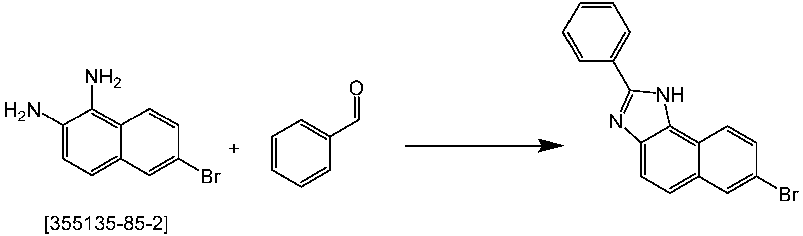

31,9 g (136 mmol) 6-Bromonaphthalene-1,2-diamin und14,4 g (136 mmol) Benzaldehyd werden in 400 ml trockene DMF gelöst und dazu gibt man bei Raumtemperatur portionsweise und 49 g (80 mmol) Kaliumhydrogenmonophosphat und rührt weiter 2 Stunden. Danach wird die Mischung mit 300 ml Wasser versetzt und die organische Phase abgetrennt, über Kieselgel filtriert und anschließend zur Trocken eingeengt. Das Produkt wird via Säulenchromatographie an Kieselgel mit Toluol/Heptan (1:2) gereinigt.

Die Ausbeute beträgt 40g (123 mmol), entsprechend 91 % der Theorie.

Analog dazu werden die folgenden Verbindungen hergestellt:

| | **Edukt 1** | **Edukt 2** | **Produkt** | **Ausbeute** |
|---|---|---|---|---|
| **1b** | 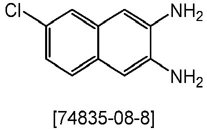 | 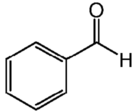 | 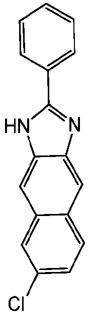 | 89% |
| **2b** | 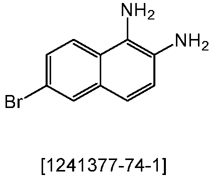 | 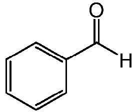 | 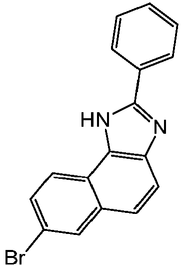 | 92% |
| **3b** | 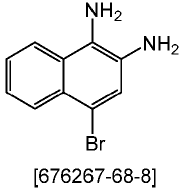 | 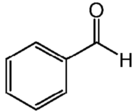 | 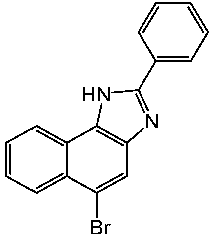 | 94% |
| **4b** | 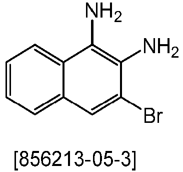 | 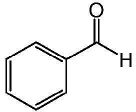 | 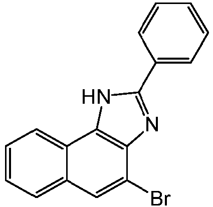 | 87% |
| **5b** | 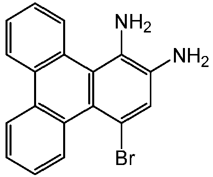 | 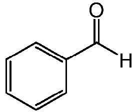 | 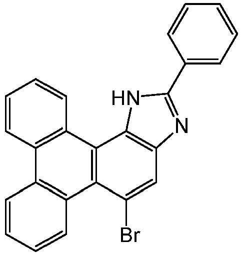 | 92% |
| **6b** | 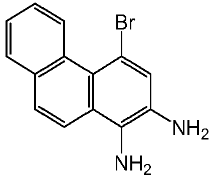 | 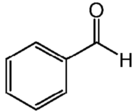 | 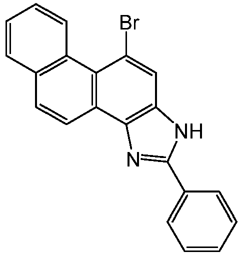 | 90% |

### c) 7-Bromo-1,2-diphenyl-benzo[e]benzimidazol

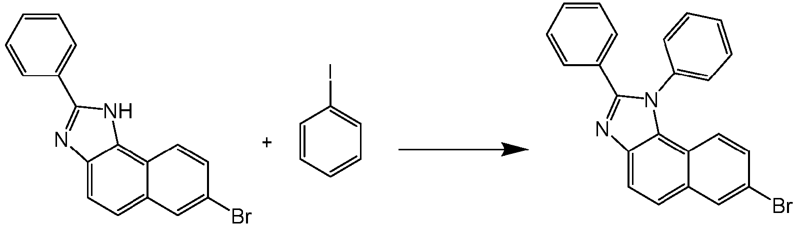

Unter inerter Atmosphäre werden 32,2 g, (100 mmol), 7-Bromo-2-phenyl-1H-benzo[e]benzimidazol, ( 20,3 g (100 mmol) lodbenzol, 34.55 g (250 mmol) Kaliumcarbonat und 1.27 g (20.0 mmol) Kupferpulver in 400 mL DMF vorgelegt, weitere 15 min mit Argon inertisiert und anschließend 32 h bei 130 °C gerührt. Man lässt den Ansatz auf Raumtemperatur abkühlen, filtriert über ein Celite-Bett, wäscht zweimal mit 200 mL DMF nach und rotiert das Filtrat bis zur Trocken am Rotationsverdampfer ein. Der Rückstand wird extraktiv mit Dichlormethan/Wasser aufgearbeitet, die organische Phase zweimal mit Wasser und einmal mit gesättigter NaCl-Lösung gewaschen und über Na₂SO₄ getrocknet. Es werden 150 mL Ethanol zugegeben, das Dichlormethan wird am Rotationsverdampfer bis 500 mbar abgezogen, der ausgefallende Feststoff abgesaugt und mit Ethanol gewaschen.

Die Ausbeute beträgt 43,4g (86 mmol), entsprechend 61 % der Theorie.

Analog werden folgende Verbindungen hergestellt:

| | **Edukt 1** | **Edukt 2** | **Produkt** | **Ausbeute** |
|---|---|---|---|---|
| **1c** | 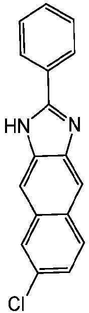 | 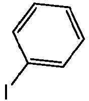 | 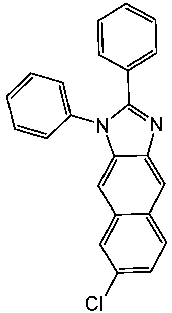 | 69% |
| **2c** | 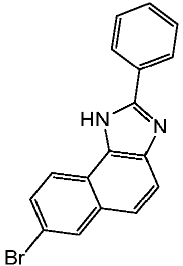 | 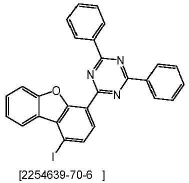 | 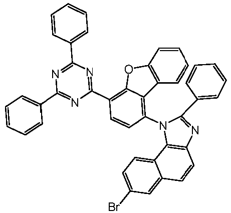 | 57% |
| **3c** | 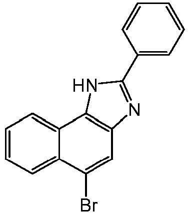 | 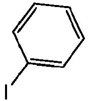 | 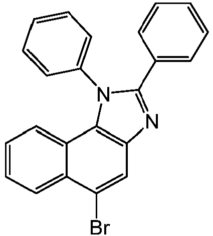 | 66% |
| **4c** | 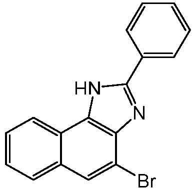 | 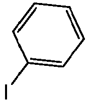 | 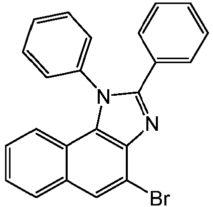 | 63% |
| **5c** | 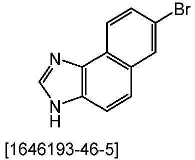 | 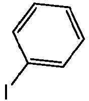 | 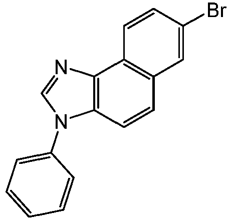 | 67% |
| **6c** | 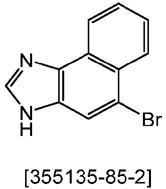 | 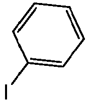 | 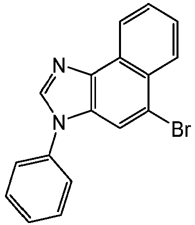 | 69% |
| **7c** | 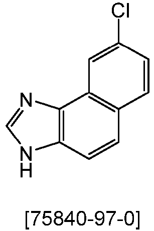 | 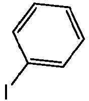 | 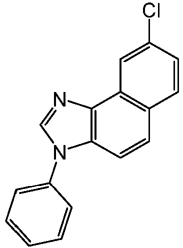 | 91% |
| **8c** | 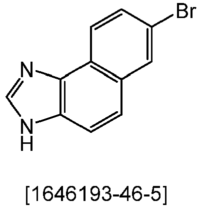 | 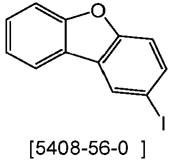 | 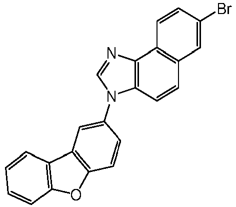 | 60% |
| **9c** | 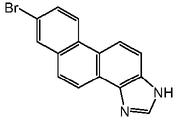 | 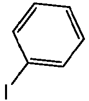 | 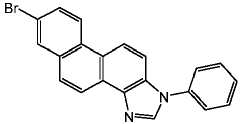 | 57% |
| **10c** | 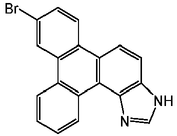 | 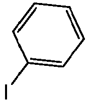 | 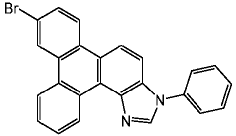 | 67% |
| **11c** | 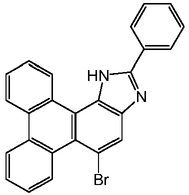 | 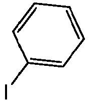 | 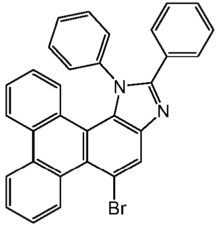 | 67% |
| **12c** | 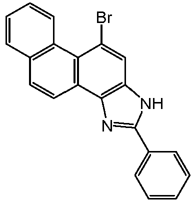 | 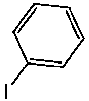 | 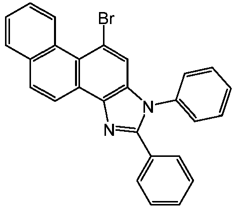 | 65% |

### d) N-(2-Chlorophenyl)-1,2-diphenyl-benzo[e]benzimidazol-7-amin

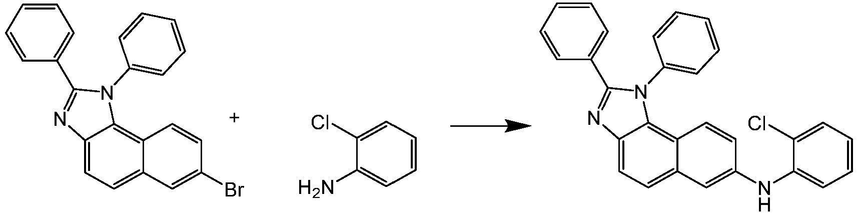

54 g (137 mmol) 7-Bromo-1,2-diphenyl-benzo[e]benzimidazol, 17,3 g (137 mmol) 2-Chloranilin, 68.2 g (710 mmol) Natrium-tert-butylat, 613 mg (3 mmol) Palladium(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 50 g (112 mmol) 83% d.Th.

Analog dazu werden die folgenden Verbindungen hergestellt:

| | **Edukt 1** | **Edukt 2** | **Produkt** | **Ausbeute** |
|---|---|---|---|---|
| **1d** | 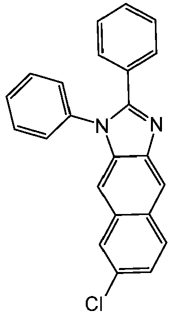 | 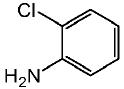 | 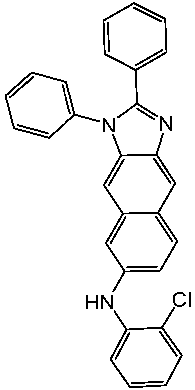 | 87% |
| **2d** | 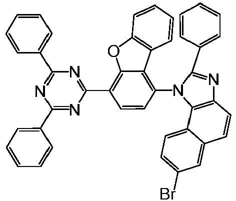 | 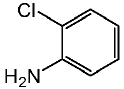 | 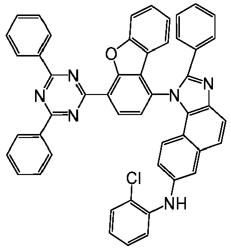 | 76% |
| **3d** | 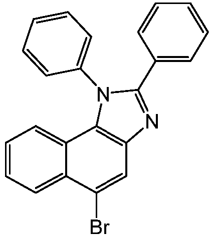 | 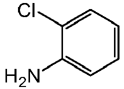 | 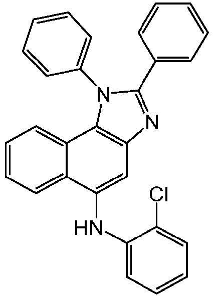 | 88% |
| **4d** | 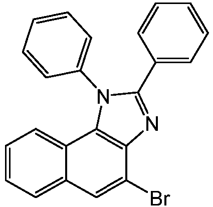 | 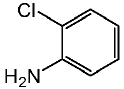 | 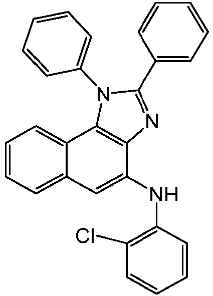 | 89% |
| **5d** | 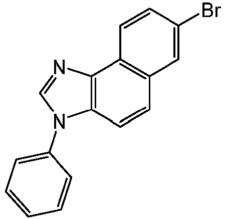 | 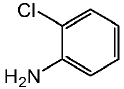 | 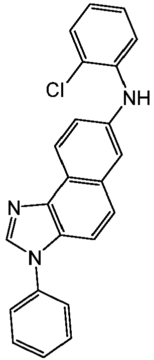 | 90% |
| **6d** | 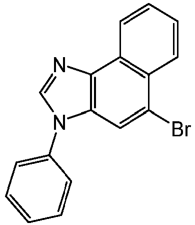 | 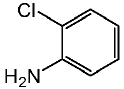 | 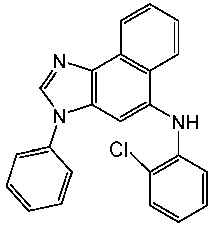 | 91% |
| **7d** | 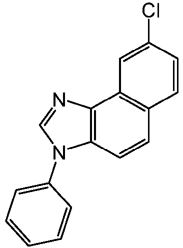 | 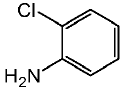 | 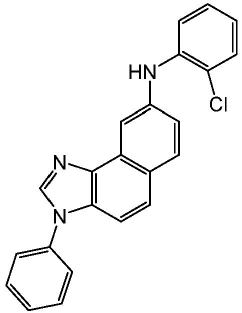 | 89% |
| **8d** | 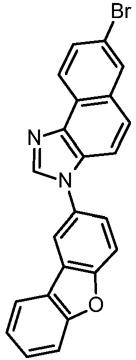 | 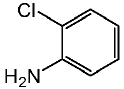 | 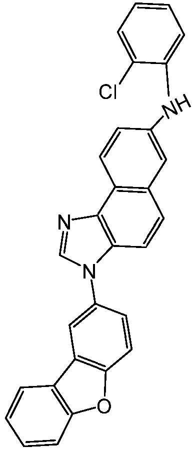 | 92% |
| **9d** | 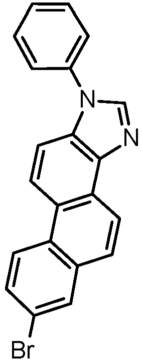 | 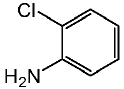 | 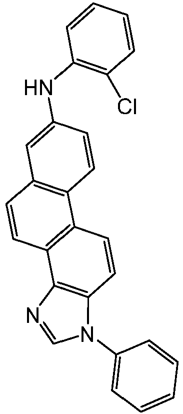 | 88% |
| **10d** | 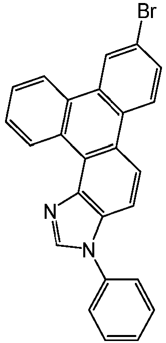 | 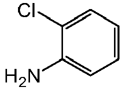 | 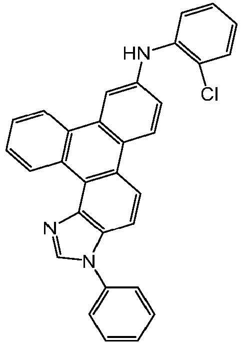 | 85% |
| **11d** | 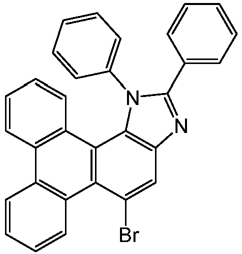 | 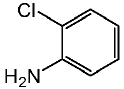 | 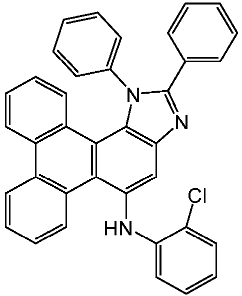 | 89% |

### e) Cyclisierung (Methode A)

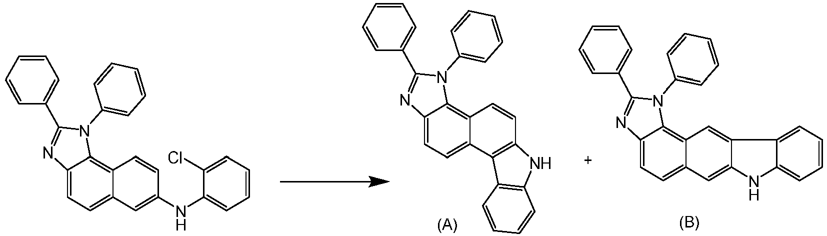

44,4 g (100 mmol) N-(2-Chlorophenyl)-1,2-diphenyl-benzo[e]benzimidazol-7-amin, 56 g (409 mmol) Kaliumcarbonat, 4.5 g (12 mmol) Tricyclohexylphosphintetrafluoroborat 1.38 g (6 mmol) Palladium(II)acetat werden in 500 mL Dimethylacetamid suspendiert und 6 Stunden unter Rückfluss gerührt. Nach Erkalten wird die Reaktionmischung mit 300 ml Wasser und 400 mL und rührt 30 min. nach, trennt die org. Phase ab, filtriert diese über ein kurzes Celite-Bett und entfernt dann das Lösungsmittel im Vakuum. Das Rohprodukt wird mit Toluol heiß extrahiertund aus Chromatographisch getrennt. Die Ausbeute beträgt 33 g (80 mmol) der Mischung A+B, entsprechend 80 % der Theorie. Nach Säulenchromatographisch getrennt und anschließender Aufarbeitung erhält man 30 % A und 42 % B.

Analog dazu werden die folgenden Verbindungen hergestellt:

| | **Edukt 1** | **Produkt A** | **Produkt B** | **Ausbeute** |
|---|---|---|---|---|
| **1e** | 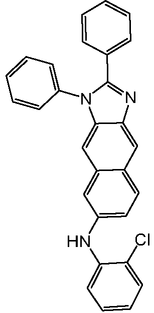 | 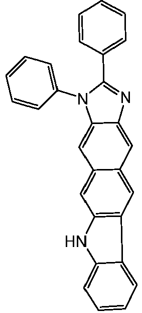 | 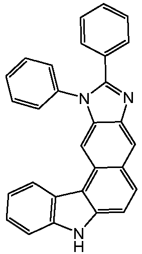 | 41 %A, 23 %B |
| **2e** | 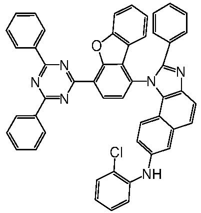 | 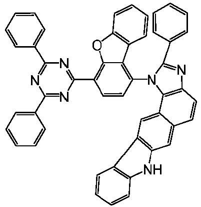 | 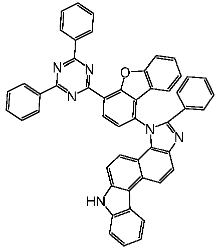 | 39 %A, 20 %B |
| **3e** | 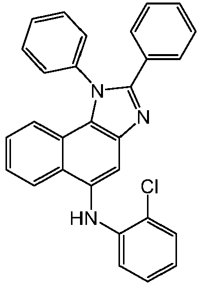 | 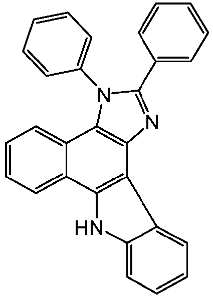 | | 82 % |
| **4e** | 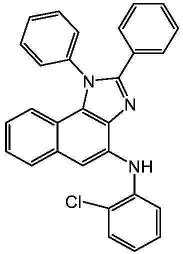 | 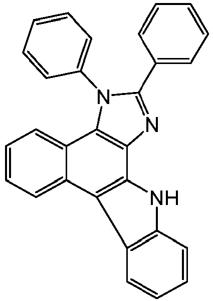 | | 84 % |
| **5e** | 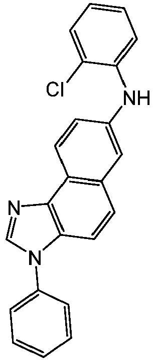 | 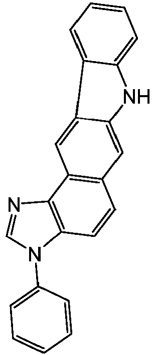 | 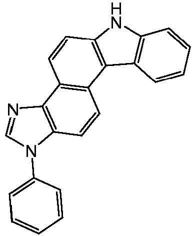 | 44 %A, 19 %B |
| **6e** | 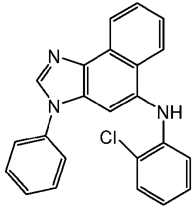 | 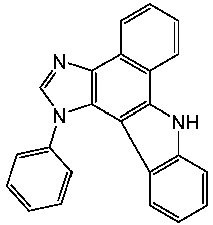 | 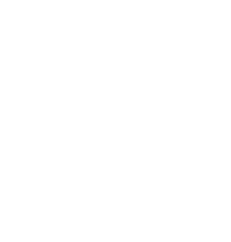 | 88% |
| **7e** | 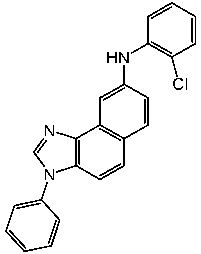 | 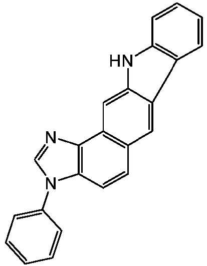 | 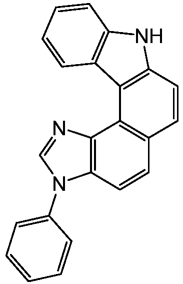 | 40 %A, 21%B |
| **8e** | 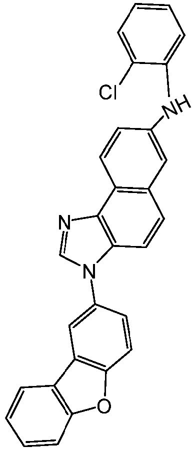 | 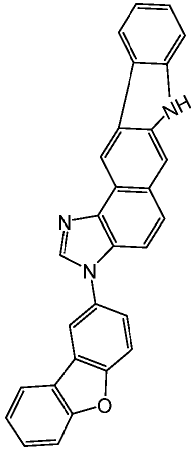 | 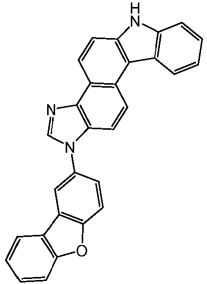 | 47 %A, 25 %B |
| **9e** | 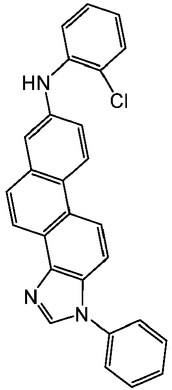 | 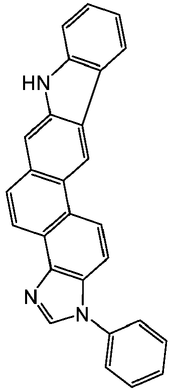 | 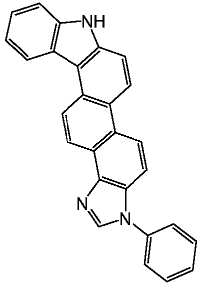 | 44 %A, 20 %B |
| **10e** | 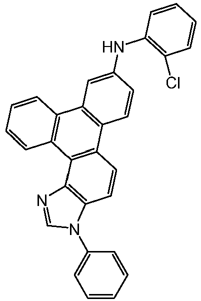 | 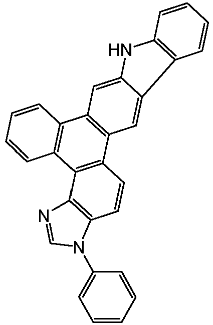 | 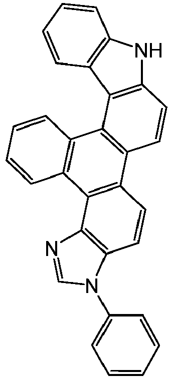 | 48 %A, 15 %B |
| **11e** | 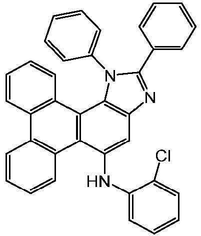 | 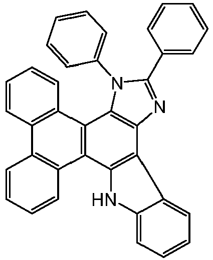 | | 79 % |

### f) 5-(2-Nitrophenyl)-1,2-diphenyl-benzo[e]benzimidazol

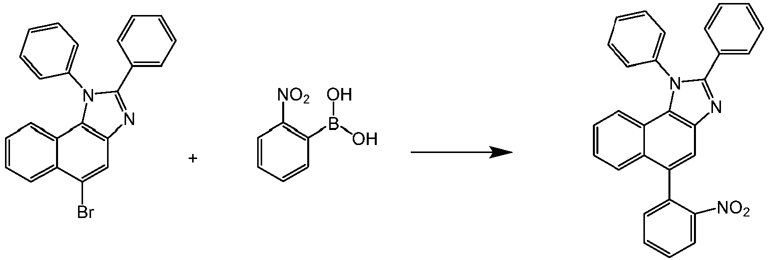

Eine gut gerührte, entgaste Suspension aus 30 g (184 mmol) 2-Nitrophenyl-benzolboronsäure, 71 g (180 mmol) 5-Bromo-1,2-diphenyl-benzo[e]benzimidazol und 66.5 g (212.7 mmol) Kaliumcarbonat in einem Gemisch aus 250 ml Wasser und 250 ml THF wird mit 1.7 g (1.49 mmol) Pd(PPh₃)₄ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet.

Ausbeute: 63 g, (144 mmol), 80 % d. Th..

Analog dazu werden die folgenden Verbindungen hergestellt:

| | **Edukt 1** | **Edukt 2** | **Produkt** | **Ausbeute** |
|---|---|---|---|---|
| 1f | 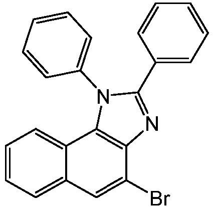 | 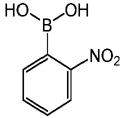 | 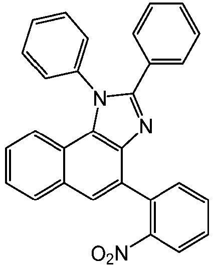 | 85% |
| 2f | 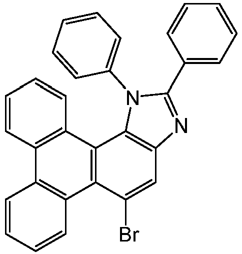 | 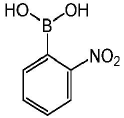 | 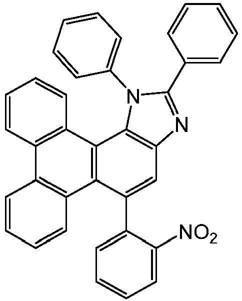 | 89% |

### g) Cyclisierung (Methode B)

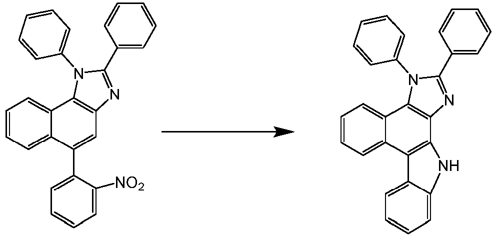

Eine Mischung aus 53 g (120 mmol) 5-(2-Nitrophenyl)-1,2-diphenyl-benzo[e]benzimidazol und 145ml (834 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert.

Ausbeute: 53 g, (86 mmol), 73 % d. Th..

heraestellt: Analog dazu werden die folgenden Verbindungen hergestellt:

| | **Edukt** | **Produkt** | **Ausbeute** |
|---|---|---|---|
| 1g | 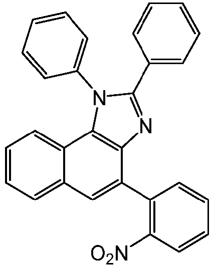 | 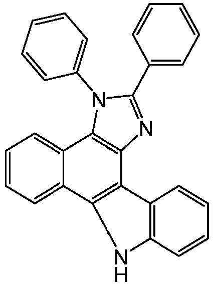 | 85% |
| 2g | 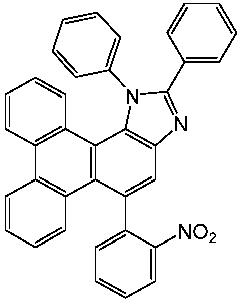 | 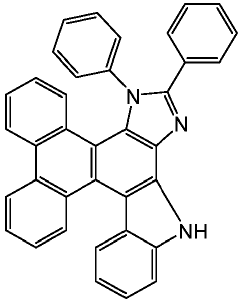 | 89% |

### h) Buchwald

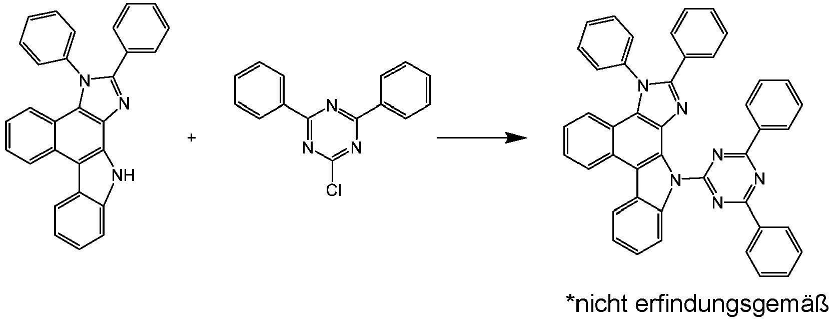

4,2 g NaH 60%ig in Mineralöl (106 mmol) werden in 300 mL Dimethylformamide unter Schutzatmosphäre gelöst. 43 g (106 mmol) von Verbindung **(g)** werden in 250 mL DMF gelöst und zu der Reaktionsmischung zugetropft. Nach 1 Stunde bei Raumtemperatur wird eine Lösung von 2-Chlor-4,6-Diphenyl-[1,3,5]triazin (34,5 g, 0,122 mol) in 200 mL THF zugetropft. Das Reaktionsgemischt wird dann 12 h bei Raumtemperatur gerührt. Nach dieser Zeit wird das Reaktionsgemisch auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert und aus Toluol / n-Heptan umkristallisiert und abschließend im Hochvakuum sublimiert, Reinheit beträgt 99,9%.

Ausbeute: 42 g, (65 mmol), 62 % d. Th..

Analog werden folgende Verbindungen hergestellt:

| | **Edukt 1** | **Edukt 2** | **Produkt** | **Ausbeute** |
|---|---|---|---|---|
| **1h** | 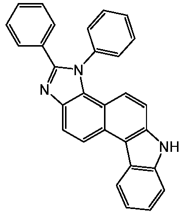 | 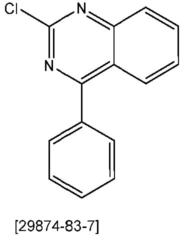 | 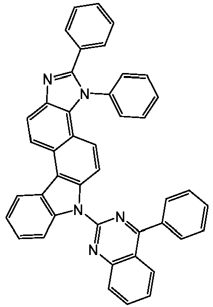 | 63% |
| **2h** | 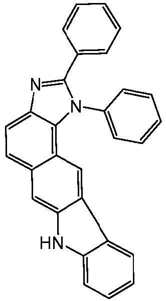 | 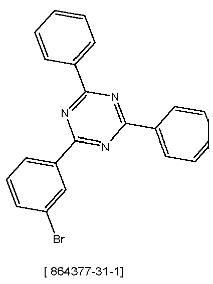 | 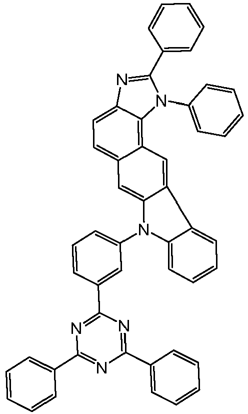 | 66% |
| **3h** | 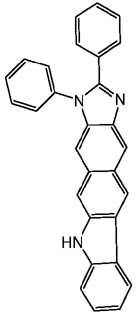 | 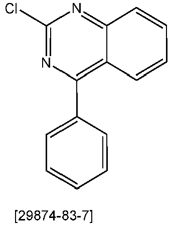 | 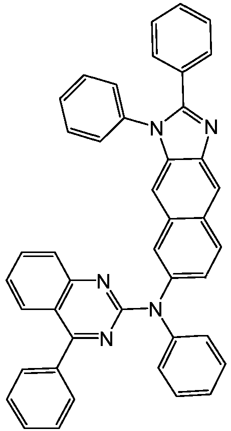 | 59% |
| **4h** | 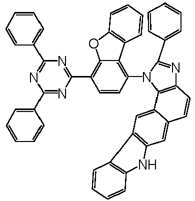 | 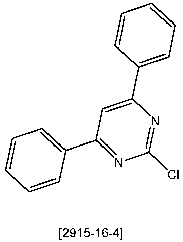 | 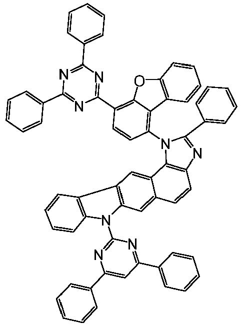 | 53% |
| **5h*** | 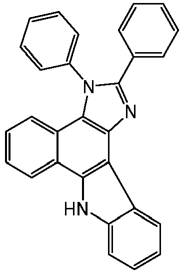 | 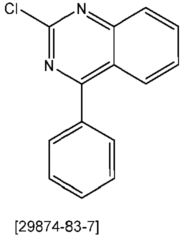 | 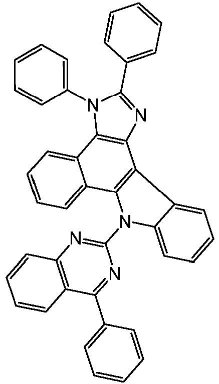 | 64% |
| **6h*** | 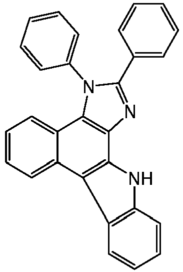 | 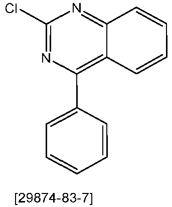 | 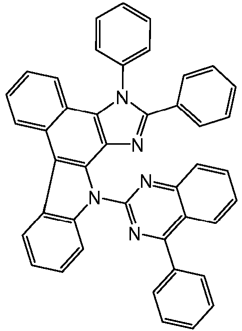 | 68% |
| **7h** | 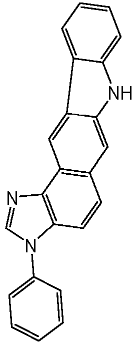 | 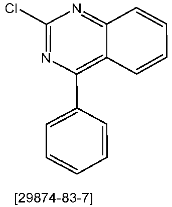 | 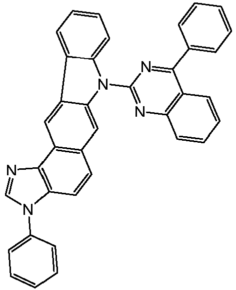 | 66% |
| **8h*** | 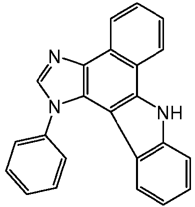 | 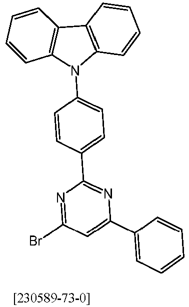 | 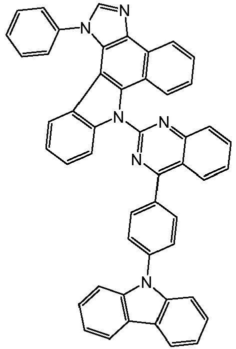 | 71% |
| **9h** | 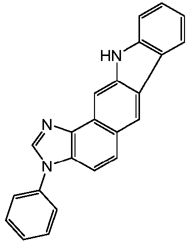 | 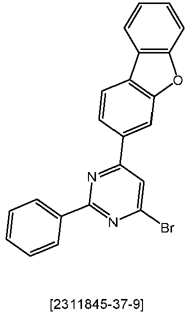 | 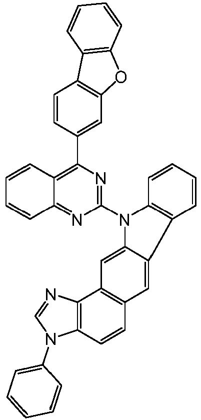 | 65% |
| **10h** | 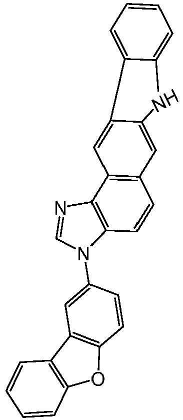 | 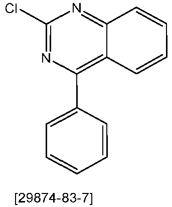 | 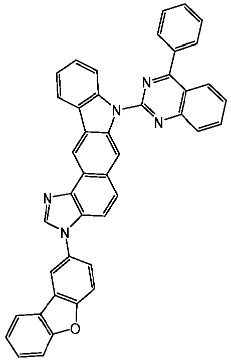 | 59% |
| **11h** | 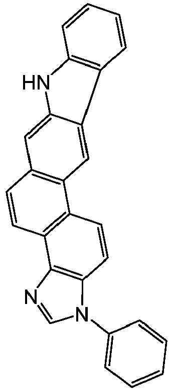 | 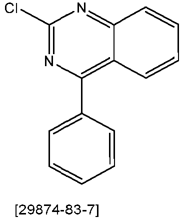 | 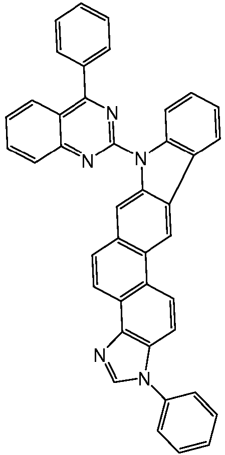 | 67% |
| **12h** | 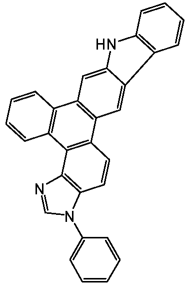 | 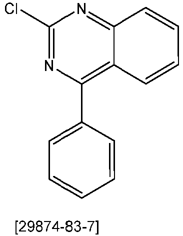 | 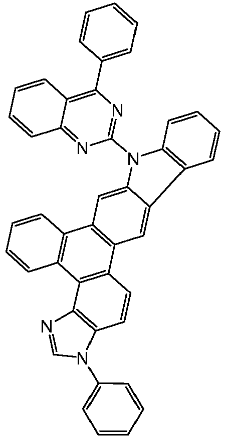 | 69% |
| **13h** | 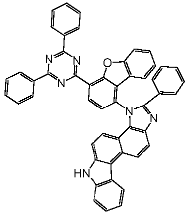 | 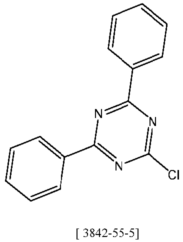 | 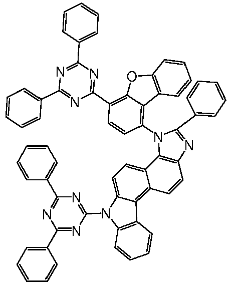 | 52% |
| **14h*** | 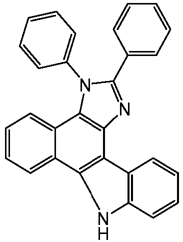 | 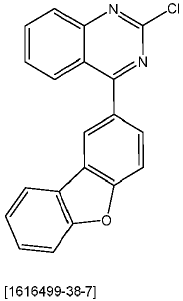 | 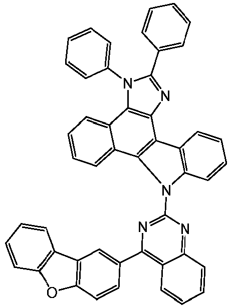 | 62% |
| **15h*** | 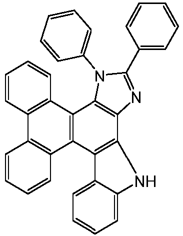 | 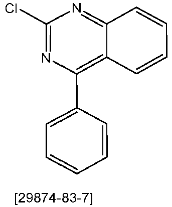 | 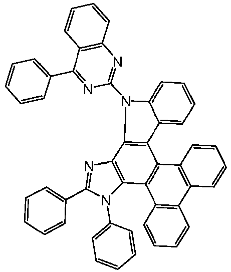 | 60% |
| **16h** | 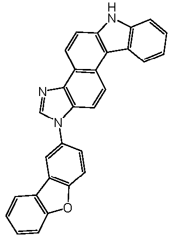 | 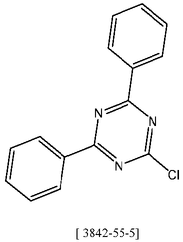 | 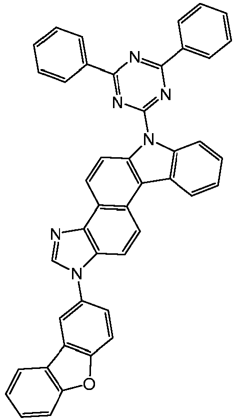 | 65% |
| **17h** | 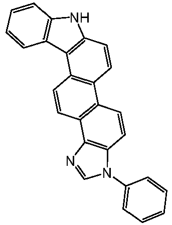 | 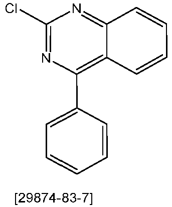 | 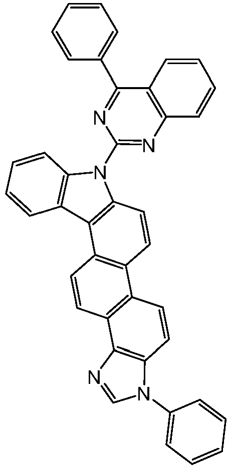 | 67% |
| **18h** | 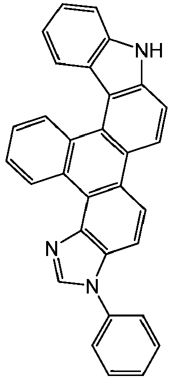 | 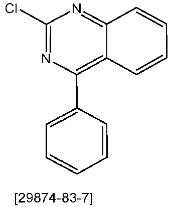 | 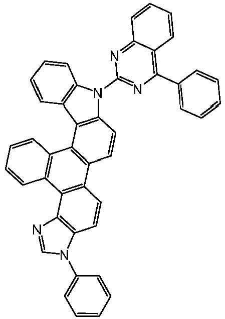 | 61% |
| **19h*** | 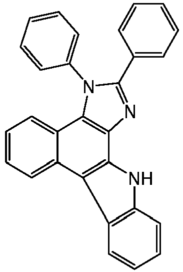 | 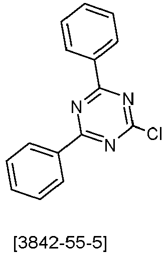 | 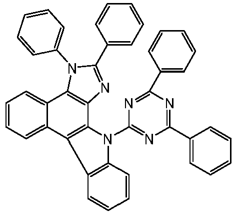 | 63% |
| **20h*** | 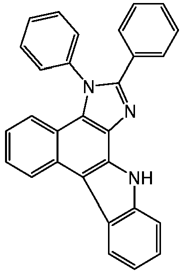 | 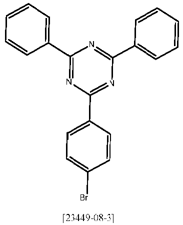 | 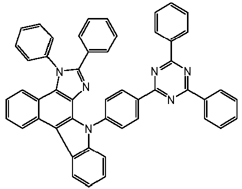 | 71% |
| **21h** | 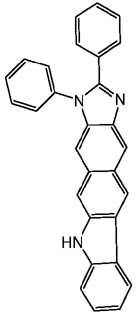 | 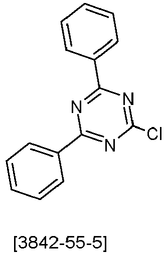 | 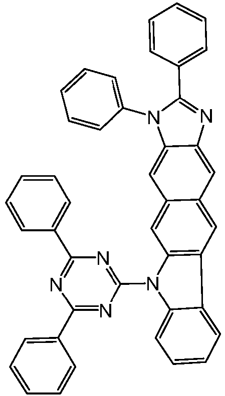 | 67% |
| **22h** | 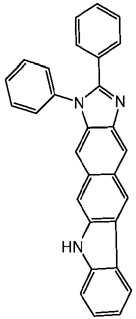 | 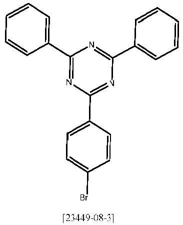 | 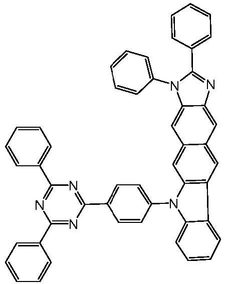 | 75% |
| **23h*** | 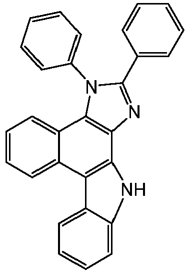 | 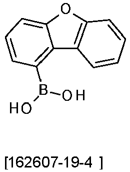 | 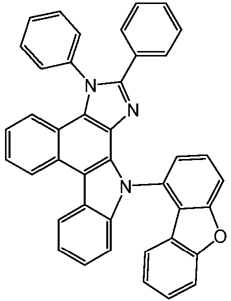 | 77% |
| **24h*** | 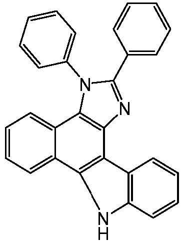 | 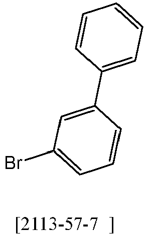 | 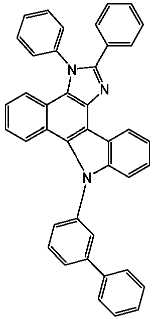 | 71% |
| **25h** | 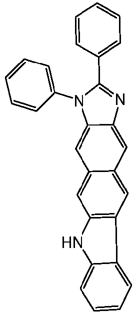 | 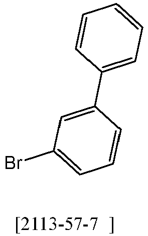 | 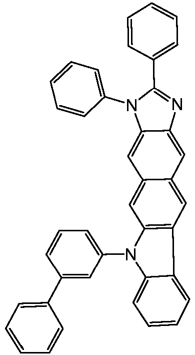 | 76% |

| | | | | |
|---|---|---|---|---|
| ***nicht erfindungsgemäß** | | | | |

### j) Arylierung

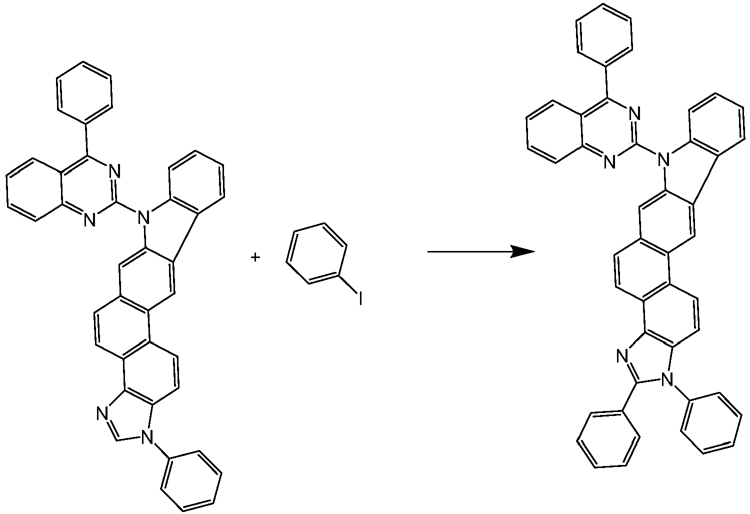

Unter Schutzgas werden 29,3 g (50 mmol) Verbindung (11h), 560 mg (25 mmol) Pd(OAc)₂, 19,3 g (118 mmol) Cul und 20,8 (100 mmol) lodbenzol in 300 ml entgastem DMF suspendiert und die Reaktionsmischung wird 24 h unter Rückfluss bei 140°C erhitzt. Nach Erkalten wird das Lösungsmittel im Vakuum entfernt, der Rückstand wird in Dichlormethan gelöst und mit Wasser versetzt. Danach wird die organische Phase abgetrennt und über Kieselgel filtriert. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99,9%). Die Ausbeute beträgt 19,9 g (30 mmol), entsprechend 60% der Theorie.

Analog werden folgende Verbindungen hergestellt:

| | **Edukt 1** | **Edukt 2** | **Produkt** | **Ausbeute** |
|---|---|---|---|---|
| **1j** | 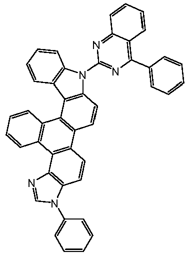 | 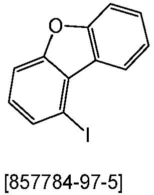 | 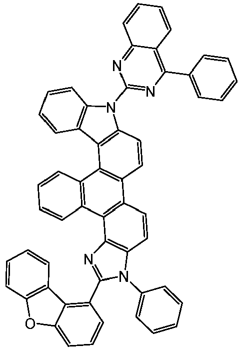 | 60% |
| **2j** | 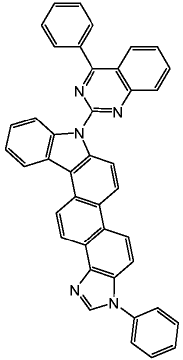 | 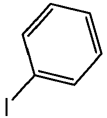 | 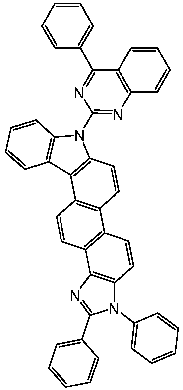 | 57% |
| **3j** | 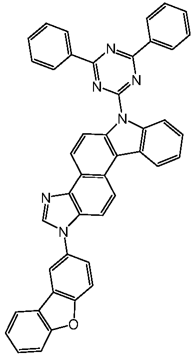 | 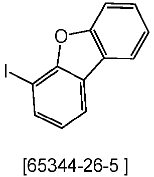 | 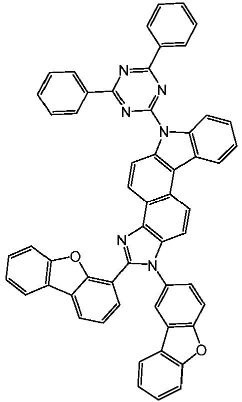 | 48% |
| **4j** | 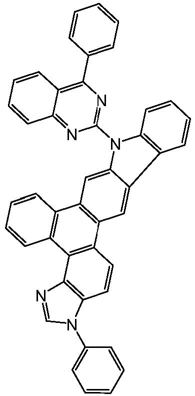 | 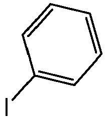 | 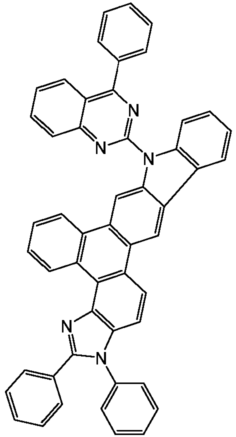 | 61% |

### Herstellung der Elektrolumineszenzvorrichtungen

**Vorbehandlung für die Beispiele V1 - E22:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die Elektrolumineszenzvorrichtungen haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der Elektrolumineszenzvorrichtungen ist Tabelle 1 zu entnehmen. Die zur Herstellung der Elektrolumineszenzvorrichtungen benötigten Materialien sind in Elektrolumineszenzvorrichtungen 2 gezeigt. Die Daten der Elektrolumineszenzvorrichtungen sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie 1e:IC2:TER5 (57%:40%:3%) bedeutet hierbei, dass das Material 1e in einem Volumenanteil von 57%, IC2 in einem Anteil von 40% und TER5 in einem Anteil von 3% in der Schicht vorliegt. Analog kann auch die Elektronentransportschicht aus einer Mischung von zwei Materialien bestehen.

Die Elektrolumineszenzvorrichtungen werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden.

Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% in Tabelle 3 bedeutet, dass die in Spalte LD angegebene Lebensdauer der Zeit entspricht, nach der die Leuchtdichte auf 95% ihres Anfangswertes absinkt.

### Verwendung von erfindungsgemäßen Mischungen in Elektrolumineszenzvorrichtungen

Die erfindungsgemäßen Materialien werden in den nicht gekennzeichneten Beispielen als Matrixmaterial in der Emissionsschicht von rot phosphoreszierenden OLEDs eingesetzt. Im Vergleich zum Stand der Technik (V1 bis V8) kann durch die Verwendung der erfindungsgemäßen Verbindungen eine deutliche Lebensdauerverbesserung erreicht werden bei ansonsten vergleichbaren Parametern.

**Tabelle 1: Aufbau der Elektrolumineszenzvorrichtungen**

| Bsp | HIL Dicke | HTL Dicke | EBL Dicke | EML Dicke | HBL Dicke | ETL Dicke | EIL Dicke |
|---|---|---|---|---|---|---|---|
| V1 | SpMA1:PD1 (95%:5%) 20nm | SpMA1 110nm | SpMA2 10nm | SdT1:TER5 (97%:3%) 35nm | ST2 10nm | ST2:LiQ (50%:50%) 30nm | LiQ 1nm |
| V2 | SpMA1:PD1 (95%:5%) 20nm | SpMA1 110nm | SpMA2 10nm | SdT2:TER5 (97%:3%) 35nm | ST2 10nm | ST2:LiQ (50%:50%) 30nm | LiQ 1nm |
| V3 | SpMA1:PD1 (95%:5%) 20nm | SpMA1 110nm | SpMA2 10nm | SdT1:IC2:TER5 (57%:40%:3%) | ST2 10nm | ST2:LiQ (50%:50%) 30nm | LiQ 1nm |
| V4 | SpMA1:PD1 (95%:5%) 20nm | SpMA1 110nm | SpMA2 10nm | SdT2:IC2:TER5 (57%:40%:3%) | ST2 10nm | ST2:LiQ (50%:50%) 30nm | LiQ 1nm |
| V5 | SpMA1:PD1 (95%:5%) 20nm | SpMA1 110nm | SpMA2 10nm | SdT3:TER5 (97%:3%) 35nm | ST2 10nm | ST2:LiQ (50%:50%) 30nm | LiQ 1nm |
| V6 | SpMA1:PD1 (95%:5%) 20nm | SpMA1 110nm | SpMA2 10nm | SdT3:IC2:TER5 (57%:40%:3%) | ST2 10nm | ST2:LiQ (50%:50%) 30nm | LiQ 1nm |
| V7 | SpMA1:PD1 (95%:5%) 20nm | SpMA1 110nm | SpMA2 10nm | SdT4:TER5 (97%:3%) 35nm | ST2 10nm | ST2:LiQ (50%:50%) 30nm | LiQ 1nm |
| V8 | SpMA1:PD1 (95%:5%) 20nm | SpMA1 110nm | SpMA2 10nm | SdT4:IC2:TER5 (57%:40%:3%) | ST2 10nm | ST2:LiQ (50%:50%) 30nm | LiQ 1nm |
| E1 | SpMA1:PD1 (95%:5%) 20nm | SpMA1 110nm | SpMA2 10nm | j:TER5 (97%:3%) 35nm | ST2 10nm | ST2:LiQ (50%:50%) 30nm | LiQ 1nm |
| E2 | SpMA1:PD1 (95%:5%) 20nm | SpMA1 110nm | SpMA2 10nm | j:IC2:TER5 (57%:40%:3%) 35nm | ST2 10nm | ST2:LiQ (50%:50%) 30nm | LiQ 1nm |
| E3 | SpMA1:PD1 (95%:5%) 20nm | SpMA1 110nm | SpMA2 10nm | 2j:TER5 (97%:3%) 35nm | ST2 10nm | ST2:LiQ (50%:50%) 30nm | LiQ 1nm |
| E4 | SpMA1:PD1 (95%:5%) 20nm | SpMA1 110nm | SpMA2 10nm | 2j:IC2:TER5 (57%:40%:3%) 35nm | ST2 10nm | ST2:LiQ (50%:50%) 30nm | LiQ 1nm |
| E5 | SpMA1:PD1 (95%:5%) 20nm | SpMA1 110nm | SpMA2 10nm | 1h:IC2:TER5 (57%:40%:3%) 35nm | ST2 10nm | ST2:LiQ (50%:50%) 30nm | LiQ 1nm |
| E6 | SpMA1:PD1 (95%:5%) 20nm | SpMA1 110nm | SpMA2 10nm | 2h:IC2:TER5 (57%:40%:3%) 35nm | ST2 10nm | ST2:LiQ (50%:50%) 30nm | LiQ 1nm |
| E7 | SpMA1:PD1 (95%:5%) 20nm | SpMA1 110nm | SpMA2 10nm | 4h:TER5 (97%:3%) 35nm | ST2 10nm | ST2:LiQ (50%:50%) 30nm | LiQ 1nm |
| E8 | SpMA1:PD1 (95%:5%) 20nm | SpMA1 110nm | SpMA2 10nm | 4h:IC2:TER5 (57%:40%:3%) 35nm | ST2 10nm | ST2:LiQ (50%:50%) 30nm | LiQ 1nm |
| E9* | SpMA1:PD1 (95%:5%) 20nm | SpMA1 110nm | SpMA2 10nm | 6h:TER5 (97%:3%) 35nm | ST2 10nm | ST2:LiQ (50%:50%) 30nm | LiQ 1nm |
| E10* | SpMA1:PD1 (95%:5%) 20nm | SpMA1 110nm | SpMA2 10nm | 6h:IC2:TER5 (57%:40%:3%) 35nm | ST2 10nm | ST2:LiQ (50%:50%) 30nm | LiQ 1nm |
| E11* | SpMA1:PD1 (95%:5%) 20nm | SpMA1 110nm | SpMA2 10nm | 14h:IC2:TER5 (57%:40%:3%) 35nm | ST2 10nm | ST2:LiQ (50%:50%) 30nm | LiQ 1nm |
| E12* | SpMA1:PD1 (95%:5%) 20nm | SpMA1 110nm | SpMA2 10nm | 15h:IC2:TER5 (57%:40%:3%) 35nm | ST2 10nm | ST2:LiQ (50%:50%) 30nm | LiQ 1nm |
| E13 | SpMA1:PD1 (95%:5%) 20nm | SpMA1 110nm | SpMA2 10nm | 1j:IC2:TER5 (57%:40%:3%) 35nm | ST2 10nm | ST2:LiQ (50%:50%) 30nm | LiQ 1nm |
| E14 | SpMA1:PD1 (95%:5%) 20nm | SpMA1 110nm | SpMA2 10nm | 4j:IC2:TER5 (57%:40%:3%) 35nm | ST2 10nm | ST2:LiQ (50%:50%) 30nm | LiQ 1nm |
| E15* | SpMA1:PD1 (95%:5%) 20nm | SpMA1 110nm | SpMA2 10nm | 19h:TER5 (97%:3%) 35nm | ST2 10nm | ST2:LiQ (50%:50%) 30nm | LiQ 1nm |
| E16* | SpMA1:PD1 (95%:5%) 20nm | SpMA1 110nm | SpMA2 10nm | 20h:TER5 (97%:3%) 35nm | ST2 10nm | ST2:LiQ (50%:50%) 30nm | LiQ 1nm |
| E17 | SpMA1:PD1 (95%:5%) 20nm | SpMA1 110nm | SpMA2 10nm | 21h:TER5 (97%:3%) 35nm 35nm | ST2 10nm | ST2:LiQ (50%:50%) 30nm | LiQ 1nm |
| E18 | SpMA1:PD1 (95%:5%) 20nm | SpMA1 110nm | SpMA2 10nm | 22h:TER5 (97%:3%) 35nm | ST2 10nm | ST2:LiQ (50%:50%) 30nm | LiQ 1nm |
| E19* | SpMA1:PD1 (95%:5%) 20nm | SpMA1 110nm | SpMA2 10nm | 19h:IC2:TER5 (57%:40%:3%) 35nm | ST2 10nm | ST2:LiQ (50%:50%) 30nm | LiQ 1nm |
| E20 | SpMA1:PD1 (95%:5%) 20nm | SpMA1 110nm | SpMA2 10nm | 21h:IC2:TER5 (57%:40%:3%) 35nm | ST2 10nm | ST2:LiQ (50%:50%) 30nm | LiQ 1nm |
| E21* | SpMA1:PD1 (95%:5%) 20nm | SpMA1 110nm | SpMA2 10nm | ST3:23h:TER5 (57%:40%:3%) 35nm | ST2 10nm | ST2:LiQ (50%:50%) 30nm | LiQ 1nm |
| E22 | SpMA1:PD1 (95%:5%) 20nm | SpMA1 110nm | SpMA2 10nm | ST3:25h:TER5 (57%:40%:3%) 35nm | ST2 10nm | ST2:LiQ (50%:50%) 30nm | LiQ 1nm |

| | | | | | | | |
|---|---|---|---|---|---|---|---|
| * nicht erfindungsgemäß | | | | | | | |

**Tabelle 2: Strukturformeln der Materialien für die Elektrolumineszenzvorrichtungen**

| | |
|---|---|
| 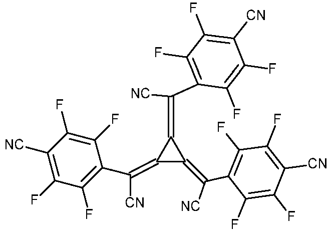 | 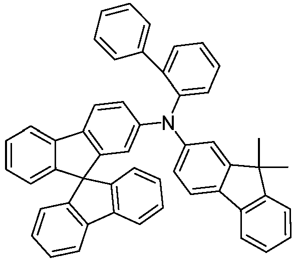 |
| PD1 (CAS Reg. No. 1224447-88-4) | SpMA1 |
| 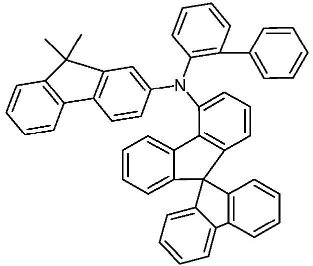 | 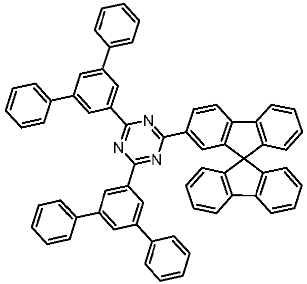 |
| SpMA2 | ST2 |
| 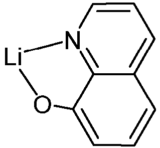 | 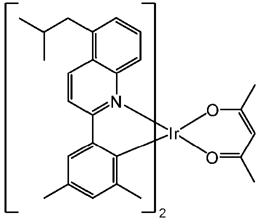 |
| LiQ | TER5 |
| 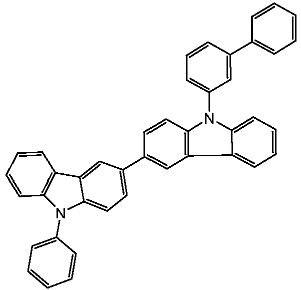 | 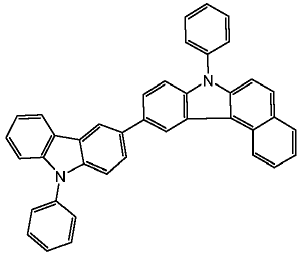 |
| IC1 | IC2 |
| 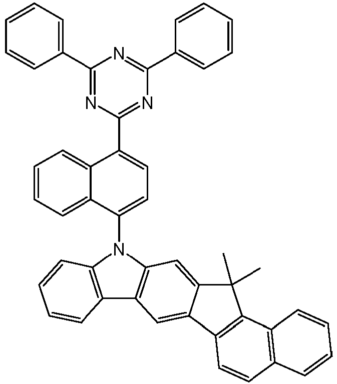 | |
| ST3 | |
| 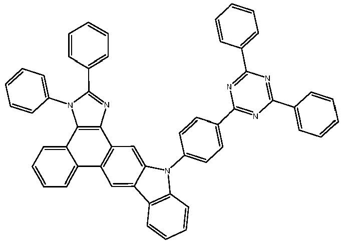 | 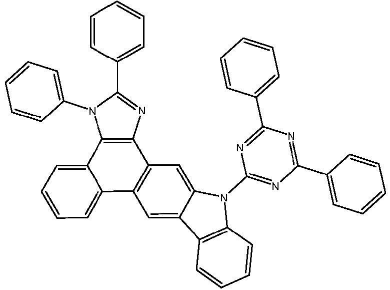 |
| SdT1 (KR 2016076881) | SdT2 (KR 2016076881) |
| 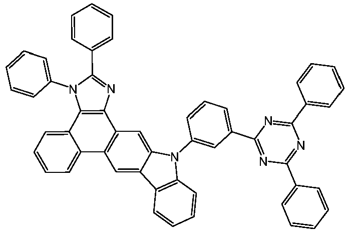 | 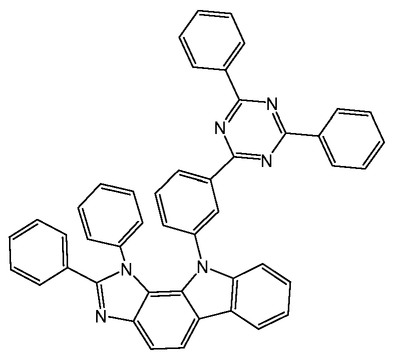 |
| SdT3 (KR 2016076881) | SdT4 (WO 2014/010810) |
| 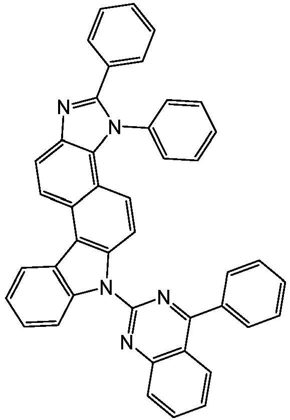 | 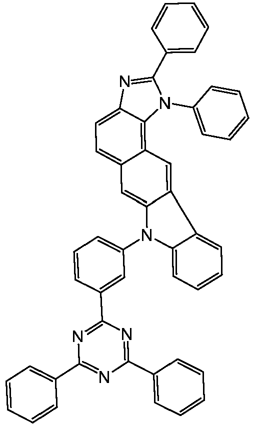 |
| 1h | 2h |
| 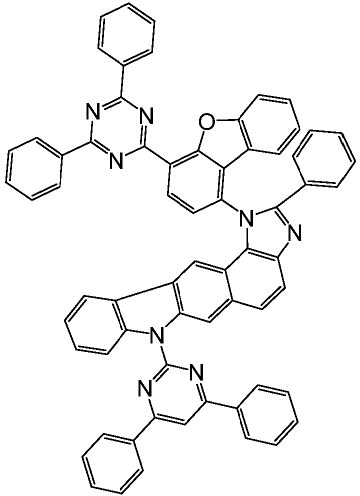 | 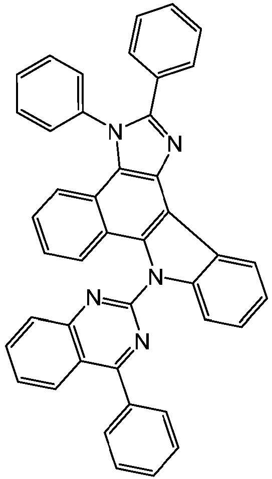 |
| 4h | 6h* |
| 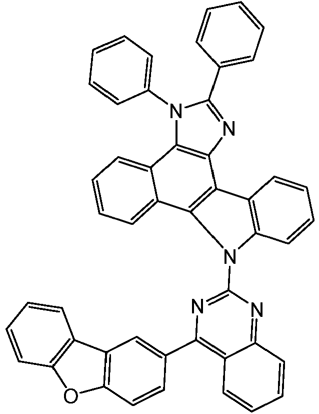 | 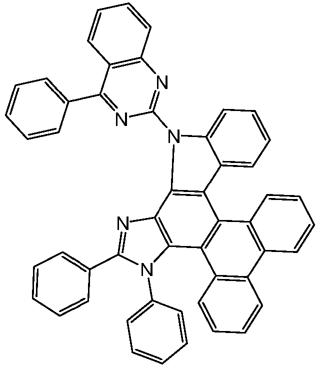 |
| 14h* | 15h* |
| 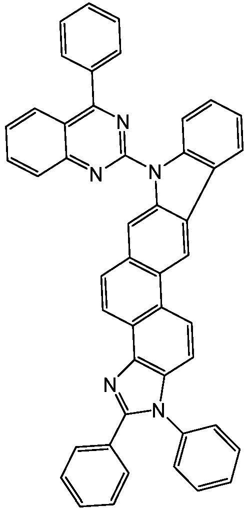 | 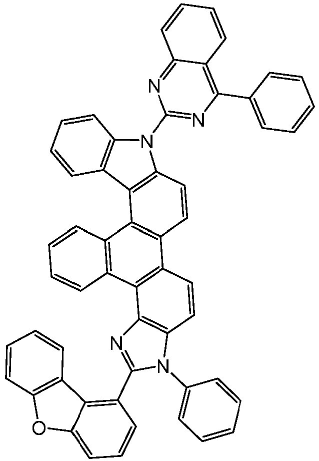 |
| j | 1j |
| 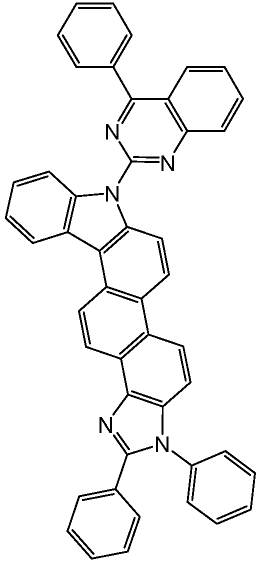 | 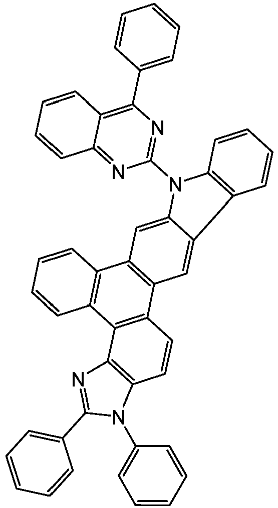 |
| 2j | 4j |
| 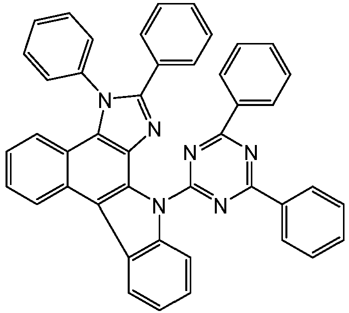 | 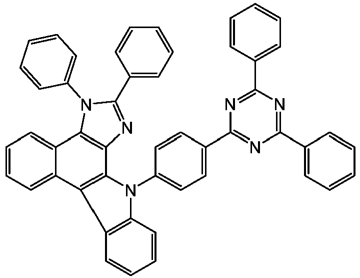 |
| 19h* | 20h* |
| 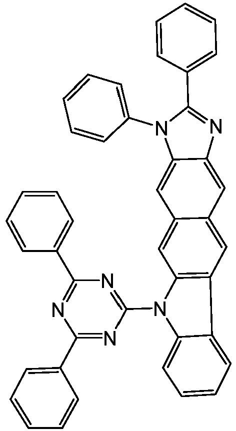 | 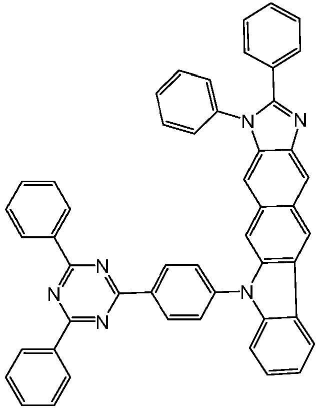 |
| 21h | 22h |
| 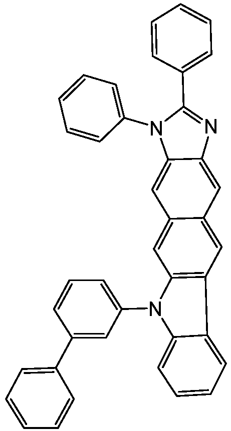 | 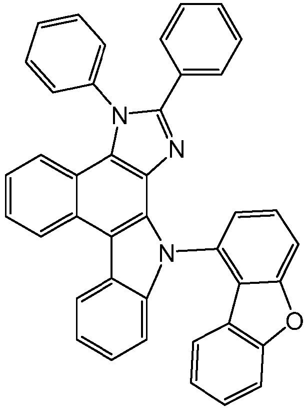 |
| 25h | 23h* |

| | |
|---|---|
| * nicht erfindungsgemäß | |

**Tabelle 3: Leistungsdaten der Elektrolumineszenzvorrichtungen**

| Bsp. | U1000 (V) | SE1000 (cd/A) | EQE 1000 (%) | CIE x/y bei 1000 cd/m² | j₀ (mA/cm²) | L1 (%) | LD (h) |
|---|---|---|---|---|---|---|---|
| V1 | 3.7 | 28 | 16.1 | 0.66/0.33 | 60 | 95 | 57 |
| V2 | 3.4 | 27 | 17.3 | 0.67/0.34 | 60 | 95 | 60 |
| V3 | 3.8 | 25 | 18.1 | 0.66/0.33 | 60 | 95 | 65 |
| V4 | 3.5 | 28 | 19.2 | 0.66/0.34 | 60 | 95 | 71 |
| V5 | 3.7 | 25 | 16 | 0.67/0.34 | 60 | 95 | 56 |
| V6 | 3.4 | 28 | 17.5 | 0.66/0.34 | 60 | 95 | 69 |
| V7 | 3.8 | 25 | 16.3 | 0.67/0.33 | 60 | 95 | 58 |
| V8 | 3.5 | 28 | 17.2 | 0.66/0.34 | 60 | 95 | 66 |
| E1 | 3.9 | 28 | 22 | 0.67/0.34 | 60 | 95 | 81 |
| E2 | 3.4 | 27 | 24.0 | 0.66/0.33 | 60 | 95 | 122 |
| E3 | 3.2 | 28 | 22 | 0.67/0.34 | 60 | 95 | 98 |
| E4 | 3.6 | 29 | 23.1 | 0.66/0.34 | 60 | 95 | 107 |
| E5 | 3.3 | 26 | 24.0 | 0.67/0.33 | 60 | 95 | 123 |
| E6 | 3.3 | 26 | 22.2 | 0.66/0.33 | 60 | 95 | 97 |
| E7 | 3.5 | 27 | 21.8 | 0.66/0.33 | 60 | 95 | 99 |
| E8 | 3.4 | 26 | 24.1 | 0.67/0.33 | 60 | 95 | 125 |
| E9* | 3.2 | 26 | 23.1 | 0.66/0.33 | 60 | 95 | 97 |
| E10* | 3.4 | 26 | 23.7 | 0.66/0.33 | 60 | 95 | 118 |
| E11* | 3.2 | 26 | 23.4 | 0.66/0.33 | 60 | 95 | 116 |
| E12* | 3.3 | 27 | 24.2 | 0.66/0.33 | 60 | 95 | 122 |
| E13 | 3.5 | 28 | 22 | 0.67/0.33 | 60 | 95 | 102 |
| E14 | 3.4 | 26 | 23.5 | 0.66/0.33 | 60 | 95 | 115 |
| E15* | 3.2 | 28 | 19 | 0.66/0.34 | 60 | 95 | 79 |
| E16* | 3.5 | 27 | 19.1 | 0.67/0.34 | 60 | 95 | 81 |
| E17 | 3.5 | 25 | 20 | 0.67/0.34 | 60 | 95 | 80 |
| E18 | 3.4 | 28 | 19 | 0.66/0.33 | 60 | 95 | 83 |
| E19* | 3.3 | 27 | 21 | 0.67/0.34 | 60 | 95 | 92 |
| E20 | 3.2 | 27 | 21 | 0.66/0.33 | 60 | 95 | 95 |
| E21* | 3.3 | 28 | 21 | 0.66/0.34 | 60 | 95 | 99 |
| E22 | 3.4 | 27 | 20 | 0.67/0.33 | 60 | 95 | 97 |

| | | | | | | | |
|---|---|---|---|---|---|---|---|
| * nicht erfindungsgemäß | | | | | | | |

Naphthyl-Strukturen mit einer kondensierten Indolgruppe und einer kondensierten Imidazolgruppe weisen im Vergleich zu Phenanthren -Strukturen bessere Eigenschaften auf. Dies gilt insbesondere für den Fall, dass die Indolgruppe und die Imidazolgruppe an verschiedenen Ringen an der Phenanthren-Struktur kondensiert ist.

Triphenylen-Strukturen mit einer kondensierten Indolgruppe und einer kondensierten Imidazolgruppe weisen im Vergleich zu Phenanthren-Strukturen bessere Eigenschaften auf.

Durch die Verwendung von Co-Hostmaterialien können unerwartete Verbesserungen erzielt werden.

## Patentansprüche

1. Verbindungen der Formeln (I'-5) bis (I'-22), (I'-31) bis (I'-78) und (I'-83) bis (I'-118), 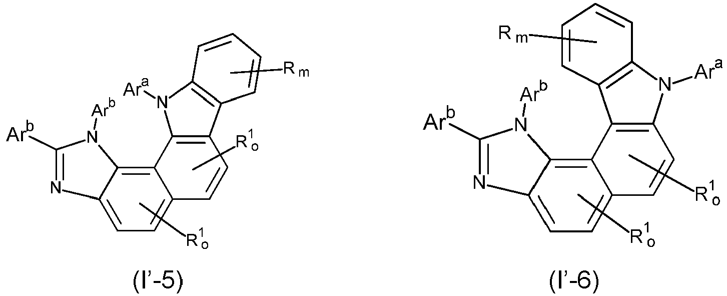 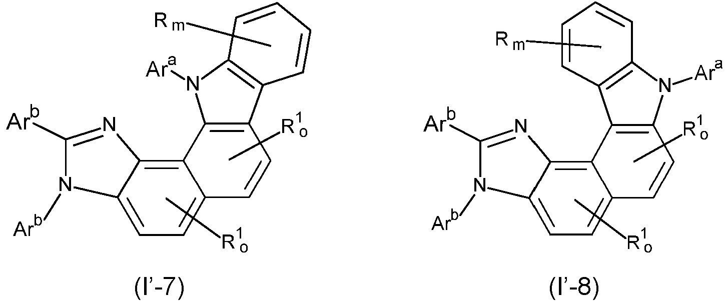 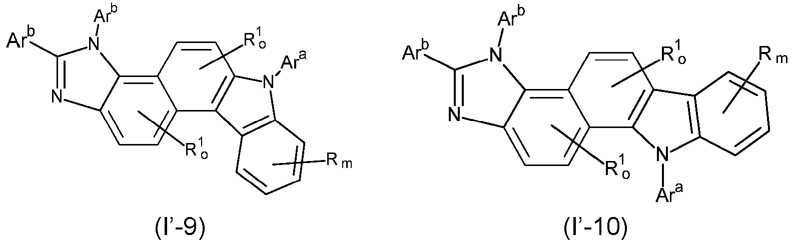 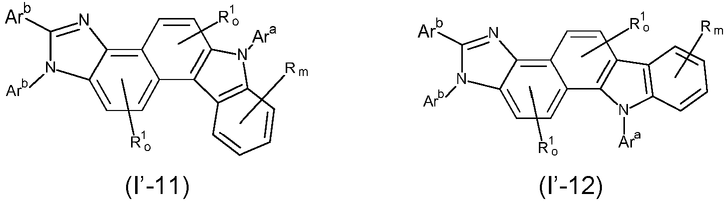 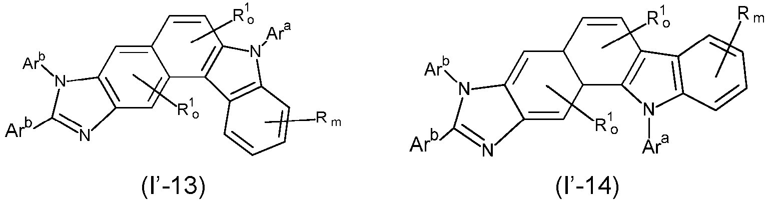 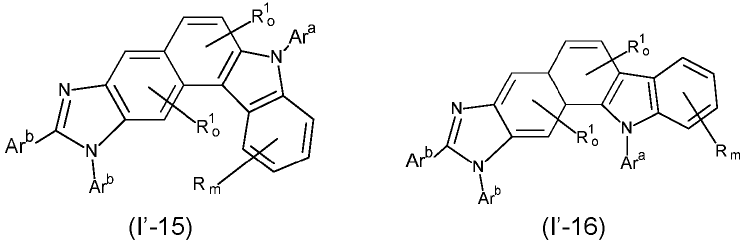 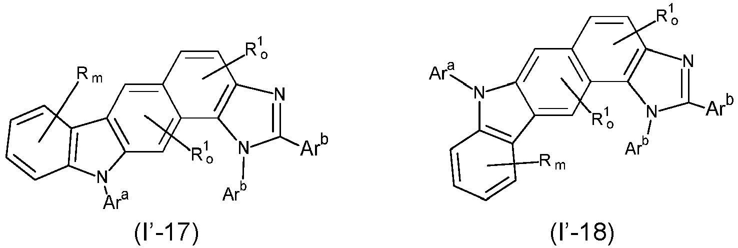 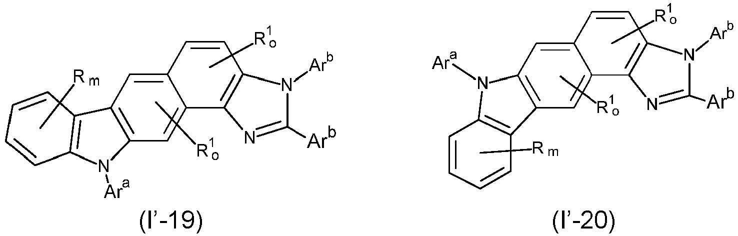 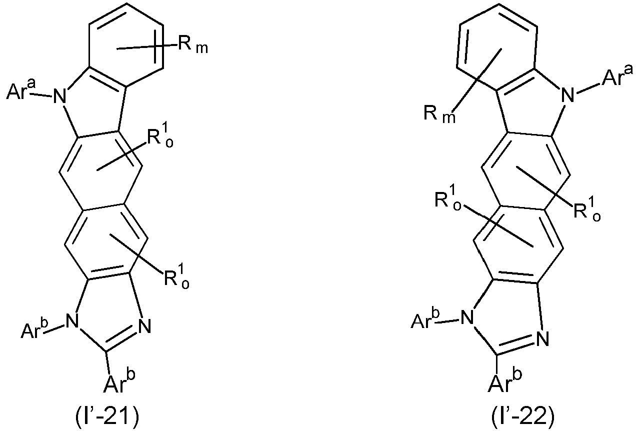 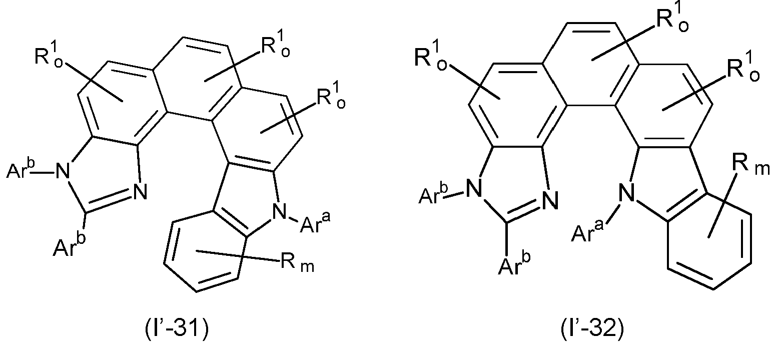 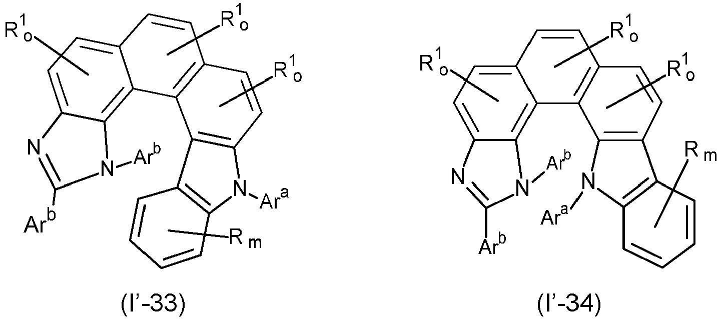 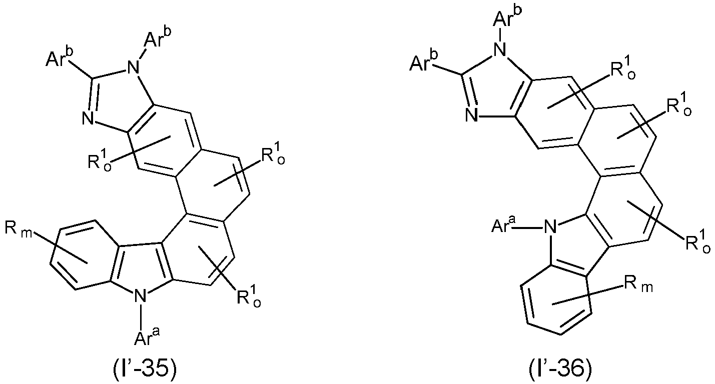 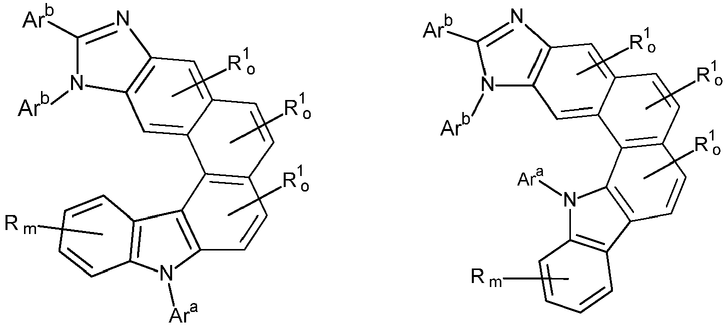 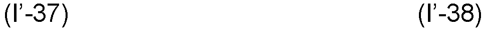 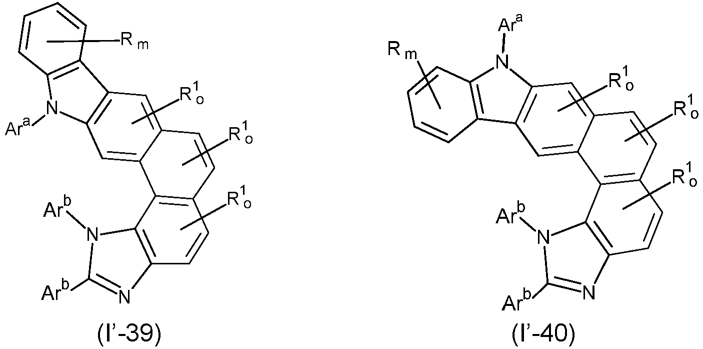 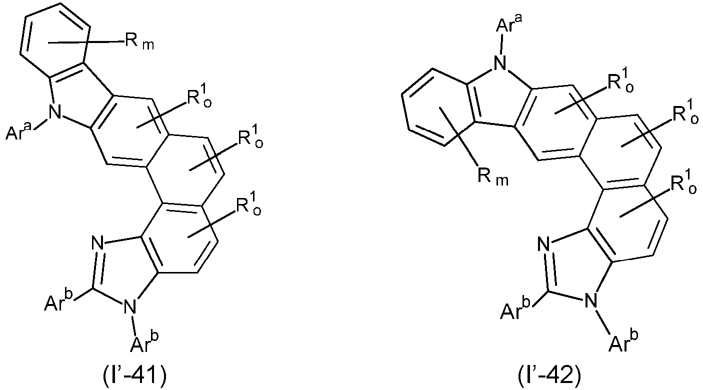 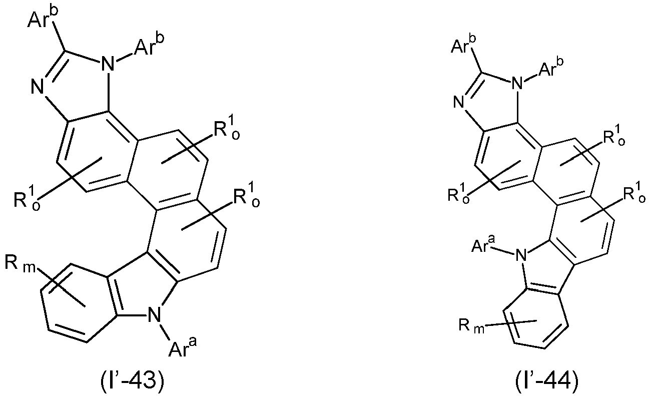 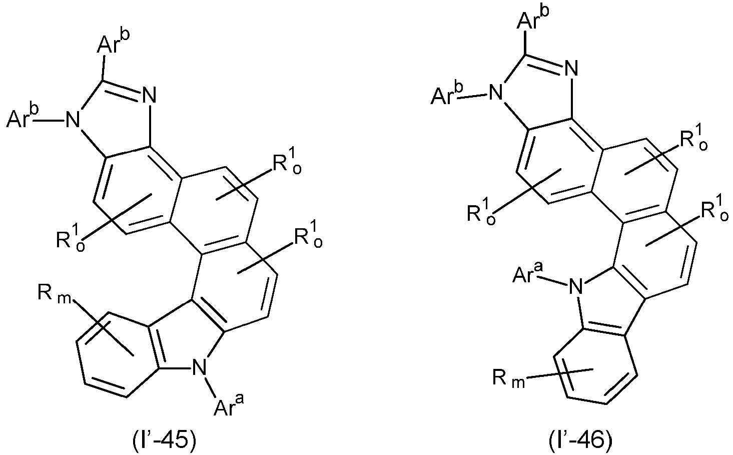 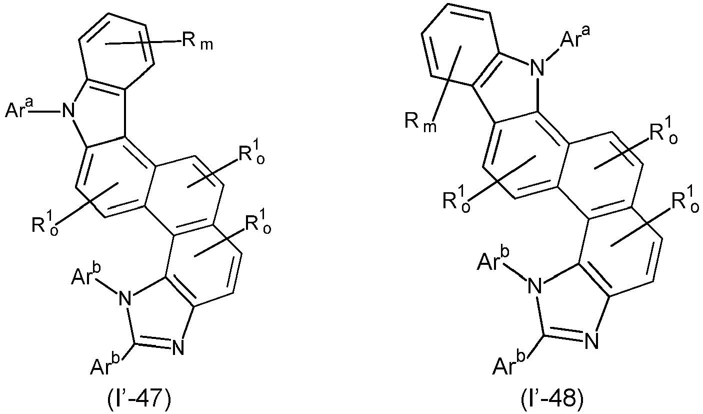 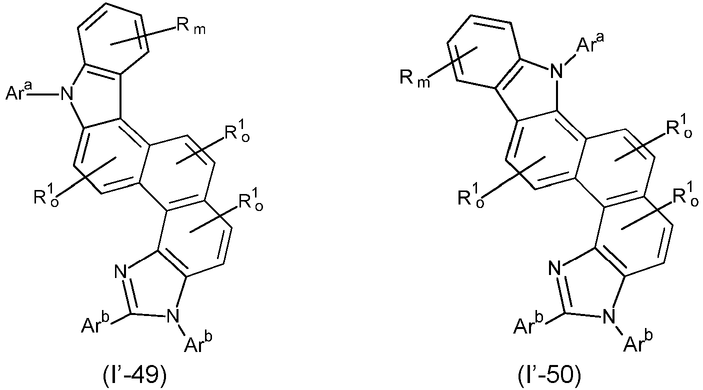 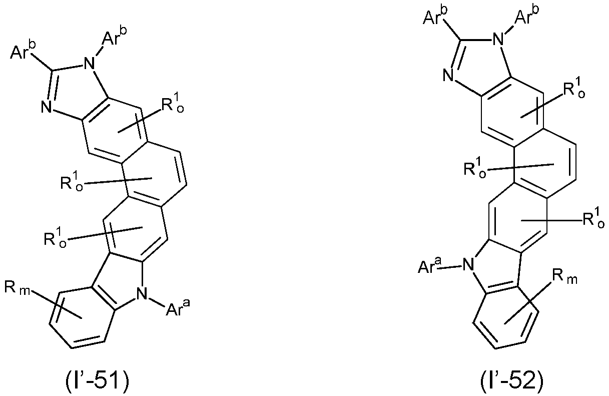 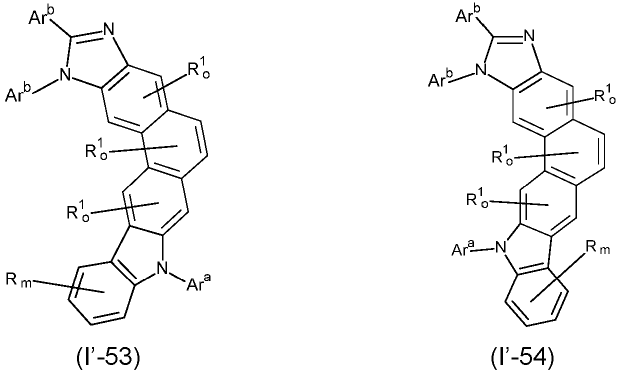 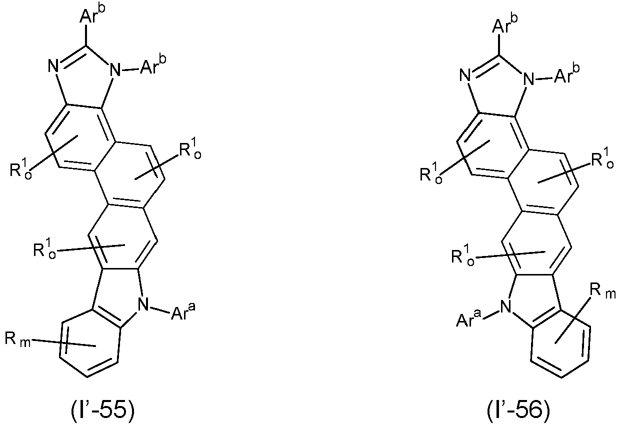 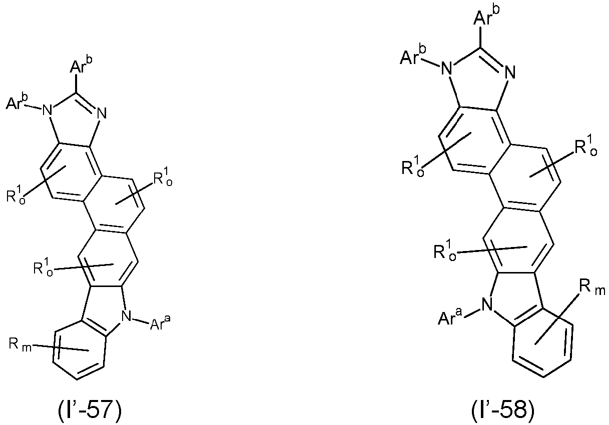 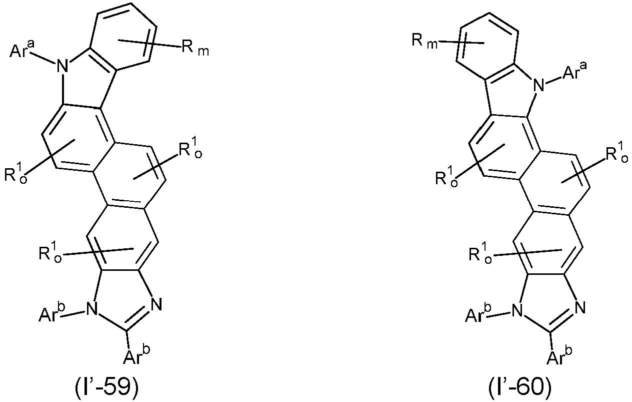 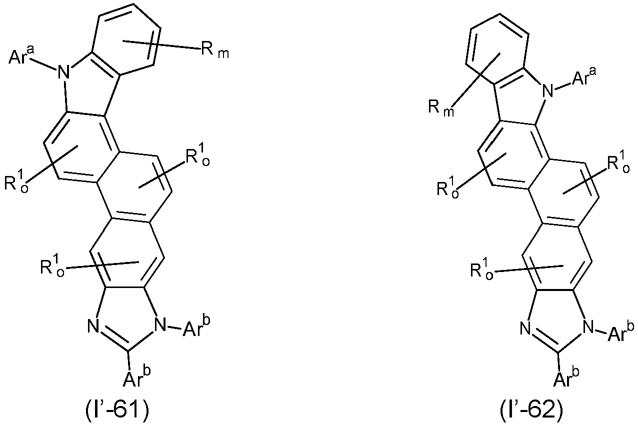 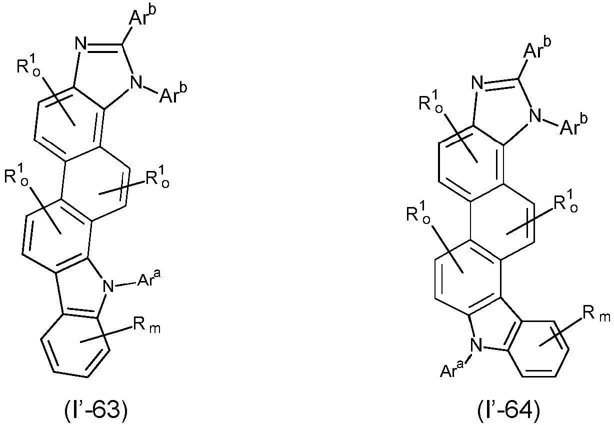 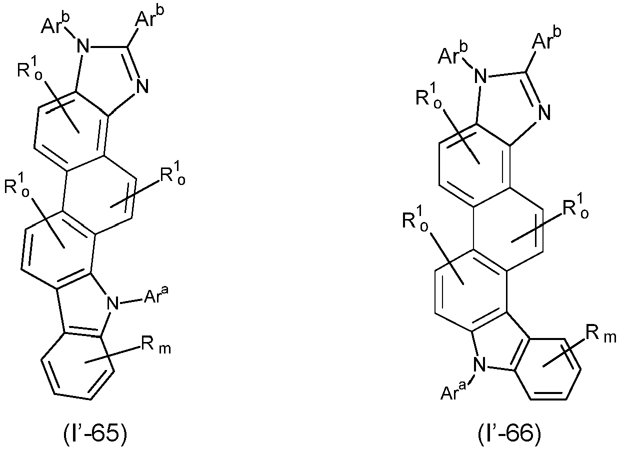 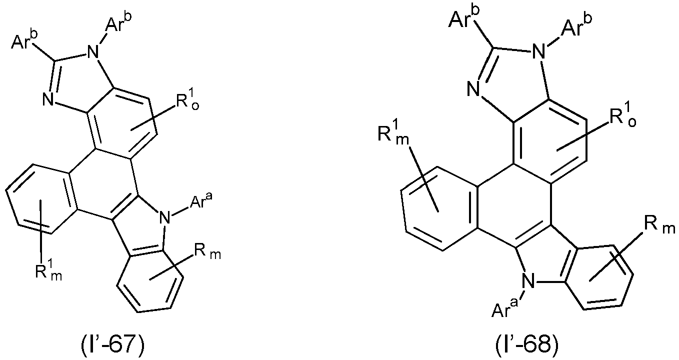 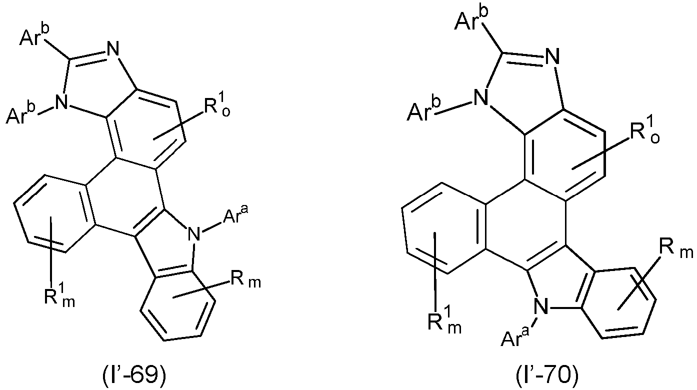 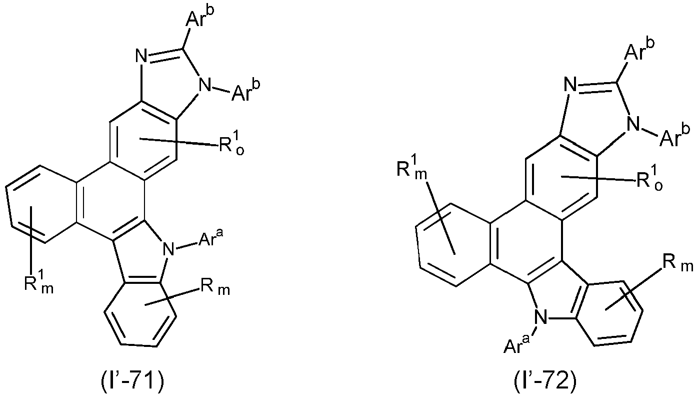 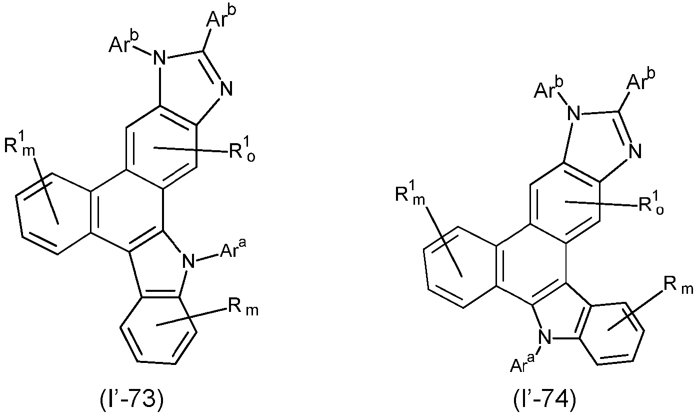 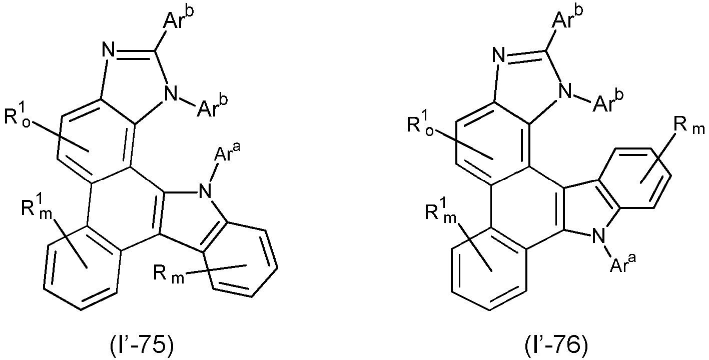 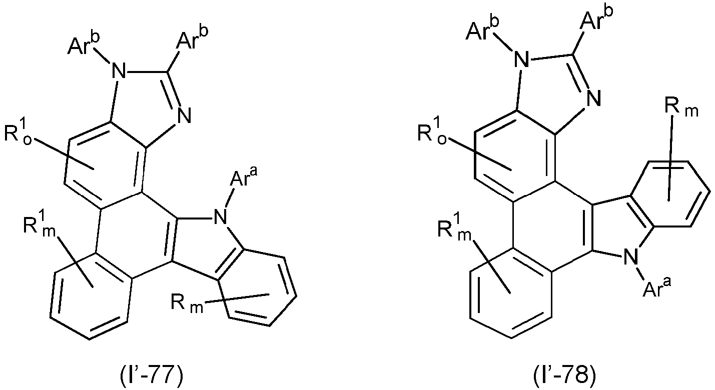 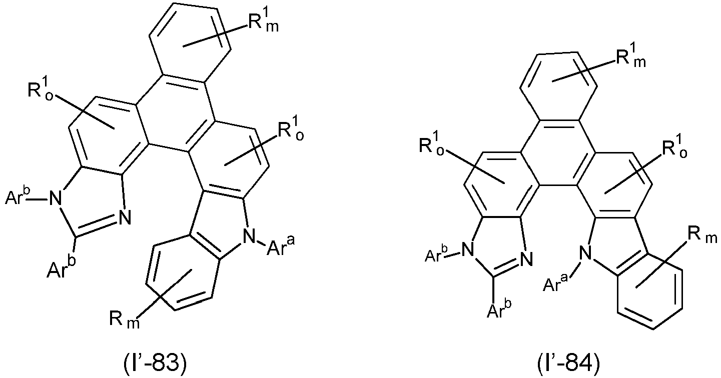 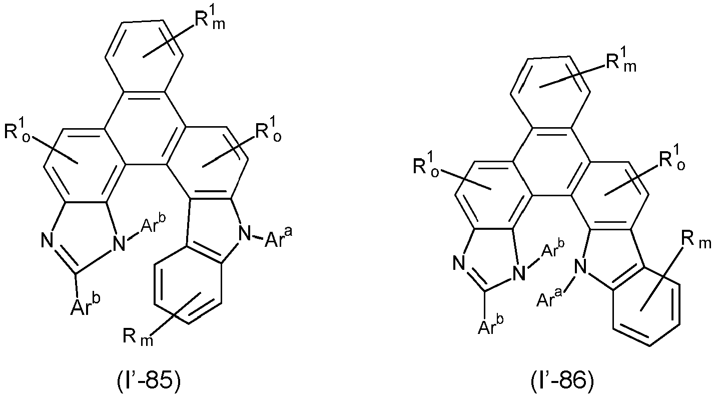 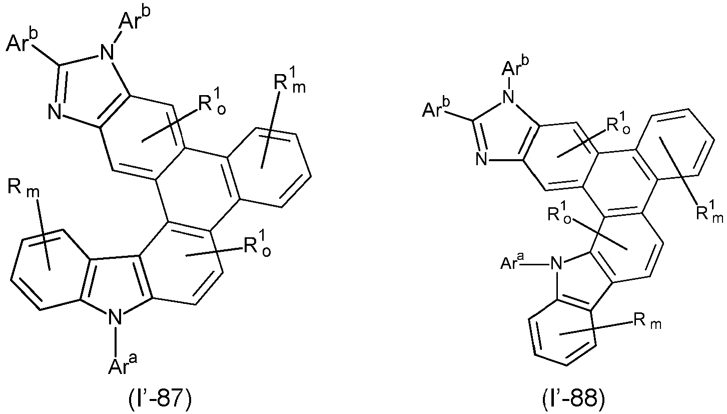 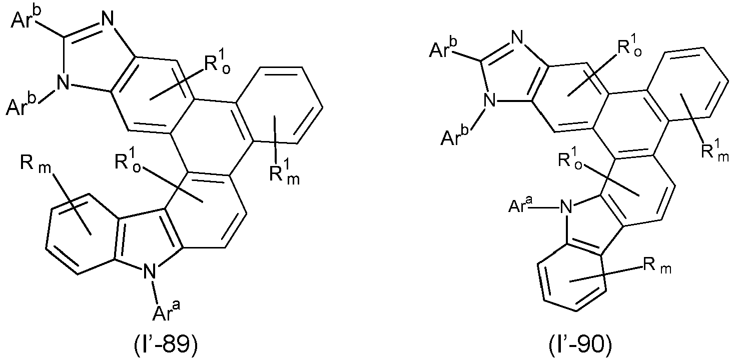 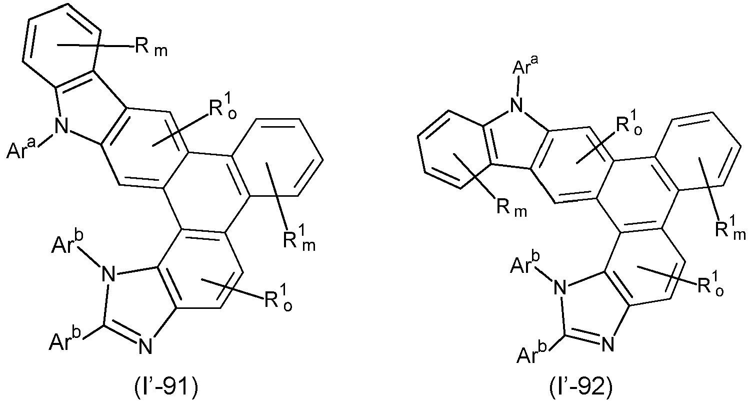 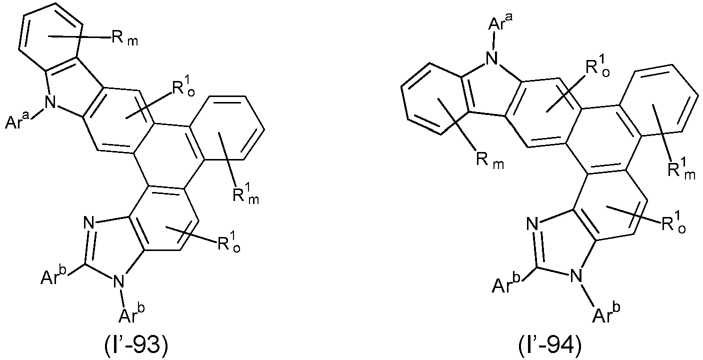 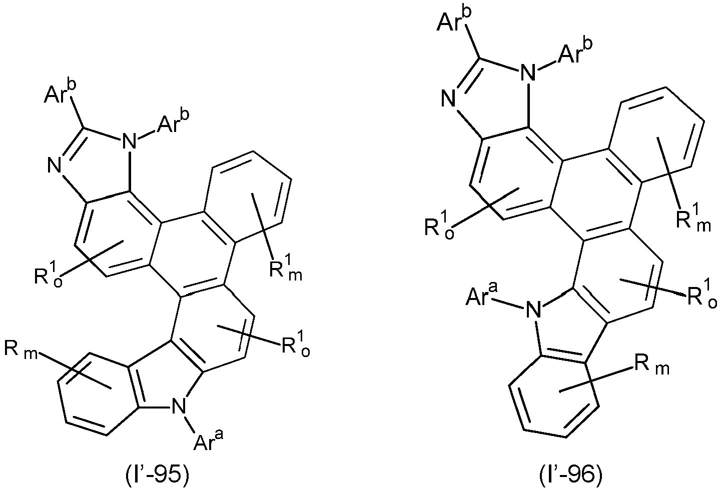 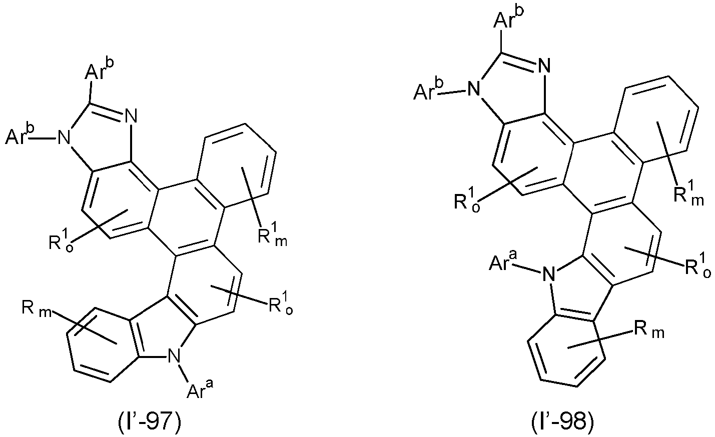 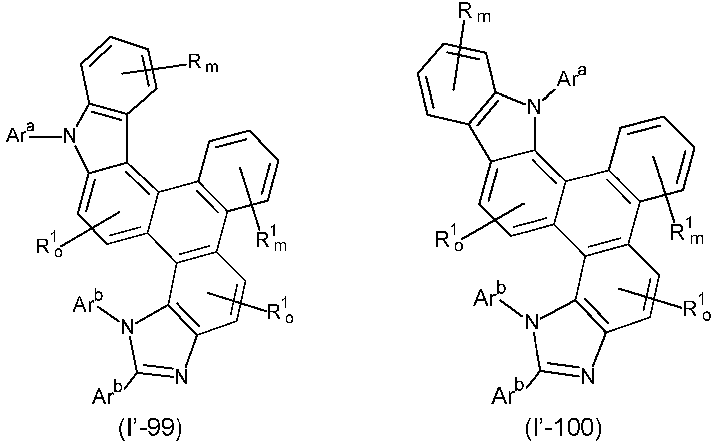 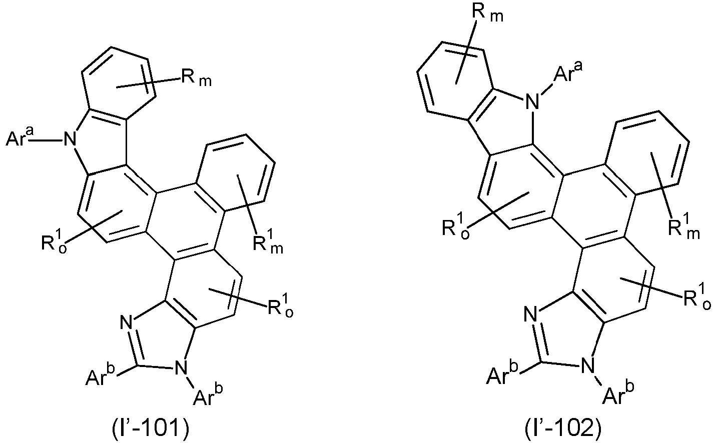 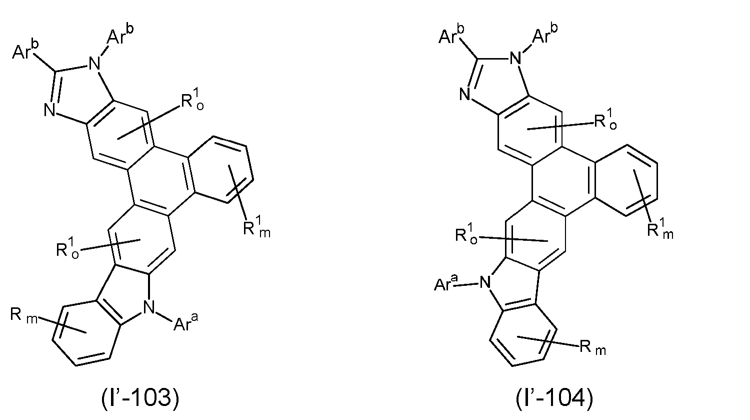 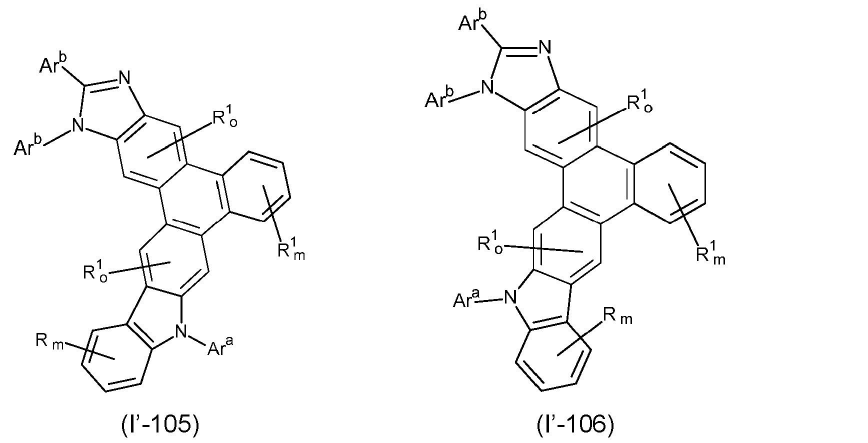 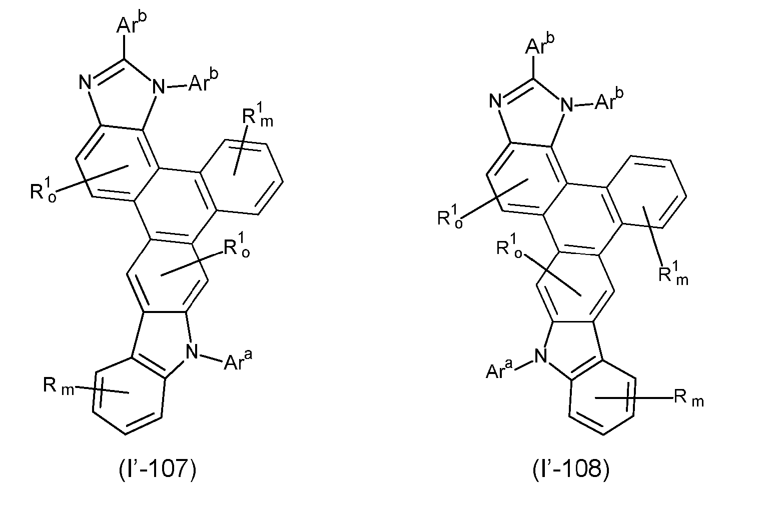 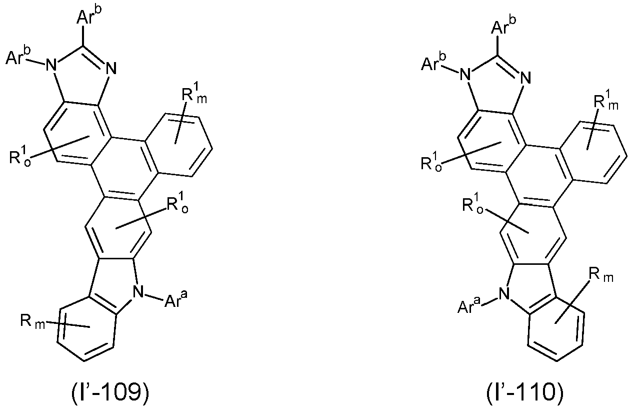 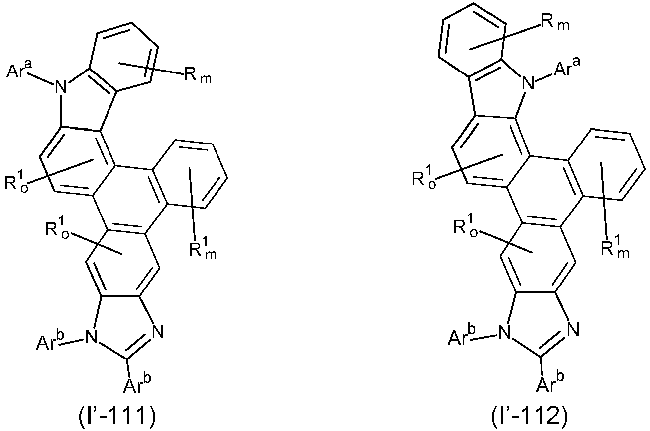 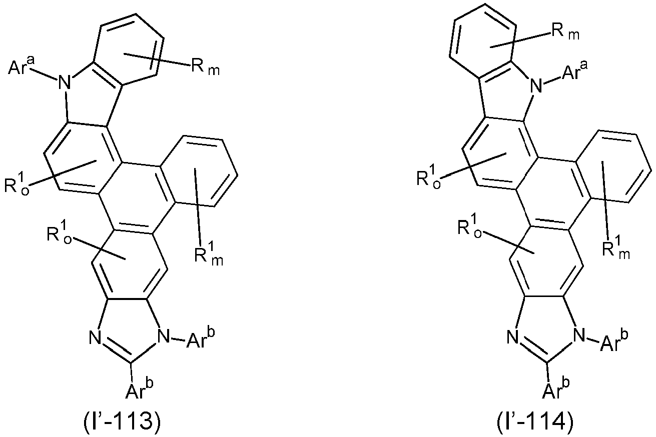 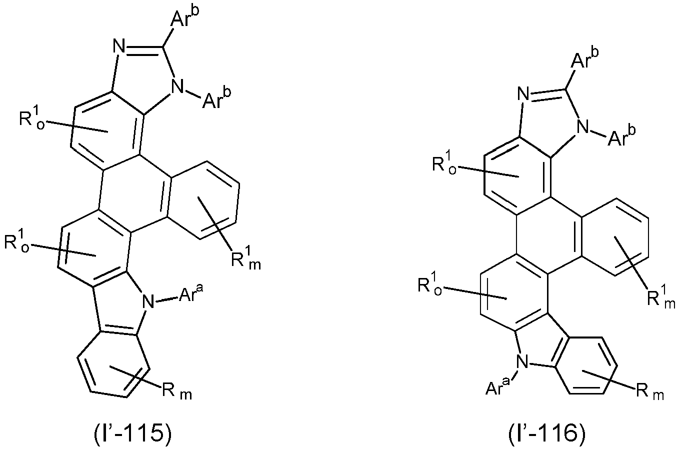 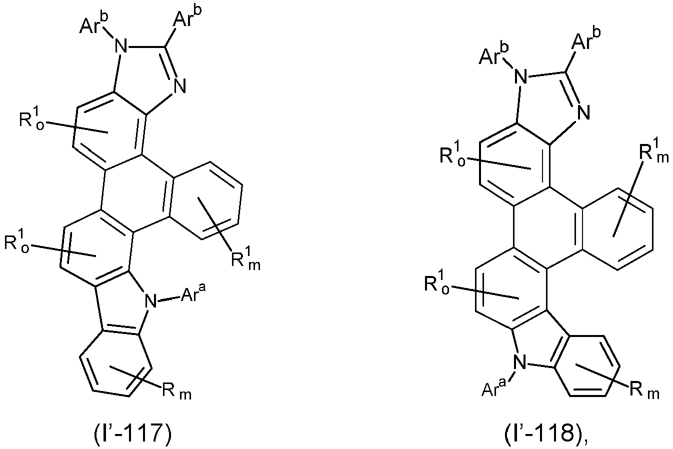 wobei für die verwendeten Symbole und Indizes gilt:
Ar^{a} ist bei jedem Auftreten gleich oder verschieden ein aromatisches oder heteroaromatisches Ringsystem mit 5 bis 40 aromatischen Ringatomen, welches mit einem oder mehreren Resten R² substituiert sein kann;
Ar^{b} ist bei jedem Auftreten gleich oder verschieden ein aromatisches oder heteroaromatisches Ringsystem mit 5 bis 40 aromatischen Ringatomen, welches mit einem oder mehreren Resten R³ substituiert sein kann;
R ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)2, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, das jeweils durch einen oder mehrere Reste R⁴ substituiert sein kann; dabei können zwei Reste R oder ein Rest R mit einem Rest R¹, R² auch miteinander ein aliphatisches, heteroaliphatisches, aromatisches oder heteroaromatisches Ringsystem bilden;
R¹ ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, das jeweils durch einen oder mehrere Reste R⁴ substituiert sein kann; dabei können zwei Reste R¹ oder ein Rest R¹ mit einem Rest R, R², R³ auch miteinander ein aliphatisches, heteroaliphatisches, aromatisches oder heteroaromatisches Ringsystem bilden;
R² ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R₄)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, das jeweils durch einen oder mehrere Reste R⁴ substituiert sein kann; dabei können zwei Reste R² oder ein Rest R² mit einem Rest R, R¹, R³ auch miteinander ein aromatisches, heteroaromatisches, aliphatisches oder heteroaliphatisches Ringsystem bilden;
R³ ist bei jedem Auftreten gleich oder verschieden H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R⁴)₂, C=O, NR⁴, O, S oder CONR⁴ ersetzt sein können, oder ein aromatisches oder heteroaromatisches Ringsystem mit 5 bis 60 aromatischen Ringatomen, das jeweils durch einen oder mehrere Reste R⁴ substituiert sein kann; dabei können zwei Reste R³ oder ein Rest R³ mit einem Rest R¹, R² auch miteinander ein aromatisches, heteroaromatisches, aliphatisches oder heteroaliphatisches Ringsystem bilden;
Ar' ist bei jedem Auftreten gleich oder verschieden ein aromatisches oder heteroaromatisches Ringsystem mit 5 bis 40 aromatischen Ringatomen, welches mit einem oder mehreren Resten R⁴ substituiert sein kann;
R⁴ ist bei jedem Auftreten gleich oder verschieden H, D, F, Cl, Br, I, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)_{2R}⁵, OSO₂R⁵, eine geradkettige Alkyl- gruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁵ substituiert sein kann, wobei eine oder mehrere nicht benachbarte CH₂-Gruppen durch Si(R⁵)₂, C=O, NR⁵, O, S oder CONR⁵ ersetzt sein können, oder ein aromatisches oder heteroaromatisches Ringsystem mit 5 bis 40 aromatischen Ringatomen, das jeweils durch einen oder mehrere Reste R⁵ substituiert sein kann; dabei können zwei oder mehrere Reste R⁴ miteinander ein aromatisches, heteroaromatisches, aliphatisches oder heteroaliphatisches Ringsystem bilden;
R⁵ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch F ersetzt sein können,
der Index o ist 0, 1 oder 2 und der Index m ist 0, 1, 2, 3 oder 4.

2. Verbindungen nach Anspruch 1, **dadurch gekennzeichnet, dass** zwei benachbarte Gruppen R¹ kein kondensiertes aromatisches oder heteroaromatisches Ringsystem mit den Gruppen bilden, an die die Gruppen R¹ binden, wobei dies die Reste R⁴ oder R⁵ einschließt, durch die die Gruppen R¹ substituiert sein können.

3. Verbindungen nach Anspruch 1 oder 2, **dadurch gekennzeichnet, dass** Ar^{a} und/oder Ar^{b} gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R² beziehungsweise R³ substituiert sein können.

4. Verbindungen nach einem oder mehreren der Ansprüche 1 bis 3,
**dadurch gekennzeichnet, dass** R, R¹, R² und/oder R³ gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-75 und/oder die Gruppe Ar' gleich oder verschieden bei jedem Auftreten ausgewählt ist aus den Gruppen der folgenden Formeln Ar-1 bis Ar-75, 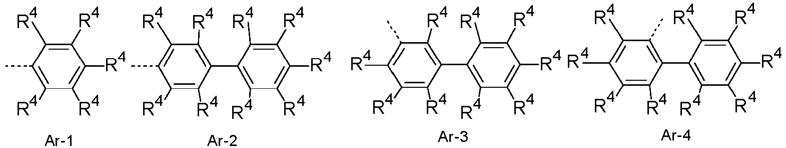 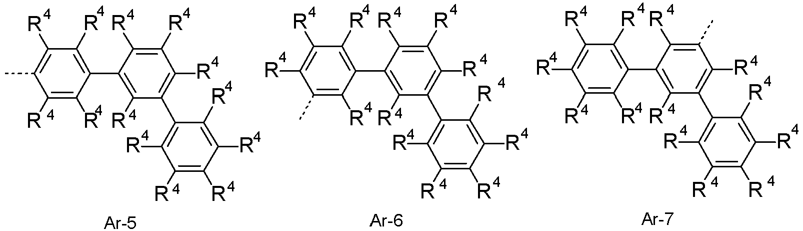 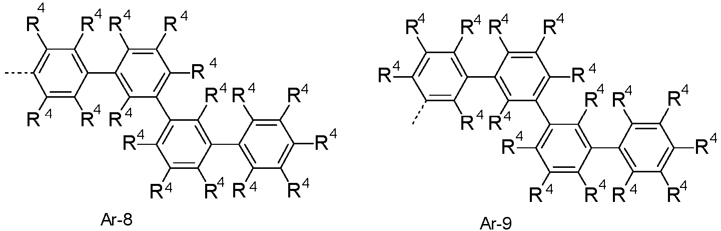 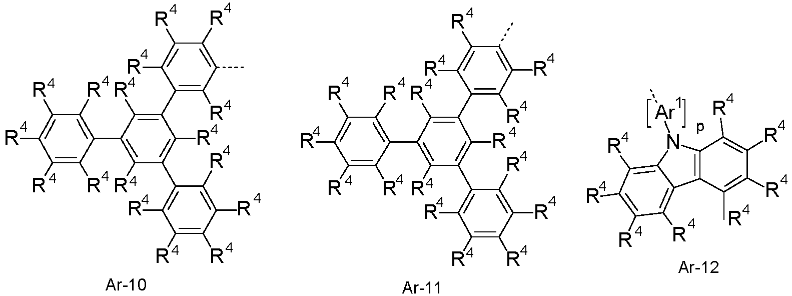 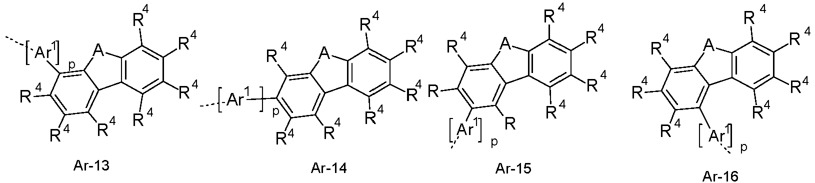 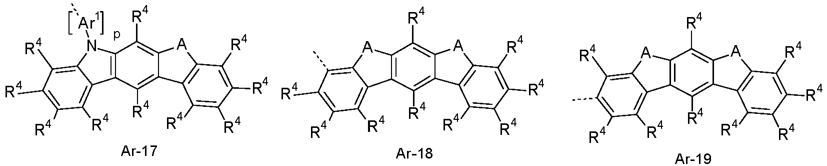 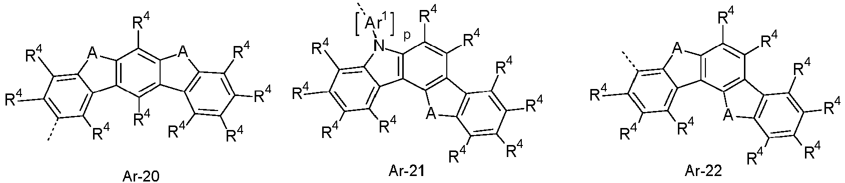 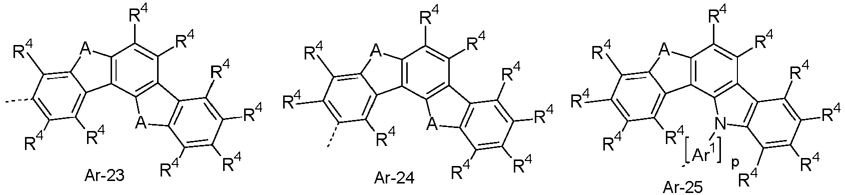 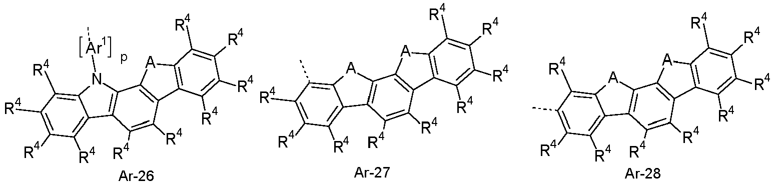 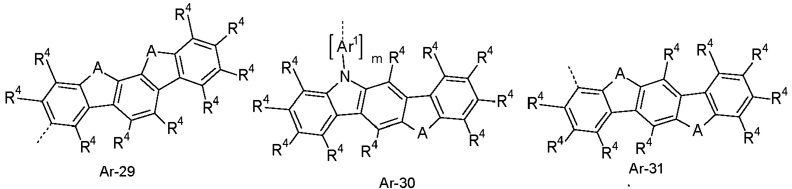 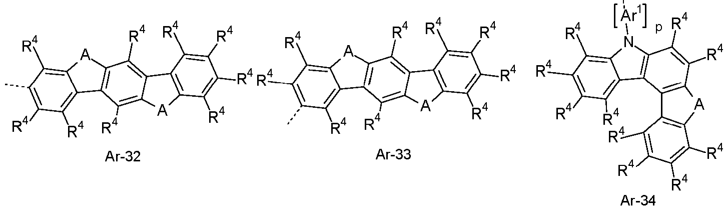 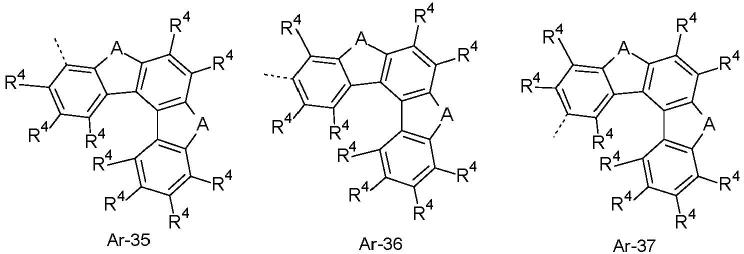 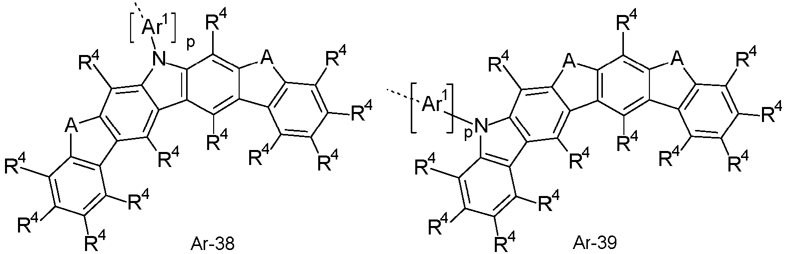 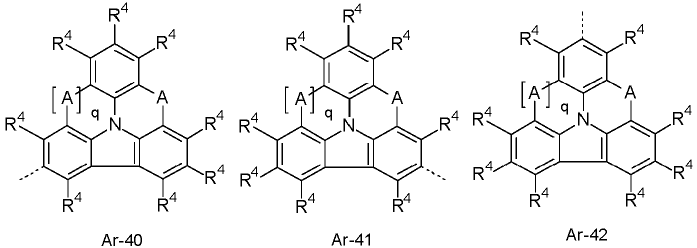 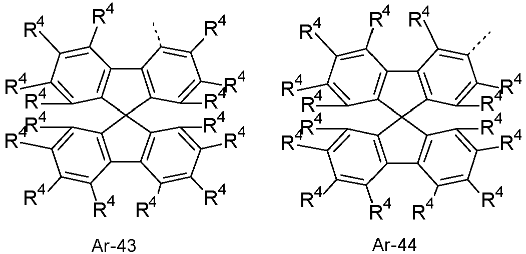 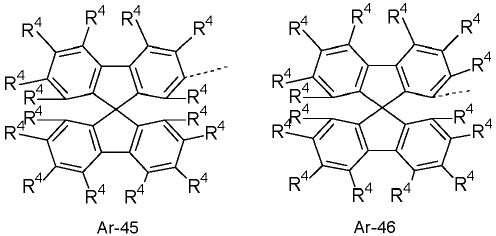 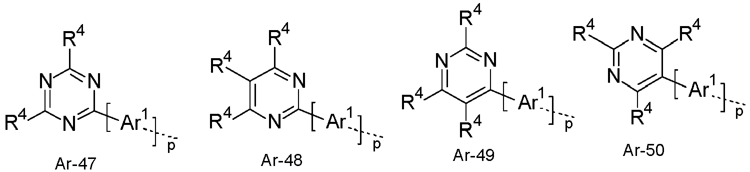 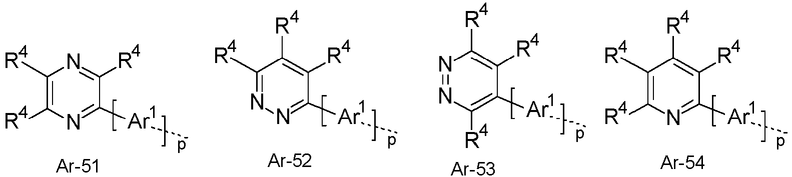 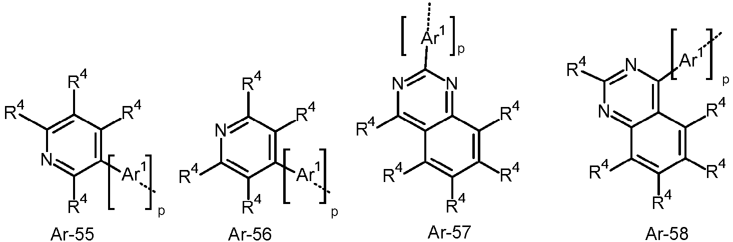 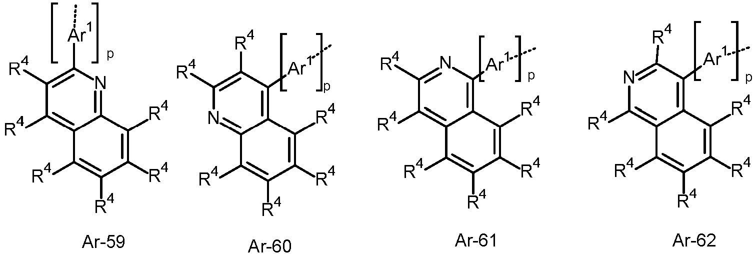 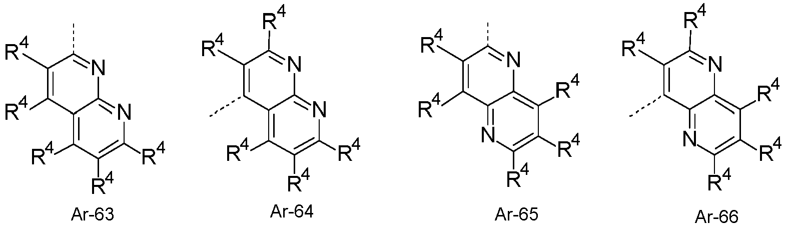 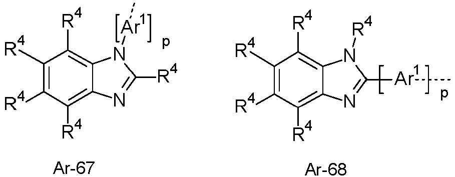 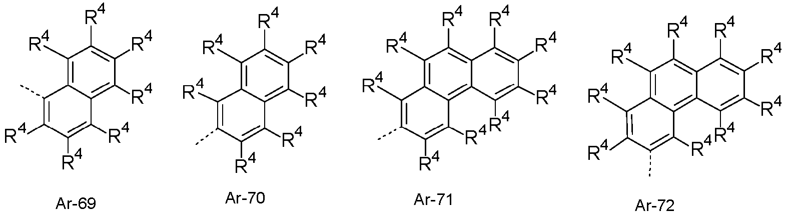 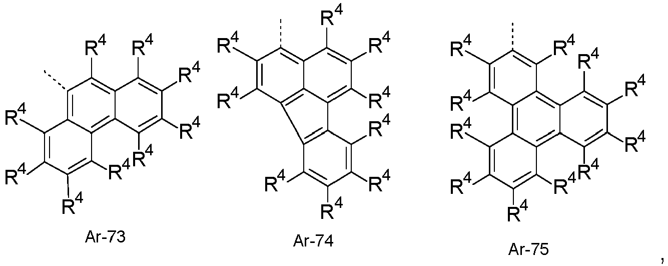 wobei R⁴ die oben genannten Bedeutungen in Anspruch 1 aufweist, die gestrichelte Bindung die Bindung an die entsprechende Gruppe darstellt und weiterhin gilt:
Ar¹ ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R⁴ substituiert sein kann;
A ist bei jedem Auftreten gleich oder verschieden C(R⁴)₂, NR⁴, O oder S;
p ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
q ist 0 oder 1, wobei q = 0 bedeutet, dass an dieser Position keine Gruppe A gebunden ist und an die entsprechenden Kohlenstoffatome stattdessen Reste R⁴ gebunden sind.

5. Verbindungen nach einem oder mehreren der Ansprüche 1 bis 4,
**dadurch gekennzeichnet, dass** die Verbindungen mindestens zwei Elektronenleitgruppen aufweisen, die ausgewählt sind aus Substituenten der in Anspruch 4 dargestellten Formeln (Ar-47) bis (Ar-51), (Ar-57), (Ar-58) und/oder (Ar-63) bis (Ar-68).

6. Verbindungen nach einem oder mehreren der Ansprüche 1 bis 5,
**dadurch gekennzeichnet, dass** der Rest Ar^{a} durch eine Gruppe der in Anspruch 4 dargestellten Formeln (Ar-47) bis (Ar-51), (Ar-57), (Ar-58) und/oder (Ar-63) bis (Ar-68) darstellbar ist, wobei die in den Formeln (Ar-47) bis (Ar-51), (Ar-57), (Ar-58) und/oder (Ar-63) bis (Ar-68) dargelegten Substituenten R⁴ durch R² zu ersetzen sind.

7. Verfahren zur Herstellung einer Verbindung nach einem oder mehreren der Ansprüche 1 bis 6, **dadurch gekennzeichnet, dass** eine aromatische oder heteroaromatische Verbindung mittels einer Kupplungsreaktion mit einer aromatischen oder heteroaromatischen Diaminoverbindung umgesetzt wird.

8. Zusammensetzung, enthaltend mindestens eine Verbindung nach einem oder mehreren der Ansprüche 1 bis 6 und mindestens ein weiteres Matrixmaterial, wobei das weitere Matrixmaterial ausgewählt ist aus Verbindungen gemäß einer der Formeln (H-1), (H-2), (H-3), (H-4) oder (H-5), 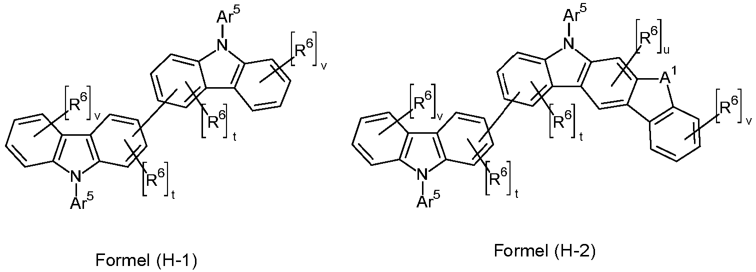 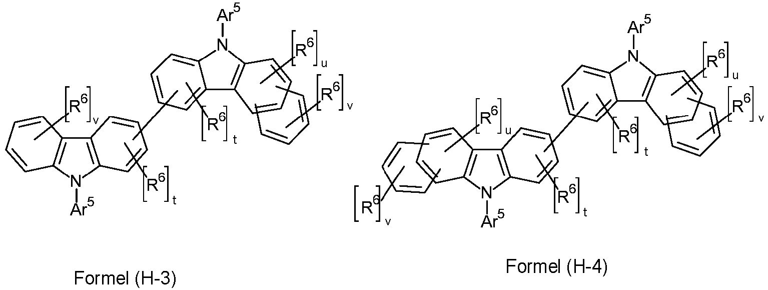 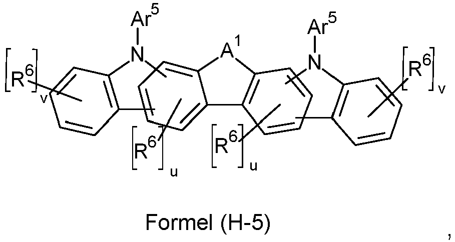 wobei für die verwendeten Symbole und Indizes gilt:
R⁶ ist bei jedem Auftreten gleich oder verschieden H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁷ substituiert sein kann und wobei eine oder mehrere nicht benachbarte CH₂-Gruppen durch Si(R⁷)₂, C=O, NR⁷, O, S oder CONR⁷ ersetzt sein können, oder ein aromatisches oder heteroaromatisches Ringsystem mit 5 bis 60 aromatischen Ringatomen, das jeweils durch einen oder mehrere Reste R⁷ substituiert sein kann; dabei können zwei Reste R⁶ auch miteinander ein aromatisches, heteroaromatisches, aliphatisches oder heteroaliphatisches Ringsystem bilden;
Ar" ist bei jedem Auftreten gleich oder verschieden ein aromatisches oder heteroaromatisches Ringsystem mit 5 bis 40 aromatischen Ringatomen, welches mit einem oder mehreren Resten R⁷ substituiert sein kann;
A¹ ist C(R⁷)₂, NR⁷, O oder S;
Ar⁵ steht gleich oder verschieden bei jedem Auftreten für ein aromatisches oder heteroaromatisches Ringsystem mit 5 bis 40 aromatischen Ringatomen, welches mit einem oder mehreren Resten R⁷ substituiert sein kann;
R⁷ ist bei jedem Auftreten gleich oder verschieden H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁸ substituiert sein kann, wobei eine oder mehrere nicht benachbarte CH₂-Gruppen durch Si(R⁸)₂, C=O, NR⁸, O, S oder CONR⁸ ersetzt sein können, oder ein aromatisches oder heteroaromatisches Ringsystem mit 5 bis 40 aromatischen Ringatomen, das jeweils durch einen oder mehrere Reste R⁸ substituiert sein kann; dabei können zwei oder mehrere Reste R⁷ miteinander ein aromatisches, heteroaromatisches, aliphatisches oder heteroaliphatisches Ringsystem bilden;
R⁸ ist bei jedem Auftreten gleich oder verschieden H, D, F oder ein aliphatischer, aromatischer oder heteroaromatischer organischer Rest, mit 1 bis 20 C-Atomen, in dem auch ein oder mehrere H-Atome durch F ersetzt sein können;
v ist bei jedem Auftreten gleich oder verschieden 0, 1, 2, 3 oder 4;
t ist bei jedem Auftreten gleich oder verschieden 0, 1, 2, oder 3;
u ist bei jedem Auftreten gleich oder verschieden 0, 1 oder 2.

9. Zusammensetzung, enthaltend mindestens eine Verbindung nach einem oder mehreren der Ansprüche 1 bis 6 und mindestens ein weiteres Matrixmaterial, wobei das weitere Matrixmaterial ausgewählt ist aus Verbindungen gemäß einer der Formeln (H-6), (H-7), (H-8), (H-9), (H-10), (H-11), (H-12) oder (H-13), 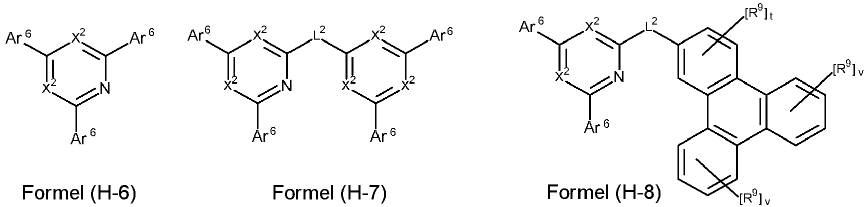 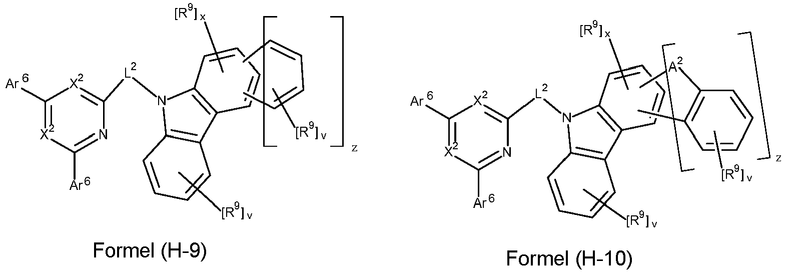 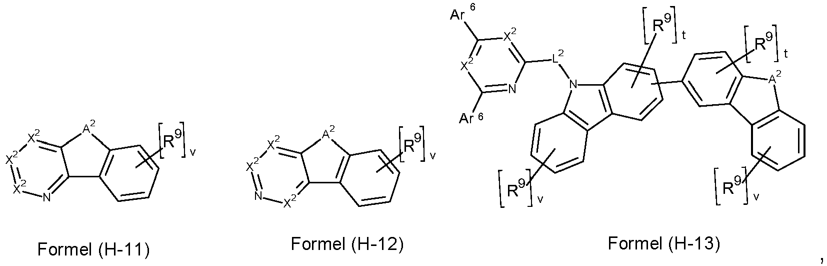 wobei für die verwendeten Symbole und Indizes gilt:
X² steht für N oder CR⁹, mit der Maßgabe, dass nicht mehr als zwei der Gruppen X² in einem Cyclus für N stehen;
L² steht für eine Verbindungsgruppe, die ausgewählt ist aus einer Bindung oder einem aromatischen oder heteroaromatischen Ringsystem mit 5 bis 40 aromatischen Ringatomen, welches mit einem oder mehreren Resten R⁹ substituiert sein kann;
Ar⁶ steht gleich oder verschieden bei jedem Auftreten für ein aromatisches oder heteroaromatisches Ringsystem mit 5 bis 40 aromatischen Ringatomen, welches mit einem oder mehreren Resten R¹⁰ substituiert sein kann;
R⁹ ist bei jedem Auftreten gleich oder verschieden H, D, F, Cl, Br, I, N(R¹⁰)₂, N(Ar‴)₂, CN, NO₂, OR¹⁰, SR¹⁰, COOR¹⁰, C(=O)N(R¹⁰)₂, Si(R¹⁰)₃, B(OR¹⁰)₂, C(=O)R¹⁰, P(=O)(R¹⁰)₂, S(=O)R¹⁰, S(=O)₂R¹⁰, OSO₂R¹⁰, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R⁴ substituiert sein kann und wobei eine oder mehrere nicht benachbarte CH₂-Gruppen durch Si(R¹⁰)₂, C=O, NR¹⁰, O, S oder CONR¹⁰ ersetzt sein können, oder ein aromatisches oder heteroaromatisches Ringsystem mit 5 bis 60 aromatischen Ringatomen, das jeweils durch einen oder mehrere Reste R¹⁰ substituiert sein kann; dabei können zwei Reste R⁹ auch miteinander ein aromatisches, heteroaromatisches, aliphatisches oder heteroaliphatisches Ringsystem bilden;
Ar‴ ist bei jedem Auftreten gleich oder verschieden ein aromatisches oder heteroaromatisches Ringsystem mit 5 bis 40 aromatischen Ringatomen, welches mit einem oder mehreren Resten R¹⁰ substituiert sein kann;
A² ist C(R¹⁰)₂, NR¹⁰, O oder S;
R¹⁰ ist bei jedem Auftreten gleich oder verschieden H, D, F, Cl, Br, I, N(R¹¹)₂, CN, NO₂, OR¹¹, SR¹¹, Si(R¹¹)₃, B(OR¹¹)₂, C(=O)R¹¹, P(=O)(R¹¹)₂, S(=O)R¹¹, S(=O)₂R¹¹, OSO₂R¹¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹¹ substituiert sein kann, wobei eine oder mehrere nicht benachbarte CH₂-Gruppen durch Si(R¹¹)₂, C=O, NR¹¹, O, S oder CONR¹¹ ersetzt sein können, oder ein aromatisches oder heteroaromatisches Ringsystem mit 5 bis 40 aromatischen Ringatomen, das jeweils durch einen oder mehrere Reste R¹¹ substituiert sein kann; dabei können zwei oder mehrere Reste R¹⁰ miteinander ein aromatisches, heteroaromatisches, aliphatisches oder heteroaliphatisches Ringsystem bilden;
R¹¹ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch F ersetzt sein können;
v ist bei jedem Auftreten gleich oder verschieden 0, 1, 2, 3 oder 4;
t ist bei jedem Auftreten gleich oder verschieden 0, 1, 2, oder 3;
x ist bei jedem Auftreten gleich oder verschieden 0, 1, 2, 3 oder 4;
z ist bei jedem Auftreten gleich oder verschieden 0, 1 oder 2, wobei die Summe aus x und z höchstens 4 ist.

10. Zusammensetzung nach Anspruch 8 oder 9, **dadurch gekennzeichnet, dass** eine Verbindung der Ansprüche 1 bis 6 in der Zusammensetzung einen Massenanteil im Bereich von 10 Gew.-% bis 95 Gew.-%, bezogen auf die Gesamtmasse der Zusammensetzung, aufweist.

11. Formulierung, enthaltend mindestens eine Verbindung nach einem oder mehreren der Ansprüche 1 bis 6 und/oder mindestens eine Zusammensetzung nach einem oder mehreren der Ansprüchen 8 bis 10 und mindestens eine weitere Verbindung, wobei die weitere Verbindung ausgewählt ist aus einem oder mehreren Lösemitteln.

12. Verwendung von Verbindungen nach einem oder mehreren der Ansprüche 1 bis 6 und/oder einer Zusammensetzung nach einem oder mehreren der Ansprüchen 8 bis 10 in einer elektronischen Vorrichtung.

13. Elektronische Vorrichtung enthaltend mindestens eine Verbindung nach einem oder mehreren der Ansprüche 1 bis 6 und/oder eine Zusammensetzung nach einem oder mehreren der Ansprüchen 8 bis 10.

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 6 als Matrixmaterial in einer emittierenden Schicht und/oder in einer Elektronentransportschicht und/oder in einer Lochblockierschicht und/oder in einer Lochtransportschicht und/oder in einer Elektronenblockierschicht, eingesetzt werden.

15. Elektronische Vorrichtung nach Anspruch 14, **dadurch gekennzeichnet, dass** die Verbindung nach einem oder mehreren der Ansprüche 1 bis 6 als Matrixmaterial für phosphoreszierende Emitter eingesetzt wird in Kombination mit einem weiteren Matrixmaterial, wobei das weitere Matrixmaterial ausgewählt ist aus Verbindungen gemäß einer der Formeln (H-1), (H-2), (H-3), (H-4) oder (H-5), 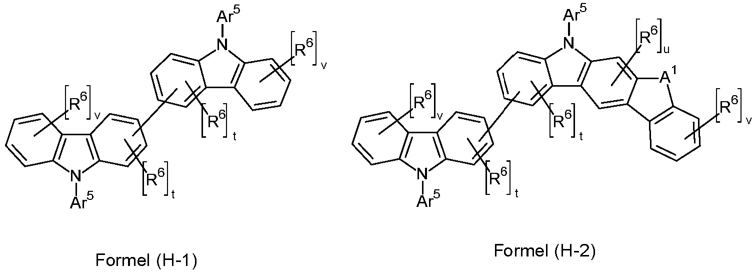 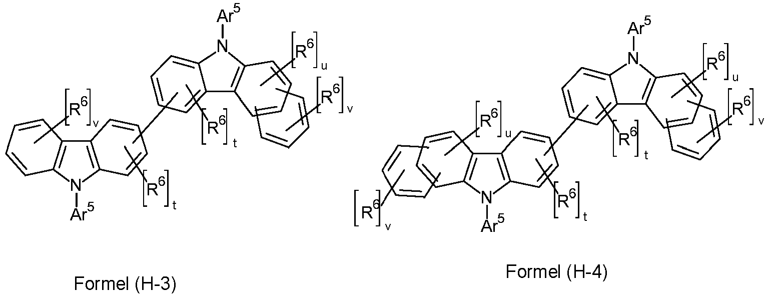 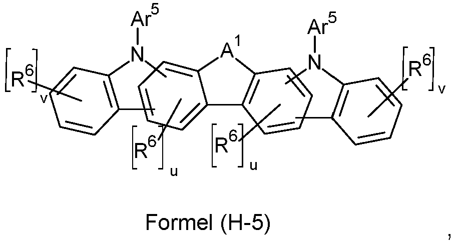
wobei die Symbole A¹, Ar⁵ und R⁶ und die Indices s, t und u die in Anspruch 8 genannten Bedeutungen aufweisen,
und/oder das das weitere Matrixmaterial ausgewählt ist aus Verbindungen gemäß einer der Formeln (H-6), (H-7), (H-8), (H-9), (H-10), (H-11), (H-12) oder (H-13), 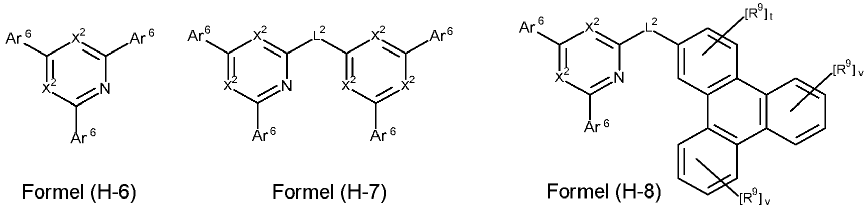 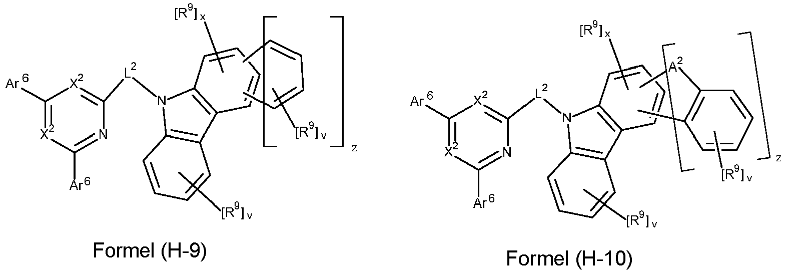 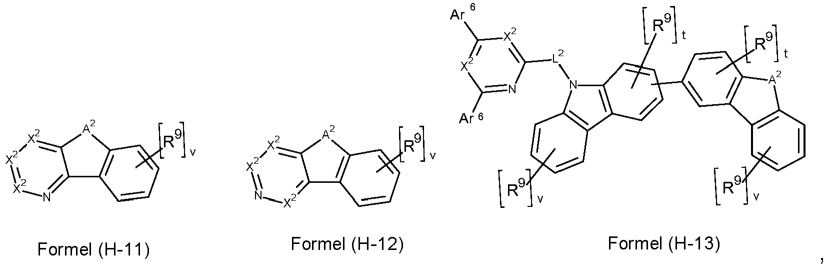
wobei die Symbole X², L², A², Ar⁶ und R⁹ und die Indices v, t, x und z die in Anspruch 9 genannten Bedeutungen aufweisen.

## Claims

1. Compounds of the formulae (I'-5) to (I'-22), (I'-31) to (I'-78) and (I'-83) to (I'-118), 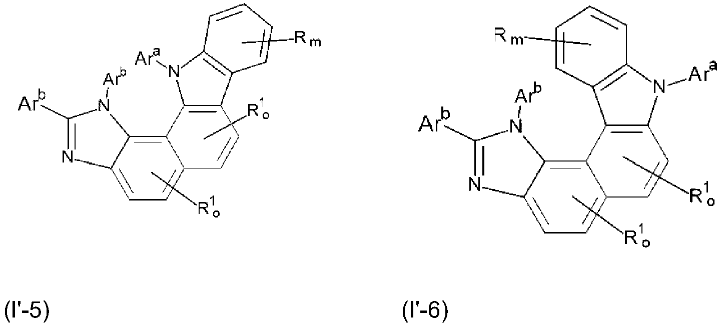 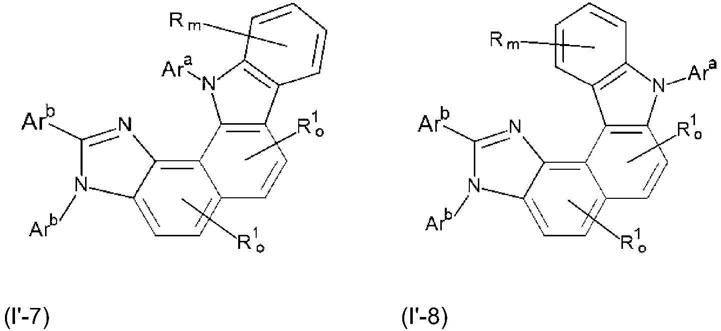 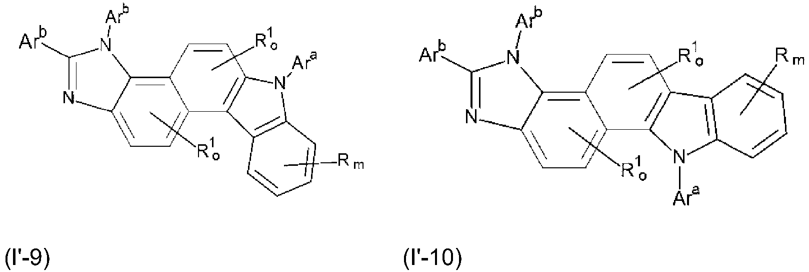 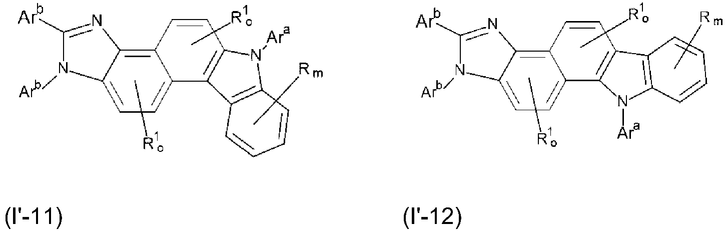 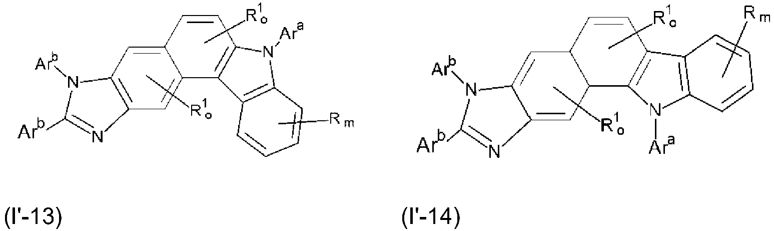 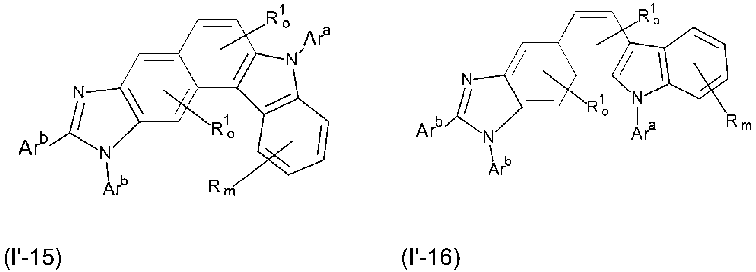 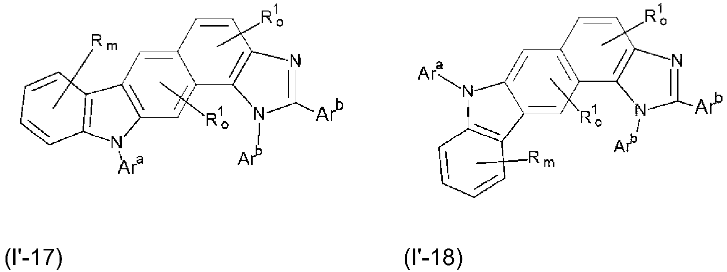 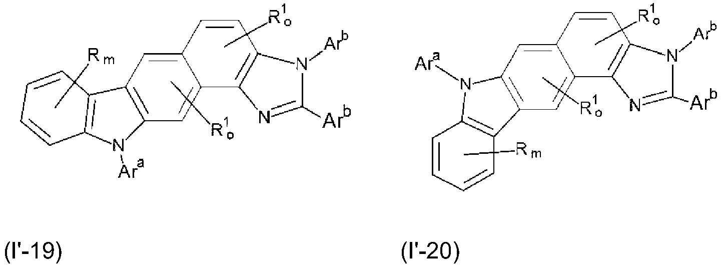 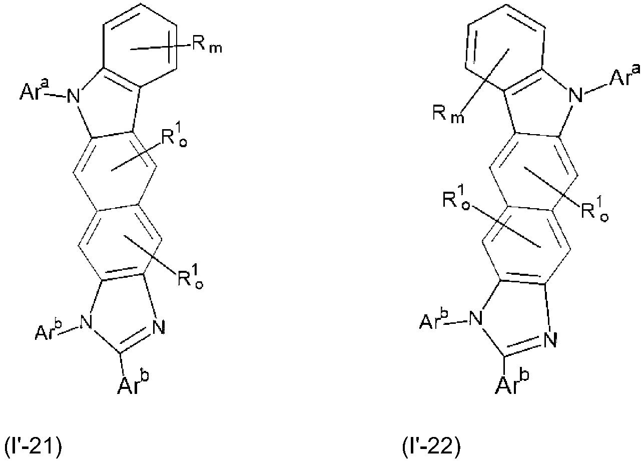 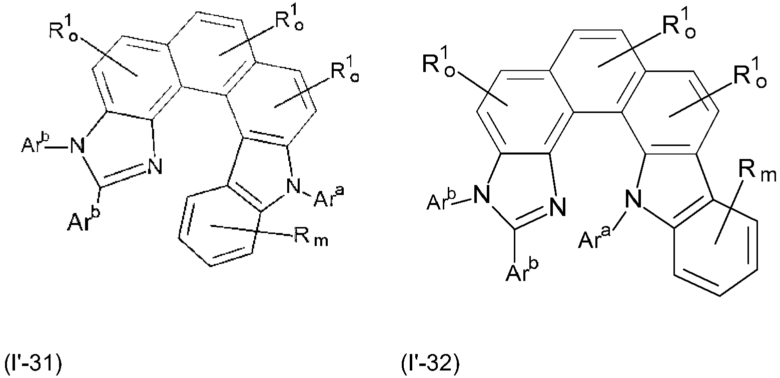 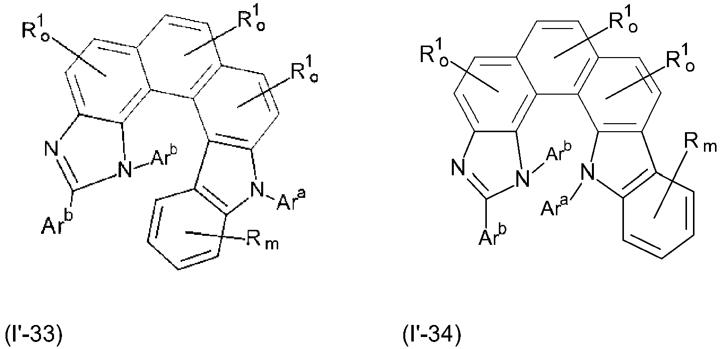 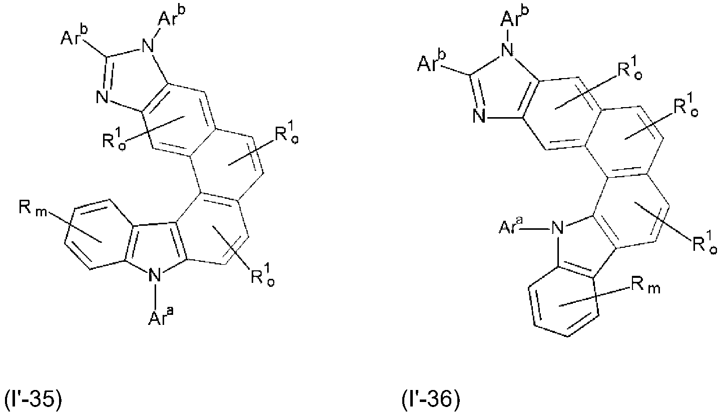 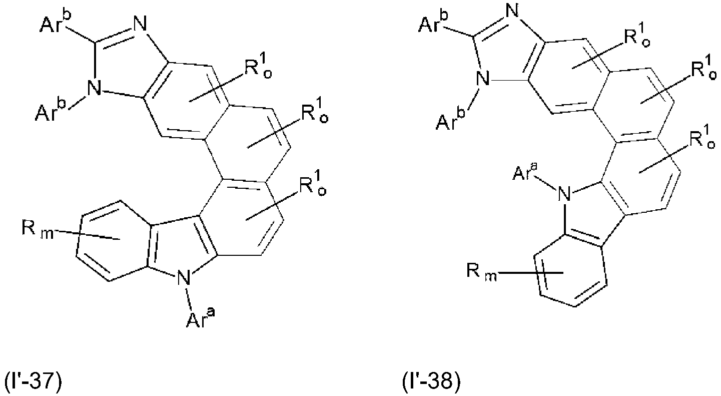 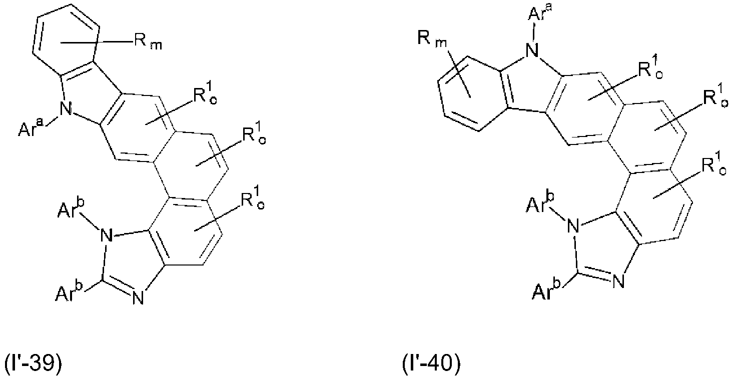 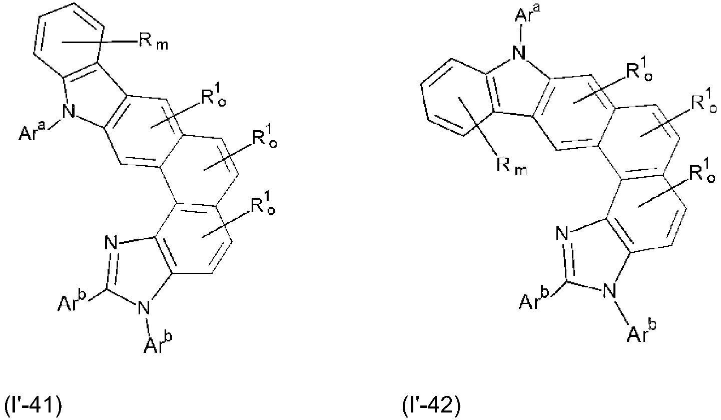 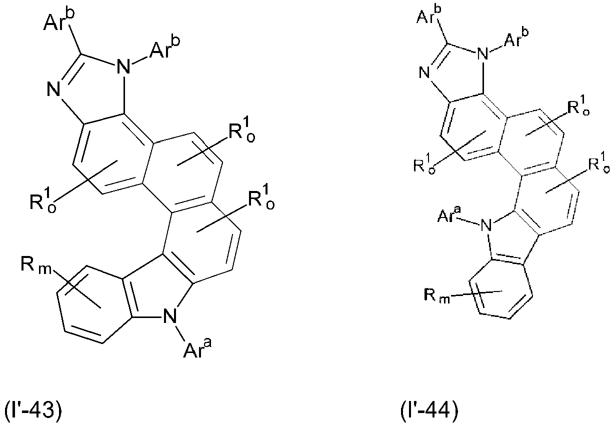 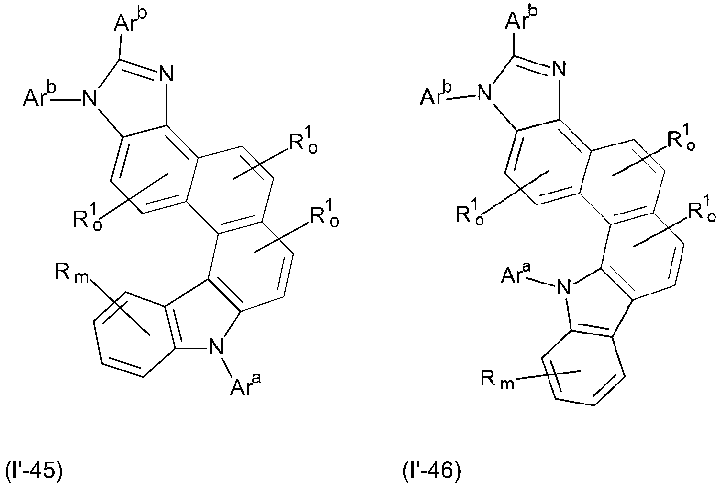 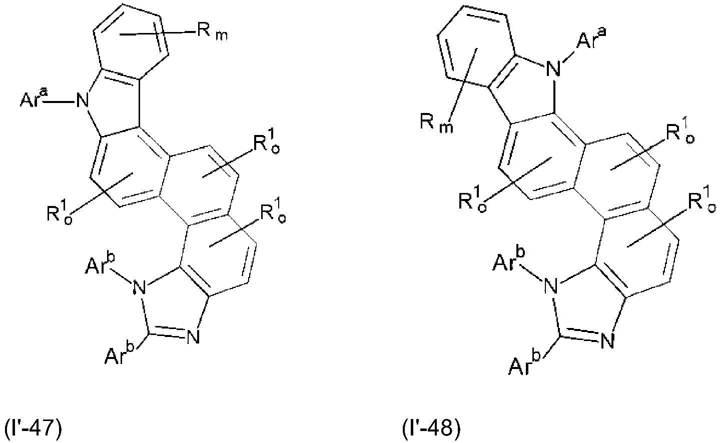 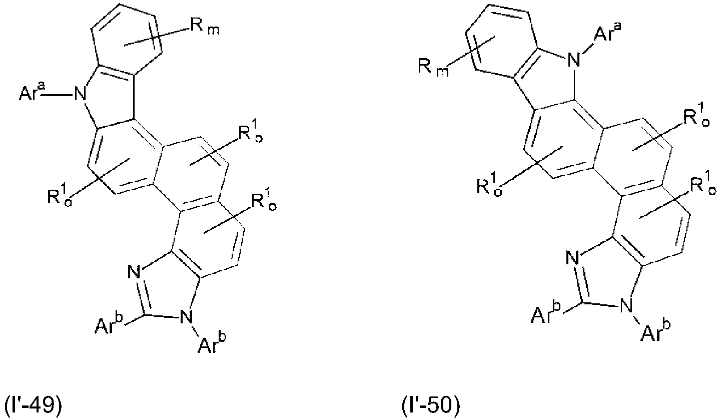 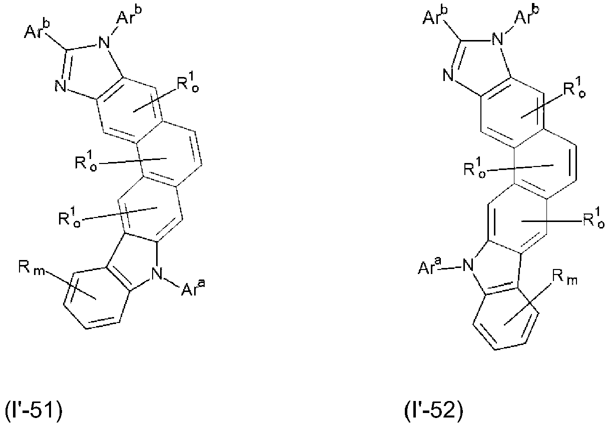 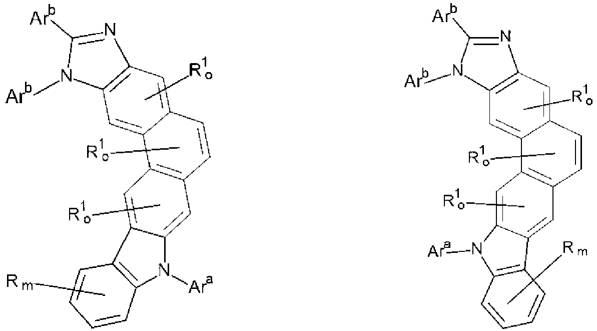 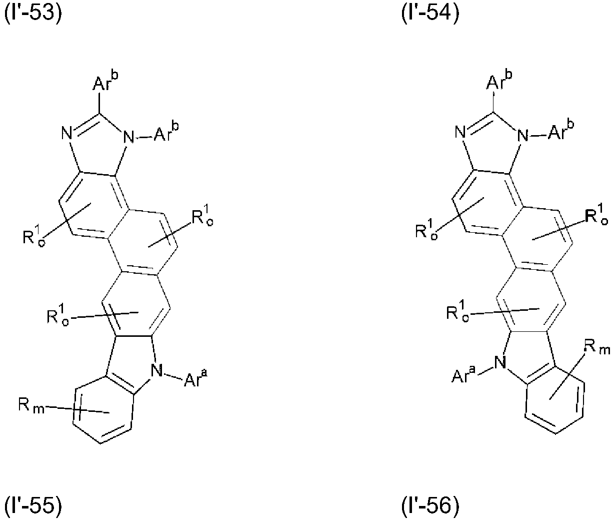 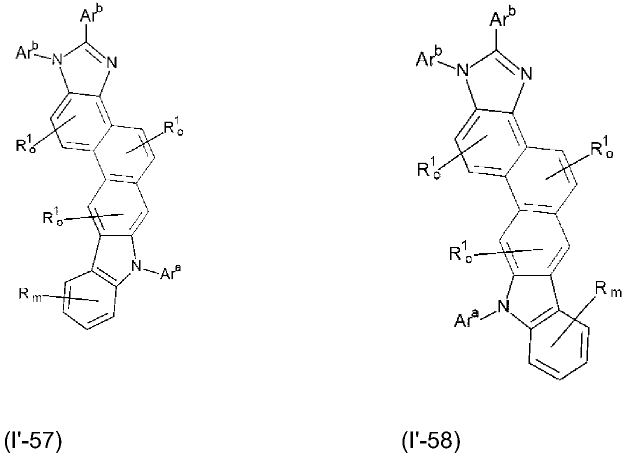 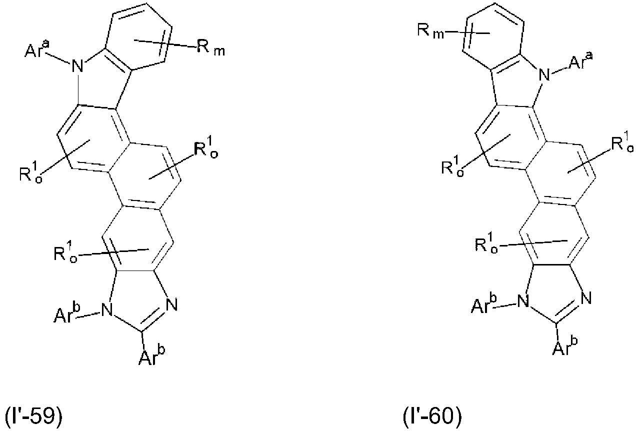 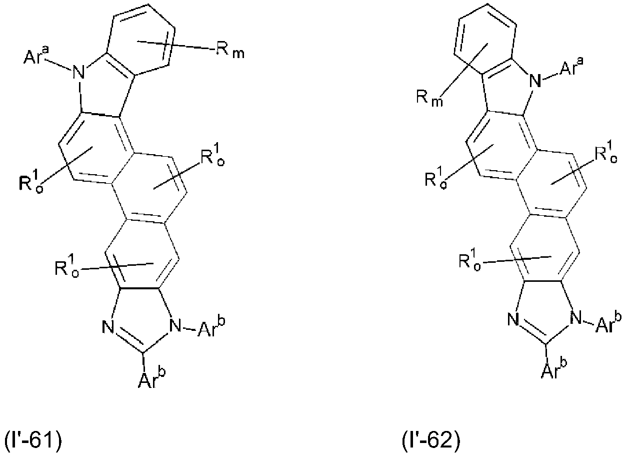 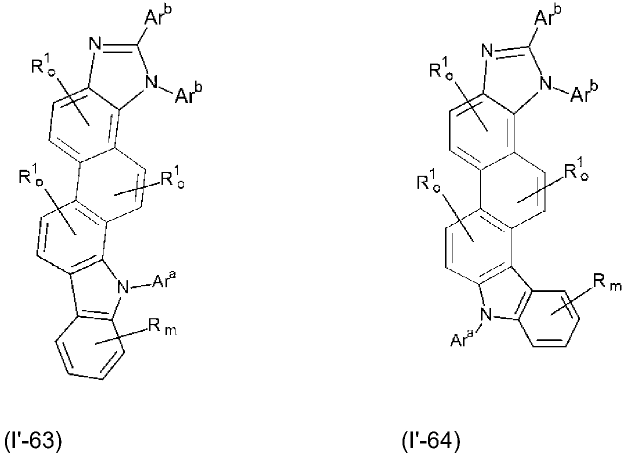 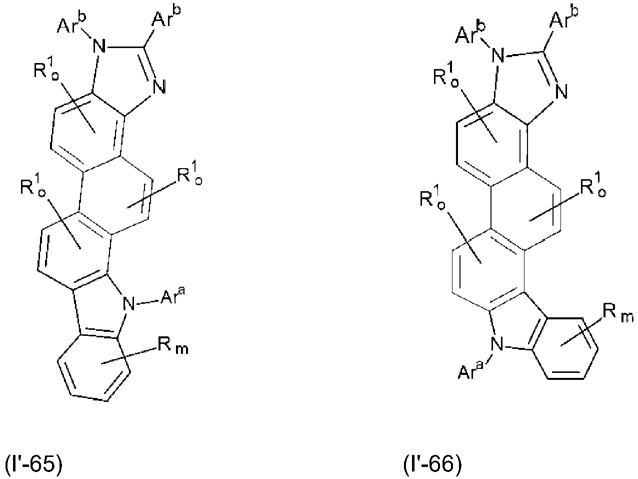 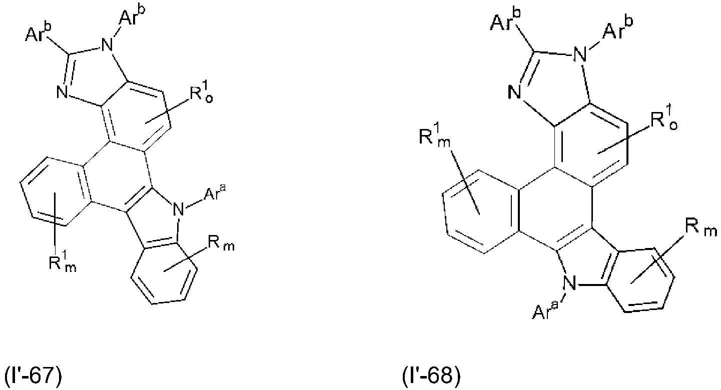 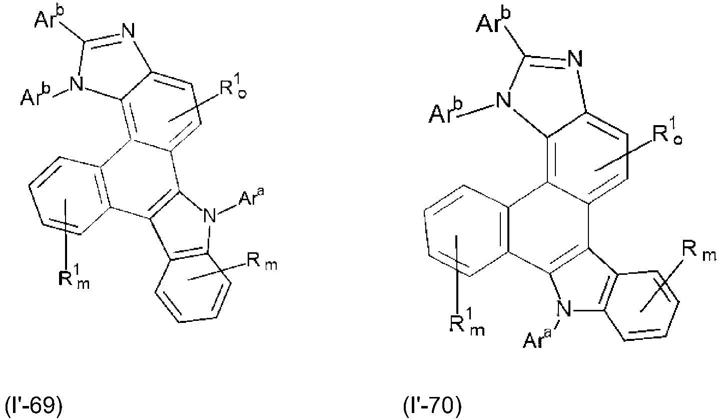 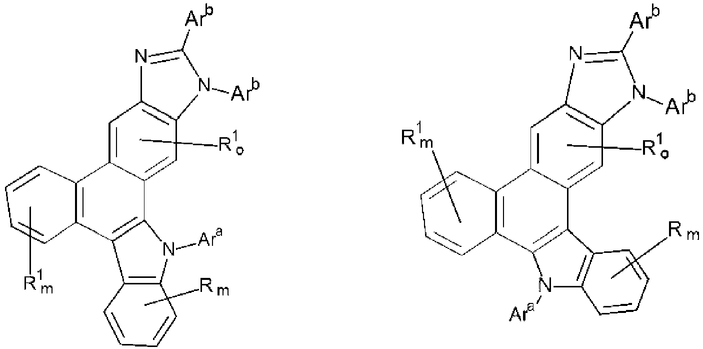 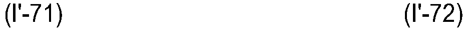 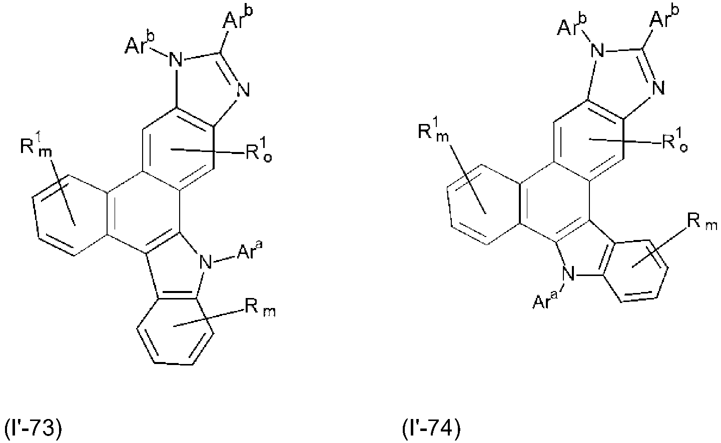 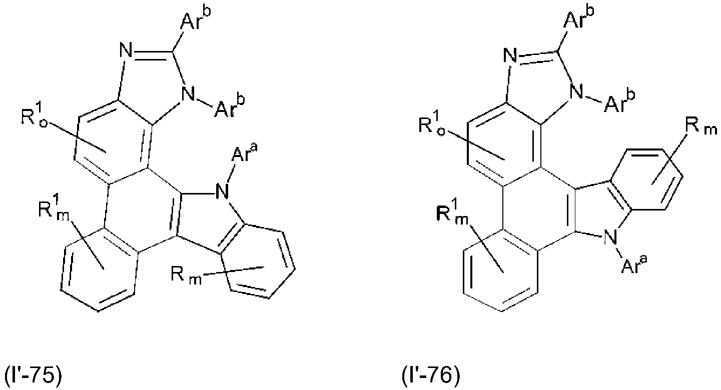 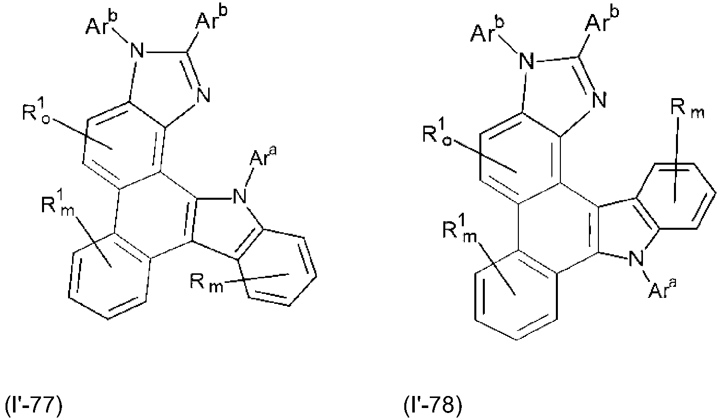 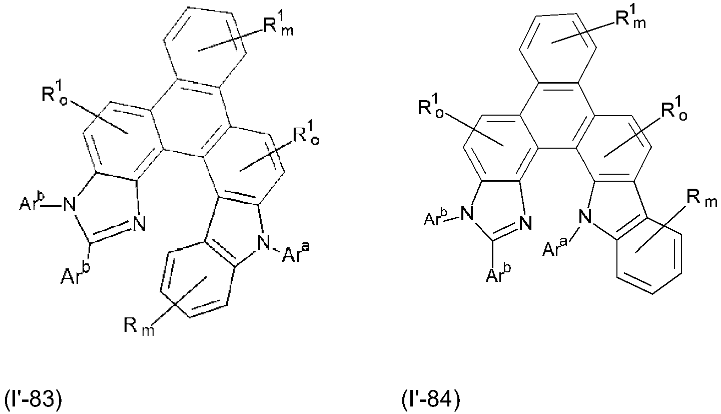 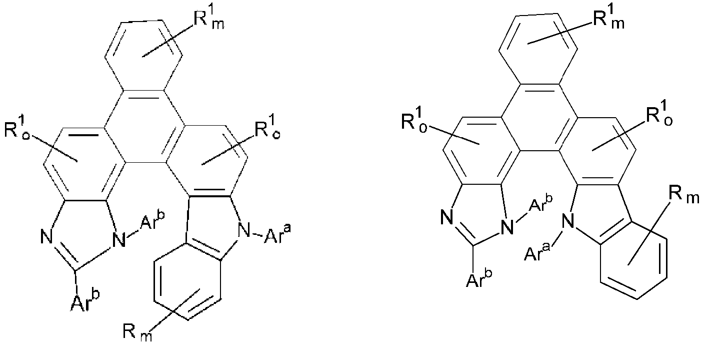 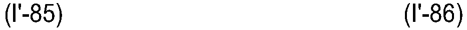 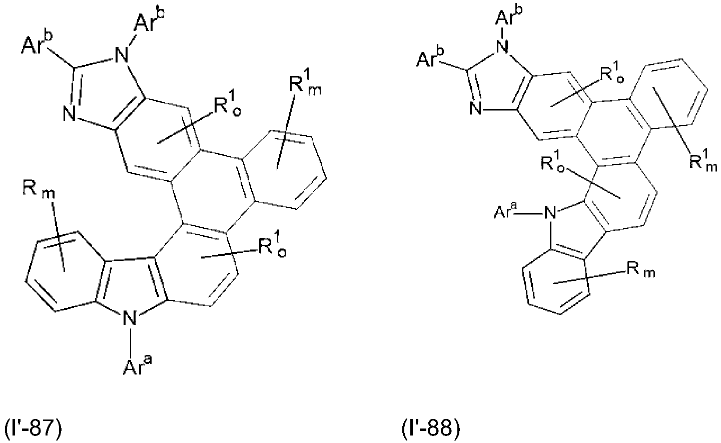 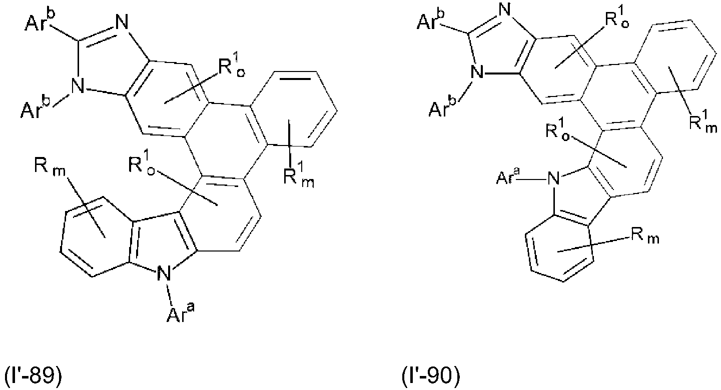 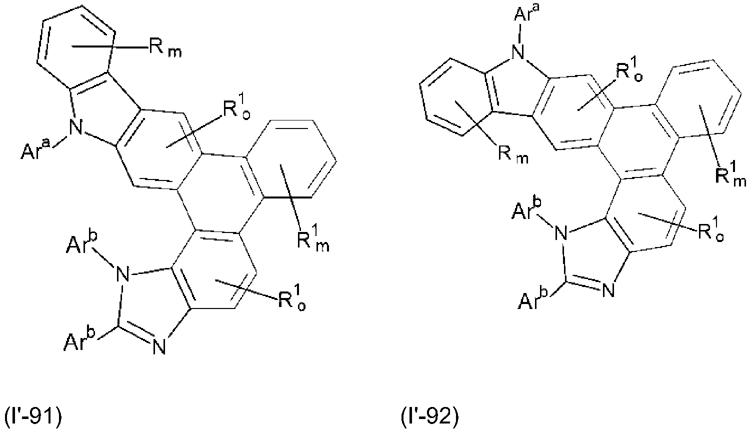 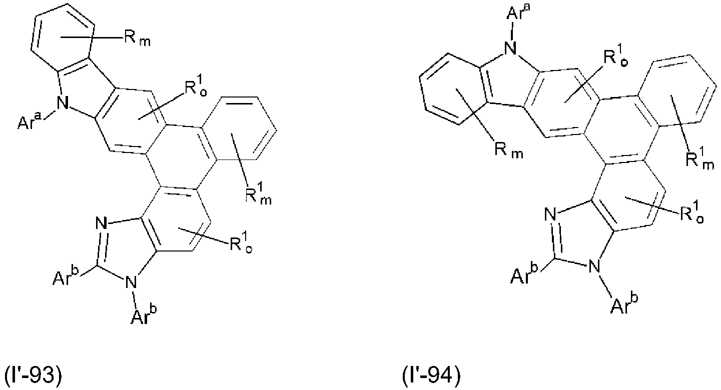 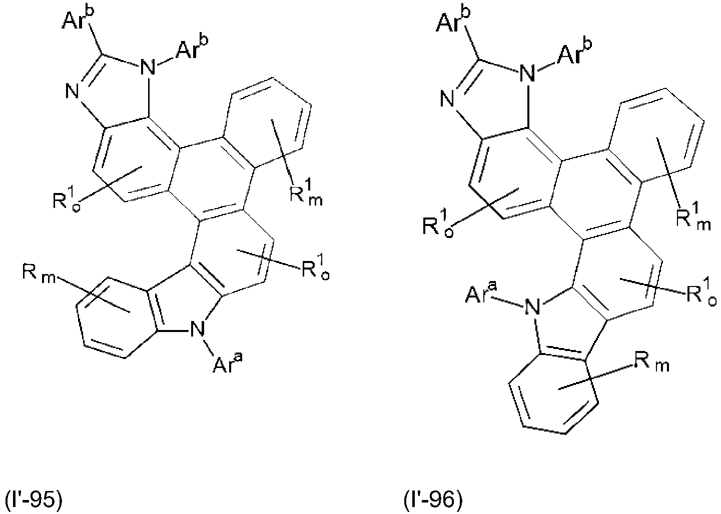 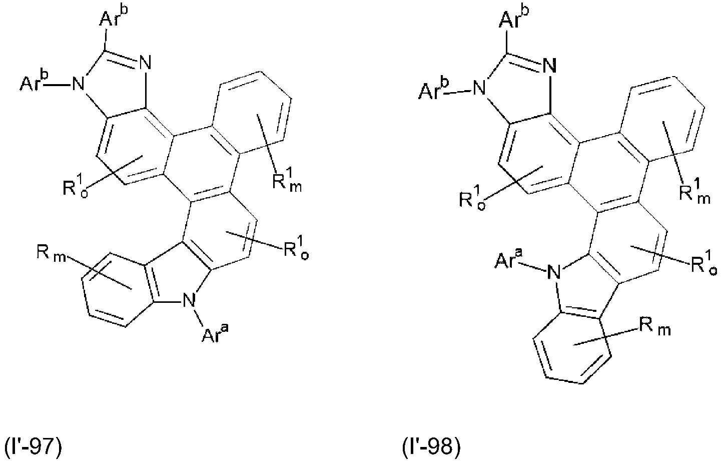 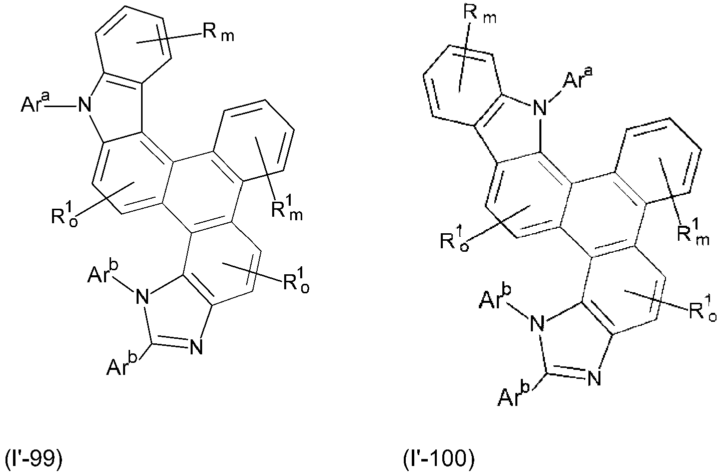 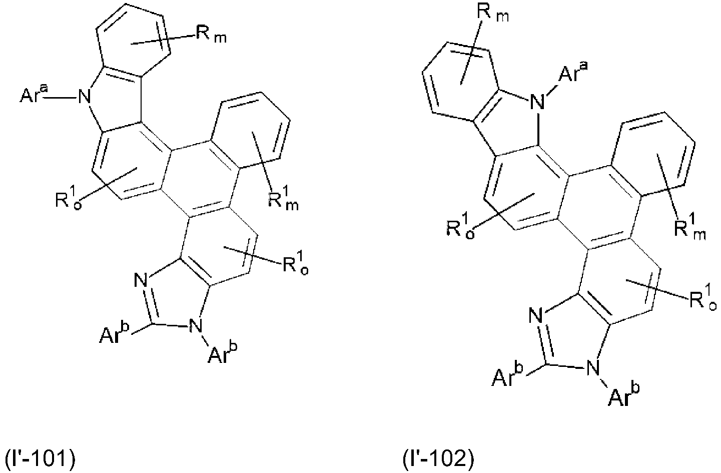 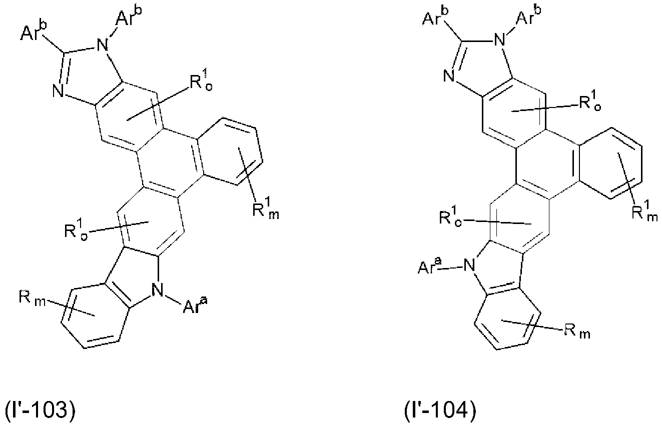 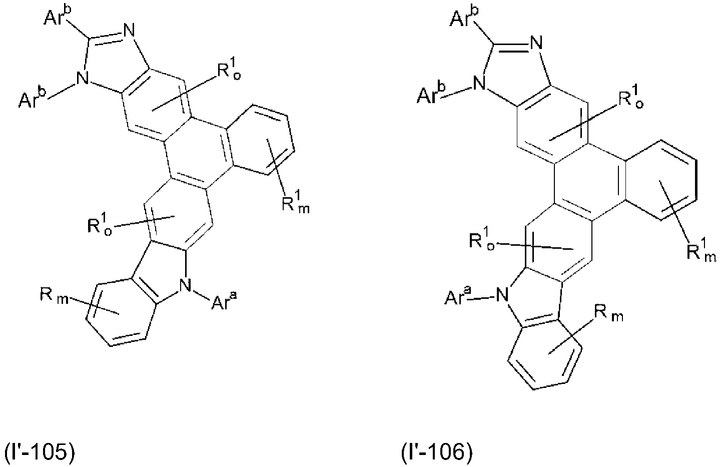 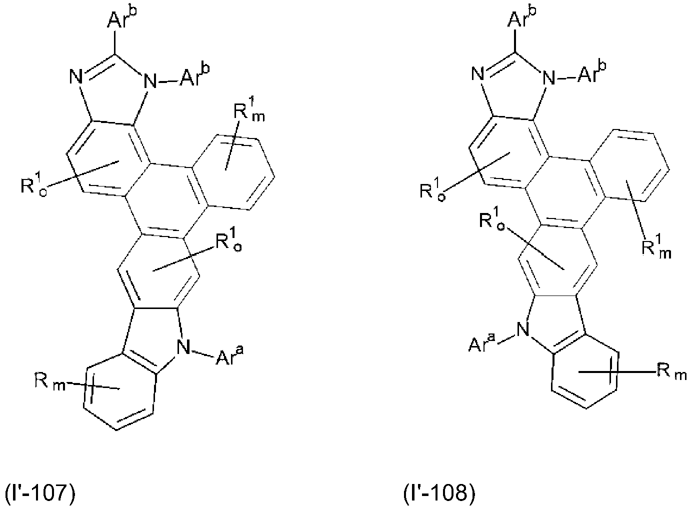 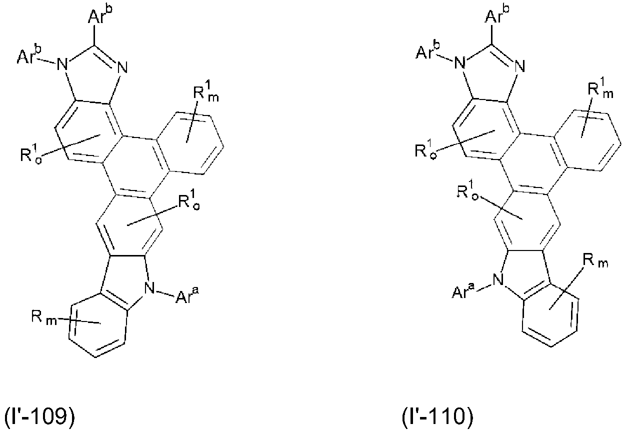 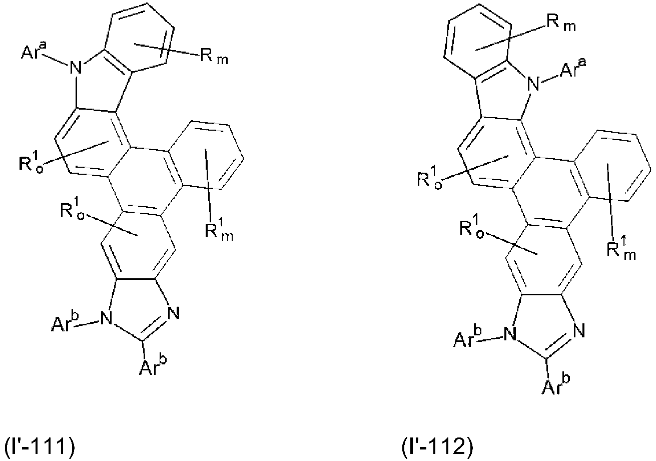 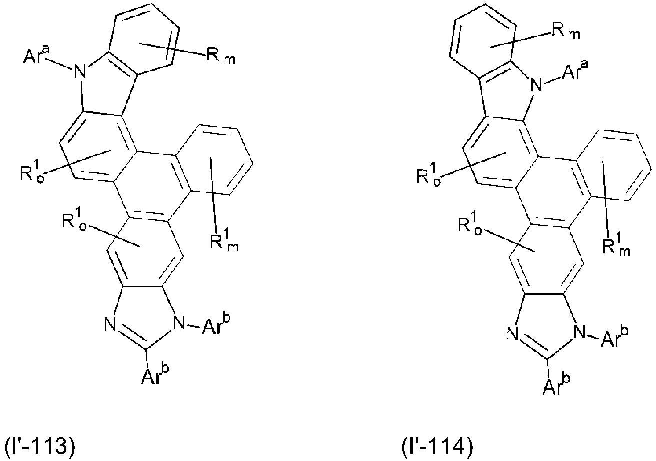 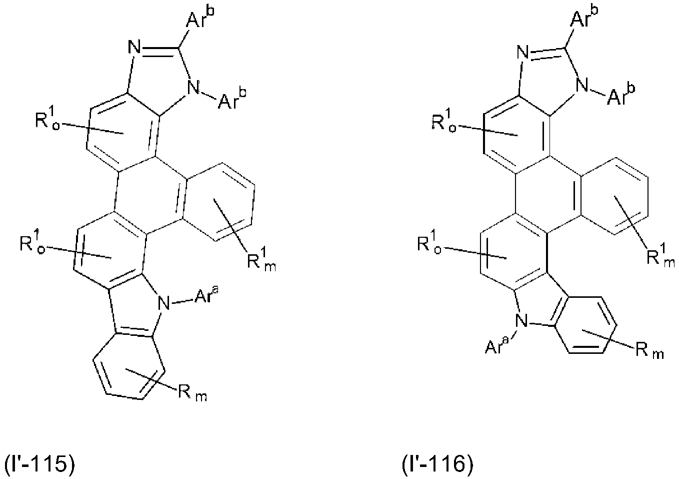 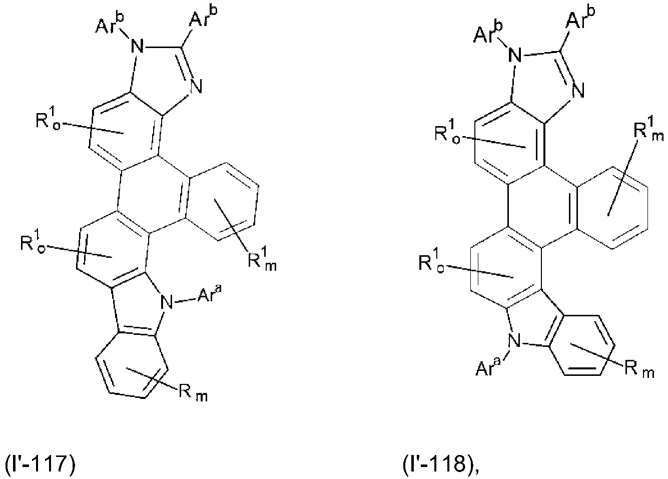 wherein the following applies to the symbols and indices used
Ar^{a} is, on each occurrence, identically or differently, an aromatic or heteroaromatic ring system with 5 to 40 aromatic ring atoms, which may be substituted with one or more radicals R²;
Ar^{b} is, on each occurrence, identically or differently, an aromatic or heteroaromatic ring system with 5 to 40 aromatic ring atoms, which may be substituted with one or more radicals R³;
R is, on each occurrence, identically or differently, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁴ and wherein one or more non-adjacent CH₂ groups may be substituted by Si(R⁴)₂, C=O, NR⁴, O, S or CONR⁴, or an aromatic or heteroaromatic ring system with 5 to 60 aromatic ring atoms, each of which may be substituted by one or more radicals R⁴; two radicals R or a radical R with a radical R¹, R² can also form an aliphatic, heteroaliphatic, aromatic or heteroaromatic ring system together;
R¹ is, on each occurrence, identically or differently, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁴ and wherein one or more non-adjacent CH₂ groups may be substituted by Si(R⁴)₂, C=O, NR⁴, O, S or CONR⁴, or an aromatic or heteroaromatic ring system with 5 to 60 aromatic ring atoms, each of which may be substituted by one or more radicals R⁴; two radicals R¹ or a radical R¹ with a radical R, R², R³ can also form an aliphatic, heteroaliphatic, aromatic or heteroaromatic ring system together;
R² is, on each occurrence, identically or differently, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁴ and wherein one or more non-adjacent CH₂ groups may be substituted by Si(R⁴)₂, C=O, NR⁴, O, S or CONR⁴, or an aromatic or heteroaromatic ring system with 5 to 60 aromatic ring atoms, each of which may be substituted by one or more radicals R⁴; two radicals R² or a radical R² with a radical R, R¹, R³ can also form an aromatic, heteroaromatic, aliphatic or heteroaliphatic ring system together;
R³ is, on each occurrence, identically or differently, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁴ and wherein one or more non-adjacent CH₂ groups may be substituted by Si(R⁴)₂, C=O, NR⁴, O, S or CONR⁴, or an aromatic or heteroaromatic ring system with 5 to 60 aromatic ring atoms, each of which can be substituted by one or more radicals R⁴; two radicals R³ or one radical R³ together with one radical R¹, R² can also form an aromatic, heteroaromatic, aliphatic or heteroaliphatic ring system;
Ar' is, on each occurrence, identically or differently, an aromatic or heteroaromatic ring system with 5 to 40 aromatic ring atoms, which may be substituted with one or more radicals R⁴;
R⁴ is, on each occurrence, identically or differently, H, D, F, Cl, Br, I, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)_{2R}⁵, OSO₂R⁵, a straight-chain alkyl group having 1 to 20 carbon atoms or an alkenyl or alkynyl group having 2 to 20 carbon atoms or a branched or cyclic alkyl group having 3 to 20 carbon atoms, wherein the alkyl, alkenyl or alkynyl group may each be substituted by one or more radicals R⁵, wherein one or more non-adjacent CH₂ groups are substituted by Si(R⁵)₂, C=O, NR⁵, O, S or CONR⁵, or an aromatic or heteroaromatic ring system with 5 to 40 aromatic ring atoms, each of which may be substituted by one or more radicals R⁵; two or more radicals R⁴ can together form an aromatic, heteroaromatic, aliphatic or heteroaliphatic ring system;
R⁵ is, on each occurrence, identically or differently, H, D, F or an aliphatic, aromatic or heteroaromatic organic radical having 1 to 20 carbon atoms, in which one or more H atoms may also be replaced by F,
the index o is 0, 1 or 2 and the index m is 0, 1, 2, 3 or 4.

2. Compounds according to claim 1, **characterised in that** two adjacent groups R¹ do not form a fused aromatic or heteroaromatic ring system with the groups to which the groups R¹ bind, wherein this includes the radicals R⁴ or R⁵ by which the groups R¹ may be substituted.

3. Compounds according to claim 1 or 2, **characterised in that** Ar^{a} and/or Ar^{b} is the same or different at each occurrence and is selected from phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, quinoline, isoquinoline, quinazoline, quinoxaline, phenanthrene or triphenylene, each of which may be substituted with one or more radicals R² or R³, respectively.

4. Compounds according to one or more of claims 1 to 3, **characterised in that** R, R¹, R² and/or R³ are the same or different at each occurrence and are selected from the group consisting of H, D or an aromatic or heteroaromatic ring system selected from the groups of the following formulae Ar-1 to Ar-75 and/or the group Ar' is the same or different at each occurrence and is selected from the groups of the following formulae Ar-1 to Ar-75, 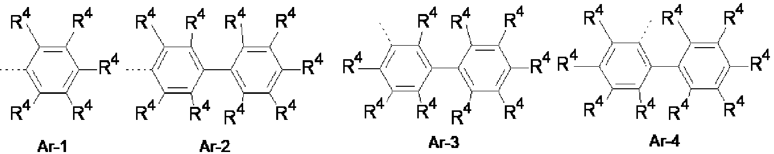 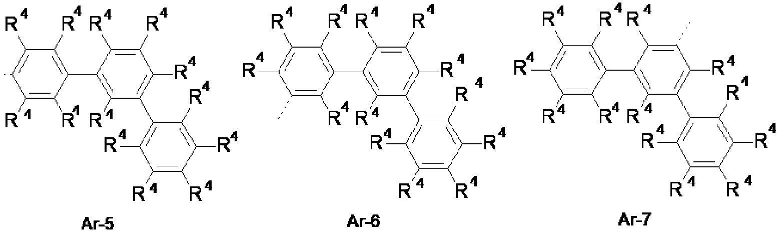 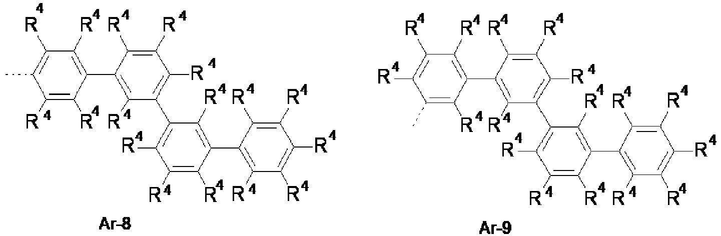 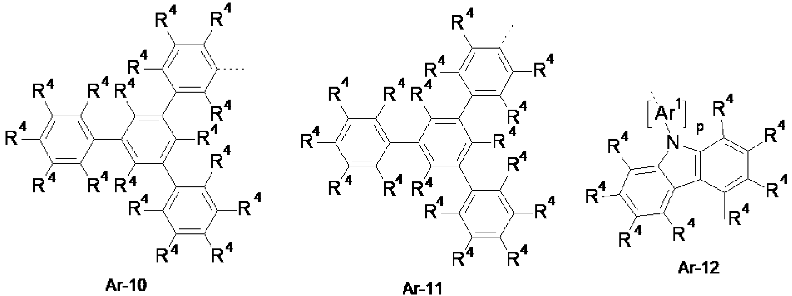 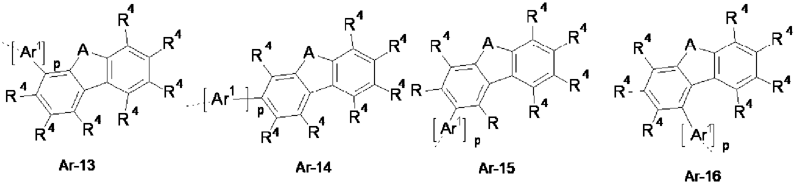 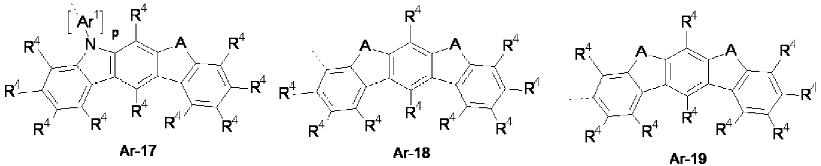 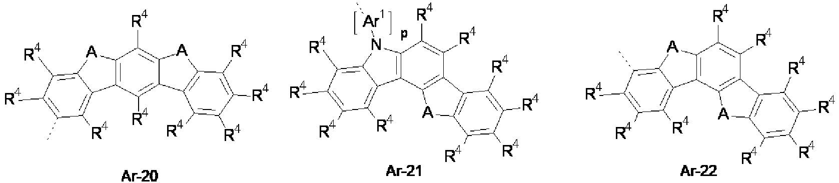 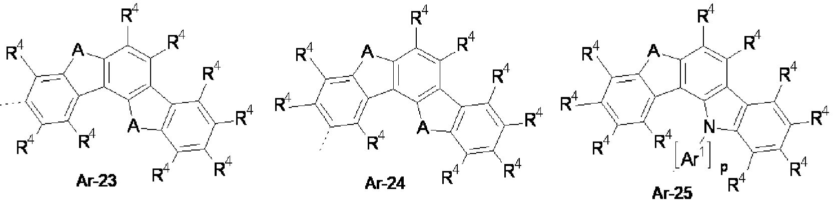 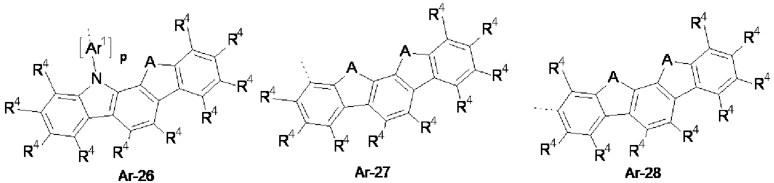 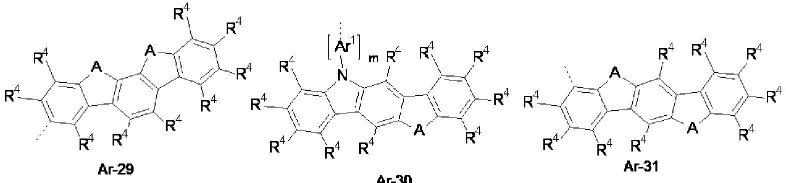 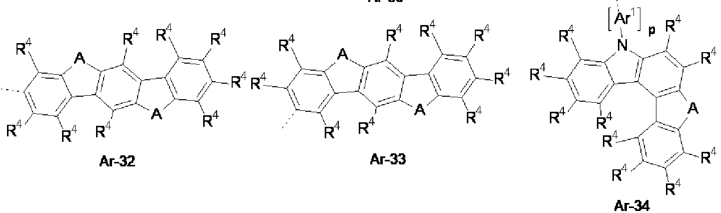 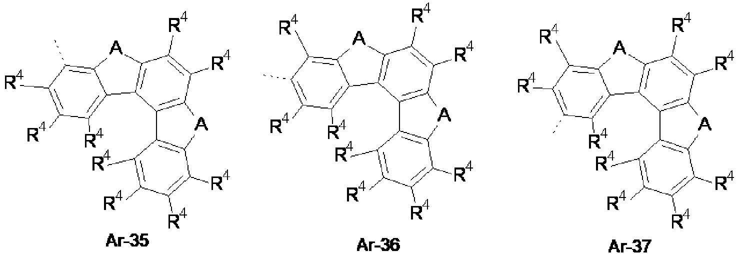 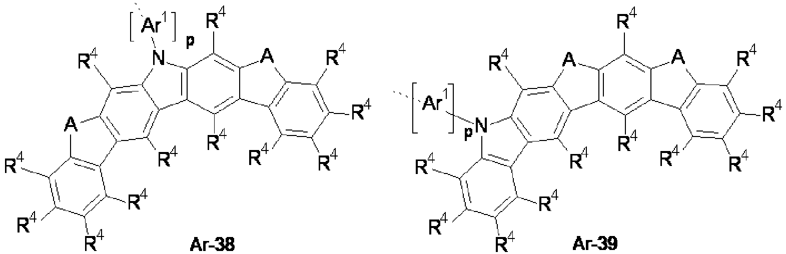 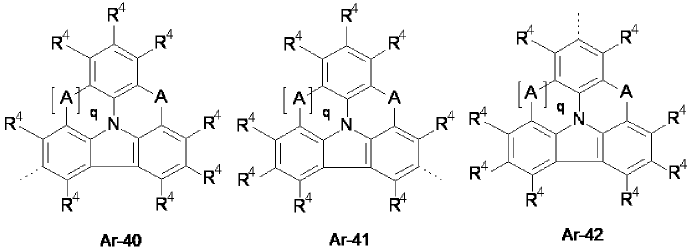 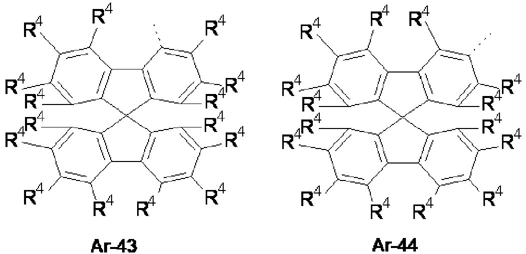 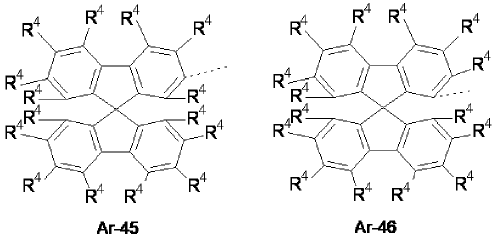 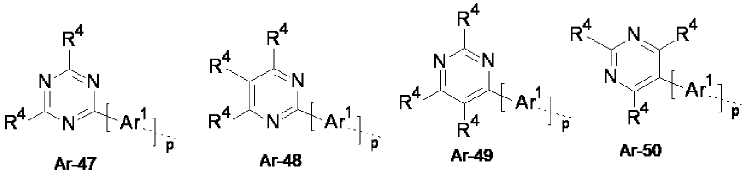 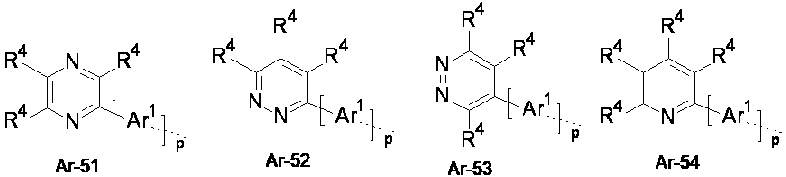 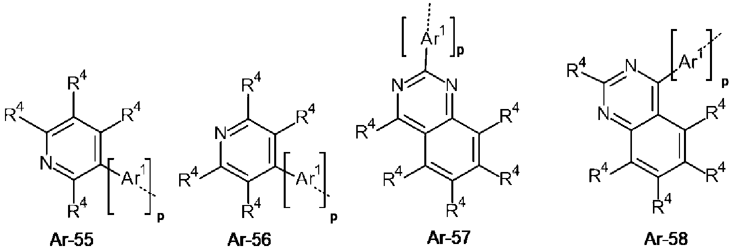 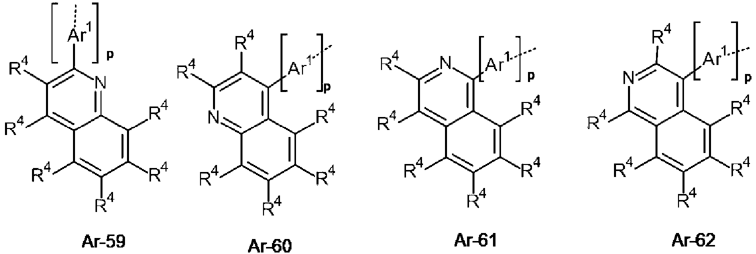 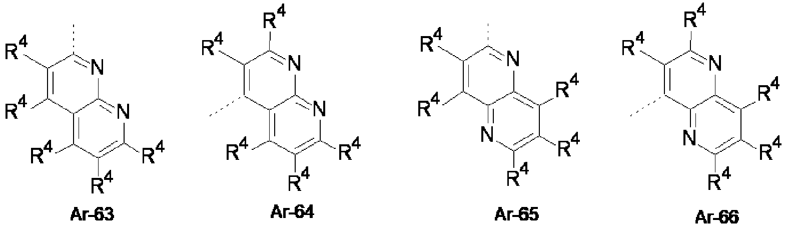 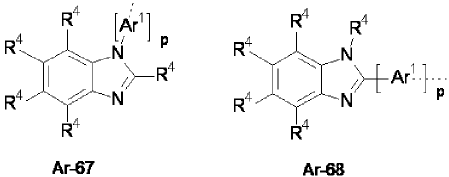 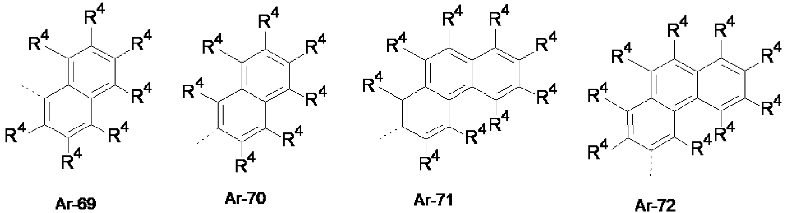 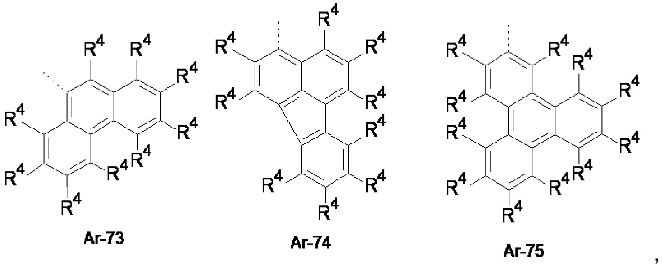 wherein R⁴ is as defined above in claim 1, the dotted bond represents the bond to the corresponding group, and further wherein
Ar¹ is, on each occurrence, identically or differently, a bivalent aromatic or heteroaromatic ring system having 6 to 18 aromatic ring atoms, which may be substituted in each case by one or more radicals R⁴;
A is, on each occurrence, identically or differently, C(R⁴)₂, NR⁴, O or S;
p is 0 or 1, wherein p = 0 means that the group Ar¹ is not present and that the corresponding aromatic or heteroaromatic group is directly bonded to the corresponding radical;
q is 0 or 1, wherein q = 0 means that no group A is bonded at this position and that residues R⁴ are bonded to the corresponding carbon atoms instead.

5. Compounds according to one or more of claims 1 to 4, **characterised in that** the compounds have at least two electron directing groups selected from substituents of the formulae (Ar-47) to (Ar-51), (Ar-57), (Ar-58) and/or (Ar-63) to (Ar-68) shown in claim 4.

6. Compounds according to one or more of claims 1 to 5, **characterised in that** the radical Ar^{a} can be represented by a group of the formulae (Ar-47) to (Ar-51), (Ar-57), (Ar-58) and/or (Ar-63) to (Ar-68) shown in claim 4, wherein the substituents R⁴ shown in the formulae (Ar-47) to (Ar-51), (Ar-57), (Ar-58) and/or (Ar-63) to (Ar-68) are to be replaced by R².

7. A process for the preparation of a compound according to one or more of claims 1 to 6, **characterised in that** an aromatic or heteroaromatic compound is reacted with an aromatic or heteroaromatic diamino compound by means of a coupling reaction.

8. A composition containing at least one compound according to one or more of claims 1 to 6 and at least one further matrix material, wherein the further matrix material is selected from compounds according to one of the formulae (H-1), (H-2), (H-3), (H-4) or (H-5), 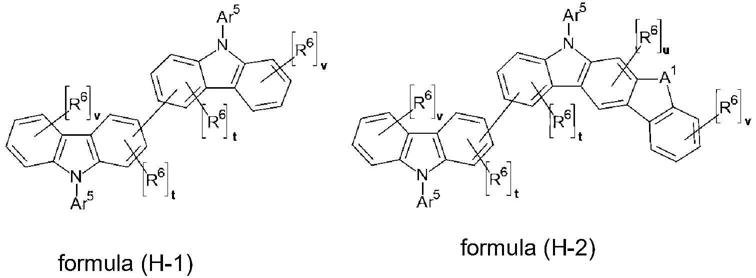 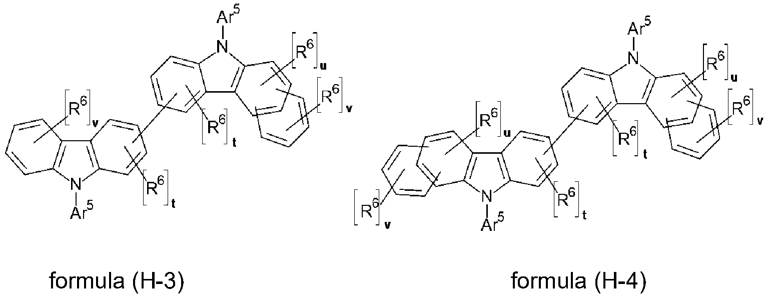 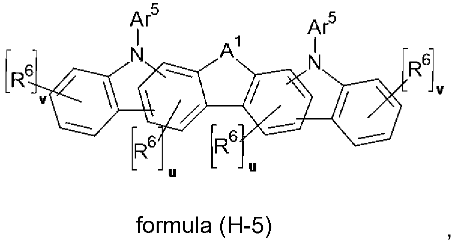 wherein the symbols and indices used are
R⁶ is, on each occurrence, identically or differently, H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁷ and wherein one or more non-adjacent CH₂ groups may be substituted by Si(R⁷)₂, C=O, NR⁷, O, S or CONR⁷, or an aromatic or heteroaromatic ring system with 5 to 60 aromatic ring atoms, each of which can be substituted by one or more radicals R⁷; two radicals R⁶ can also form an aromatic, heteroaromatic, aliphatic or heteroaliphatic ring system together;
Ar" is an aromatic or heteroaromatic ring system with 5 to 40 aromatic ring atoms, which may be substituted with one or more radicals R⁷;
A¹ is C(R⁷)₂, NR⁷, O or S;
Ar⁵ is, on each occurrence, identically or differently, an aromatic or heteroaromatic ring system with 5 to 40 aromatic ring atoms, which can be substituted with one or more radicals R⁷;
R⁷ is, on each occurrence, identically or differently, H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁸, wherein one or more non-adjacent CH₂ groups are substituted by Si(R⁸)₂, C=O, NR⁸, O, S or CONR⁸, or an aromatic or heteroaromatic ring system with 5 to 40 aromatic ring atoms, each of which may be substituted by one or more radicals R⁸; two or more radicals R⁷ can together form an aromatic, heteroaromatic, aliphatic or heteroaliphatic ring system;
R⁸ is, on each occurrence, identically or differently, H, D, F or an aliphatic, aromatic or heteroaromatic organic radical having 1 to 20 carbon atoms, in which one or more H atoms may also be replaced by F;
v is, on each occurrence, identically or differently, 0, 1, 2, 3 or 4;
t is, on each occurrence, identically or differently, 0, 1, 2 or 3;
u is, on each occurrence, identically or differently, 0, 1 or 2.

9. A composition containing at least one compound according to one or more of claims 1 to 6 and at least one further matrix material, wherein the further matrix material is selected from compounds according to one of the formulae (H-6), (H-7), (H-8), (H-9), (H-10), (H-11), (H-12) or (H-13), 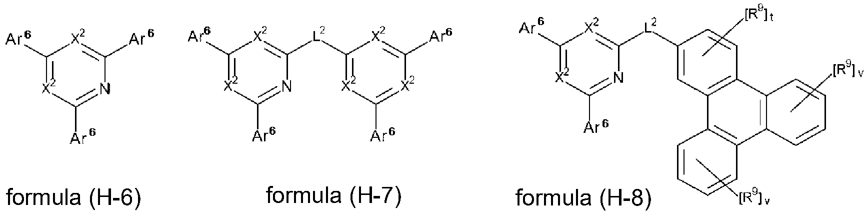 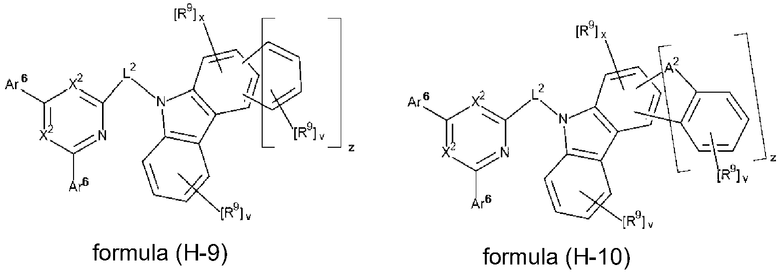 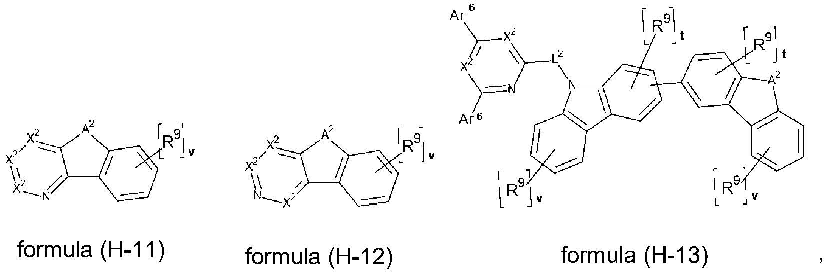 wherein the following applies to the symbols and indices used
X² stands for N or CR⁹, with the proviso that no more than two of the groups X² in a cycle stand for N;
L² represents a linking group selected from a bond or an aromatic or heteroaromatic ring system with 5 to 40 aromatic ring atoms, which may be substituted with one or more radicals R⁹;
Ar⁶ is, on each occurrence identically or differently, an aromatic or heteroaromatic ring system with 5 to 40 aromatic ring atoms, which can be substituted with one or more radicals R¹⁰;
R⁹ is, on each occurrence, identically or differently, H, D, F, Cl, Br, I, N(R¹⁰)₂, N(Ar‴)₂, CN, NO₂, OR, SR¹⁰, COOR¹⁰, C(=O)N(R¹⁰)₂, Si(R¹⁰)₃, B(OR¹⁰)₂, C(=O)R¹⁰, P(=O)(R¹⁰)₂, S(=O)R¹⁰, S(=O)₂R¹⁰, OSO₂R¹⁰, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group may each be substituted by one or more radicals R⁴ and wherein one or more non-adjacent CH₂ groups may be substituted by Si(R¹⁰)₂, C=O, NR¹⁰, O, S or CONR¹⁰, or an aromatic or heteroaromatic ring system with 5 to 60 aromatic ring atoms, each of which may be substituted by one or more radicals R¹⁰; two radicals R⁹ can also form an aromatic, heteroaromatic, aliphatic or heteroaliphatic ring system together;
Ar‴ is, on each occurrence, identically or differently, an aromatic or heteroaromatic ring system with 5 to 40 aromatic ring atoms, which may be substituted with one or more radicals R¹⁰;
A² is C(R¹⁰)₂, NR¹⁰, O or S;
R¹⁰ is, on each occurrence, identically or differently, H, D, F, Cl, Br, I, N(R¹¹)₂, CN, NO₂, OR¹¹, SR¹¹, Si(R¹¹)₃, B(OR¹¹)₂, C(=O)R¹¹, P(=O)(R¹¹)₂, S(=O)R¹¹, S(=O)₂R¹¹, OSO₂R¹¹, a straight-chain alkyl group having 1 to 20 carbon atoms or an alkenyl or alkynyl group having 2 to 20 carbon atoms or a branched or cyclic alkyl group having 3 to 20 carbon atoms, wherein the alkyl, alkenyl or alkynyl group may each be substituted by one or more radicals R¹¹, wherein one or more non-adjacent CH₂ groups are substituted by Si(R¹¹)₂, C=O, NR¹¹, O, S or CONR¹¹, or an aromatic or heteroaromatic ring system with 5 to 40 aromatic ring atoms, each of which may be substituted by one or more radicals R¹¹; two or more radicals R¹⁰ can together form an aromatic, heteroaromatic, aliphatic or heteroaliphatic ring system;
R¹¹ is, on each occurrence, identically or differently, H, D, F or an aliphatic, aromatic or heteroaromatic organic radical having 1 to 20 carbon atoms, in which one or more H atoms may also be replaced by F;
v is, on each occurrence, identically or differently, 0, 1, 2, 3 or 4;
t is, on each occurrence, identically or differently, 0, 1, 2 or 3;
x is, on each occurrence, identically or differently, 0, 1, 2, 3 or 4;
z is, on each occurrence, identically or differently, 0, 1 or 2, where the sum of x and z is at most 4.

10. The composition according to claim 8 or 9, **characterised in that** a compound of claims 1 to 6 has a mass fraction in the composition in the range from 10% by weight to 95% by weight, based on the total mass of the composition.

11. A formulation containing at least one compound according to one or more of claims 1 to 6 and/or at least one composition according to one or more of claims 8 to 10 and at least one further compound, wherein the further compound is selected from one or more solvents.

12. Use of compounds according to one or more of claims 1 to 6 and/or a composition according to one or more of claims 8 to 10 in an electronic device.

13. An electronic device containing at least one compound according to one or more of claims 1 to 6 and/or a composition according to one or more of claims 8 to 10.

14. The electronic device according to claim 13, wherein it is an organic electroluminescent device, **characterised in that** the compound according to one or more of claims 1 to 6 is used as a matrix material in an emitting layer and/or in an electron transport layer and/or in a hole blocking layer and/or in a hole transport layer and/or in an electron blocking layer.

15. The electronic device according to claim 14, **characterised in that** the compound according to one or more of claims 1 to 6 is used as a matrix material for phosphorescent emitters in combination with a further matrix material, wherein the further matrix material is selected from compounds according to one of the formulae (H-1), (H-2), (H-3), (H-4) or (H-5), 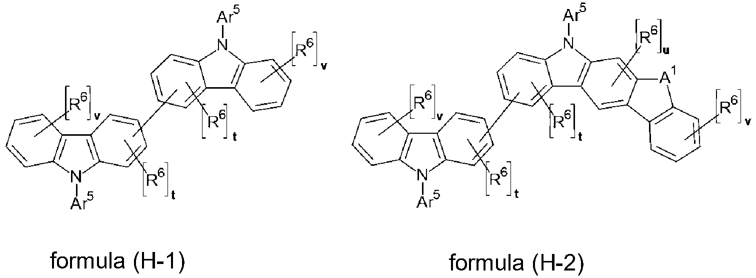 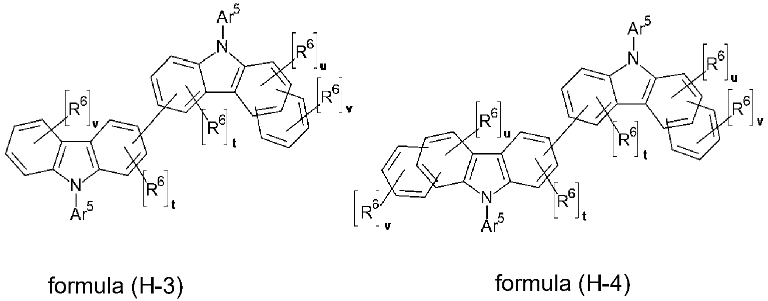 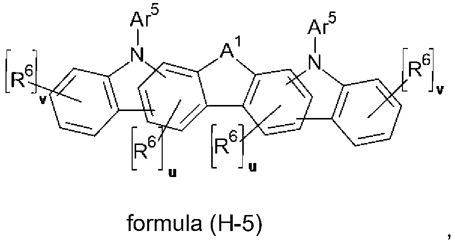
wherein the symbols A¹, Ar⁵ and R⁶ and the indices s, t and u have the meanings given in claim 8,
and/or that the further matrix material is selected from compounds according to one of the formulae (H-6), (H-7), (H-8), (H-9), (H-10), (H-11), (H-12) or (H-13), 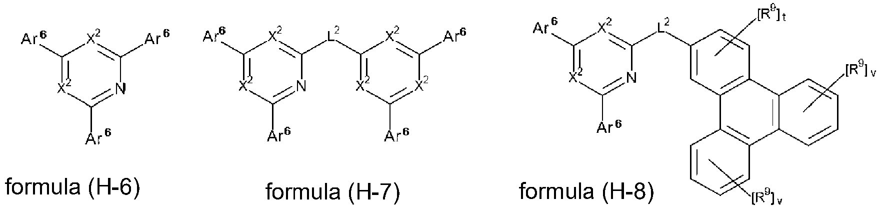 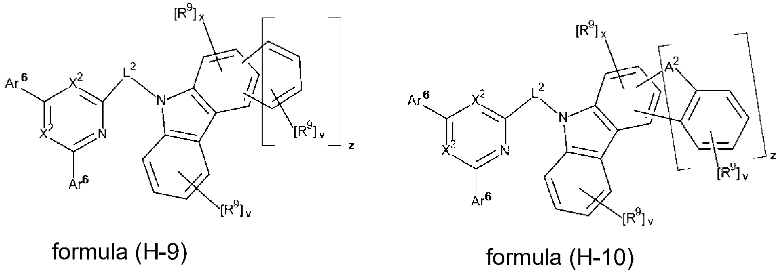 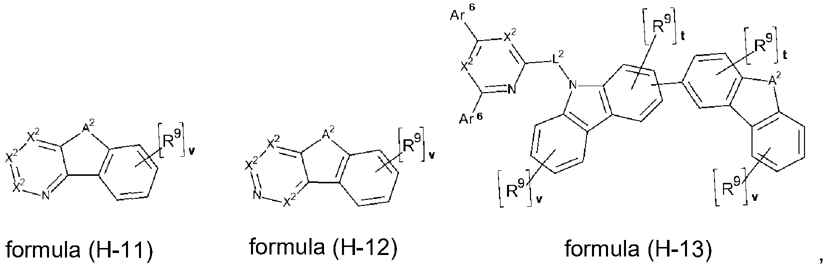
wherein the symbols X², L², A², Ar⁶ and R⁹ and the indices v, t, x and z have the meanings given in claim 9.

## Revendications

1. Composés de formules (I'-5) à (I'-22), (I'-31) à (I'-78) et (I'-83) à (I'-118), 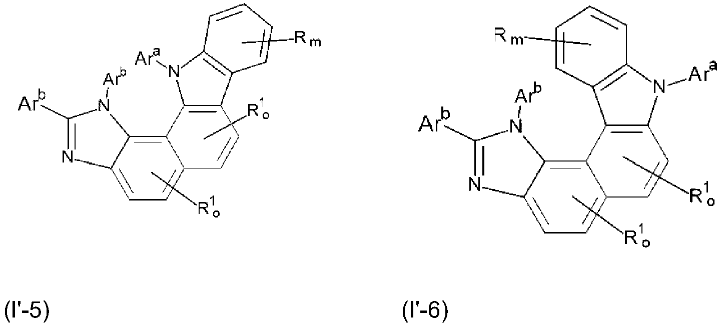 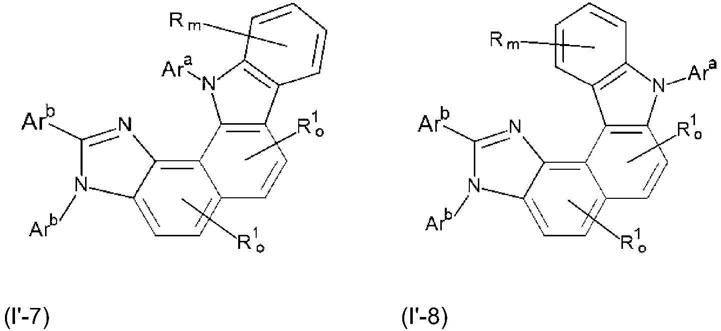 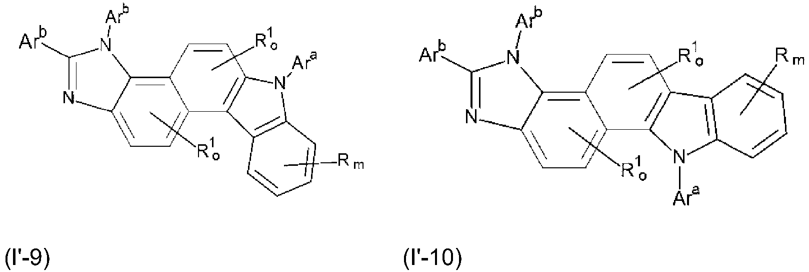 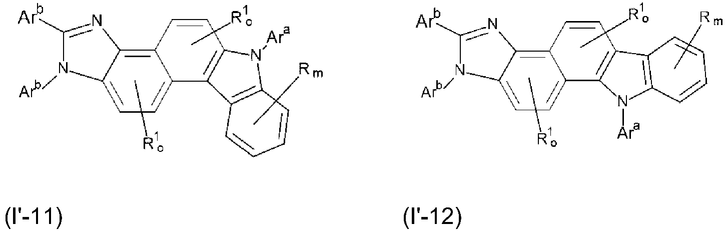 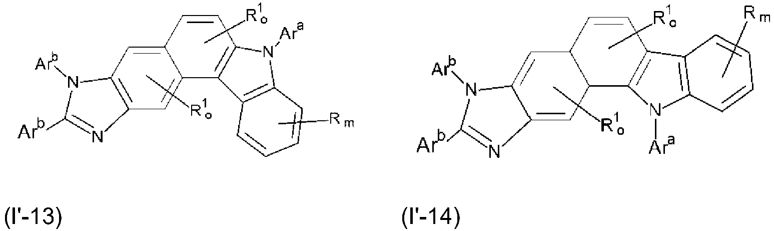 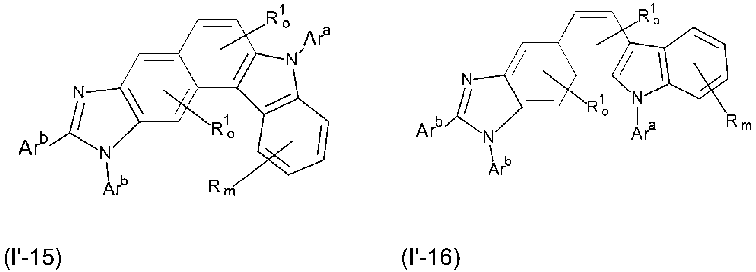 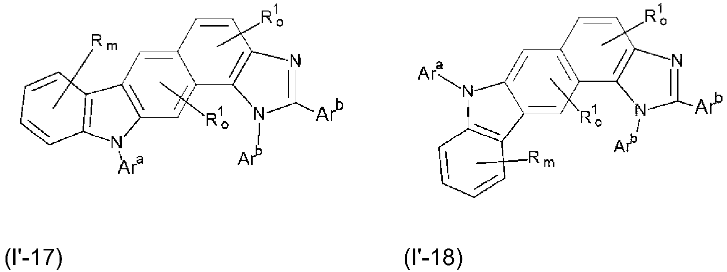 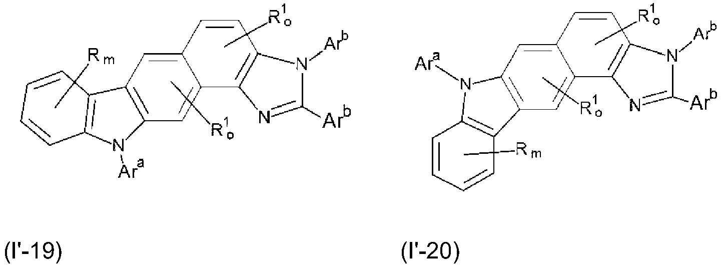 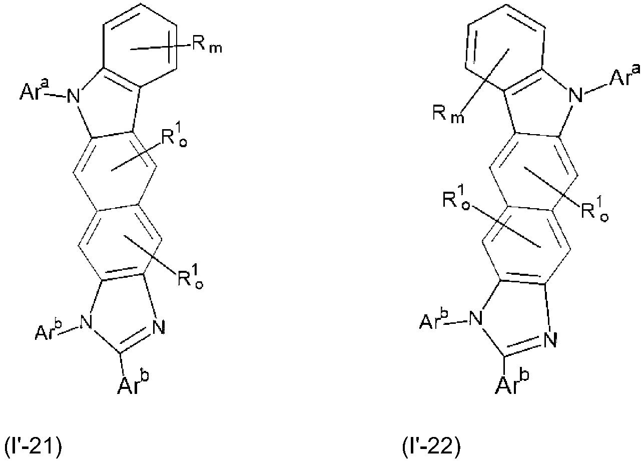 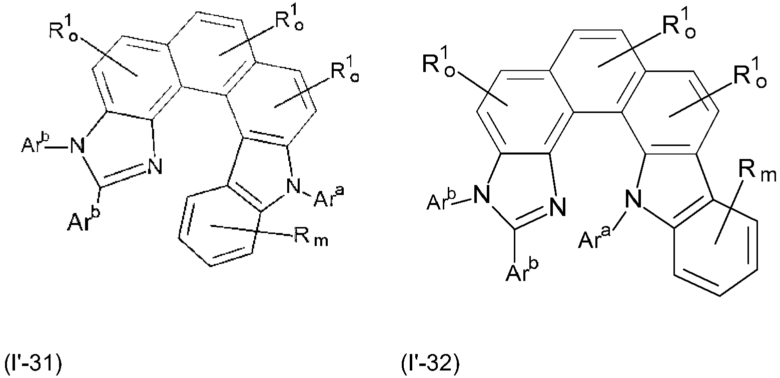 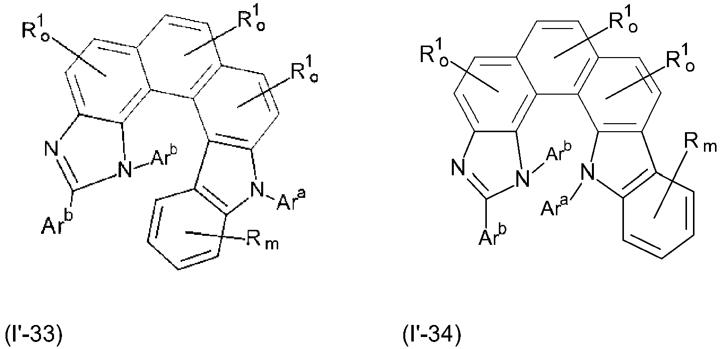 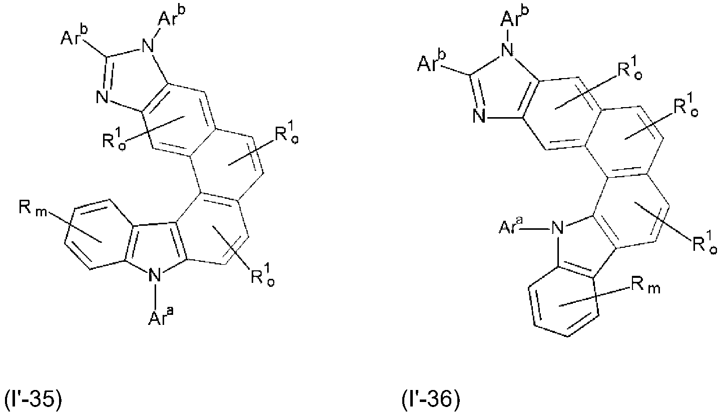 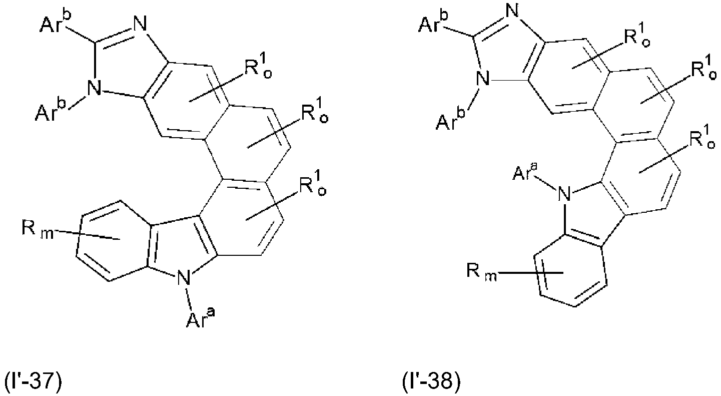 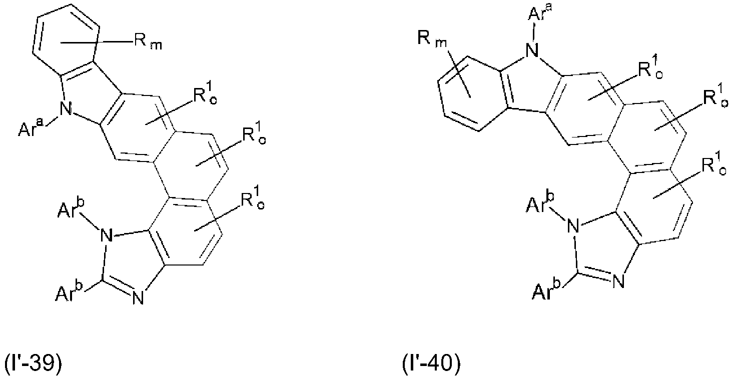 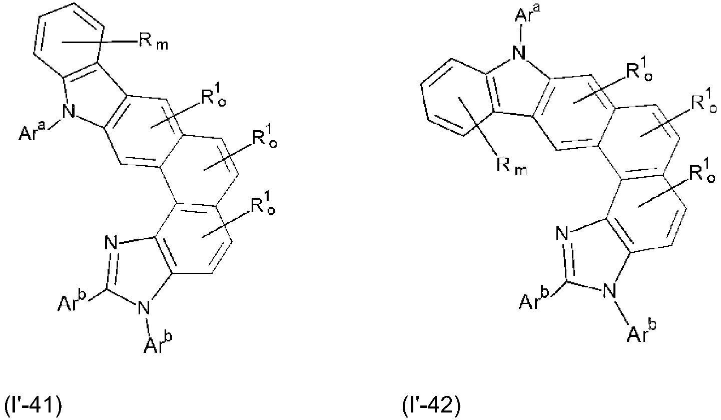 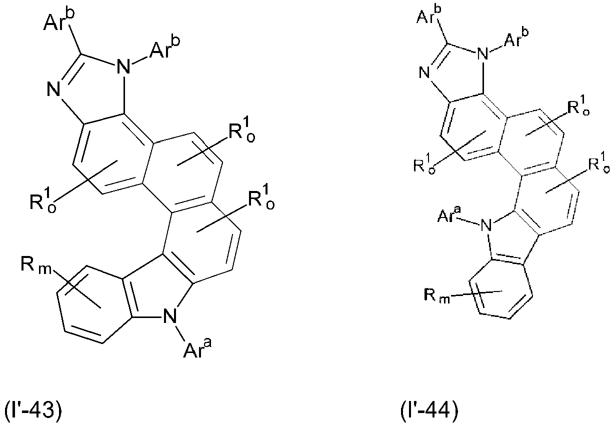 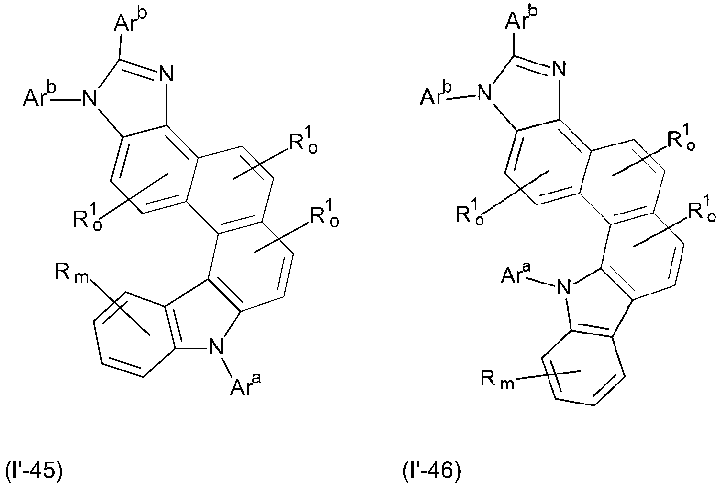 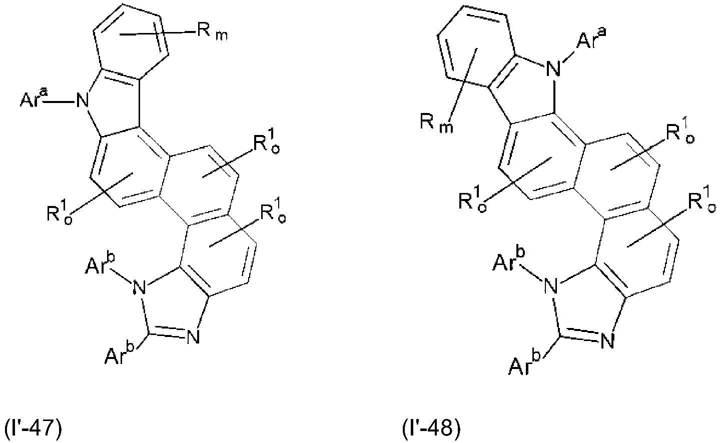 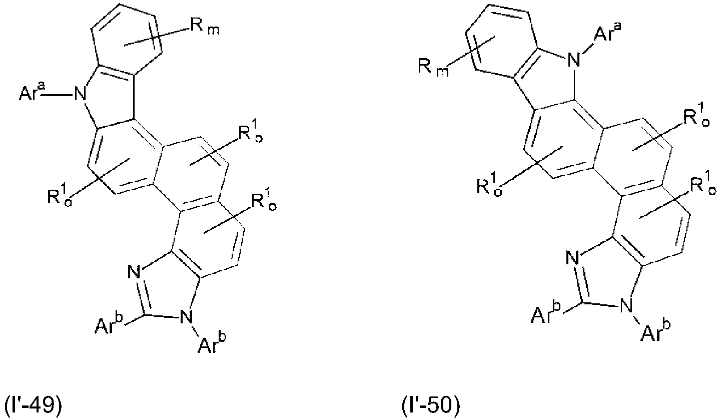 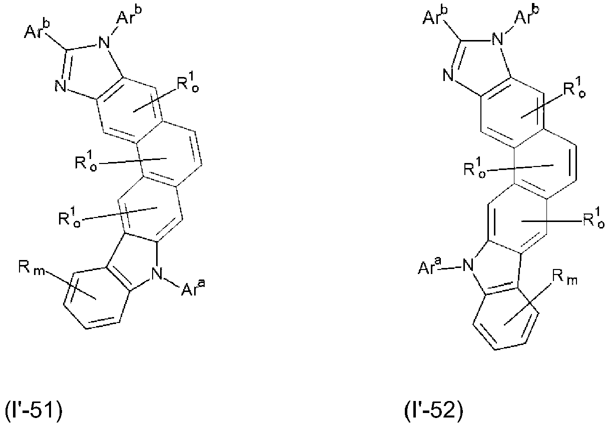 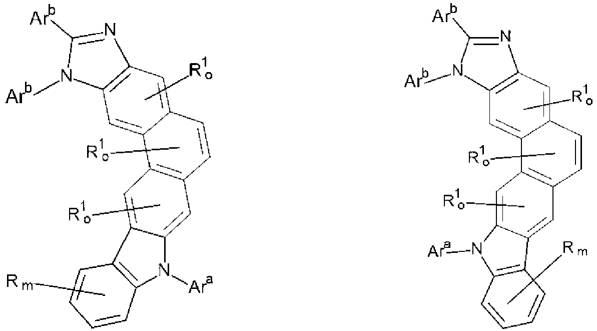 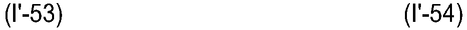 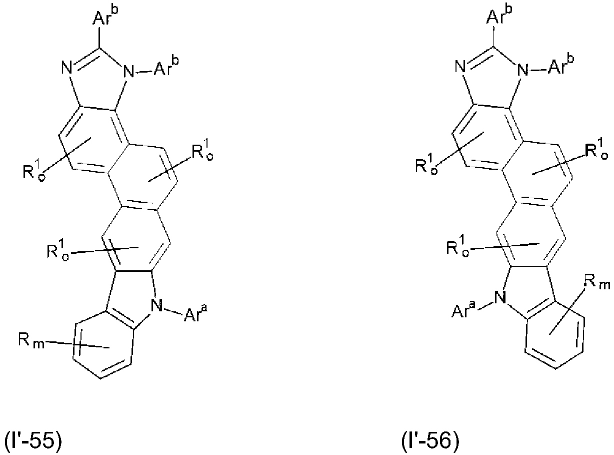 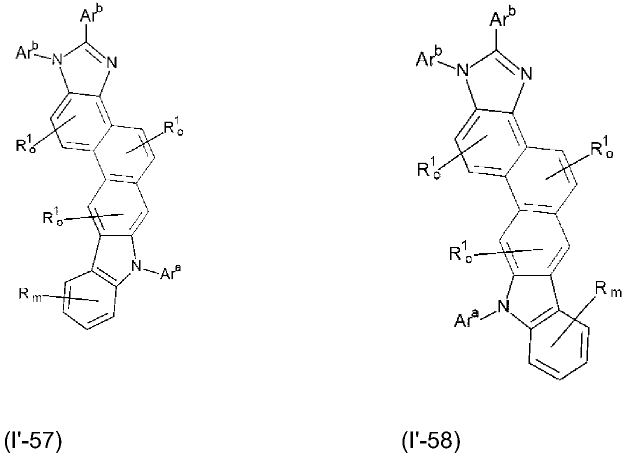 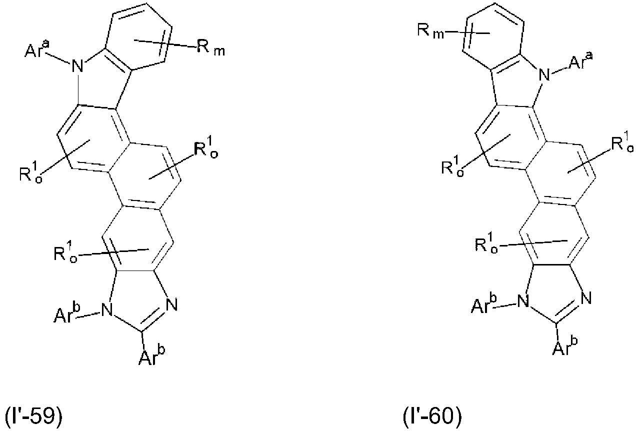 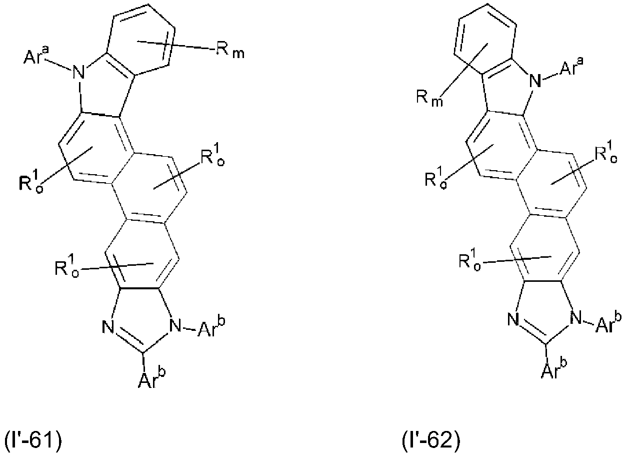 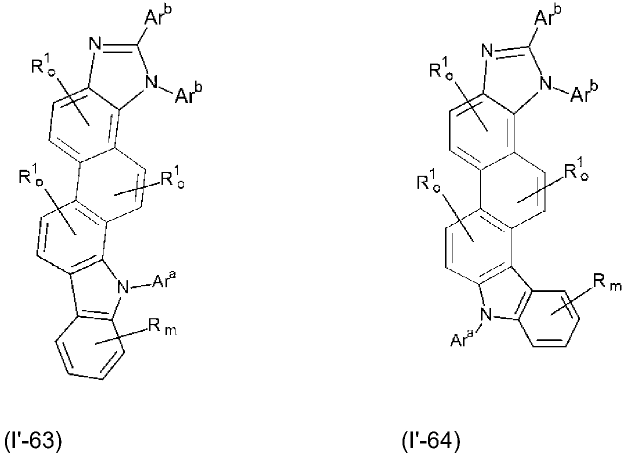 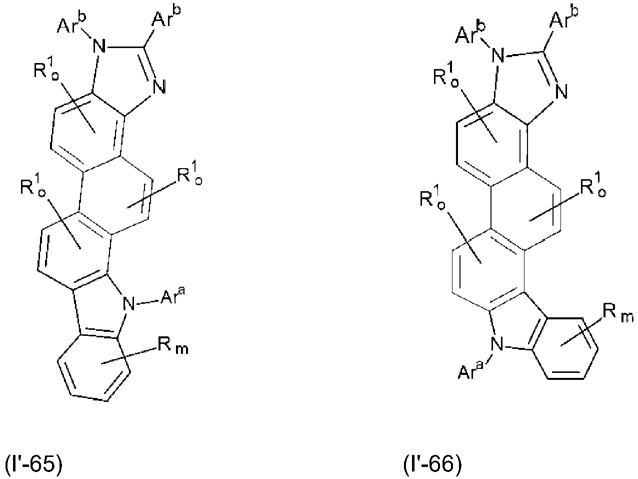 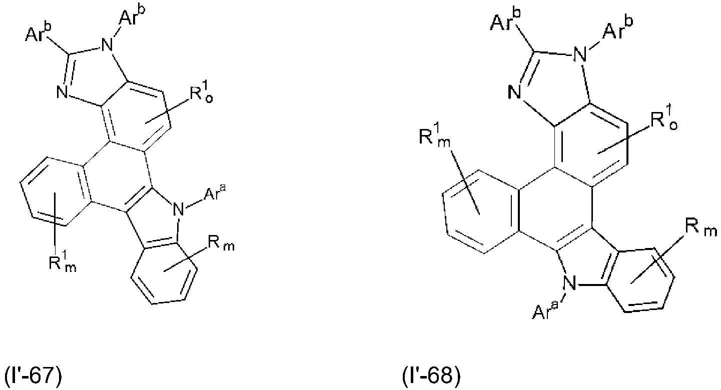 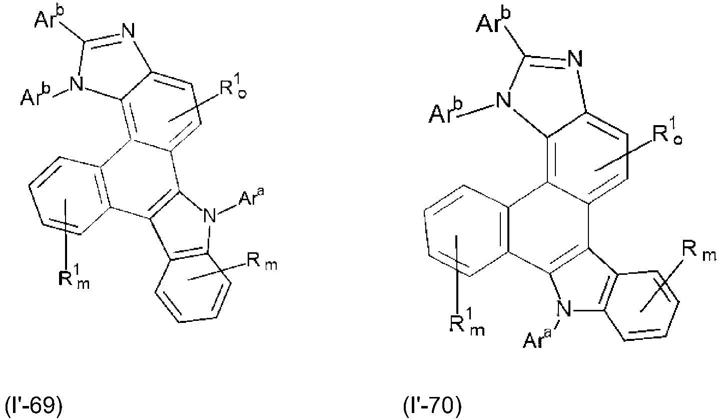 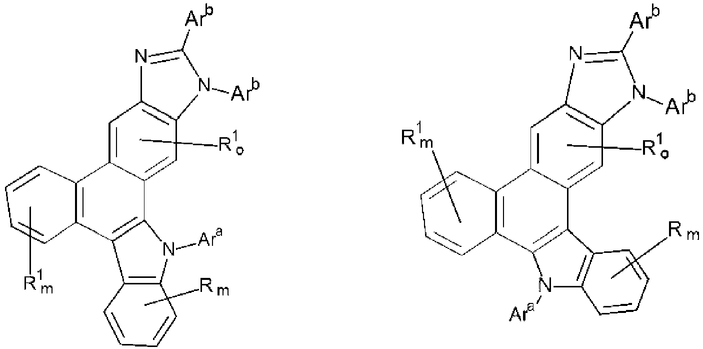 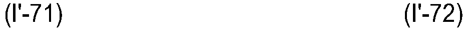 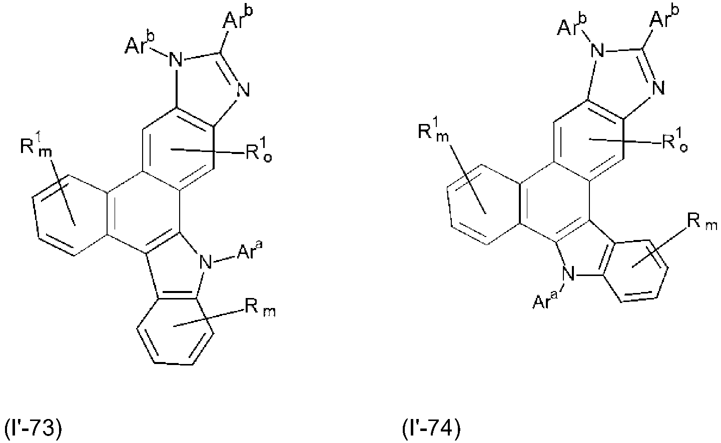 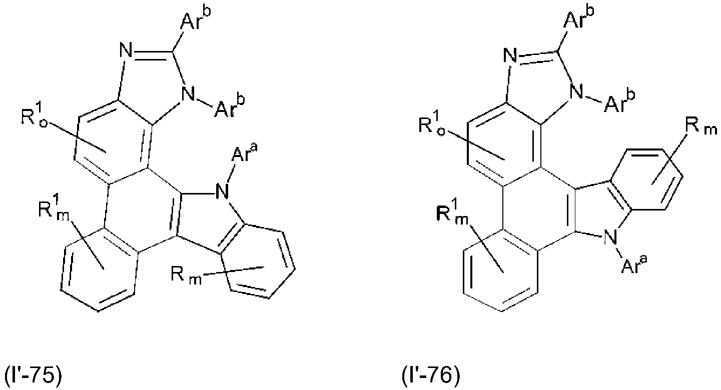 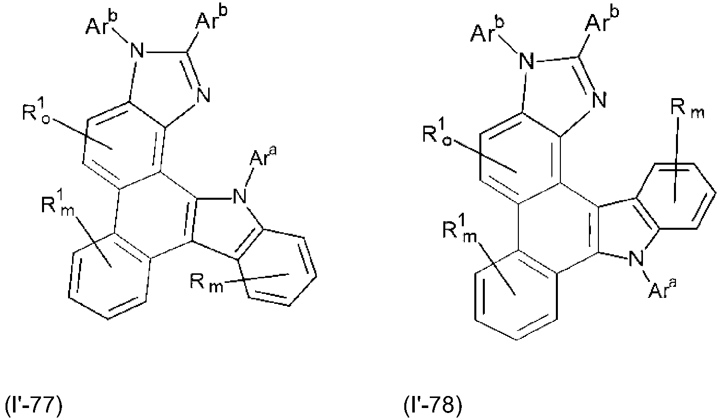 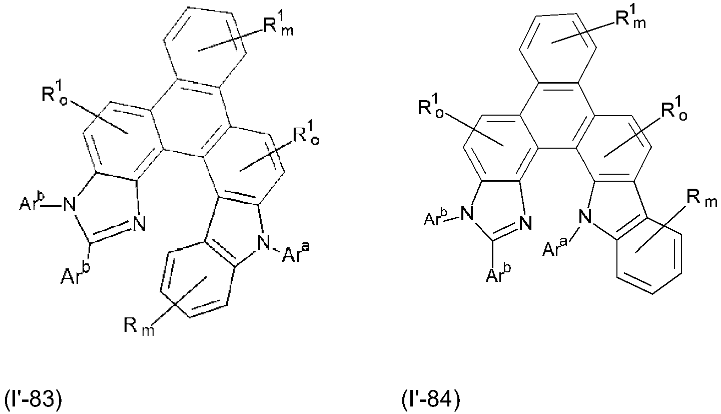 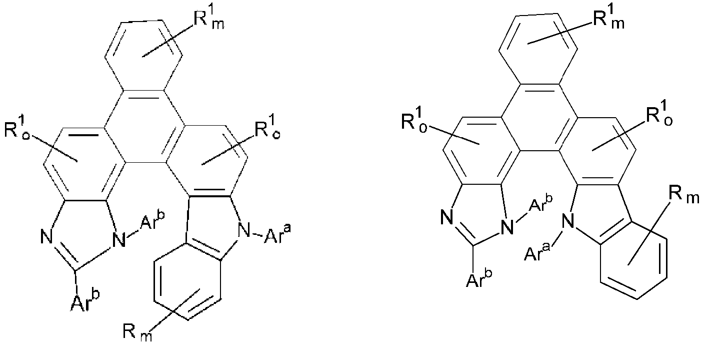 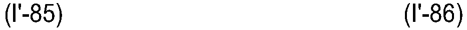 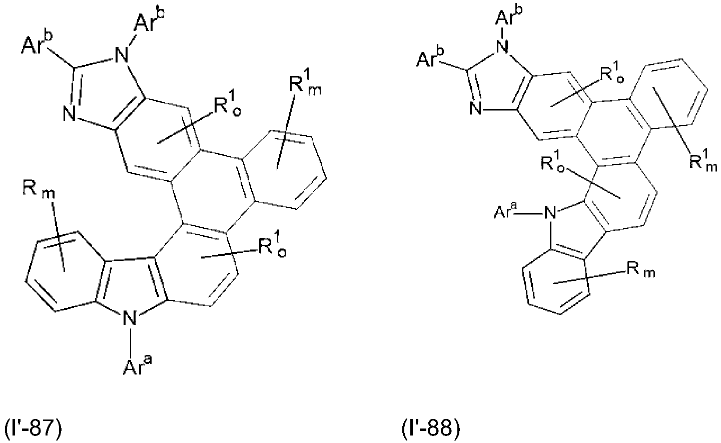 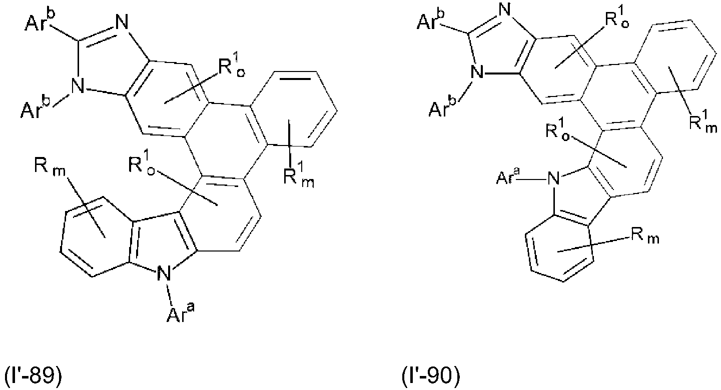 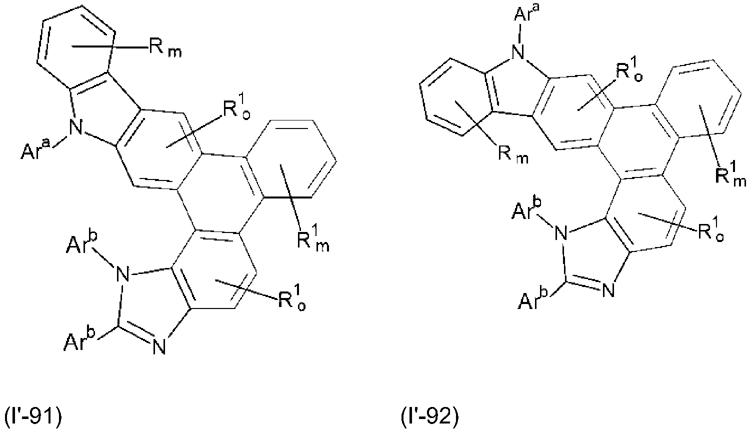 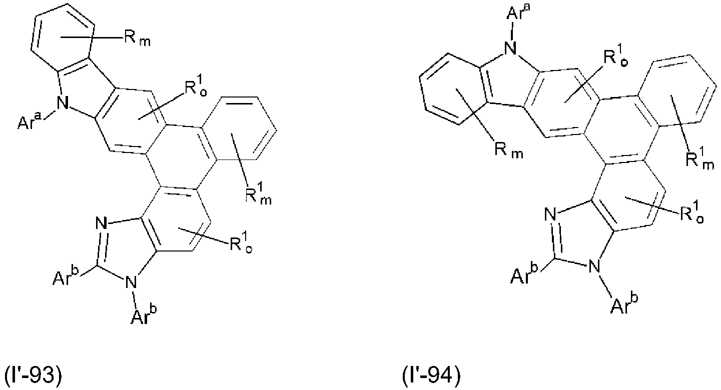 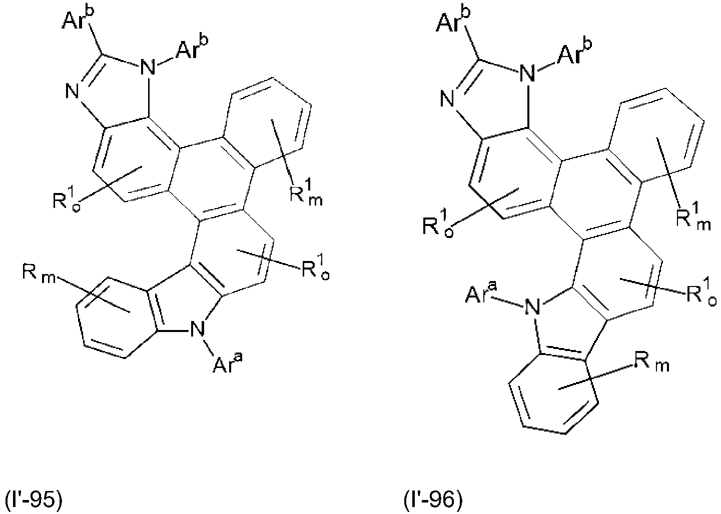 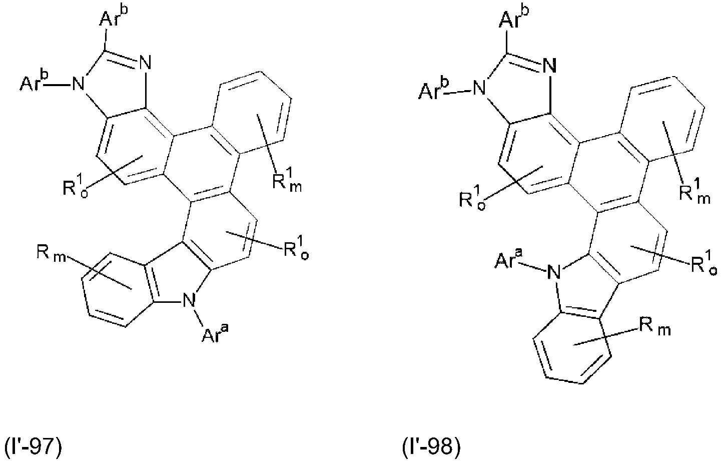 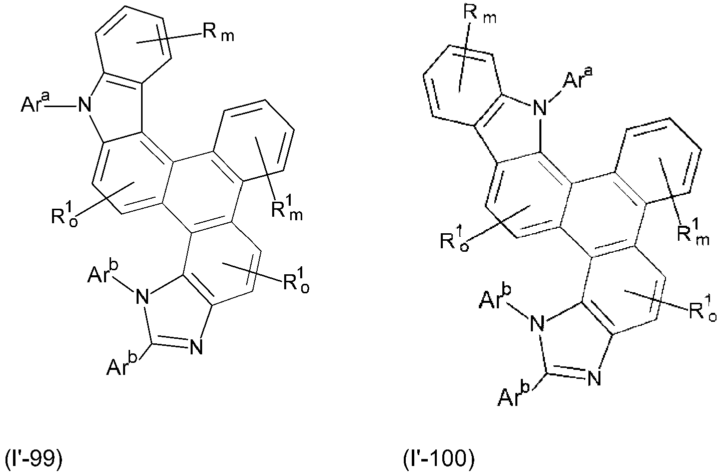 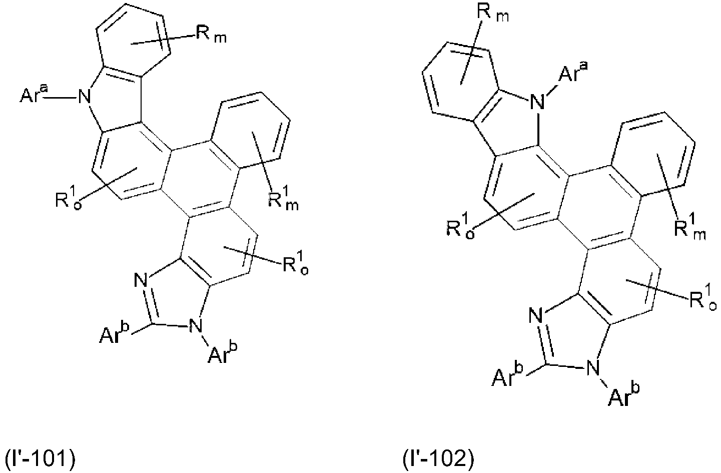 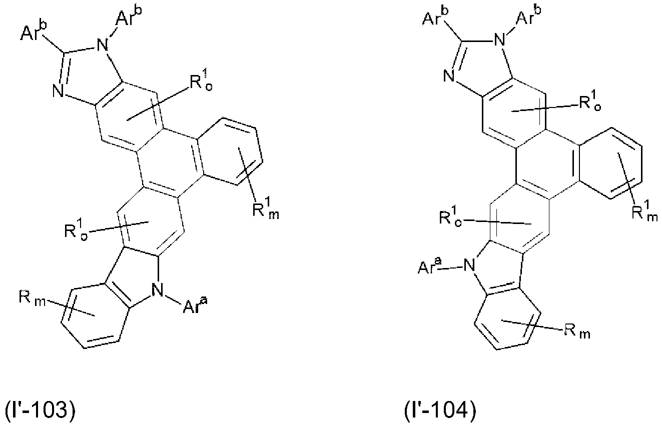 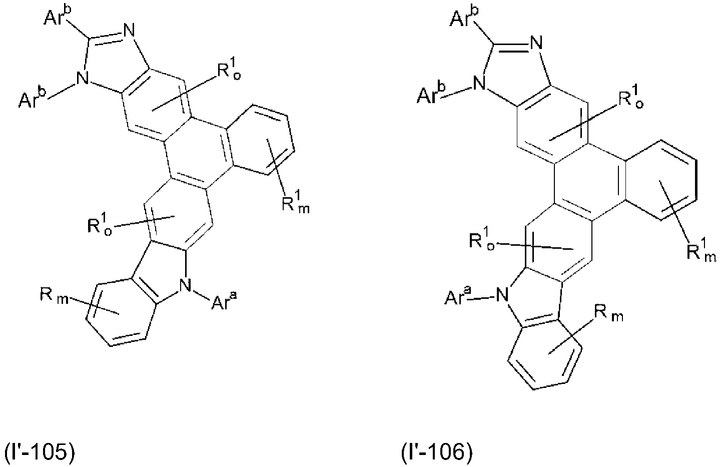 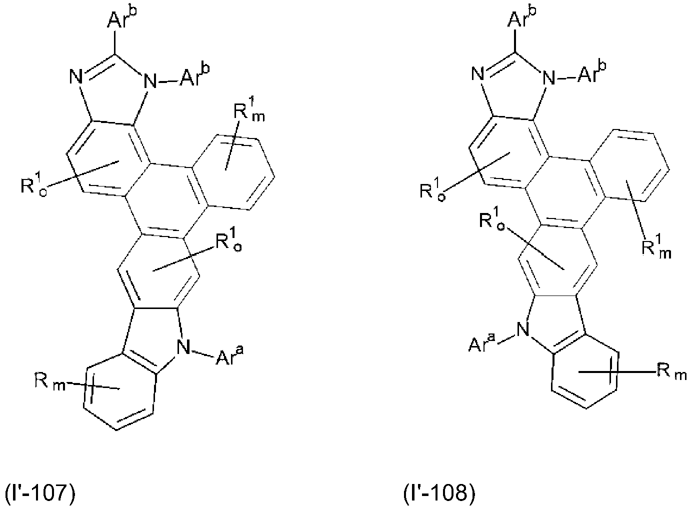 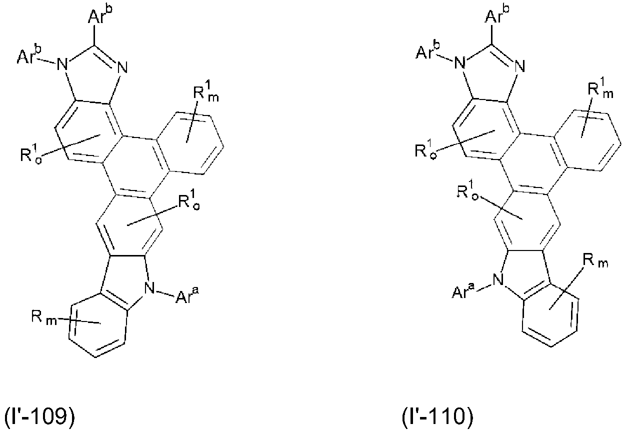 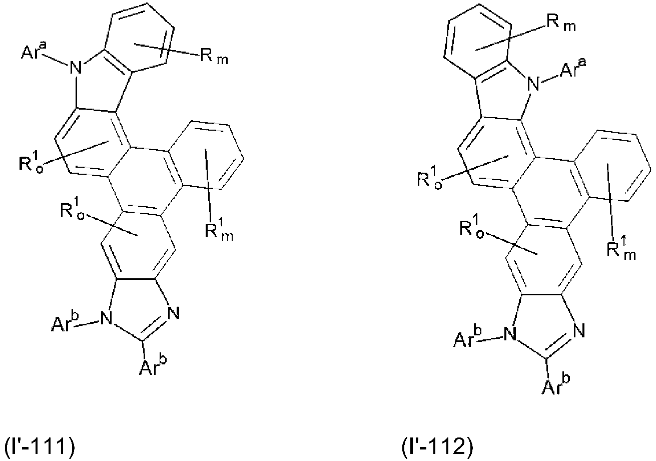 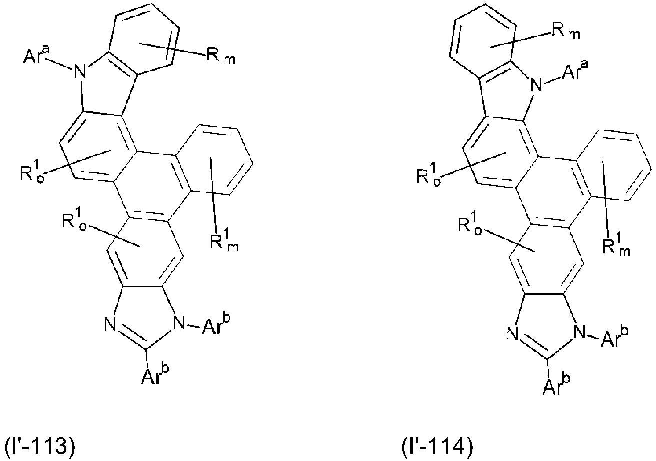 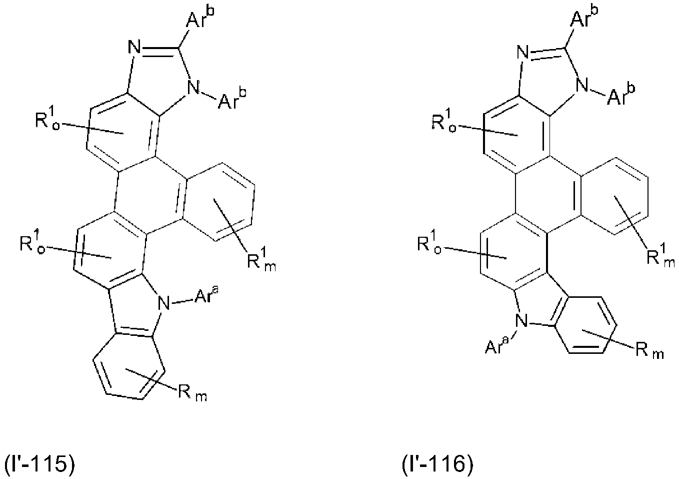 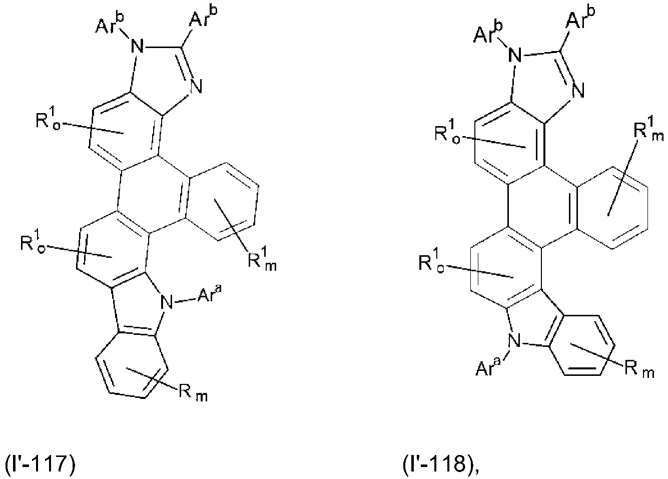 dans laquelle, pour les symboles et les indices utilisés, on a:
Ar^{a} est à chaque occurrence, identique ou différente, un système cyclique aromatique ou hétéroaromatique avec 5 à 40 atomes cycliques aromatiques, qui peut être substitué par un ou plusieurs radicaux R² ;
Ar^{b} est à chaque occurrence, identique ou différente, un système cyclique aromatique ou hétéroaromatique ayant de 5 à 40 atomes cycliques aromatiques, qui peut être substitué par un ou plusieurs radicaux R³ ;
R est à chaque occurrence, identique ou différente, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁴ et dans lequel un ou plusieurs groupes CH₂ non adjacents peuvent être remplacés par Si(R⁴)₂, C=O, NR⁴, O, S ou CONR⁴, ou un système cyclique aromatique ou hétéroaromatique avec 5 à 60 atomes cycliques aromatiques, qui peuvent être substitués chacun par un ou plusieurs restes R⁴ ; où deux radicaux R ou un radical R avec un radical R¹, R² peuvent également former ensemble un système cyclique aliphatique, hétéroaliphatique, aromatique ou hétéroaromatique ;
R¹ est à chaque occurrence, identique ou différente, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁴ et dans lequel un ou plusieurs groupes CH₂ non adjacents peuvent être remplacés par Si(R⁴)₂, C=O, NR⁴, O, S ou CONR⁴, ou un système cyclique aromatique ou hétéroaromatique avec 5 à 60 atomes cycliques aromatiques, qui peuvent être substitués chacun par un ou plusieurs radicaux R⁴ ; où deux radicaux R¹ ou un radical R¹ avec un radical R, R², R³ peuvent également former ensemble un système cyclique aliphatique, hétéroaliphatique, aromatique ou hétéroaromatique ;
R² est à chaque occurrence, identique ou différente, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁴ et dans lequel un ou plusieurs groupes CH₂ non adjacents peuvent être remplacés par Si(R⁴)₂, C=O, NR⁴, O, S ou CONR⁴, ou un système cyclique aromatique ou hétéroaromatique avec 5 à 60 atomes cycliques aromatiques, qui peuvent être substitués chacun par un ou plusieurs radicaux R⁴ ; où deux radicaux R² ou un radical R² avec un radical R, R¹, R³ peuvent également former ensemble un système cyclique aromatique, hétéroaromatique, aliphatique ou hétéroaliphatique ;
R³ est à chaque occurrence, identique ou différente, H, D, F, Cl, Br, I, N(R⁴)₂, N(Ar')₂, CN, NO₂, OR⁴, OAr', SR⁴, SAr', COOR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁴ et dans lequel un ou plusieurs groupes CH₂ non adjacents peuvent être remplacés par Si(R⁴)₂, C=O, NR⁴, O, S ou CONR⁴, ou un système cyclique aromatique ou hétéroaromatique avec 5 à 60 atomes cycliques aromatiques, qui peuvent être substitués chacun par un ou plusieurs radicaux R⁴ ; où deux radicaux R³ ou un radical R³ avec un radical R¹, R² peuvent également former ensemble un système cyclique aromatique, hétéroaromatique, aliphatique ou hétéroaliphatique ;
Ar' est, à chaque occurrence, identique ou différente, un système cyclique aromatique ou hétéroaromatique comportant de 5 à 40 atomes cycliques aromatiques, qui peut être substitué par un ou plusieurs radicaux R⁴ ;
R⁴ est à chaque occurrence, identique ou différente, H, D, F, Cl, Br, I, N(R⁸)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁸, un groupe alkyle à chaîne droite ayant de 1 à 20 atomes de carbone ou un groupe alcényle ou alcynyle ayant de 2 à 20 atomes de carbone ou un groupe alkyle ramifié ou cyclique ayant de 3 à 20 atomes de carbone, dans lequel chaque groupe alkyle, alcényle ou alcynyle peut être substitué par un ou plusieurs radicaux R⁵, un ou plusieurs groupes CH₂ non adjacents pouvant être remplacés par Si(R⁵)₂, C=O, NR⁵, O, S ou CONR⁵, ou un système cyclique aromatique ou hétéroaromatique avec 5 à 40 atomes cycliques aromatiques, qui peuvent être substitués chacun par un ou plusieurs restes R⁵ ; où deux ou plusieurs radicaux R⁴ peuvent former ensemble un système cyclique aromatique, hétéroaromatique, aliphatique ou hétéroaliphatique ;
R⁵ est à chaque occurrence, identique ou différente, H, D, F ou un radical organique aliphatique, aromatique ou hétéroaromatique avec 1 à 20 atomes de C, dans lequel un ou plusieurs atomes de H peuvent également être remplacés par F,
l'indice o est 0, 1 ou 2 et l'indice m est 0, 1, 2, 3 ou 4.

2. Composés selon la revendication 1, **caractérisés en ce que** deux groupes R¹ adjacents ne forment pas un système cyclique aromatique ou hétéroaromatique condensé avec les groupes auxquels les groupes R¹ sont liés, dans lequel ceci inclut les résidus R⁴ ou R⁵ par lesquels les groupes R¹ peuvent être substitués.

3. Composés selon la revendication 1 ou 2, **caractérisés en ce que** Ar^{a} et/ou Ar^{b}, identiques ou différents à chaque occurrence, sont choisis parmi les groupes phényle, biphényle, terphényle, quaterphényle, fluorène, spirobifluorène, naphtalène, indole, benzofurane, benzothiophène, carbazole, dibenzofurane, dibenzothiophène, indénocarbazole, indolocarbazole, pyridine, pyrimidine, pyrazine, pyridazine, triazine, quinoléine, isoquinoléine, quinazoline, quinoxaline, phénanthrène ou triphénylène, dont chacun peut être substitué par un ou plusieurs radicaux R² et R³, respectivement.

4. Composés selon l'une ou plusieurs des revendications 1 à 3, **caractérisés en ce que** R, R¹, R² et/ou R³, identiques ou différents à chaque occurrence, sont choisis dans le groupe constitué par H, D ou un système cyclique aromatique ou hétéroaromatique choisi parmi les groupes de formules suivantes Ar-1 à Ar-75 et/ou le groupe Ar', identique ou différent à chaque occurrence, est choisi parmi les groupes de formules suivantes Ar-1 à Ar-75, 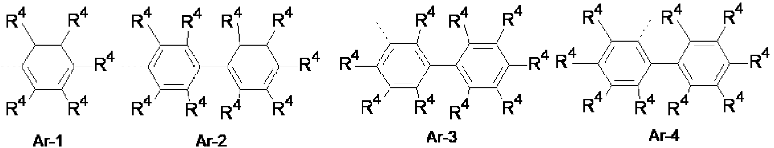 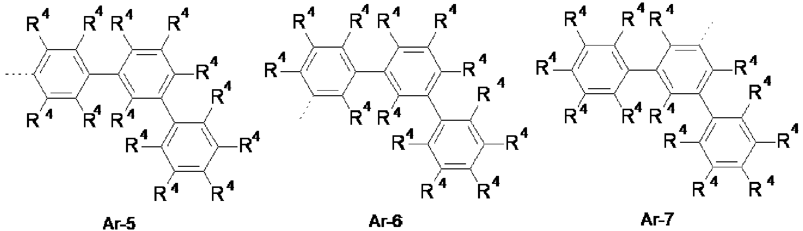 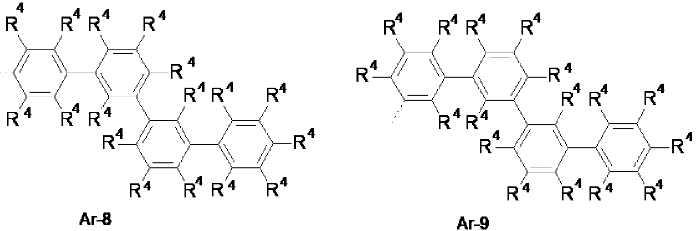 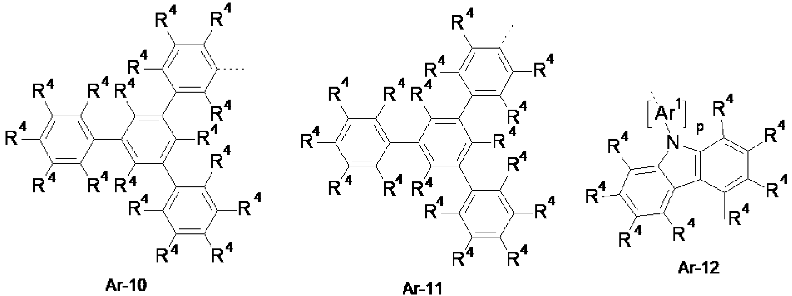 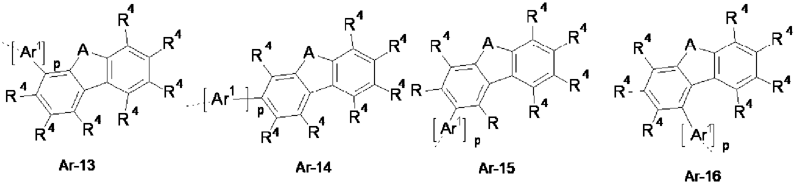 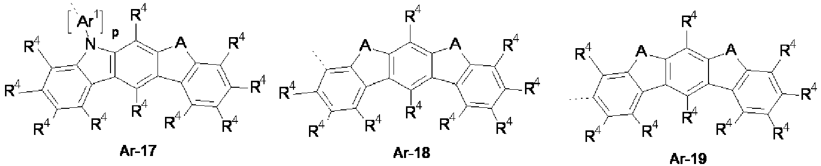 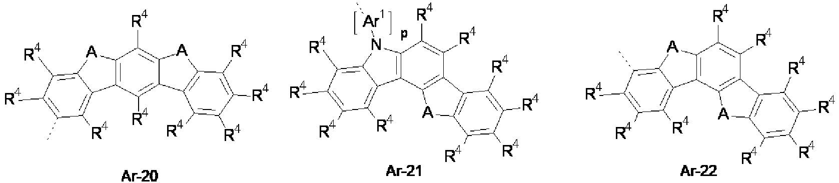 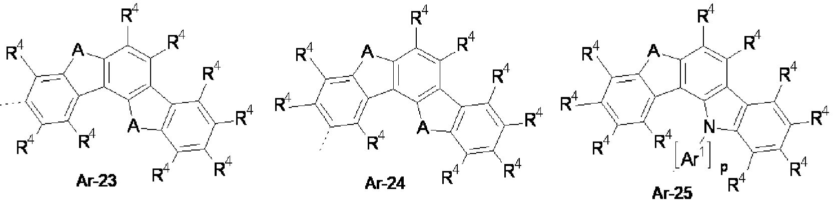 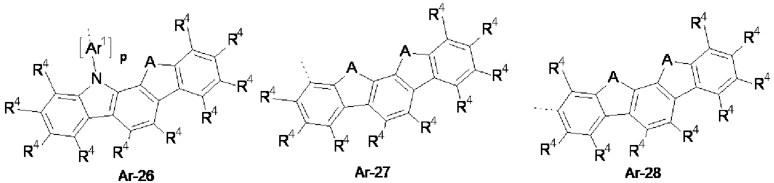 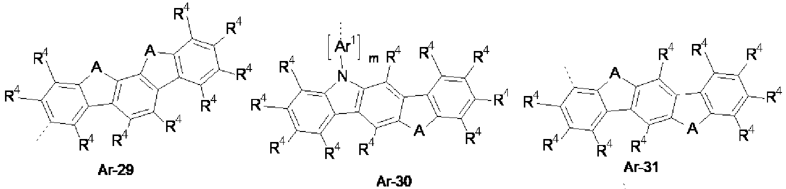 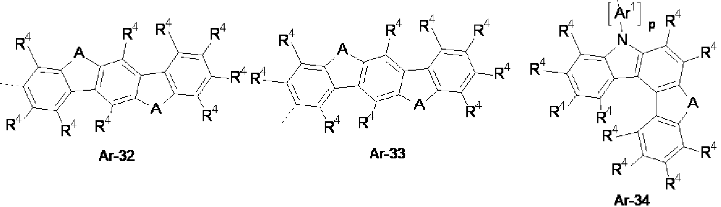 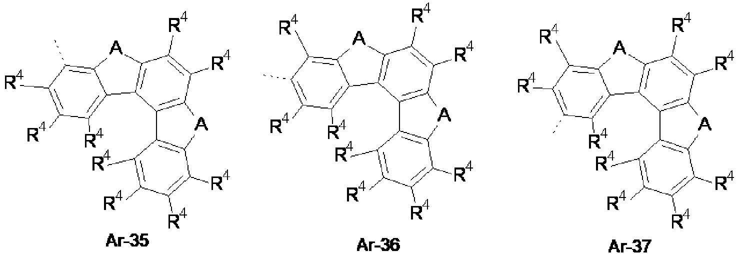 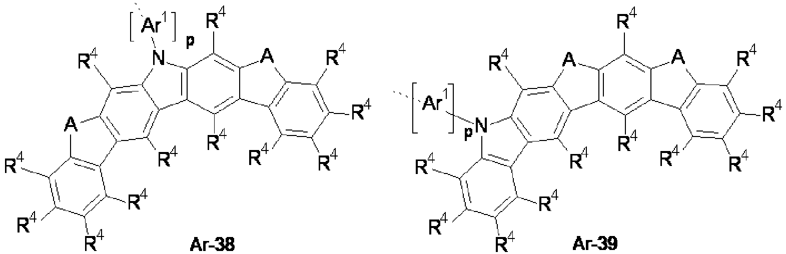 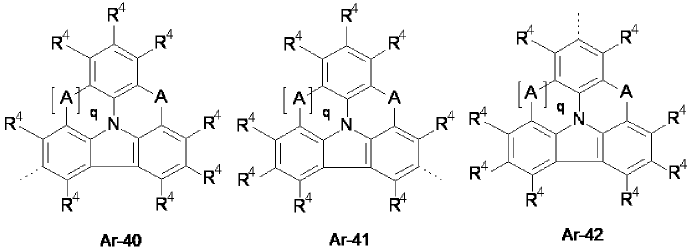 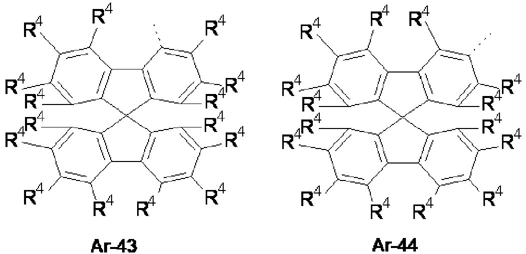 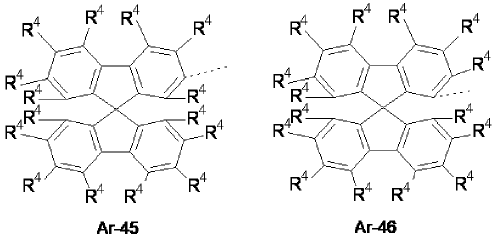 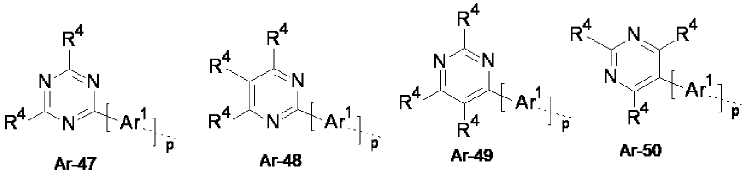 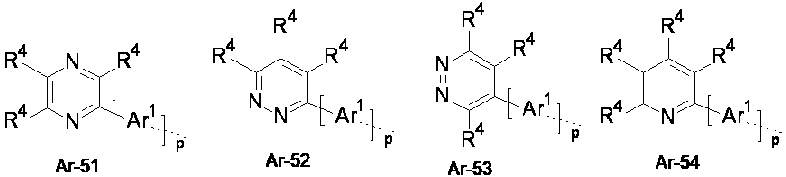 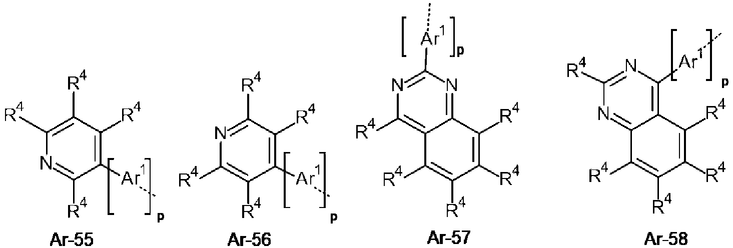 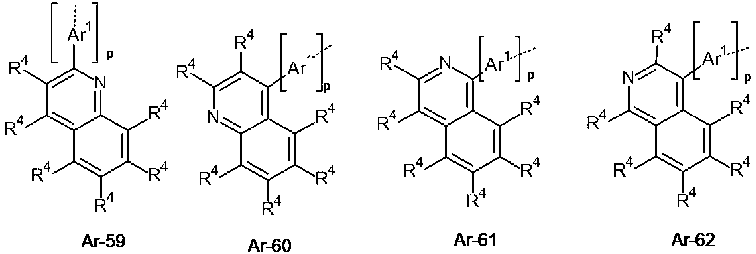 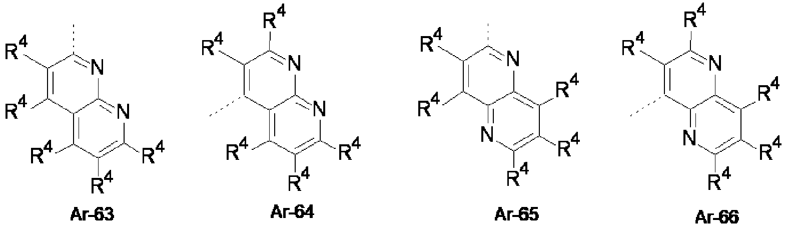 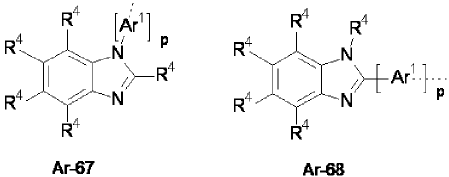 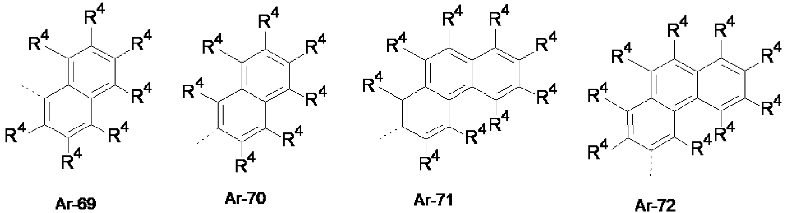 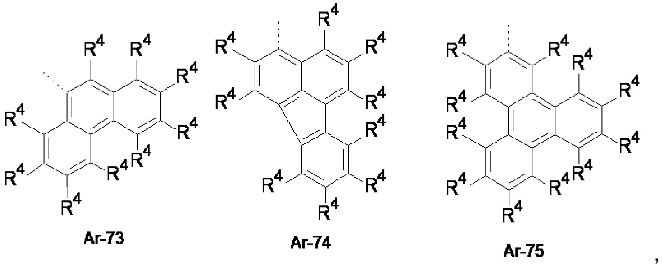 dans laquelle R⁴ a les significations données ci-dessus dans la revendication 1, la liaison en pointillés représente la liaison au groupe correspondant et reste valable :
Ar¹ est à chaque occurrence, identique ou différente, un système cyclique aromatique ou hétéroaromatique bivalent avec 6 à 18 atomes cycliques aromatiques, qui peut à chaque fois être substitué par un ou plusieurs radicaux R⁴ ;
A est à chaque occurrence, identique ou différente, C(R⁴)₂, NR⁴, O ou S ;
p est 0 ou 1, dans lequel p = 0 signifie que le groupe Ar¹ n'est pas présent et que le groupe aromatique ou hétéroaromatique correspondant est directement lié au radical correspondant ;
q est 0 ou 1, dans lequel q = 0 signifie qu'aucun groupe A n'est lié à cette position et que des radicaux R⁴ sont liés aux atomes de carbone correspondants à la place.

5. Composés selon une ou plusieurs des revendications 1 à 4, **caractérisés en ce que** les composés ont au moins deux groupes conducteurs d'électrons choisis parmi les substituants représentés par les formules (Ar-47) à (Ar-51), (Ar-57), (Ar-58) et/ou (Ar-63) à (Ar-68) représentées dans la revendication 4.

6. Composés selon une ou plusieurs des revendications 1 à 5, **caractérisés en ce que** le radical Ar^{a} est représenté par un groupe de formules (Ar-47) à (Ar-51), (Ar-57), (Ar-58) et/ou (Ar-63) à (Ar-68) représentées dans la revendication 4, dans lequel les substituants R⁴ représentés dans les formules (Ar-47) à (Ar-51), (Ar-57), (Ar-58) et/ou (Ar-63) à (Ar-68) doivent être remplacés par R².

7. Procédé de préparation d'un composé selon une ou plusieurs des revendications 1 à 6, **caractérisé en ce qu'**un composé aromatique ou hétéroaromatique est mis à réagir avec un composé diaminé aromatique ou hétéroaromatique au moyen d'une réaction de couplage.

8. Composition contenant au moins un composé selon une ou plusieurs des revendications 1 à 6 et au moins un autre matériau de matrice, dans laquelle l'autre matériau de matrice est choisi parmi les composés selon l'une des formules (H-1), (H-2), (H-3), (H-4) ou (H-5), 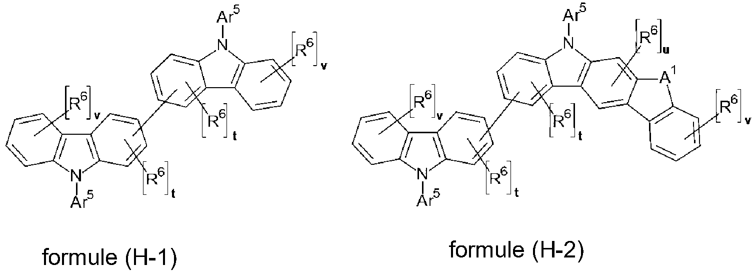 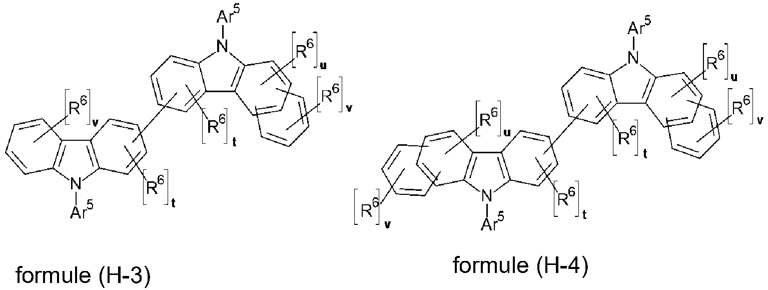 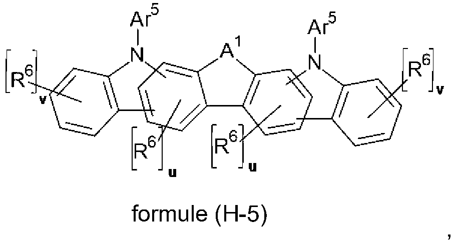 dans laquelle, pour les symboles et les indices utilisés, on a :
R⁶ est à chaque occurrence, identique ou différente, H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R')₂, S(=O)R⁷, S(=O)₂R⁷, OSO₂R⁷, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁷ et dans lequel un ou plusieurs groupes CH₂ non adjacents peuvent être remplacés par Si(R⁷)₂, C=O, NR⁷, O, S ou CONR⁷, ou un système cyclique aromatique ou hétéroaromatique avec 5 à 60 atomes cycliques aromatiques, qui peuvent être substitués chacun par un ou plusieurs radicaux R⁷ ; où deux radicaux R⁶ peuvent également former ensemble un système cyclique aromatique, hétéroaromatique, aliphatique ou hétéroaliphatique ;
Ar" est à chaque occurrence, identique ou différente, un système cyclique aromatique ou hétéroaromatique comportant de 5 à 40 atomes cycliques aromatiques, qui peut être substitué par un ou plusieurs radicaux R⁷ ;
A¹ est C(R⁷)₂, NR⁷, O ou S ;
Ar⁵ est à chaque occurrence, identique ou différente, un système cyclique aromatique ou hétéroaromatique ayant de 5 à 40 atomes dans le cycle aromatique, qui peut être substitué par un ou plusieurs radicaux R⁷ ;
R⁷ est à chaque occurrence, identique ou différente, H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, un groupe alkyle à chaîne droite ayant de 1 à 20 atomes de carbone ou un groupe alcényle ou alcynyle ayant de 2 à 20 atomes de carbone ou un groupe alkyle ramifié ou cyclique ayant de 3 à 20 atomes de carbone, dans lequel chaque groupe alkyle, alcényle ou alcynyle peut être substitué par un ou plusieurs radicaux R⁸, un ou plusieurs groupes CH₂ non adjacents pouvant être remplacés par Si(R⁸)₂, C=O, NR⁸, O, S ou CONR⁸, ou un système cyclique aromatique ou hétéroaromatique avec 5 à 40 atomes cycliques aromatiques, qui peuvent être substitués chacun par un ou plusieurs restes R⁸ ; où deux ou plusieurs radicaux R⁷ peuvent former ensemble un système cyclique aromatique, hétéroaromatique, aliphatique ou hétéroaliphatique ;
R⁸ est à chaque occurrence, identique ou différente, H, D, F ou un radical organique aliphatique, aromatique ou hétéroaromatique, avec 1 à 20 atomes de C, dans lequel un ou plusieurs atomes de H peuvent également être remplacés par F ;
v est à chaque occurrence, identique ou différente, 0, 1, 2, 3 ou 4 ;
t est à chaque occurrence, identique ou différente, 0, 1, 2 ou 3 ;
u est à chaque occurrence, identique ou différente, 0, 1 ou 2.

9. Composition contenant au moins un composé selon une ou plusieurs des revendications 1 à 6 et au moins un autre matériau de matrice, dans laquelle l'autre matériau de matrice est choisi parmi les composés selon l'une des formules (H-6), (H-7), (H-8), (H-9), (H-10), (H-11), (H-12) ou (H-13), 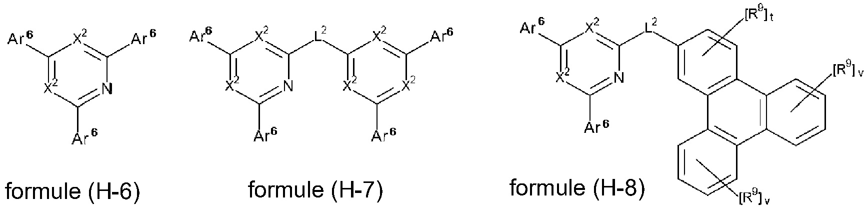 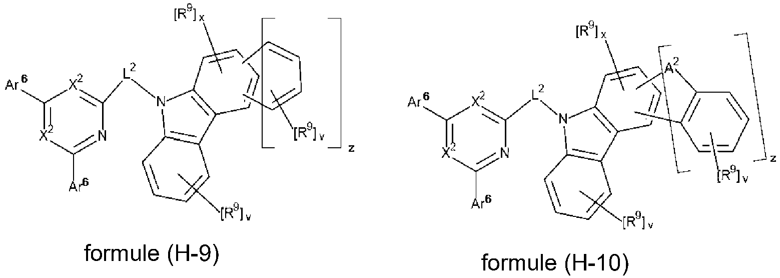 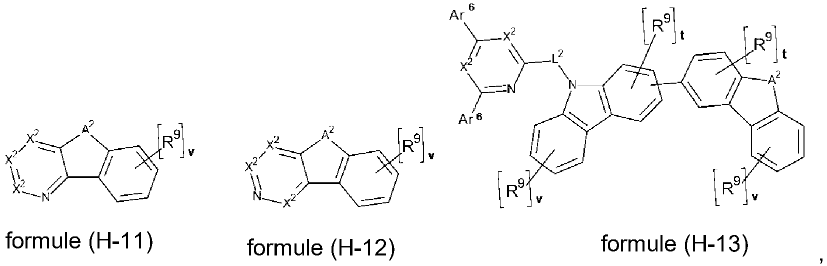 dans laquelle, pour les symboles et les indices utilisés, on a :
X² représente N ou CR⁹, à condition que pas plus de deux des groupes X² dans un cycle représentent N ;
L² représente un groupe de liaison choisi parmi une liaison ou un système cyclique aromatique ou hétéroaromatique ayant de 5 à 40 atomes dans le cycle aromatique, qui peut être substitué par un ou plusieurs radicaux R⁹ ;
Ar⁶ est à chaque occurrence, identique ou différente, un système cyclique aromatique ou hétéroaromatique ayant de 5 à 40 atomes dans le cycle aromatique, qui peut être substitué par un ou plusieurs radicaux R¹⁰ ;
R⁹ est à chaque occurrence, identique ou différente, H, D, F, Cl, Br, I, N(R¹⁰)₂, N(Ar‴)₂, CN, NO₂, OR¹⁰, SR¹⁰, COOR¹⁰, C(=O)N(R¹⁰)₂, Si(R¹⁰)₃, B(OR¹⁰)₂, C(=O)R¹⁰, P(=O)(R¹⁰)₂, S(=O)R¹⁰, S(=O)₂R¹⁰, OSO₂R¹⁰, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel chaque groupe alkyle, alcényle ou alcynyle peut être substitué par un ou plusieurs radicaux R⁴ et dans lequel un ou plusieurs groupes CH₂ non adjacents peuvent être remplacés par Si(R¹⁰)₂, C=O, NR¹⁰, O, S ou CONR¹⁰, ou un système cyclique aromatique ou hétéroaromatique avec 5 à 60 atomes cycliques aromatiques, qui peuvent être substitués chacun par un ou plusieurs radicaux R¹⁰ ; où deux radicaux R⁹ peuvent également former ensemble un système cyclique aromatique, hétéroaromatique, aliphatique ou hétéroaliphatique ;
Ar‴ est à chaque occurrence, identique ou différente, un système cyclique aromatique ou hétéroaromatique comportant de 5 à 40 atomes cycliques aromatiques, qui peut être substitué par un ou plusieurs radicaux R¹⁰ ;
A² est C(R¹⁰)₂, NR¹⁰, O ou S ;
R¹⁰ est à chaque occurrence, identique ou différente, H, D, F, Cl, Br, I, N(R¹¹)₂, CN, NO₂, OR¹¹, SR¹¹, Si(R¹¹)₃, B(OR¹¹)₂, C(=O)R¹¹, P(=O)(R¹¹)₂, S(=O)R¹¹, S(=O)₂R¹¹, OSO₂R¹¹, un groupe alkyle à chaîne droite ayant de 1 à 20 atomes de carbone ou un groupe alcényle ou alcynyle ayant de 2 à 20 atomes de carbone ou un groupe alkyle ramifié ou cyclique ayant de 3 à 20 atomes de carbone, dans lequel chaque groupe alkyle, alcényle ou alcynyle peut être substitué par un ou plusieurs radicaux R¹¹, un ou plusieurs groupes CH₂ non adjacents pouvant être remplacés par Si(R¹¹)₂, C=O, NR¹¹, O, S ou CONR¹¹, ou un système cyclique aromatique ou hétéroaromatique avec 5 à 40 atomes cycliques aromatiques, qui peuvent être substitués chacun par un ou plusieurs restes R¹¹ ; où deux ou plusieurs radicaux R¹⁰ peuvent former ensemble un système cyclique aromatique, hétéroaromatique, aliphatique ou hétéroaliphatique ;
R¹¹ est à chaque occurrence, identique ou différente, H, D, F ou un radical organique aliphatique, aromatique ou hétéroaromatique avec 1 à 20 atomes de C, dans lequel un ou plusieurs atomes de H peuvent également être remplacés par F ;
v est à chaque occurrence, identique ou différente, 0, 1, 2, 3 ou 4;
t est à chaque occurrence, identique ou différente, 0, 1, 2 ou 3;
x est à chaque occurrence, identique ou différente,0, 1, 2, 3 ou 4;
z est à chaque occurrence, identique ou différente, 0, 1 ou 2, dans lequel la somme de x et z est au plus égale à 4.

10. Composition selon la revendication 8 ou 9, **caractérisée en ce qu'**un composé visé aux revendications 1 à 6 est présent dans la composition en une proportion en masse comprise entre 10 % et 95 %, par rapport à la masse totale de la composition.

11. Formulation contenant au moins un composé selon une ou plusieurs des revendications 1 à 6 et/ou au moins une composition selon une ou plusieurs des revendications 8 à 10 et au moins un autre composé, dans laquelle l'autre composé est choisi parmi un ou plusieurs solvants.

12. Utilisation de composés selon une ou plusieurs des revendications 1 à 6 et/ou d'une composition selon une ou plusieurs des revendications 8 à 10 dans un dispositif électronique.

13. Dispositif électronique contenant au moins un composé selon une ou plusieurs des revendications 1 à 6 et/ou une composition selon une ou plusieurs des revendications 8 à 10.

14. Dispositif électronique selon la revendication 13, dans lequel il s'agit d'un dispositif électroluminescent organique, **caractérisé en ce que** le composé selon une ou plusieurs des revendications 1 à 6 est utilisé comme matériau de matrice dans une couche émettrice et/ou dans une couche de transport d'électrons et/ou dans une couche de blocage de trous et/ou dans une couche de transport de trous et/ou dans une couche de blocage d'électrons.

15. Dispositif électronique selon la revendication 14, **caractérisé en ce que** le composé selon une ou plusieurs des revendications 1 à 6 est utilisé comme matériau de matrice pour des émetteurs phosphorescents en combinaison avec un autre matériau de matrice, l'autre matériau de matrice étant choisi parmi les composés selon l'une des formules (H-1), (H-2), (H-3), (H-4) ou (H-5), 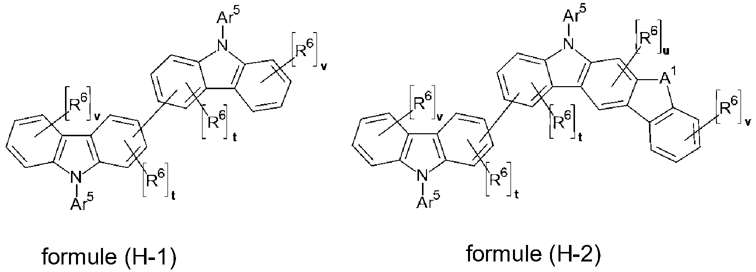 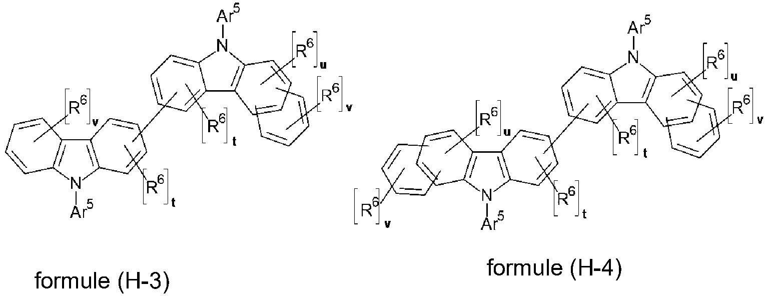 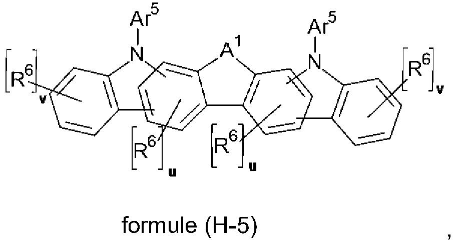
dans laquelle les symboles A¹, Ar⁵ et R⁶ et les indices s, t et u ont les significations indiquées dans la revendication 8,
et/ou **en ce que** l'autre matériau de matrice est choisi parmi les composés selon l'une des formules (H-6), (H-7), (H-8), (H-9), (H-10), (H-11), (H-12) ou (H-13), 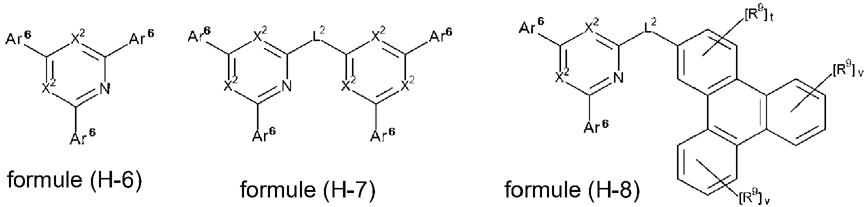 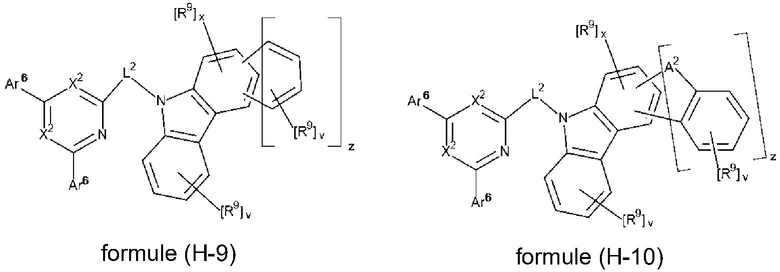 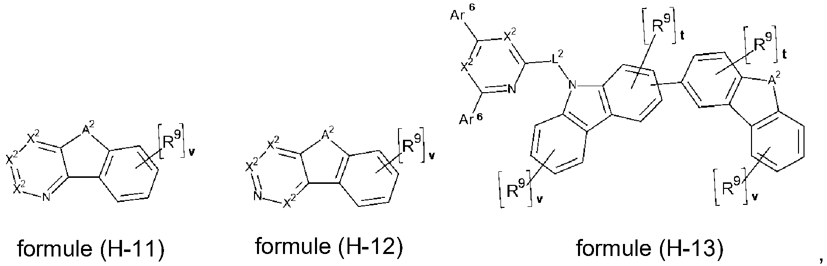
dans laquelle les symboles X², L², A², Ar⁶ et R⁹ et les indices v, t, x et z ont les significations indiquées dans la revendication 9.
